# EUROPEAN PATENT APPLICATION

(11) **EP 4 410 801 A1**
(43) Date of publication of application: **07.08.2024**
(21) Application number: 22875185.5
(22) Date of filing: 30.09.2022
(51) Int. Cl.: C07F 9/74, A61K 31/00, A61P 1/16, A61P 25/00, A61P 29/00, A61P 35/00

(54) **HALOGENATED PHENYLARSINE OXIDE COMPOUND AND APPLICATION THEREOF**

(30) Priority: 30.09.2021 CN 202111162542
(71) Applicant: Nuo-Beta Pharmaceutical Technology (Shanghai) Co., Ltd., Shanghai 201210 (CN)
(72) Inventor: HUANG, Fude, Shanghai 201210 (CN); HONG, Feng, Shanghai 201210 (CN); WANG, Wenan, Shanghai 201210 (CN); ZHANG, Jiangang, Shanghai 201210 (CN); XIE, Yuyu, Shanghai 201210 (CN); ZHANG, Hao, Shanghai 201210 (CN); JIAO, Changping, Shanghai 201210 (CN); CAO, Luxiang, Shanghai 201210 (CN); ZHENG, Linan, Shanghai 201210 (CN); HUANG, Changde, Shanghai 201210 (CN); HOU, Lijuan, Shanghai 200131 (CN); MA, Liping, Shanghai 200131 (CN); LU, Jinlian, Shanghai 200131 (CN); FANG, Li, Shanghai 200131 (CN); AN, Peiyun, Shanghai 200131 (CN)
(74) Representative: Simmons & Simmons LLP (Munich)
(86) International application number: PCT/CN2022/123419
(87) International publication number: WO 2023/051805

(57) **Abstract**

A fluorinated phenylarsine oxide or a pharmaceutically acceptable salt thereof, relating to a pharmaceutical composition containing a pharmaceutically acceptable carrier and a fluorinated phenylarsine oxide. The fluorinated phenylarsine oxide can be used for treating and preventing cancer and related diseases.

## Description

### FIELD OF INVENTION

The present disclosure belongs to the field of chemical synthesis, and specifically relates to a novel halogenated phenylarsine oxide compound and preparation method and application thereof.

### BACKGROUND

Phenylarsine oxide (PAO) is a known biological inhibitor, and the arsenic atom in this molecule has high affinity for the sulfur atom of sulfhydryl groups in biomolecules. Recent studies have found that PAO is a PI4KIIIα inhibitor and can be used to treat Alzheimer's disease. More research work is needed to determine if PAO can be used to treat other diseases. Deuterium substitution can change the half-life of a drug, reduce the frequency of administration, while maintain the original activity and selectivity. Deuterated drugs have become a new direction of new drug research and development and drug development mode. In 2017, the U.S. Food and Drug Administration approved the world's first deuterated drug, namely deuterated tetrabenazine (AUSTEDOTM, for the treatment of Huntington's disease and its related motor dysfunction). Several deuterated drugs have already entered clinical studies. However, it still needs further study whether the *in vivo and in vivo* activity and *in vivo* metabolic properties of the substitution-modified PAO would be changed when the hydrogen atom on the benzene ring is substituted by a halogen.

Radiotherapy is one of the main means of treating malignant tumors. Its main principle is to use high-energy rays to destroy the chromosomes of the cancer cells, thus causing the cancer cells (especially those that are growing and dividing) to be necrotic, and thus achieving the result of inhibiting the development of tumors. Radiation includes α, β, γ rays produced by radioactive isotopes and x-rays, electron rays, proton beams and other particle beams produced by various types of x-ray therapy machines or gas pedals, etc. 70% of the cancer patients need to be treated with radiation therapy during treatment, and 40% of the cancers can be cured by radiation therapy, so radiation therapy is the first choice as well as the main treatment means for some tumor types, such as oral cavity cancer, nasopharyngeal cancer, mid-stage esophageal cancer, inoperable lung cancer, skin cancer, early lymphoma. Most of the nervous system tumors require surgery followed by radiation therapy. For highly radiation-sensitive tumors such as malignant lymphoma, testicular seminoma, nephroblastoma, neuroblastoma, medulloblastoma, small-cell lung cancer, late-stage lung cancer, esophageal cancer and other types of tumors, combination with radiotherapy can significantly improve the therapeutic effect. Because radiation has penetrability, it will cause damage or necrosis of normal cells before it passes through normal tissues to reach cancer cells, thus causing different degrees of side effects, such as skin damage, vomiting, hair loss, headache, fever, cough, difficulty in swallowing, decreased appetite, changes in the mouth (dryness), diarrhea, tissue necrosis and fibrosis, and decrease in peripheral blood count. Some side effects disappear, but some are long-lasting or even permanent. Side effects on the one hand can affect the effectiveness of the treatment and can even lead to ineffective treatment and ultimately to death. Therefore, adjuvant therapy (including drugs) administered during radiotherapy can protect normal cells and thus enhance the therapeutic effect by improving the patient's physical condition.

Currently, amifostine is mainly used in clinical practice as a normal cell protectant against cellular damage induced by tumor therapy. Amifostine removes phosphoric acid by alkaline phosphatase in tissues and becomes a free thiol metabolite with pharmacological activity, which can reduce the toxicity caused by radiotherapy and chemotherapy. However, its use is limited to a few types of cancer, and the report regarding its protective effect on radiotherapy-induced damage is also limited, as well as its use causes many side effects. Therefore, it is an urgent task to find a safer and more effective normal cell protectant so as to improve the efficiency of tumor therapy.

### SUMMARY OF THE INVENTION

In one aspect, the present disclosure provides a compound of Formula (I) or a pharmaceutically acceptable salt thereof, 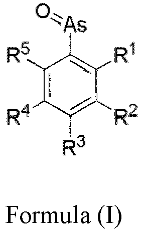 wherein R¹, R², R³, R⁴, R⁵ are independently selected from hydrogen, deuterium, fluorine, chlorine, bromine, iodine, methyl, amino, nitro, mono-deuteromethyl, di-deuteromethyl, tri-deuteromethyl, mono-fluoromethyl, di-fluoromethyl, or tri-fluoromethyl, and at least one of R¹, R², R³, R⁴, R⁵ is halogen or deuterium.

Preferably, R¹, R², R³, R⁴, R⁵ are independently selected from hydrogen, deuterium, fluorine, chlorine, bromine, methyl, amino, nitro, mono-fluoromethyl, di-fluoromethyl, or tri-fluoromethyl, and at least one of R¹, R², R³, R⁴, R⁵ is fluorine or fluorinated.

In one embodiment, R¹, R², R³, R⁴, R⁵ are independently selected from hydrogen, deuterium, or halogen, and at least one of R¹, R², R³, R⁴, R⁵ is halogen, at least two of R¹, R², R³, R⁴, R⁵ are halogens, preferably at least one of R¹, R², R³, R⁴, R⁵ is fluorine, preferably at least two, three, four or five of R¹, R², R³, R⁴, R⁵ are fluorine.

In one embodiment, the compound of Formula (I) optionally forms a compound of Formula (I') when dissolved in a solvent, in particular an aqueous solvent, wherein R¹, R², R³, R⁴, R⁵ have the same meaning as described above.

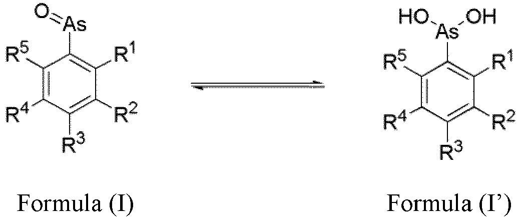

In one specific embodiment, the compound is selected from the group consisting of the following compounds:

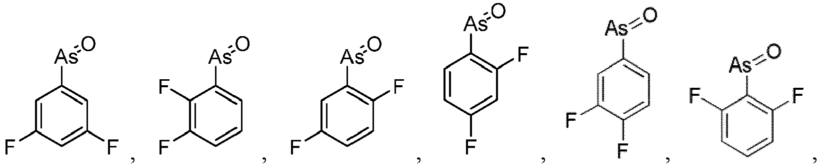

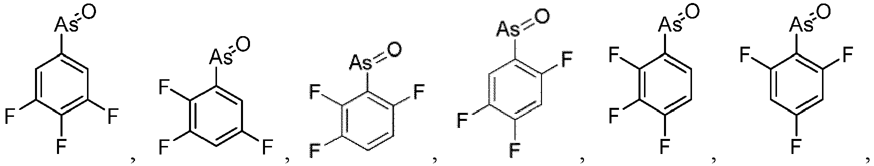

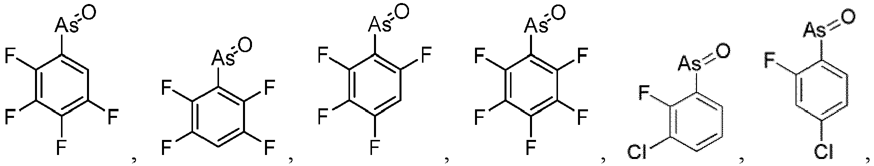

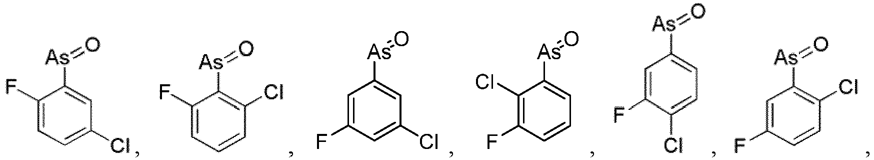

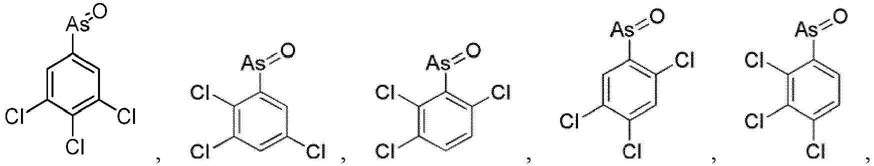

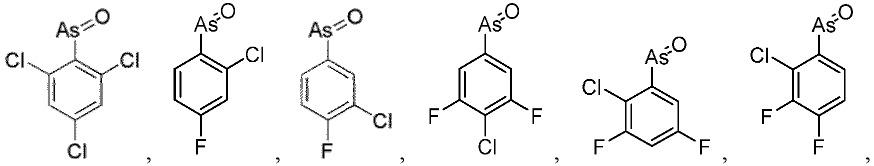

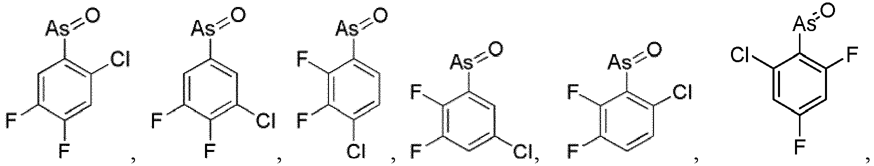

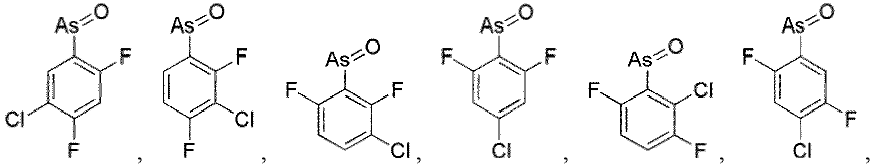

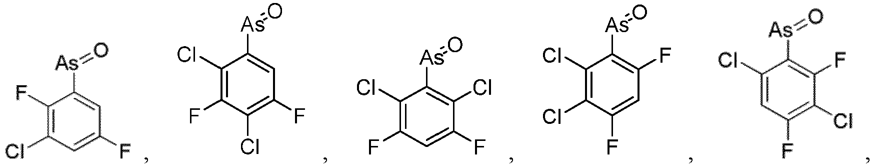

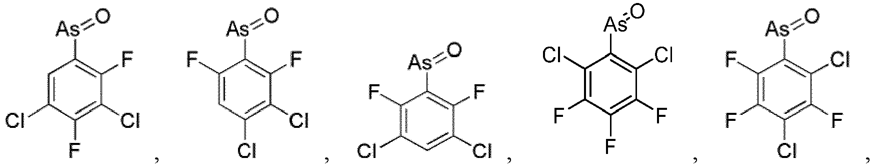

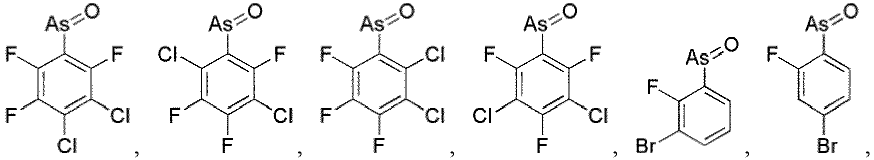

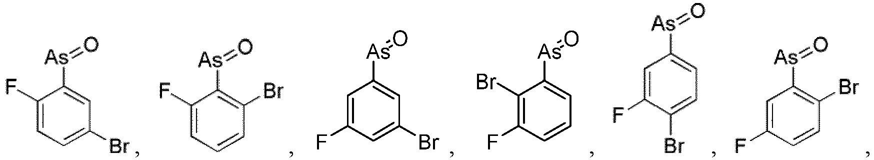

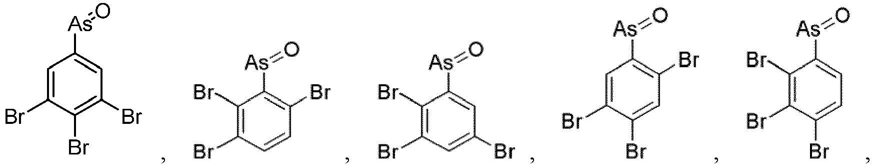

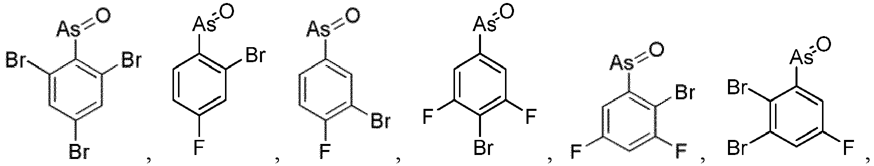

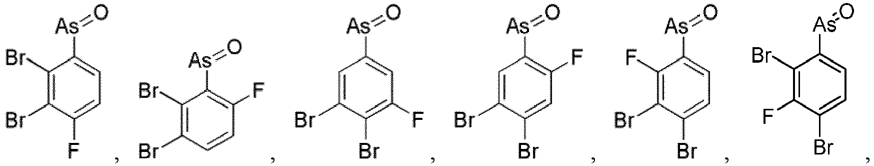

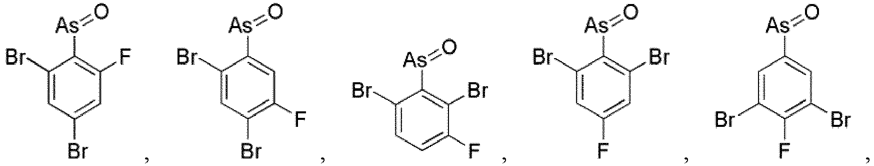

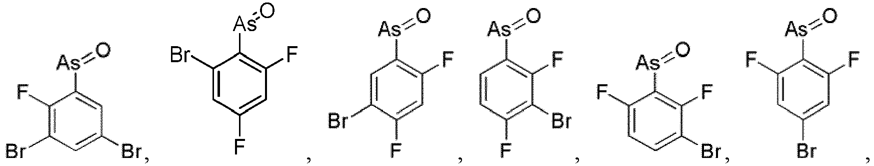

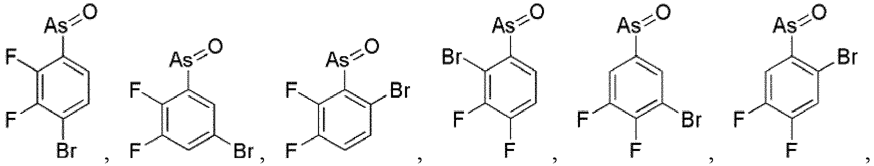

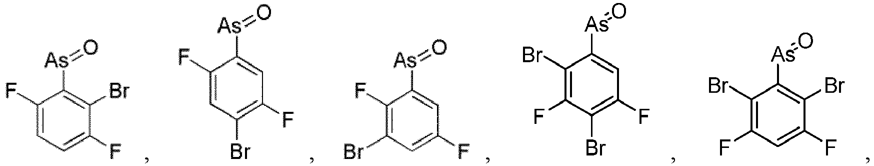

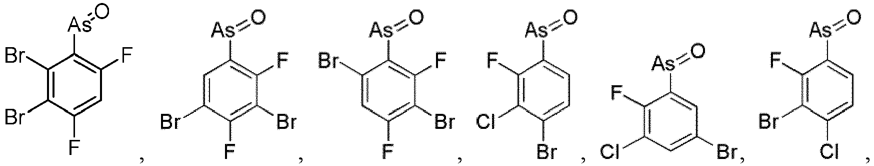

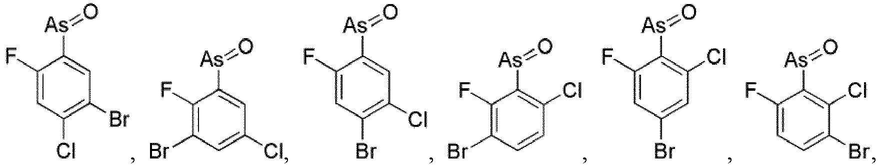

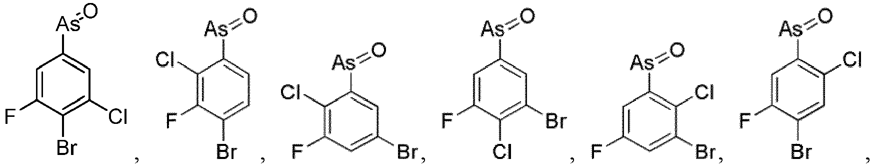

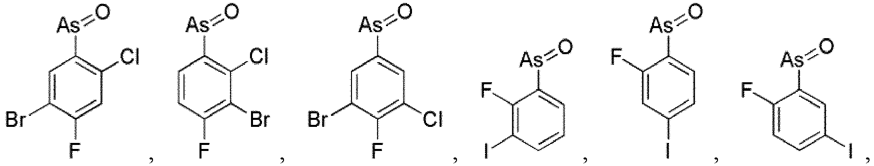

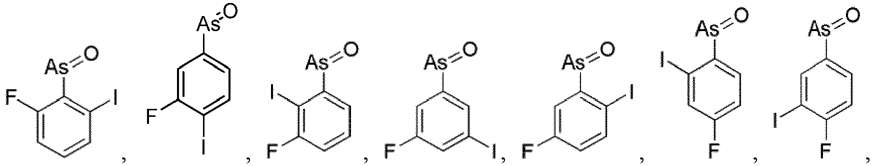

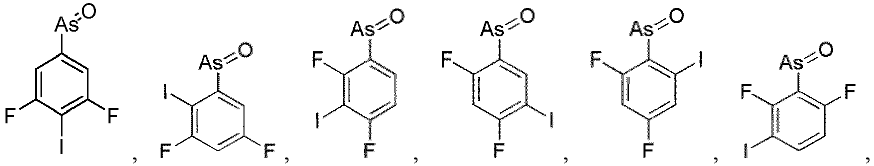

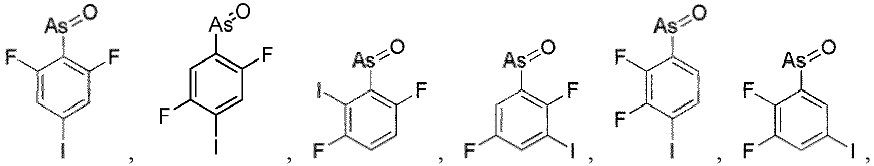

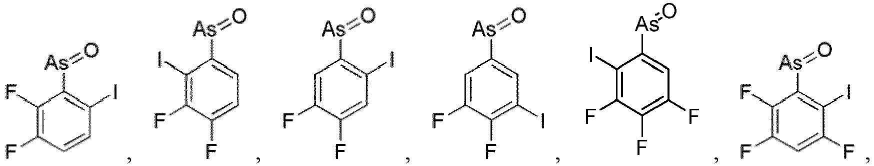

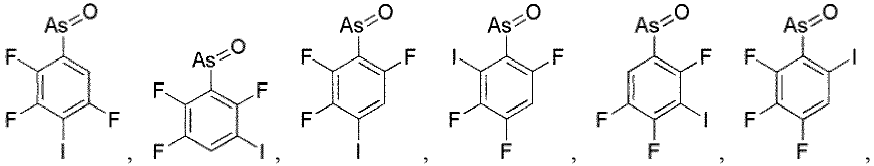

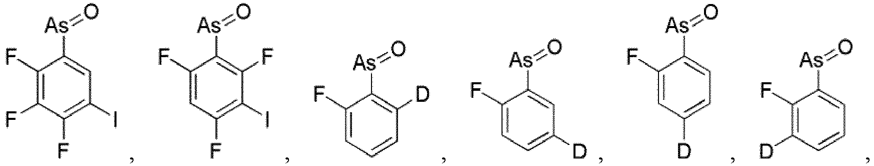

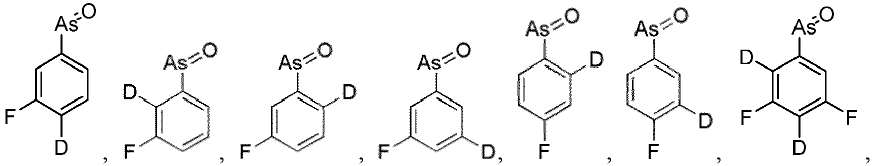

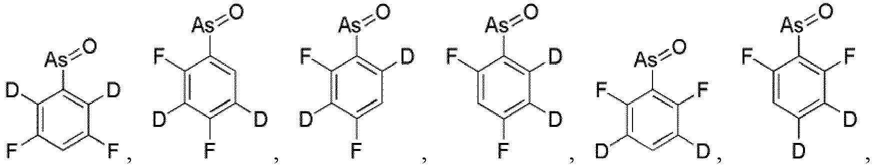

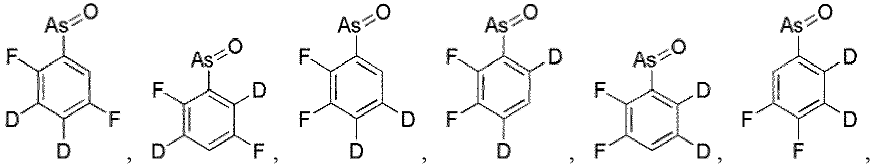

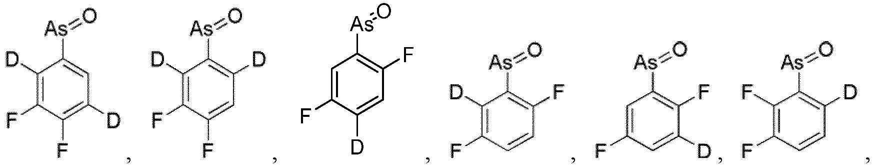

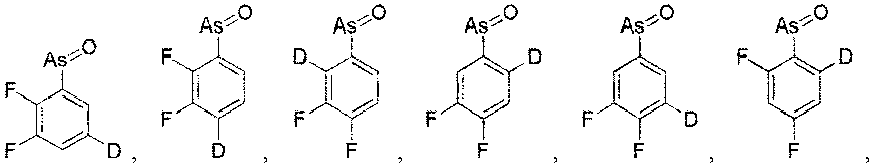

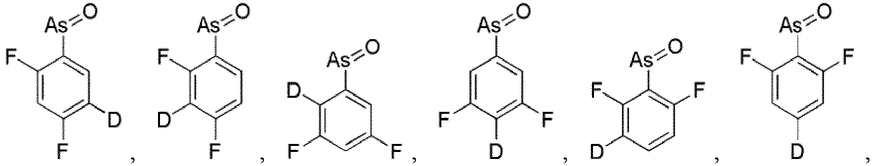

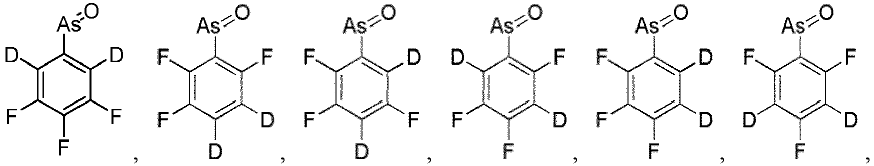

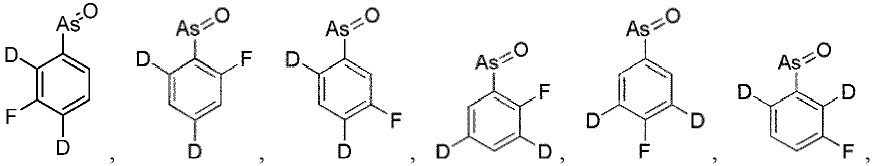

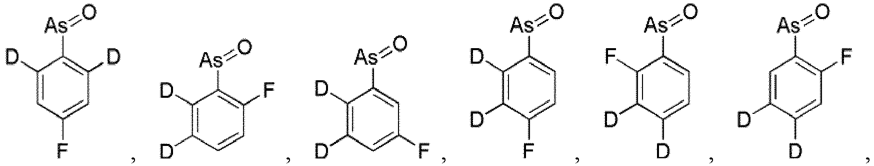

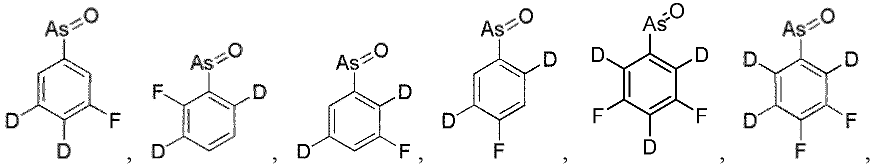

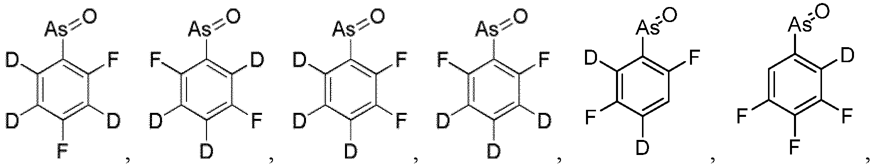

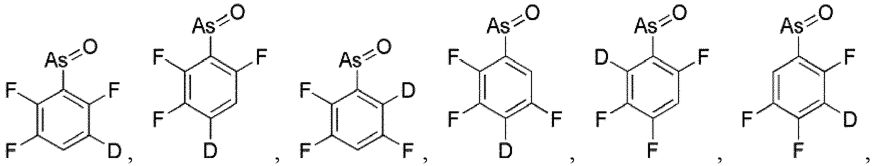

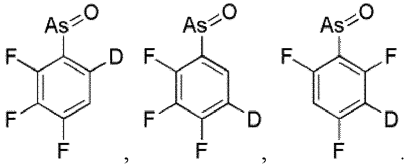

In another aspect, the present disclosure provides use of the foregoing compound or a pharmaceutically acceptable salt thereof in the manufacture of a medicament for preventing or treating a disease or a pathological reaction in a subject.

In one embodiment, the disease is selected from at least one of: tumor, malignant disease such as malignant tumor or malignant disease caused by chemotherapeutic for the treatment of tumor, Alzheimer's disease, intracellular protein misfolding related disease, lysosomal storage disease, inflammatory response, tissue or organ fibrosis, virus-infected disease, neurosis, microbial infection such as bacterial or fungal infections, preferably infection of drug-resistant bacteria or fungus, cellular tissue damage resulting from exposure to radiation, atherosclerosis, diabetes and asthma.

In one embodiment, the subject is human or non-human mammal.

In one specific embodiment, the tumor is selected from lymphomas (e.g., acute non-B or non-T cell lymphoma), cervical cancer, endometrial cancer, liver cancer, breast cancer (e.g., triple-negative breast cancer), lung cancer (e.g., non-small cell lung cancer or small cell lung cancer), colorectal cancer, gastric cancer, skin cancer (e.g., melanoma), bone cancer, osteosarcoma, myeloma, blood cancer (e.g., acute or chronic leukemia), ovarian cancer, prostate tumor, brain tumor (e.g., cerebral astrocytoma), neuroblastoma, thyroid cancer, malignant embryonal rhabdomyosarcoma.

In one specific embodiment, the intracellular protein misfolding related disease is Parkinson's disease, dementia with Lewy bodies, multiple system atrophy, inclusion body myositis, frontotemporal dementia, Huntington's disease, polyglutamine disease, amyotrophic lateral sclerosis, or prion disease.

In one specific embodiment, the lysosomal storage disease is a disorder of sphingolipid metabolism such as Gaucher disease, Niemann's disease type C, mucopolysaccharidosis, glycogen storage disease, glycoprotein storage disease, lipid storage disease, post-translational modification defects, intrinsic membrane protein deletion disorder, neuronal waxy plasma lipofuscinosis, or lysosome-associated organelle disorder.

In one specific embodiment, the inflammatory response is an increase of inflammatory factors such as TNFα or IL-6 in a local tissue or in blood throughout a body. The inflammatory response is inflammatory response caused by pathogens such as bacteria, fungi, viruses, or parasites, or the inflammatory response is inflammatory response caused by non-pathogens such as tumors, allergies, damage, etc.

In one specific embodiment, the tissue or organ fibrosis is selected from: cardiac fibrosis, renal fibrosis, pulmonary fibrosis, liver fibrosis, fibrosis during wound healing, or restenosis due to scar formation or fibrosis after arterial stent placement.

In one specific embodiment, the virus comprises coronavirus and non-coronavirus, preferably the coronavirus is selected from chicken infectious bronchitis virus, porcine epidemic diarrhea virus, porcine infectious gastroenteritis virus, porcine hemagglutinating encephalomyelitis virus, porcine δ-coronavirus, canine respiratory coronavirus, murine hepatitis virus, feline coronavirus, human coronavirus, human papillomavirus, severe acute respiratory syndrome virus, Middle East Respiratory Syndrome virus, or novel coronavirus; the non-coronavirus is selected from Hepatitis C virus or HIV.

In one specific embodiment, the neurosis is selected from neurasthenia, anxiety, depression, or mania.

In one embodiment, the cellular tissue damage resulting from exposure to radiation is selected from structural and functional changes in normal cells, tissues and organs at the site of radiation, e.g., damage to normal cells or necrosis, caused by α, β, γ rays produced by radioisotopes, x-rays produced by various x-ray therapy machines or accelerator-generated x-rays, electron beams, proton beams or other particle beams, etc., e.g., cell or tissue and organ damage caused by X-ray radiation.

In one embodiment, the microbial infection is selected from bacterial or fungal infection, preferably drug-resistant bacterial infection or drug-resistant fungal infection, more preferably wherein the microorganism is selected from Acinetobacter baumannii, Enterobacter cloacae, Escherichia coli, Klebsiella oxytoca, Klebsiella pneumoniae, Morganella morgani, Pseudomonas aeruginosa, Serratia marcescens, Staphylococcus aureus, Enterococcus faecalis, Enterococcus faecium, Streptococcus pneumoniae, Candida albicans, Candida parapsilosis, Aspergillus fumigatus, Neisseria gonorrhoeae, Clostridium tetani, Propionibacterium acnes, Cryptococcus neoformans, Helicobacter pylori, Bacterioides fragilis, Clostridium difficile, Gardnerella vaginalis, Atopobium vaginae, Peptostreptococcus anaerobius, Trichomonas vaginalis, and at least one of the drug-resistant organisms thereof; or, preferably, the microbial infection is selected from a microorganism infecting surface of body or mucosal site such as the site of acne wound, pimples, wound, burn or scald.

In one embodiment, the symptom of cellular tissue damage resulting from exposure to radiation is selected from at least one of decreased salivation, dry mouth, mucositis caused by X-ray induced salivary gland damage, and leukopenia caused by X-ray radiation.

In yet another aspect, the present disclosure provides use of the foregoing compound or a pharmaceutically acceptable salt thereof in the manufacture of a medicament for preventing or treating a disease in a subject, further comprising administering a second reagent to a subject in need thereof. The present disclosure provides use of the foregoing compound or a pharmaceutically acceptable salt thereof in combination with a second reagent in the manufacture of a medicament for preventing or treating a disease in a subject.

In one embodiment, the disease is selected from tumor, and the second reagent is used for the treatment of tumors.

In one embodiment, the drug for treating tumors is preferably selected from an antineoplastic agent interfering with mitosis such as paclitaxel or albumin paclitaxel, a drug directly affecting DNA structure and function such as carboplatin or cisplatin, or a Kras inhibitor.

In one specific embodiment, the disease is selected from pulmonary fibrosis, and the second reagent is used for the treatment of pulmonary fibrosis.

In one embodiment, the foregoing compound, or a pharmaceutically acceptable salt thereof, is administered before, after or simultaneously with the second reagent.

In another aspect, the present disclosure provides a pharmaceutical composition, comprising the foregoing compound or a pharmaceutically acceptable salt thereof, and a pharmaceutically acceptable carrier.

In one embodiment, the pharmaceutical composition further comprises a drug used for the treatment of tumors.

In another aspect, the present disclosure provides a method of preparing the foregoing compound, or a pharmaceutically acceptable salt thereof, comprising the steps of: 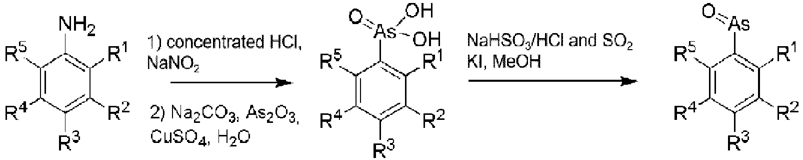
1) adding concentrated hydrochloric acid, aqueous sodium nitrite successively to an aqueous solution of aniline having the structure of Formula (I) or the salt thereof at 0°C-10°C and keeping the temperature below 5°C;
2) heating an aqueous solution of sodium carbonate, arsenic trioxide, copper sulfate to 90-100°C and then cooling down the aqueous solution, adding the solution prepared in step 1) into the aqueous solution, stirring, filtering, and adding acid to the filtrate to adjust the pH, and separating precipitated solid;
3) stirring the above precipitated solid, potassium iodide, sodium bisulfite or hydrochloric acid and sulfur dioxide in methanol until the reaction is complete and post-treating to obtain the compound.

In one embodiment, the post-treatment in the step 3) comprises adjusting the pH to an appropriate value with acid or base, extracting with ethyl acetate, combining the organic phases and evaporating to dryness.

In another aspect, the present disclosure provides use of a PI4KIIIα-specific inhibitor in the manufacture of a medicament for preventing or treating tissue or organ fibrosis such as cardiac fibrosis.

Use of a PI4KIIIα-specific inhibitor in the manufacture of a medicament for preventing or treating cellular tissue damage caused by radiation exposure in a tumor patient.

Use of a PI4KIIIα-specific inhibitor in the manufacture of a medicament for preventing or treating atherosclerosis or atherosclerosis-induced disease, wherein the atherosclerosis-induced disease is preferably coronary heart disease, heart attack or cerebral infarction.

Use of a PI4KIIIα-specific inhibitor in the manufacture of a medicament for preventing or treating diabetes in a tumor patient.

Use of a PI4KIIIα-specific inhibitor the manufacture of a medicament for preventing or treating a microbial infection, wherein the microbial infection is selected from bacterial or fungal infection, preferably drug-resistant bacterial infection or drug-resistant fungal infection, more preferably wherein the microorganism is selected from Acinetobacter baumannii, Enterobacter cloacae, Escherichia coli, Klebsiella oxytoca, Klebsiella pneumoniae, Morganella morgani, Pseudomonas aeruginosa, Serratia marcescens, Staphylococcus aureus, Enterococcus faecalis, Enterococcus faecium, Streptococcus pneumoniae, Candida albicans, Candida parapsilosis, Aspergillus fumigatus, Neisseria gonorrhoeae, Clostridium tetani, Propionibacterium acnes, Cryptococcus neoformans, Helicobacter pylori, Bacterioides fragilis, Clostridium difficile, Gardnerella vaginalis, Atopobium vaginae, Peptostreptococcus anaerobius, Trichomonas vaginalis, and at least one of the drug-resistant organisms thereof; or, preferably, the microbial infection is selected from a microorganism infecting surface of body or mucosal site such as the site of acne wound, pimple, wound, burn or scald.

Use of a PI4KIIIα-specific inhibitor in the manufacture of a medicament for preventing or treating asthma.

Use of a PI4KIIIα-specific inhibitor in the manufacture of a medicament for preventing or treating nonalcoholic steatohepatitis.

In one embodiment, wherein phenylarsine oxide and a derivative thereof are an antibody, a small molecule compound, a RNAi molecule or an antisense nucleic acid, preferably the antibody is a monoclonal or polyclonal antibody, more preferably the antibody is a chimeric antibody, a humanized antibody or a fully human antibody; preferably the RNAi molecule is a small interfering RNA (siRNA), a short hairpin RNA (shRNA) or microRNA (miRNA), more preferably the RNAi molecule has a length of 18-100 bases and/or the RNAi molecule is modified to enhance its stability; preferably the small molecule compound is phenylarsine oxide or a derivative thereof, G1 and an analogue thereof, A1 and an analogue thereof (J Med Chem 2014 Vol. 57 Issue 5 Pages 2091-106 and J Biol Chem 2014 Vol. 289 Issue 9 Pages 6120-32), or simepivir and an analogue thereof (Int J Radiation Oncol Biol Phys, Vol. 96, No. 4, pp. 867e876, 2016), TG-1478 ( Tyrphostin AG-1478, NSC-693255), more preferably phenylarsine oxide and the derivative thereof have a structure of Formula (II), or a pharmaceutically acceptable salt thereof: 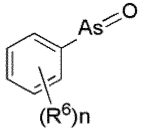 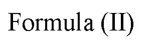
wherein R⁶ is each independently selected from (a) hydrogen, deuterium, methyl, mono-deuteromethyl, di-deuteromethyl, tri-deuteromethyl, halogen, nitro, cyano, hydroxy, amino, carbamoyl, C1-6 alkylsulfonyl, C1-6 alkyl, C1-6 cycloalkyl, C2-6 alkynyl, C2-6 alkenyl, C1-6 alkoxy, C1-6 haloalkyl, C1-6 alkylene-NH2, C1-6 alkylene-NH-C(O)H, -As(O), -N=NH, N-(C1-6 alkyl) amino, N,N-(C1-6 alkyl)2 amino, -NH-C(O)H, -NH-S(O)2H, -C(O)OH, -OC(O)H, -SH, -S(O)2H, -S(O)2-NH2 or heterocyclyl, which is optionally substituted by R⁷ or R⁸, wherein R⁷ and R⁸ are each independently selected from amino, C1-6 alkyl, C1-6 alkoxy, C1-6 haloalkyl, N-(C1-6 alkyl) amino, N-(6-12-membered-aryl) amino, N,N-(C1-6 alkyl)2 amino, C3-6 cycloalkyl, 6-12-membered aryl, or 3-12-membered heterocyclyl, which is optionally substituted by one or more halogen, nitro, cyano, hydroxyl, amino, carbamoyl, -NH-C(O)-R¹⁰, -C(O)OR⁹, 6-12-membered aryl, C1-6 alkyl, C2-6 alkynyl, C2-6 alkenyl, C1-6 alkoxy, C1-6 haloalkyl, 3-6-membered heterocyclyl, C3-6 cycloalkyl, or Bn-O-, and R⁹ is C1-6 alkyl, which is optionally substituted by one or more halogen, nitro, cyano, hydroxyl, amino, carbamoyl, 6-12-membered aryl, C1-6 alkyl, C2-6 alkynyl, C2-6 alkenyl, C1-6 alkoxyl, C1-6 haloalkyl, 3-6-membered heterocyclyl, C3-6 cycloalkyl, or Bn-O-, and R¹⁰ is selected from H, C1-6 alkyl, C2-6 alkynyl, C2-6 alkenyl, C1-6 alkoxy or C1-6 haloalkyl, and/or
(b) R⁶ on two adjacent carbon atoms forms 5-12-membered cycloalkyl, aryl or heterocyclyl, which is optionally substituted by one or more halogen, nitro, cyano, hydroxyl, amino, carbamoyl, 6-12-membered aryl, C1-6 alkyl, C2-6 alkynyl, C2-6 alkenyl, C1-6 alkoxyl, C1-6 haloalkyl, 3-6-membered heterocyclyl, C3-6 cycloalkyl or Bn-O-,
wherein n is an integer from 0 to 5.

In one embodiment, wherein n is an integer from 0 to 2, R⁶ is each independently selected from H, deuterium, methyl, mono-deuteromethyl, di-deuteromethyl, tri-deuteromethyl, halogen, nitro, cyano, hydroxy, amino, carbamoyl, C1-6 alkylsulfonyl, C1-6 alkyl, C1-6 cycloalkyl, C1-6 alkoxy, C1-6 haloalkyl, -As(O), N-(C1-6 alkyl) amino, N,N-(C1-6 alkyl)2 amino, -NH-C(O)H or -NH-S(O)2H, which is optionally substituted by R⁷ or R⁸.

In one embodiment, wherein n is an integer from 0 to 2, R⁶ is each independently selected from H, deuterium, methyl, mono-deuteromethyl, di-deuteromethyl, tri-deuteromethyl, halogen, nitro, cyano, hydroxyl, amino, C1-6 alkylsulfonyl, C1-6 alkyl, C1-6 cycloalkyl, C1-6 alkoxy, C1-6 haloalkyl, -As(O), -NH-C(O)H or -NH-S(O)2H, which is optionally substituted by R⁷ or R⁸.

In one embodiment, wherein n is 1 or 2, R⁶ is each independently selected from H, deuterium, methyl, mono-deuteromethyl, di-deuteromethyl, tri-deuteromethyl, halogen, amino, C1-6 alkylsulfonyl, C1-6 cycloalkyl, C1-6 alkoxy, C1-6 haloalkyl, -NH-C(O)R⁷ or -NH-S(O)2 R⁸, wherein R⁷ is C1-6 alkyl which is optionally substituted by 6-12-membered aryl, R⁸ is 6-12-membered aryl which is optionally substituted by one halogen, C1-6 alkoxyl or C1-6 haloalkyl.

In one embodiment, wherein the R⁶ is located at an ortho position and/or para position of the -As(O) group.

In one embodiment, wherein n is 0.

In one embodiment, wherein R¹, R², R³, R⁴, R⁵ are independently selected from hydrogen, deuterium, halogen, methyl, mono-deuteromethyl, di-deuteromethyl or tri-deuteromethyl, and at least one of R¹, R², R³, R⁴, R⁵ is deuterium or deuterated; preferably, R¹, R², R³, R⁴, R⁵ are independently selected from hydrogen or deuterium, and at least one of R¹, R², R³, R⁴, R⁵ is deuterium, at least two of R¹, R², R³, R⁴, R⁵ are deuterium, preferably, at least three, four or five of R¹, R², R³, R⁴, R⁵ are deuterium; more preferably, the compound is selected from the group consisting of the following compounds:

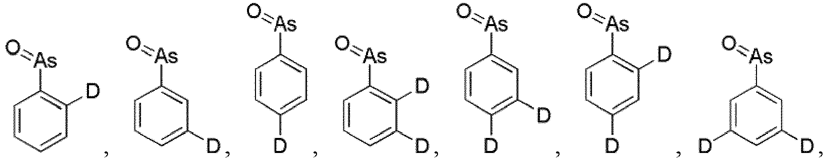

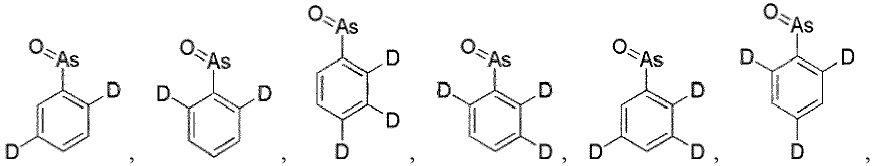

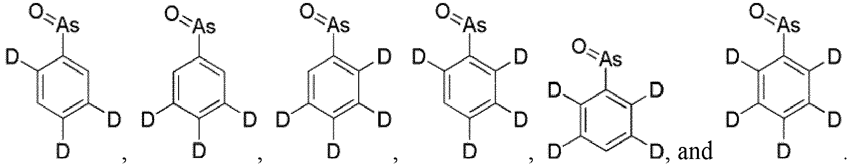

In one embodiment, the compound is selected from the group consisting of the following compounds:

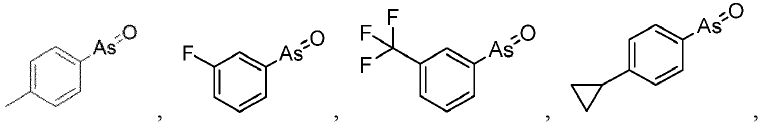

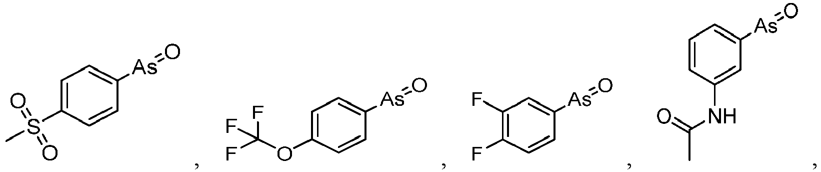

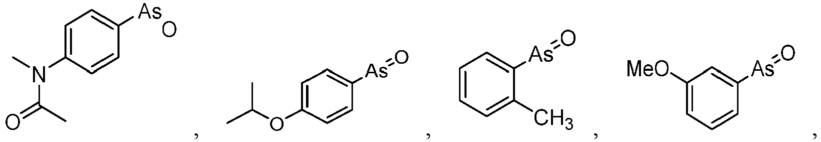

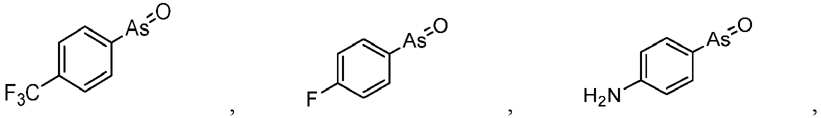

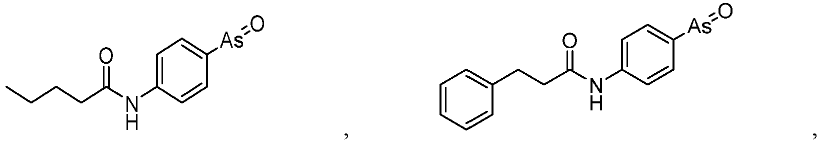

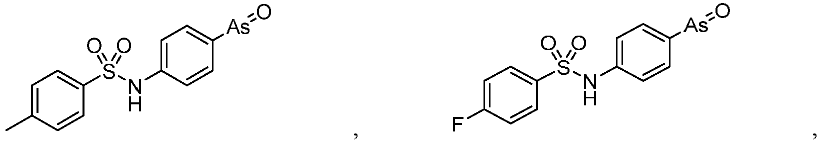

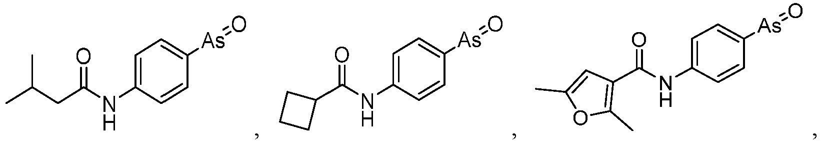

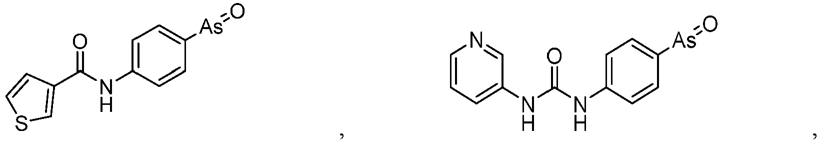

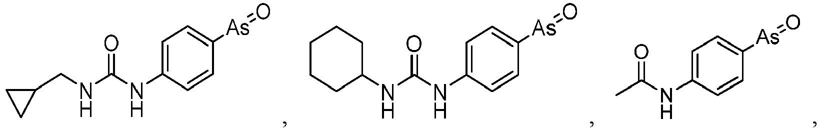

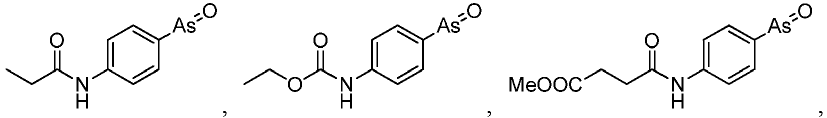

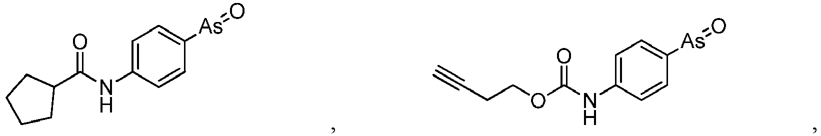

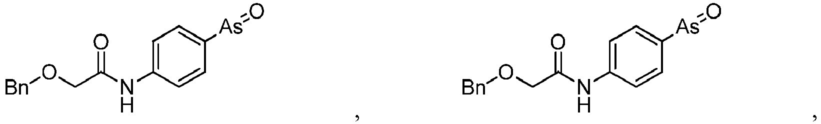

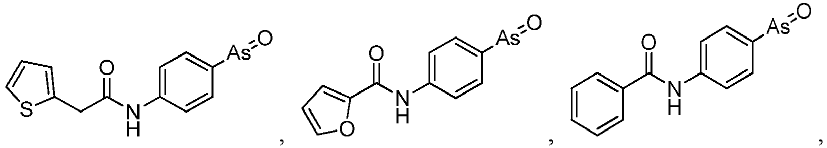

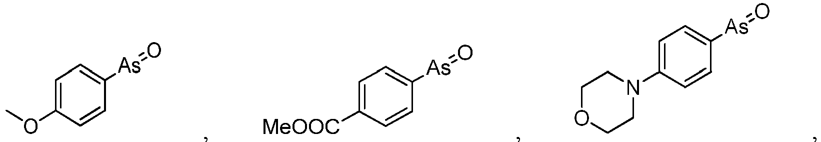

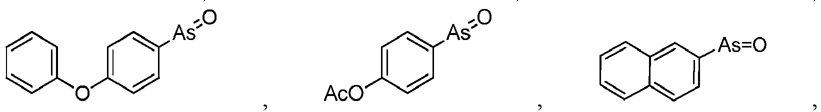

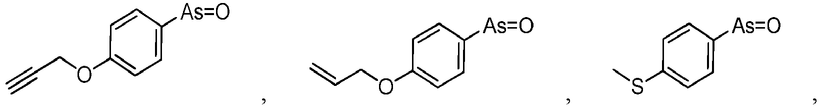

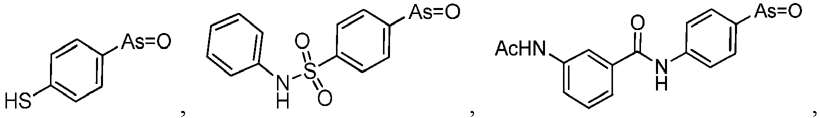

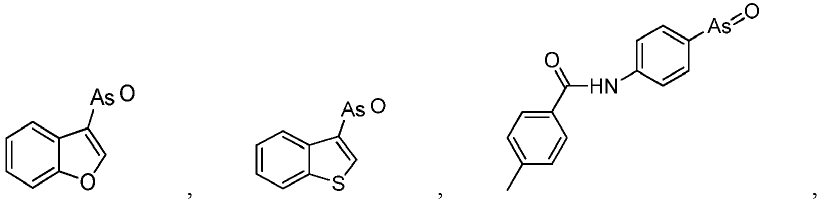

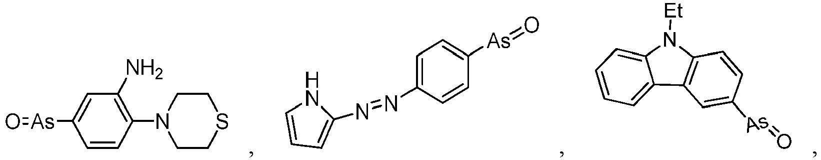

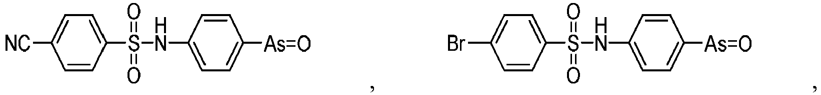

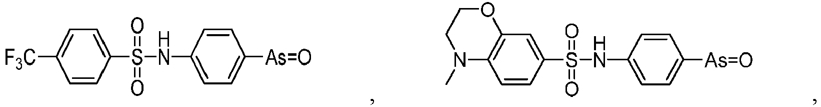

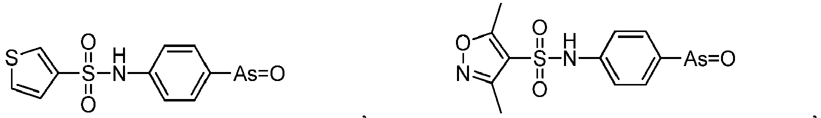

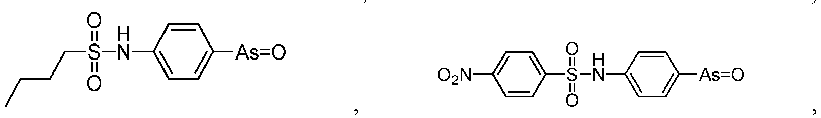

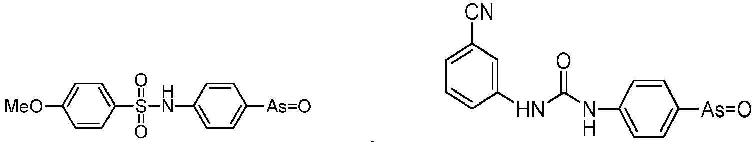

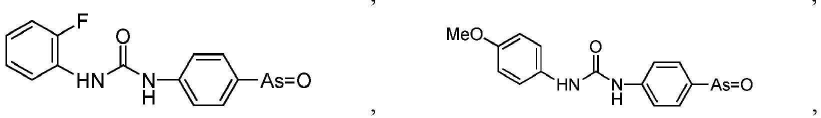

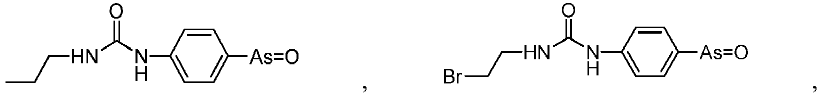

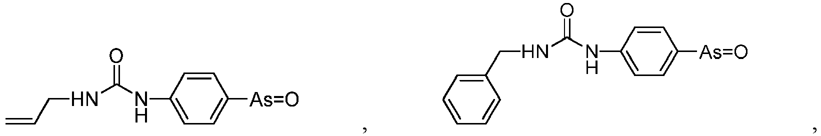

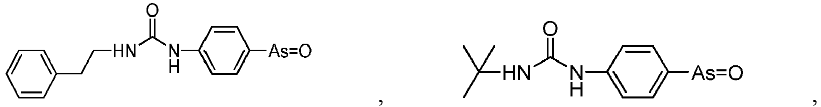

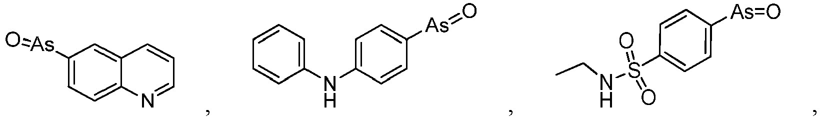

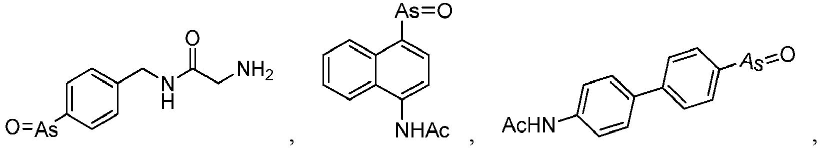

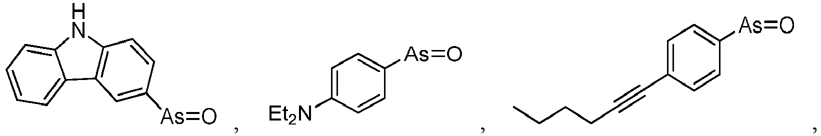

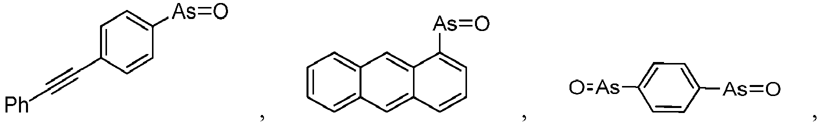

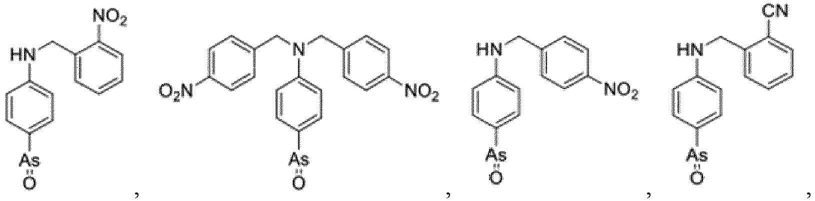

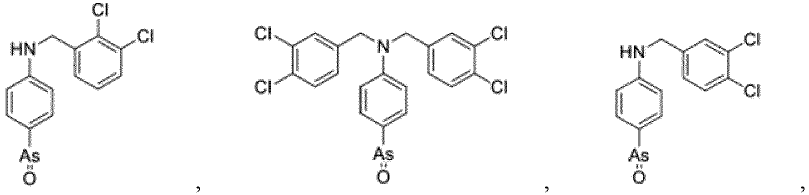

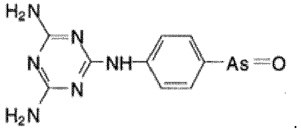

In one embodiment, the subject is human or non-human mammal.

In one embodiment, the cellular tissue damage resulting from exposure to radiation is selected from X-ray-induced damage to cells or tissues and organs.

In one embodiment, the symptom of cellular tissue damage resulting from exposure to radiation is selected from at least one of decreased salivation, dry mouth, mucositis caused by X-ray induced salivary gland damage, tissue and organ fibrosis and leukopenia caused by X-ray radiation.

In one embodiment, wherein the tumor is selected from lymphomas (e.g., acute non-B or non-T cell lymphoma), cervical cancer, endometrial cancer, liver cancer, breast cancer (e.g., triple-negative breast cancer), lung cancer (e.g., non-small cell lung cancer or small cell lung cancer), colorectal cancer, gastric cancer, skin cancer (e.g., melanoma), bone cancer, osteosarcoma, myeloma, blood cancer (e.g., acute or chronic leukemia), ovarian cancer, prostate tumor, brain tumor (e.g., cerebral astrocytoma), neuroblastoma, thyroid cancer, malignant embryonal rhabdomyosarcoma.

In one embodiment, further comprising administering a second reagent to a subject in need thereof, preferably the second reagent is a reagent used for treating tumor.

In one embodiment, wherein the second reagent is a reagent used for treating tumor.

In one embodiment, the drug for treating tumors is preferably selected from an antineoplastic agent interfering with mitosis such as paclitaxel or albumin paclitaxel, a drug directly affecting DNA structure and function such as carboplatin or cisplatin, or a Kras inhibitor.

In one embodiment, the compound is administered before, after or simultaneously with the second reagent.

The present disclosure further discloses a method of screening drugs for preventing or treating a disease, comprising contacting a candidate drug with a PI4KIIIα protein or nucleic acid or PI4KIIIα and testing whether the candidate drug is capable of inhibiting the formation or activity of the PI4KIIIα, the disease is selected from cardiac fibrosis, cellular tissue damage due to radiation exposure, atherosclerosis or arterial restenosis, diabetes mellitus or asthma.

The present disclosure further discloses an analytical method for measuring the concentration of PAO or a derivative thereof in blood, the derivative comprise a compound of Formula (I) or Formula (II), comprising the steps of:
Step 1: preparing three stock solutions by dissolving PAO or derivatives thereof, a control standard and sodium 2,3-dimercaptopropanesulfonate, respectively, in separate containers;
Step 2: mixing the blood comprising PAO or the derivative thereof to be tested with the stock solution of sodium 2,3-dimercaptopropanesulfonate, adjusting the solution to acidity, and incubating at 25-80°C for an appropriate time;
Step 3: adding the stock solution of the control standard to the solution obtained in step 2, shaking for a period of time and centrifuging, and taking the supernatant to test the derivatization product of PAO or the derivative thereof.
wherein in the step 2, the incubation time is 10-80 minutes, the acidity is pH less than 6, the solution is adjusted to acidity by adding appropriate amount of an acid such as formic acid, potassium hydrogen phosphate, acetic acid or hydrochloric acid, and the reaction in the step 2 is as follows: 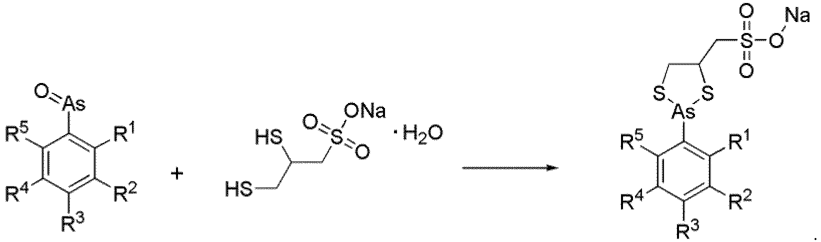
wherein in the step 1, PAO or the derivative thereof and the control standard are dissolved in DMSO, the DMSO solution of the control standard is diluted with acetonitrile, and sodium 2,3-dimercaptopropanesulfonate is dissolved in pure water.
wherein the control standard is tolbutamide, and in the step 3, the derivatization product of PAO or the derivative thereof is measured by a liquid chromatography-mass spectrometry method.

The derivative of PAO is selected from deuterated or halogenated compound, such as a compound of Formula (I) or Formula (II), or any of the foregoing PAO derivatives.

### BRIEF DESCRIPTION OF THE DRAWINGS

Figure 1 shows the drug concentration-time curve of pharmacokinetics in male SD rats after a single intravenous administration of 0.1 mg/Kg PAO, d5PAO (or d5-PAO) and F2m-PAO (or F2mPAO).
Figure 2 shows the drug concentration-time curve of pharmacokinetics in male SD rats after a single oral gavage administration of 0.2 mg/Kg PAO, d5PAO and F2m-PAO.

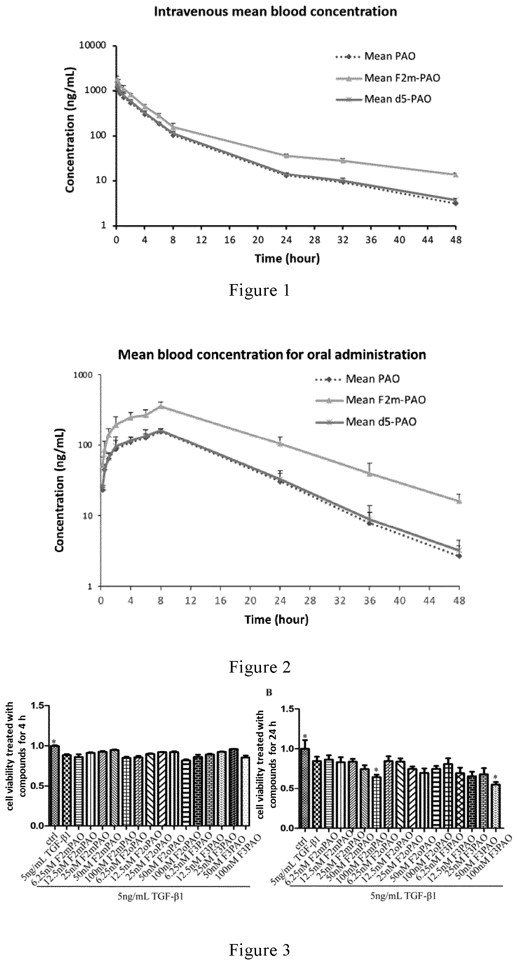

Figure 3 shows the cell viability of MRC-5 cells treated with a series of fluorinated compounds for 4 h and 24 h. MRC-5 cells were cultured for 4 h or 24 h with MEM medium (without FBS) containing 5 ng/mL TGF-β1 and different concentrations of a series of fluorinated compounds according to grouping. Cell viability was assayed using MTT. (A) Different concentrations of each fluorinated compounds were used for treatment for 4 h to detect cell viability; (B) Different concentrations of each fluorinated compound were used for treatment for 24 h to detect cell viability. Compared with 5 ng/mL TGF-β1 treatment group, * indicates p<0.03.

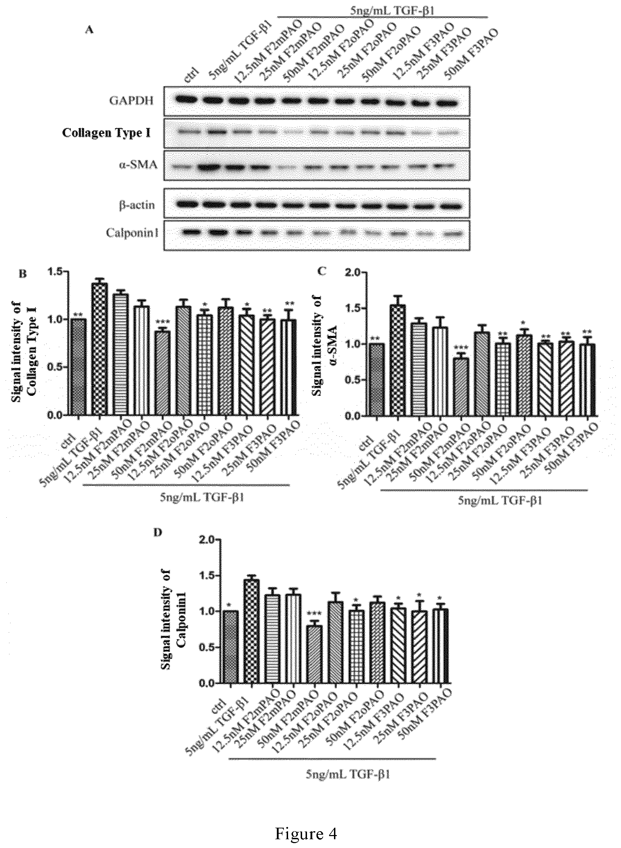

Figure 4 shows the inhibition of the expression of Collagen Type I, α-SMA and Calponin1 by a series of fluorinated compounds in MRC-5 model cells. (A) The expression levels of α-SMA, Calponin1 and Collagen Type I detected by protein immunoblotting. (B) Results of statistical analysis of Collagen Type I expression in each group. (C) Results of statistical analysis of α-SMA expression in each group. (D) Results of statistical analysis of Calponin1 expression in each group. Protein signal intensity was analyzed using Image J software, and the signal intensity of ctrl group was taken as 1, n=3, and processed using One-way ANOVA, the data were expressed as mean±SEM, and compared with the 5 ng/mL TGF-β1 treatment group, *p<0.03, **p<0.001, ***p<0.0001, and compared with ctrl group, #p<0.03.
Figure 5 shows the inhibition of the expression of α-SMA by a series of fluorinated compounds in MRC-5 model cells. (A) Immunofluorescence plots of α-SMA in each group, green: α-SMA, blue: DAPI (nuclear). Scale bar: 50 µm. (B) Analysis of statistical results of α-SMA immunofluorescence intensity. The mean value of ctrl group was taken as 1, n=5, and processed using one-way ANOVA; the data were expressed as mean±SEM, and compared with 5 ng/mL TGF-β1 treatment group, **p<0.001, ***p<0.0001.
Figure 6 shows the inhibition of the expression of Calponin1 by a series of fluorinated compounds in MRC-5 model cells. (A) Immunofluorescence plots of Calponin1 in each group, red: Calponin1, blue: DAPI (nuclear). Scale bar: 50 µm. (B) Analysis of statistical results of Calponin1 immunofluorescence intensity. The mean value of ctrl group was taken as 1, n=5, and processed using one-way ANOVA; the data were expressed as mean±SEM, and compared with 5 ng/mL TGF-β1 treatment group, *p<0.03, **p<0.001, ***p<0.0001.
Figure 7 shows that shRNA interference lentiviral vectors reduced PI4KIIIα expression. shRNA interference lentiviral vectors was co-incubated with MRC-5 cells for 48 hours and proteins were collected for protein immunoblotting experiments. (A) Detection of PI4KIIIα protein; (B) Data were analyzed using Image J software, normalized with sh-ctrl group as 1, n=3; data are expressed as mean±SEM; compared with sh-ctrl group, ****p<0.0001.

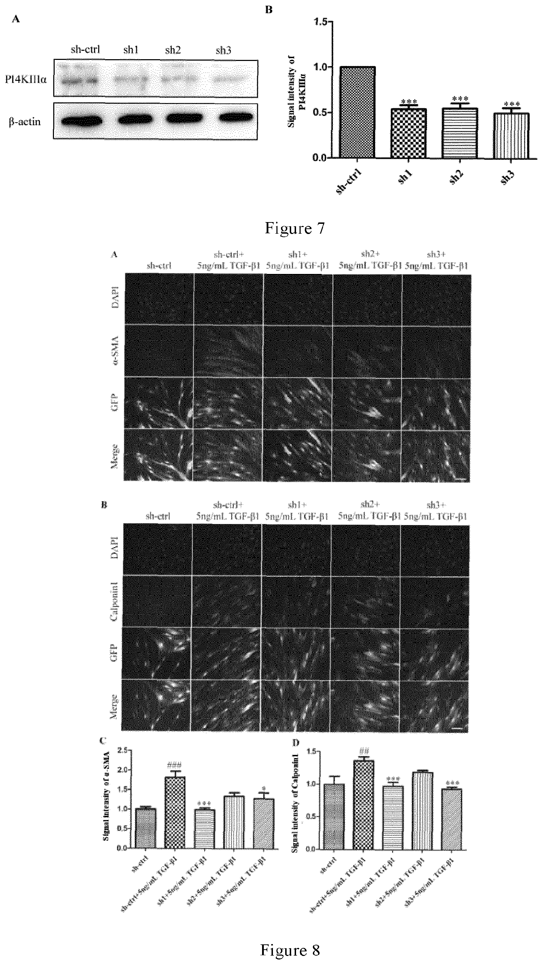

Figure 8 shows the inhibition of the expression of Calponin1 and α-SMA by knockdown of PI4KA in TGF-β1-treated MRC-5 cells. MRC-5 cells were cultured for 24 hours by adding lentiviral vectors with different sequences after cell adherence, and then treated with or without 5 ng/mL TGF-β1 for 24 hours according to the grouping, and observed by immunofluorescence staining. (A) Immunofluorescence staining of α-SMA in each group, red: α-SMA, blue: DAPI (nuclear), green: green fluorescent protein GFP. scale bar: 50 µm. (B) Immunofluorescence staining of Calponin1 in each group, red: Calponin1, blue: DAPI (nuclear), green: green fluorescent protein GFP. scale bar: 50 µm. (C) Analysis of statistical results of α-SMA and Calponin1 immunofluorescence intensity, normalized with the mean value of sh-ctrl group as 1, n=5, data are expressed as mean±SEM; compared with the group co-treated with sh-ctrl and 5 ng/mL TGF-β1, *p<0.03, ***p<0.0001, compared with sh-ctrl group, ##p< 0.001, ###p<0.0001.
Figure 9 shows the effects of different concentrations of d5PAO and PAO on cultured cardiac fibroblasts. n=3, processed using one-way ANOVA; T-test analysis was used among groups, and data are expressed as mean±SEM; compared with the 24-hour anoxia-treated group, the effects of 100 nmol/L of d5PAO and PAO on cardiac fibroblast proliferation and migration (***p<0.0001).
Figure 10 shows the qPCR result analysis graph of inhibition of expression of α-SMA and Col 1A genes by PAO in hypoxic stimulus-induced cells. n=3, processed using one-way ANOVA; T-test analysis was used among groups, and the data are expressed as mean±SEM; compared with the 24-hour anoxia-treated group, 50 nmol/L and 100 nmol/L of PAO may play a role in inhibiting fibrosis of cardiac fibroblasts (*p<0.05,**p<0.001).
Figure 11 shows the WB results that 24 hours of anoxia significantly increased the expression of Collagen Type I protein in cardiomyocytes. (A, B) Signal intensity values of α-SMA and Collagen Type I, (C) WB result plots of various concentrations of PAO. n=3, processed using one-way ANOVA, T-test analysis was used among groups, and data are expressed as mean±SEM; compared with the 24-hour anoxia-treated group, 20 nmol/L, 50 nmol/L and 100 nmol/L of PAO may play a role in inhibiting fibrosis in cardiac fibroblasts (*p<0.05,**p<0.001).
Figure 12 shows the inhibition of Collagen-I and α-SMA protein expression by A1 and G1 in hypoxic stimulus-induced cells. (A, B) Signal intensity values of α-SMA and Collagen Type I; (C) WB result plots of A1 and G1 at 100 nmol/L. n=3, processed with one-way ANOVA, and analyzed using T-test among groups; data are expressed as mean±SEM; compared with the 24-hour anoxia-treated group, 100 nmol/L of A1 and G1 may play a role in inhibiting fibrosis in cardiac fibroblasts (**p<0.001,***p<0.0001).
Figure 13 shows the inhibition of migration of anoxia-induced cardiac fibroblast by PAO and d5PAO. (A, C): the performance of 100 nmol/L of d5PAO/PAO in cell scratch assay, (B, D) are the statistical analysis: n=3, T-test analysis was used among groups; data were expressed as mean±SEM; compared with the 24-hour anoxia-treated group, 100 nmol/L of d5PAO and PAO may play a role in inhibiting fibrosis in cardiac fibroblasts (*p<0.05).
Figure 14 shows that PAO effectively inhibited the increase in α-SMA and Collagen-1 protein expression in the infarct zone. (A, B) Signal intensity values of α-SMA and Collage Type I protein; (C) WB result graph of 0.3 mg/kg PAO. n=3, T-test analysis was used among the groups; the data were expressed as mean±SEM; compared with infarct modeling group, 0.3 mg/kg PAO may play a role in inhibiting fibrosis in cardiac fibroblasts (*p<0.05).
Figure 15 shows that PAO effectively inhibited the increase in the fibrosis area of the infarct zone containing Collagen area. (A, B) Statistical graphs of HE and Masson staining results; (C) graphs of HE staining and Masson staining results. n=5, T-test analyses were used among the groups; the data were expressed as mean±SEM; compared with the infarct modeling group, 0.3 mg/kg PAO may play a role in inhibiting fibrosis of cardiac fibroblasts (**p<0.001).
Figure 16 shows the α-synuclein over-expression plasmid information.
Figure 17 shows the standard curve preparation of the samples .

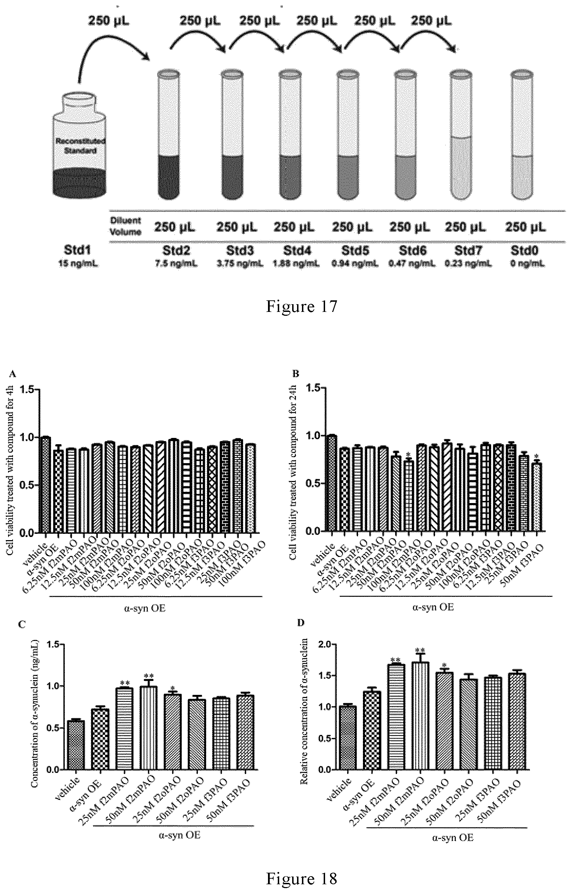

Figure 18 shows a series of fluorinated compounds have an effect on cell viability and promote α-synuclein efflux. SH-sy5y cells were transfected with the empty (vehicle) plasmid or the α-synuclein overexpression plasmid by Fugene HD transfection reagent, after 24 h of transfection, starved for 24 h, respectively treated with different concentrations of F2mPAO, F2oPAO and F3PAO according to the grouping. (A) Cell viability was measured by MTT after treatment with different concentrations of the series of fluorinated compounds for 4 hours; (B) Cell viability was measured by MTT after treatment with different concentrations of the series of fluorinated compounds for 24 hours; (C) The content of α-synuclein in the supernatant of the culture medium was detected by ELISA kits, and α-synuclein concentration for each group was calculated by the standard curve; (D) the data were normalized with the mean value of α-synuclein concentration in the empty plasmid (vehicle) group as 1, n=3, mean±SEM, One-way ANOVA, compared with the α-synuclein overexpression (α-syn OE) group, *p<0.03, **p<0.001.

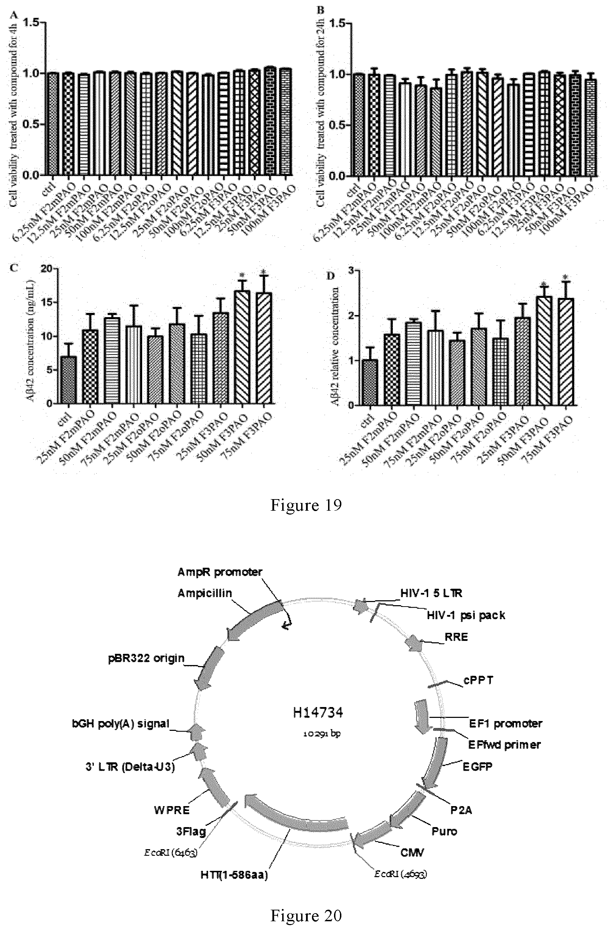

Figure 19 shows that a series of fluorinated compounds did not affect the viability of stable transfection APP (SW) HEK293 cells and promoted Aβ release. (A) the cell viability of stable transfection APP (SW) HEK293 cells treated with a certain concentration of a series of fluorinated compounds for 4 h was detected by MTT assay; (B) the cell viability of stable transfection APP (SW) HEK293 cells treated with a certain concentration of a series of fluorinated compounds for 24 h was detected by MTT assay, n=5, mean±SEM, One-way ANOVA; (C) the Aβ42 content in the supernatant of the culture medium was detected by ELISA kit, and the Aβ42 values of each group were calculated by standard curve; (D) data were normalized with the mean value of Aβ42 concentration in the control group (ctrl) as 1, and the multiplicity value of the change of Aβ42 content in each group was calculated, n=3, mean±SEM, One-way ANOVA, compared with the control group, *p<0.03.
Figure 20 shows the empty vector map of H14734.
Figure 21 shows the full map of H14734.
Figure 22 shows the empty vector map of H303 pcDNA3.1.
Figure 23 shows the full map of H303 pcDNA3.1.

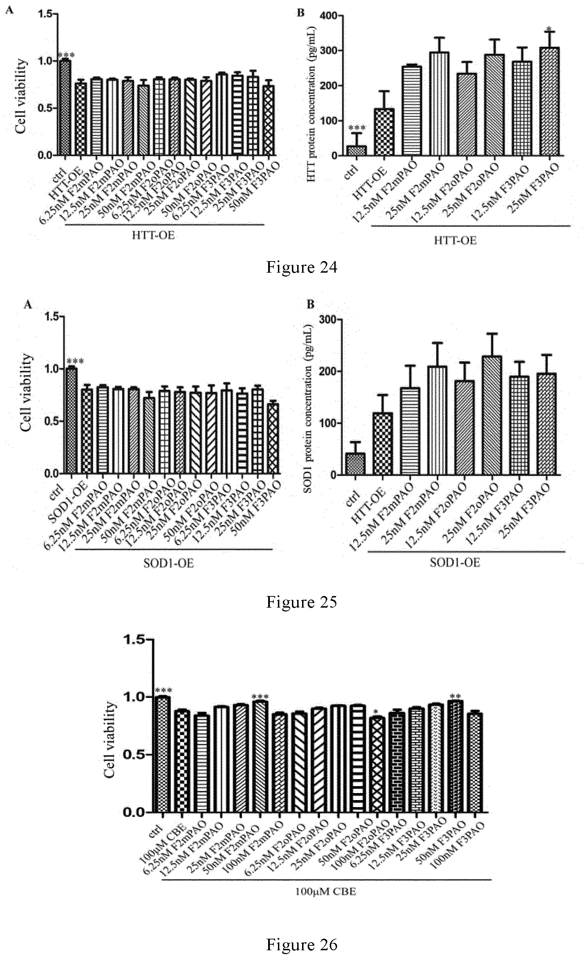

Figure 24 shows that a series of fluorinated compounds promote HTT secretion. (A) Cell lines overexpressing HTT for 24 hours were starved for 24 hours, then incubated with different concentrations of a series of fluorinated compounds for 24 hours, MTT was added and incubated for 4 hours, and the absorbance values were detected. n=5, data were expressed as MEAN±SEM, One-way ANOVA, compared with the control group, ***p<0.0001. (B) Cell lines overexpressing HTT for 24 hours were starved for 24 hours, then incubated with different concentrations of a series of fluorinated compounds for 4 hours, cell culture medium supernatant was collected and HTT protein concentration was detected by ELISA. n=3, data are expressed as mean±SEM, One-way ANOVA, compared with the control group, *p<0.03, ***p<0.01.
Figure 25 shows that a series of fluorinated compounds promote SOD1 secretion. (A) Cell lines overexpressing SOD1 for 24 hours were starved for 24 hours, then incubated with different concentrations of a series of fluorinated compounds for 24 hours, MTT was added and incubated for 4 hours, and the absorbance values were detected. n=5, data were expressed as mean±SEM, One-way ANOVA, compared with the control group, ***p<0.0001. (B) Cell lines overexpressing SOD1 for 24 hours were starved for 24 hours, then incubated with different concentrations of a series of fluorinated compounds for 4 hours, cell culture medium supernatant was collected and SOD1 protein concentration was detected by ELISA. n=3, data are expressed as mean±SEM, One-way ANOVA.
Figure 26 shows that a series of fluorinated compounds exerted a protective effect in the SH-SY5Y cell model constructed by CBE. SH-SY5Y cells were treated with 100 µM CBE for 24 h, starved (high-glucose DMEM without FBS) and co-treated with 100 µM CBE for 24 h, and then treated with different concentrations of a series of fluorinated compounds for 24 h. Cell viability was detected by MTT assay, n=5, data were expressed as mean±SEM, compared with 100 µM CBE-treated group, *p<0.03,**p<0.001, ***p<0.0001.
Figure 27 shows the inhibition of CBE-induced lysosomal storage by a series of fluorinated compounds. (A) SH-SY5Y cells were co-incubated with lysosome tracker for 30 min, and then the supernatant was aspirated and replaced with PAO in different concentrations and incubated for 10 min, and the Lyso-tracker was observed by immunofluorescence; (B) statistical analysis of the fluorescence intensity of the Lyso-tracker in each group; n=5; One-way ANOVA analysis, compared with 100 µM CBE-treated group, *p<0.03, **p<0.001, ***p<0.0001.
Figure 28 shows the bright field of cells treated with a series of fluorinated compounds for 12 h. BV2 cells were cultured for 48 h. The medium was removed and replaced with high-glucose DMEM medium containing 1 µg/mL LPS. The cells were treated with different concentrations of the series of fluorinated compounds according to the grouping for a total of 12 h, and were photographed using an Olympus IX50 fluorescence microscope (Olympus, Japan). Scale bar: 200 µm.

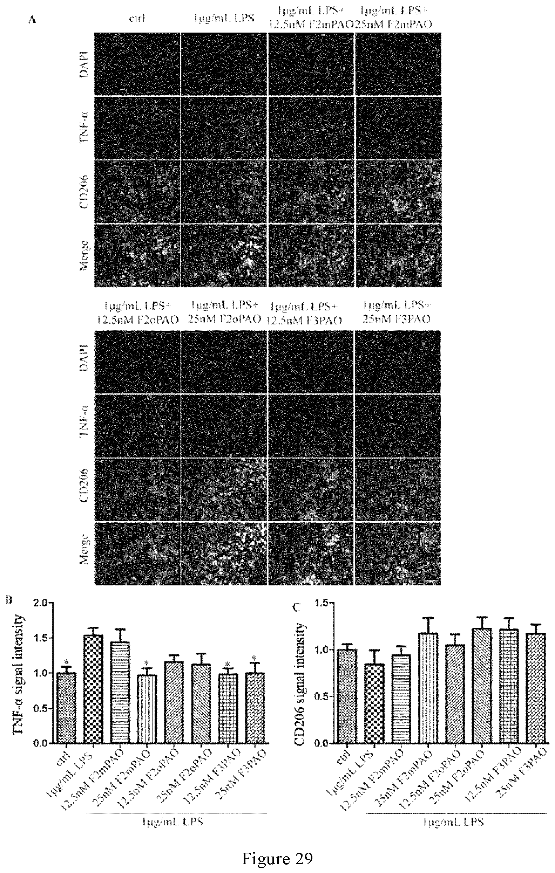

Figure 29 shows that a series of fluorinated compounds inhibited TNF-α and promoted CD206 expression in BV2 cell model. BV2 cells were cultured for 48 h. The medium was removed and replaced with high-glucose DMEM medium containing 1 µg/mL LPS. The cells were treated with different concentrations of the series of fluorinated compounds according to the grouping for a total of 12 h. (A) Immunofluorescence staining plots of different treatment groups; blue: DAPI, red: TNF-α, green: CD206. scale bar: 50 µm. (B) Statistical analysis of TNF-α immunofluorescence intensity. (C) Statistical analysis of CD206 immunofluorescence intensity. The mean value of the ctrl group was taken as 1, n=5, and processed using one-way ANOVA, and the data were expressed as mean±SEM, compared with the 1 µg/mL LPS-treated group, *p<0.03.
Figure 30 shows the ratio of CD206 fluorescence intensity to TNF-α fluorescence intensity. The CD206 immunofluorescence intensity was compared with the corresponding TNF-α immunofluorescence intensity, normalized with the mean value of the ctrl group ratio as 1, n=5, and processed using one-way ANOVA, and the data were expressed as mean±SEM, and compared with the 1 µg/mL LPS-treated group, *p<0.03.

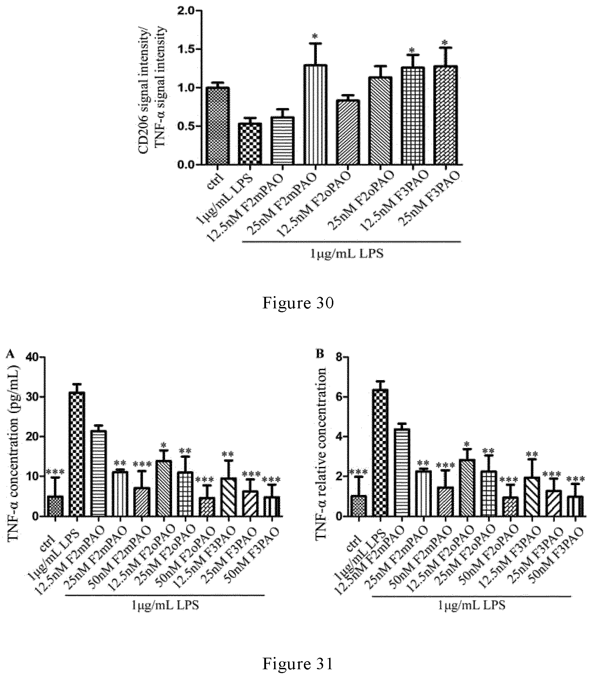

Figure 31 shows the inhibition of TNF-α efflux by a series of fluorinated compounds. BV2 cells were cultured for 48 h. The medium was removed and replaced with high-glucose DMEM medium containing 1 µg/mL LPS. The cells were treated with different concentrations of the series of fluorinated compounds according to the grouping for a total of 4 h, and the supernatants were collected for ELISA assay. (A) TNF-α concentration in the supernatant of the cell culture medium and TNF-α content (pg/mL) was calculated according to the standard curve; (B) the relative concentration change of TNF-α in each group was calculated by taking the mean value of the concentrations of the ctrl group as 1. n=3, processed using one-way ANOVA, and the data were expressed as mean±SEM, and compared with the 1 µg/mL LPS-treated group, *p<0.03, **p<0.001,***p<0.0001.
Figure 32 shows the bright field diagram of oil red O staining.
Figure 33 shows that the signal of oil red O staining of THP-1 cells differentiated by ox-LDL treatment (model) was stronger than that of cells not treated with ox-LDL (control).
Figure 34 shows the effect of F3PAO and d5PAO on body weight of ApoE^{-/-}mice fed with high-fat diet. t-test, *p<0.05 and **p<0.001 (compared with MCT group).
Figure 35 shows the effect of PAO on body weight of ApoE^{-/-} mice fed with high-fat diet. t-test, *p<0.05 (compared with MCT group).
Figure 36 shows the effect of PAO, F3PAO and d5PAO on fasting blood glucose of ApoE^{-/-} mice fed with high-fat diet. t-test, **p<0.01 and ***p<0.001 (compared with MCT group).
Figure 37 shows a derivatization reaction of PAO using sodium dimercaptosulphonate.
Figure 38 shows the LC-MS/MS chromatogram of representative SD rat whole blood where PAO (1000 ng/mL) was added and derivatized.
Figure 39 shows the LC-MS/MS chromatogram of representative SD rat whole blood where PAA (1000 ng/mL) was added and derivatized.
Figure 40 shows the tissue distribution properties of PAO in SD rats after a single gavage administration.
Figure 41 shows a mouse atherosclerotic plaque oil red O staining showing that PAO reduces the amount of atherosclerotic plaque.
Figure 42 shows that PAO reduced the proportion of atherosclerotic plaque area in the ApoE^{-/-} mice fed with a high-fat diet. n=4-6, *: P<0.05.
Figure 43 shows the effect of PAO on weight changes in asthma model mice.
Figure 44 shows that PAO is capable of improving the respiratory function in the asthma model mice: airway responsiveness monitoring-Penh Value statistical analysis.
Figure 45 shows that PAO is capable of inhibiting the rise in total leukocyte count in the asthma model mice.
Figure 46 shows that PAO is capable of inhibiting the rise of different types of leukocytes in the asthma model mice.
Figure 47 shows PAO improves respiratory pathology changes resulting from asthma modeling.
Figure 48 shows that PAO, d5PAO and F3PAO reduce lipid droplet and triglyceride accumulation in hepatocytes caused by oleic acid.
Figure 49 shows that F3PAO inhibits Ang2-induced proliferation of smooth muscle A7R5 cells.
Figure 50 shows that F3PAO inhibits Ang2-induced PCNA, MMP9 gene expression in A7R5 cells.
Figure 51 shows that F3PAO inhibits Ang2-induced expression of PCNA and MMP9 proteins in A7R5 cells (WB).
Figure 52 shows that PAO/F3PAO/GSK-A1 (A1) inhibits Ang2 stimulation-induced cellular Collagen-1 protein expression (WB).
Figure 53 shows that F3PAO inhibits the increase in the Ang2-induced migration ability of vascular smooth muscle cells (scratch assay).

### DETAILED DESCRIPTION OF THE INVENTION

The present disclosure is described in detail below in accordance with examples, and in combination with the accompanying drawings. The foregoing aspects of the present disclosure and other aspects of the present disclosure will be apparent from the following detailed description. The scope of the present disclosure is not limited to the following examples.

The term "compound" as used herein is intended to include all stereoisomers (e.g., enantiomers and diastereomers), geometric isomers, tautomers and isotopes of the structure shown.

The compounds described herein may be asymmetric (e.g., having one or more stereocenters). Unless otherwise indicated, all stereoisomers, e.g., enantiomers and diastereomers, are intended to be included. A variety of geometric isomers such as alkenes, carbon-carbon double bonds may also be present in the compounds described herein and all such stable isomers have been considered herein. The *cis* and *trans* geometric isomers of the compounds are described herein and can be isolated as mixtures of isomers or as individual isomers.

The compounds herein also include tautomeric forms. The tautomeric forms are caused by the interconversion between a single bond and an adjacent double bond accompanied by the migration of protons. The tautomeric forms include tautomers of protons in an isomeric protonated state with the same chemical formula and total charge. Examples of the proton tautomers include a keto-enol pair, an amide-imidic acid pair, a lactam-lactim pair, an enamine-imine pair, and an annular form in which protons can occupy two or more positions of a heterocyclic system, such as 1H- and 3H-imidazole, 1H-, 2H- and 4H-1,2,4-triazole, 1H- and 2H-isoindole, and 1H- and 2H-pyrazole. The tautomeric forms can be balanced or sterically locked into one form through appropriate substitution.

In certain embodiments, the small molecule compounds herein can be obtained by organic synthesis. The compound provided herein, including salts, esters, hydrates or solvates thereof, may be prepared by any well-known organic synthesis technology and may be synthesized according to many possible synthesis routes.

### PI4KIIIα-specific inhibitors

As used herein, the term "PI4KIIIα-specific inhibitor" refers to a class of substances that are capable of specifically reducing, decreasing, or eliminating the transcription or translation of the PI4KIIIα gene, and/or the activity of the PI4KIIIα protein. In some embodiments, the PI4KIIIα-specific inhibitor is capable of reducing the activity of PI4KIIIα by at least 5%, 10%, 20%, 40%, 50%, 80%, 90%, 95% or more. As used herein, when "activity" is used in conjunction with an increase or decrease, it refers to the detected functional activity, which may be characterized by a change in content, or no change in content but a change in functional activity.

As used herein, the activity of PI4KIIIα refers to the activity of the PI4KIIIα protein to phosphorylate phosphatidylinositol (PI) at a specific site (e.g., to convert to phosphatidylinositol 4-phosphate (PI4P)). The PI4KIIIα-specific inhibitor has a binding constant to the PI4KIIIα protein that is at least two times greater than its binding constant to other non-specific binding proteins. In some embodiments, the PI4KIIIα-specific inhibitor is capable of preferably recognizing PI4KIIIα proteins in complex mixtures, including mixtures with other PI4K subtype proteins.

In some embodiments, compared to other subtypes of PI4K proteins (e.g., PI4K proteins, including PI4KIIα or PI4KIIβ), the PI4KIIIα-specific inhibitor inhibits PI4KIII at least 1-fold, 2-fold, 4-fold, 5-fold, 10-fold, 20-fold, 30-fold, 50-fold, 100-fold, 200-fold, 500-fold or 10,000-fold more strongly. For example, in some embodiments, a PI4KIIIα-specific inhibitor substantially does not inhibit PI4KII (e.g., PI4KIIα or PI4KIIβ), e.g., with an IC50 greater than or equal to 10 µM, 20 µM, 30 µM, 40 µM, 50 µM, 60 µM, 80 µM, 100 µM, 150 µM, 200 µM or 500 µM.

In some embodiments, compared with other subtypes of PI4KIII (e.g., PI4KIIIβ), the PI4KIIIα-specific inhibitor inhibits PI4KIIIα at least 1-fold, 2-fold, 4-fold, 5-fold, 10-fold, 20-fold, 30-fold, 50-fold, 100-fold, 200-fold, 500-fold, or 10,000-fold more strongly.

In some embodiments, the IC50 of the PI4KIIIα-specific inhibitor against PI4KIIIα is less than or equal to 100 µM, 80 µM, 50 µM, 30 µM, 20 µM, 10 µM, 5 µM, 3 µM, 2 µM, 1 µM, 0.5 µM, 0.2 µM, 0.1 µM, 0.05 µM, 0.02 µM, 0.01 µM, 0.005 µM, 0.002 µM or 0.001 µM. In some embodiments, the PI4KIIIα-specific inhibitor is an antibody, a small molecule compound, an RNAi molecule, or an antisense nucleic acid.

In some embodiments, the PI4KIIIα-specific inhibitor is an antibody.

The term "antibody" as used herein includes any immunoglobulin, monoclonal antibody, polyclonal antibody, polyvalent antibody, bivalent antibody, monovalent antibody or antibody that can bind to a specific antigen. The term "antibody" herein is intended to broadly encompass conventional antibodies with four chains and unconventional antibodies without four chains (for example, antibodies that naturally lack light chains).

A conventional intact antibody is a heterotetramer, which contains two heavy (H) chains and two light (L) chains. Mammalian heavy chains are categorized as α, δ, ε, γ, and µ. Each heavy chain consists of one variable domain (V_{H}) and first, second, and third constant domains (C_{H1}, C_{H2}, and C_{H3}, respectively). Mammalian light chains are categorized as λ or κ. Each light chain consists of one variable domain (V_{L}) and one constant domain. Conventional antibodies have the "Y"-shaped structure with neck consisting of the second and third constant domains of two heavy chains joined by disulfide bonds. Each arm of the "Y"-shaped structure comprises the variable domain and the first constant domain of one of the two heavy chains that are joined with the variable domain and the constant domain of one light chain. The variable domains of the light chain and heavy chain determine the binding to an antigen. The variable domain of each chain contains three hypervariable regions referred to as complementarity-determining regions (CDRs) (CDRs of the light chain include LCDR1, LCDR2, and LCDR3, and CDRs of the heavy chain include HCDR1, HCDR2, and HCDR3). Wherein three CDRs are separated by a lateral contiguous portion of a framework region (FR), and the FR is more highly conserved than the CDR and forms a scaffold to support the hypervariable loop. The constant region of the heavy chain and light chain is not involved in the binding to an antigen but has multiple effector functions.

In some embodiments of the present disclosure, the antibody is a full-length antibody or an antigen-binding fragment.

As used herein, the term "antigen-binding fragment" refers to an antibody fragment formed by an antibody fragment comprising one or more CDRs but without an entire antibody structure. For example, the antigen-binding fragment includes, but is not limited to, a Fab fragment, a Fab' fragment, a F(ab')₂ fragment, an Fv fragment, a single-chain antibody molecule (scFv), an scFv dimer, a camelized single domain antibody, and a nanobody. The antigen-binding fragment can bind to the same antigen as the parent antibody.

The "Fab" fragment of an antibody refers to the portion of the antibody consisting of one light chain (including a variable domain and a constant domain) and a variable domain and a first constant domain of one heavy chain, which are jointed by disulfide bonds.

The "Fab'" fragment refers to a Fab fragment that contains part of the hinge region.

The "F(ab')₂" fragment refers to a dimer of the Fab'.

The "Fv" fragment of an antibody consists of the variable domain of one light chain and the variable domain of one heavy chain.

The "single-chain antibody molecule" or "scFv" refers to an engineered antibody made from the variable domain of a light chain and the variable domain of a heavy chain that are jointed directly or by a peptide chain. Details can be found in Huston JS et al., Proc Natl Acad Sci USA, 85:5879 (1988).

The "scFv dimer" refers to an oligomer formed by two scFvs.

The "camelized single domain antibody" (also referred to as "heavy-chain antibody" or "heavy-chain-only antibody (HCAb)") refers to an antibody that contains two heavy chain variable domains and no light chain. The heavy-chain antibody is originally derived from the family Camelidae (camelids, dromedaries, and llamas). Although lack of the light chain, the camelized single domain antibody has all the functions for the binding to an antigen.

The "nanobody" consists of one heavy chain variable domain from the heavy-chain antibody and two constant domains C_{H2} and C_{H3}.

In some embodiments, the antibody is a monoclonal antibody or a polyclonal antibody.

In some embodiments, the antibody is a murine antibody, a rabbit antibody, a chimeric antibody, a humanized antibody, or a fully human antibody.

As used herein, the term "fully human", when used in the antibody or antigen-binding fragment, means that the amino acid sequence of the antibody or antigen-binding fragment corresponds to the amino acid sequence of an antibody produced by a human or human immune cells or derived from non-human sources such as transgenic non-human animals based on the human antibody library, or other sequences encoding human antibodies.

As used herein, the term "humanized", when used in the antibody or antigen-binding fragment, refers to an antibody or antigen-binding fragment including CDRs derived from non-human animals, FRs derived from humans, and, where applicable, constant domains derived from humans. The humanized antibody or antigen-binding fragment may be used in some embodiments as a therapeutic agent for humans since it is less immunogenic. In some embodiments, the non-human animals are mammals (such as mice, rats, rabbits, goats, sheep, guinea pigs or hamsters). In some embodiments, the humanized antibody or antigen-binding fragment consists essentially entirely of human sequences, except that the CDR sequences are non-human.

As used herein, the term "chimeric", when used in the antibody or antigen-binding fragment, refers to an antibody or antigen-binding fragment including a heavy chain and/or a light chain with one portion derived from the same species and the other portions derived from different species. In some embodiments, the chimeric antibody may include a constant domain derived from human and a variable domain derived from a non-human animal (such as a mouse or a rabbit).

In some embodiments, the antibody described herein is a monospecific, bispecific, or multispecific antibody.

In some embodiments, the antibody described herein may be further marked.

In some embodiments, the PI4KIIIα-specific inhibitor is an RNAi molecule.

As used herein, the term "RNAi molecule" refers to an RNA or analog thereof that is sufficiently complementary in sequence to a target RNA to direct RNA interference. In some embodiments, DNA that can be used to produce RNA is also involved. RNAi is a process of sequence-specific selection in which target molecules (such as target gene, protein, or RNA) are downregulated.

In some embodiments, the RNAi molecule is capable of reducing the expression of PI4KIIIα, e.g., knocking down the PI4KA gene.

In some embodiments, the RNAi molecule has a length of 18-100 bases.

In some embodiments, the RNAi molecule is modified to enhance its stability.

In some embodiments, the RNAi molecule is a small interfering RNA (siRNA), a short hairpin RNA (shRNA) or a microRNA (miRNA).

As used herein, the term "small interfering RNA (siRNA)" refers to an RNA molecule, preferably a double-stranded molecule, with a length of about 10-50 nucleotides, preferably a length of about 15-25 nucleotides, more preferably a length of about 17, 18, 19, 20, 21, 22, 23, 24, or 25 nucleotides. Optionally, the strand has an overhanging end including, for example, one, two, or three overhanging nucleotides (or nucleotide analogs) that can direct or mediate the degradation of RNA.

As used herein, the term "short hairpin RNA (shRNA)" refers to an RNA molecule with a stem-loop structure that includes a first region and a second region that are complementary to each other. The degree of complementarity and the orientation of the regions are sufficient for base pairs to occur between the regions. The first region and the second region are linked by a loop region. The loop results from the absence of base pairing between nucleotides (or nucleotide analogs) in a loop region.

As used herein, the term "microRNA" or "miRNA" is a short and naturally occurring single-stranded non-coding RNA molecule with a length of about 16-26 nucleotides (nt) (for example, about 16-29 nt, 19-22 nt, 20-25 nt, or 21-23 nt), which usually functions in regulation of gene expression in vivo. In eukaryotic cells, a miRNA gene is transcribed by DNA transcriptase II into a "primary product" (pri-miRNA), the pri-miRNA is quickly processed by a ribonuclease III (Drosha) into a miRNA "precursor" (pre-miRNA), the pre-miRNA is transported from the nucleus to the cytoplasm, and then recognized and cleaved by another ribonuclease III (Dicer) into a mature miRNA. The mature miRNA molecule is partially complementary to one or more mRNAs and regulates expression of protein. Known miRNA sequences can be obtained from public databases, for example, the miRBase database (www.mirbase.org), providing miRNA sequence information, functional annotations, predicted gene targets, and other information. In the present disclosure, miRNA also includes RNA molecules with similar structure and function to natural miRNAs that are expressed in cells by artificially synthesized plasmids, and target corresponding mRNAs like natural miRNA molecules to prevent the translation of the mRNAs into proteins.

In some embodiments, the PI4KIIIα-specific inhibitor is an antisense nucleic acid.

As used herein, the term "antisense nucleic acid" includes nucleotides that are fully complementary to a target sequence and nucleotides with one or more nucleotide mismatches, so long as the antisense nucleic acid can specifically hybridize to the target sequence. For example, the antisense nucleic acid in the present application includes polynucleotides with at least 70% or more, preferably 80% or more, more preferably 90% or more, even more preferably 95% or more homology in a length of at least 15 contiguous nucleotides. Transcription of the target gene and/or translation of the target mRNA are/is reduced or blocked due to the formation of a hybrid.

In some embodiments, the PI4KIIIα inhibitor is a small molecule compound.

As used herein, the term "small molecule compound" means an organic compound with a molecular weight of less than 3000, 2500, 2000, 1500, 1000 or 500 daltons, which may be natural or chemically synthesized.

As used herein, the term "phenylarsine oxide" (PAO) refers to a small molecule compound with the following specific chemical structure:

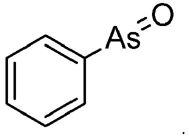

As used herein, the term "substituted", when used in reference to a chemical group, means that one or more hydrogen atoms of the chemical group are removed and substituted with a substituent.

As used herein, the term "substituent", having its common meaning known in the art, refers to a chemical moiety that is covalently linked or, where appropriate, fused to a parent group.

As used herein, the term "Cₙ-Cₘ" represents a range of carbon atom numbers, wherein n and m are integers and the range of carbon numbers includes the endpoints (i.e., n and m) and integer points therebetween. For example, C₁-C₆ represents a range of 1 to 6 carbon atoms, including 1, 2, 3, 4, 5, and 6 carbon atoms.

As used herein, the term "alkyl", whether used as part of another term or alone, refers to a saturated hydrocarbyl group that may be straight-chained or branched-chained. The term "Cₙ-Cₘ alkyl" refers to an alkyl group with n to m carbon atoms. In some embodiments, the alkyl group contains 1 to 12, 1 to 8, 1 to 6, 1 to 4, 1 to 3, or 1 to 2 carbon atoms. For example, the alkyl group includes, but is not limited to, chemical groups such as methyl, ethyl, n-propyl, isopropyl, n-butyl, tert-butyl, isobutyl, sec-butyl, 2-methyl-1-butyl, n-pentyl, 3-pentyl, n-hexyl, and 1,2,2-trimethylpropyl, etc.

As used herein, the term "alkenyl", whether used as part of another term or alone, refers to an unsaturated hydrocarbyl group that may be straight-chained or branched-chained, with at least one carbon-carbon double bond. In some embodiments, the alkenyl group contains 2 to 12, 2 to 10, 2 to 8, 2 to 6, 2 to 5, 2 to 4, or 2 to 3 carbon atoms. In some embodiments, the alkenyl group may also contain 1 to 6, 1 to 5, 1 to 4, 1 to 3, 1 to 2, or 1 carbon-carbon double bond. For example, the alkenyl group includes, but is not limited to, chemical groups such as vinyl, n-propenyl, isopropenyl, n-butenyl, and sec-butenyl, etc.

As used herein, the term "alkynyl", whether used as part of another term or alone, refers to an unsaturated alkynyl group that may be straight-chained or branched-chained, with at least one carbon-carbon triple bond. In some embodiments, the alkynyl group contains 2 to 12, 2 to 10, 2 to 8, 2 to 6, 2 to 5, 2 to 4, or 2 to 3 carbon atoms. In some embodiments, the alkynyl group may also contain 1 to 6, 1 to 5, 1 to 4, 1 to 3, 1 to 2, or 1 carbon-carbon triple bond. For example, the alkynyl group includes, but is not limited to, chemical groups such as ethynyl, propynyl, and butynyl, etc.

As used herein, the term "cycloalkyl" refers to a ring alkyl group consisting of at least 3 atoms. The term "n- to m- membered cycloalkyl" refers to a cycloalkyl group with n to m members for the formation of the ring. In addition, the ring may also contain one or more double bonds without a fully conjugated system. In some embodiments, the cycloalkyl group contains 3 to 8, 3 to 6, or 4 to 6 carbon atoms for the formation of the ring. For example, the cycloalkyl group includes, but is not limited to, cyclopropane, cyclobutane, and cyclopentyl, etc.

As used herein, the term "heterocyclyl" refers to a ring group in which at least one atom in the ring is a heteroatom and the remaining atoms are carbon atoms. The term "n- to m- membered heterocyclyl" refers to a heterocyclyl group with n to m members for the formation of the ring. In the present application, the term "heterocyclyl" includes heteroaryl and heterocycloalkyl. In addition, the ring may also contain one or more double bonds. In some embodiments, the heterocyclyl is a saturated heterocycloalkyl group. For example, the heteroatom includes, but is not limited to, oxygen, sulfur, nitrogen, and phosphorus, etc.

As used herein, the term "heterocycloalkyl" refers to a cycloalkyl group in which at least one atom in the ring is a heteroatom and the remaining atoms are carbon atoms. The term "n- to m- membered heterocycloalkyl" refers to a heterocycloalkyl group with n to m members for the formation of the ring. In addition, the ring may also contain one or more double bonds without a fully conjugated system. In some embodiments, the heterocycloalkyl is a saturated heterocycloalkyl group. For example, the heteroatom includes, but is not limited to, oxygen, sulfur, nitrogen, and phosphorus. In some embodiments, the heterocycloalkyl group contains 3 to 8, 3 to 6, or 4 to 6 carbon atoms for the formation of the ring. For example, the heterocycloalkyl group includes, but is not limited to, azetidine, aziridine, pyrrolidinyl, piperidinyl, piperazinyl, morpholinyl, thiomorpholine, and homopiperazine, etc.

As used herein, the term "aryl", whether used as part of another term or alone, refers to a mono- or poly-carbocyclic group with alternating double and single bonds between the carbon atoms forming the ring. The term "Cₙ-Cₘ aryl" refers to an aryl group with n to m carbon atoms for the formation of the ring. In some embodiments, the aryl ring system contains 6 to 12, 6 to 10, or 6 to 8 carbon atoms in one or more rings. In some embodiments, the aryl ring system contains two or more fused rings. For example, the aryl group includes, but is not limited to, chemical groups such as phenyl, naphthyl, tetrahydronaphthyl, indanyl, and indenyl, etc.

As used herein, the term "heteroaryl" refers to an aryl group in which at least one ring atom is a heteroatom and the remaining ring atoms are carbon atoms. The term "n- to m- membered heteroaryl" refers to a heteroaryl group with n to m members for the formation of the ring. For example, the heteroatom includes, but is not limited to, oxygen, sulfur, nitrogen, and phosphorus, etc. In some embodiments, the heteroaryl group may contain 5 to 10, 5 to 8, or 5 to 6 members for the formation of the ring. In some embodiments, the heteroaryl group is 5- or 6- membered heteroaryl. For example, the heteroaryl group includes, but is not limited to, furyl, thienyl, pyridyl, quinolyl, pyrrolyl, N-lower alkylpyrrolyl, pyridyl-N-oxide, pyrimidinyl, pyrazinyl, imidazolyl, and indolyl.

As used herein, the term "alkoxy", whether used as part of another term or alone, refers to the "-O-alkyl" group. The term "Cₙ-Cₘ alkoxy" means that the alkyl portion of the alkoxy group contains n to m carbon atoms. In some embodiments, the alkyl portion contains 1 to 6, 1 to 4, or 1 to 3 carbon atoms. For example, the alkoxy group includes, but is not limited to, chemical groups such as methoxy, ethoxy, propoxy (e.g., n-propoxy and isopropoxy), and t-butoxy, etc.

As used herein, the term "haloalkyl", whether used as part of another term or alone, refers to the "-alkyl-X" group, wherein X is a halogen selected from the group consisting of fluorine, chlorine, bromine and iodine. The term "Cₙ-Cₘ haloalkyl" means that the alkyl portion of the haloalkyl group contains n to m carbon atoms. In some embodiments, the alkyl portion contains 1 to 6, 1 to 4, or 1 to 3 carbon atoms. For example, the haloalkyl group includes, but is not limited to, chemical groups such as halomethyl, haloethyl, halopropyl (e.g., n-halopropyl and isohalopropyl), and t-halobutyl, etc.

As used herein, the term "n-membered", wherein n is an integer, is generally used with a ring system to describe the number of atoms in the ring system that form the ring. For example, piperidinyl is one of 6-membered heterocycloalkyl groups, pyrazolyl is one of 5-membered heteroaryl groups, pyridyl is one of 6-membered heteroaryl groups, and 1,2,3,4-tetrahydro-naphthalene is one of 10-membered aryl groups. As used herein, the term "n-m-membered" is generally used with a ring system to describe the number of members in the ring system that form the ring, wherein n and m are integers and the range of ring-forming members includes the endpoints (i.e., n and m) and each integer point therebetween. For example, 3-8-membered denotes a range of 3 to 8 ring-forming members, including 3 members, 4 members, 5 members, 6 members, 7 members, and 8 members.

As used herein, the term "halogen" refers to an atom selected from the group consisting of fluorine, chlorine, bromine and iodine.

As used herein, the term "cyano" refers to the "-CN" group.

As used herein, the term "hydroxyl" refers to the "-OH" group.

As used herein, the term "nitro" refers to the "-NO₂" group.

As used herein, the term "amino" refers to the "-NH₂" group.

As used herein, the term "carbamoyl" refers to the "-HNCONH₂" group.

As used herein, the terms "A1" and "G1" are both small molecule inhibitors of the PI4KIIIα protein with similar structures, wherein A1 has the formula:

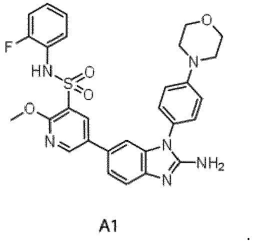

### 5-(2-amino-1-(4-(4-morpholinyl)phenyl)-1H-benzimidazol-6-yl)-N-(2-fluoro phenyl)-2-methoxy-3-pyridinesulfonamide

G1 has the formula:

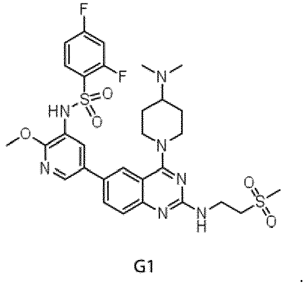

### (aS)-5-(2-amino-4-oxo-3-(2-(trifluoromethyl)phenyl)-3,4-dihydroquinazolin-6-yl)-N-(2,4-difluorophenyl)-2-methoxypyridine-3-sulfonamide.

### Cellular tissue damage caused by radioactive radiation exposure

Radiation therapy is one of the main means of treating malignant tumors, and its main principle is to use high-energy rays to destroy the chromosomes of the cancer cells, thus causing the cancer cells (especially those that are growing and dividing) to necrotic, and thus achieving the result of inhibiting the development of tumors. Radiation includes α, β, γ rays produced by radioisotopes and x-rays, electron rays, proton beams and other particle beams produced by various types of x-ray therapy machines or gas pedals, etc. 70% of the cancer patients need radiation therapy in the treatment process, and 40% of the cancers can be cured by radiation therapy. Therefore, radiation therapy is the first choice and the main means of treatment for some tumor types, such as oral cavity cancer, nasopharyngeal cancer, mid-stage esophageal cancer, inoperable lung cancer, skin cancer, and early lymphoma. Most tumors of the nervous system require surgery followed by radiation therapy. Tumors that are highly sensitive to radiation such as malignant lymphoma, testicular seminoma, nephroblastoma, neuroblastoma, medulloblastoma, small cell lung cancer, advanced lung cancer, and esophageal cancer.

As used herein, the term "radioactive rays" includes α, β, γ rays produced by radioisotopes, x-rays produced by various types of x-ray therapy machines or accelerator-generated x-rays, electron beams, proton beams or other particle beams, etc.

As used herein, the term "cell and tissue damage caused by radioactive radiation exposure" means that radioactive rays lead to changes in the structure and function of normal cells, tissues and organs in the irradiated area, such as damage or necrosis of normal cells, and the symptoms of cell and tissue damage caused by radioactive radiation exposure include skin damage, vomiting, hair loss, headache, fever, cough, difficulty in swallowing, decreased appetite, changes in the oral cavity (dryness), diarrhea, tissue necrosis, decreased peripheral blood counts, etc, for example, decreased salivary secretion due to damage to salivary glands caused by X-ray irradiation of the head and neck, dryness of the oral cavity, mucositis, and decreased leukocytes due to X-ray irradiation.

### Intracellular protein misfoldins related disease

As used herein, the term "intracellular protein misfolding related disease" refers to diseases that are characterized by aggregations of abnormally folded proteins in the cytoplasm and are also diagnosed as protein aggregation (aggregation, accumulation) or protein misfolding diseases. In addition, the term "intracellular protein misfolding related disease" also encompasses intracellular inclusion body lesions, such as protein inclusion body aggregation diseases, where the inclusion body is mainly composed of a core protein that aggregates due to misfolding, with various stress proteins involved in responding to the unfolded protein.

The intracellular protein misfolding related diseases include, but are not limited to, Parkinson's disease (PD), Lewy body dementia (LBD), multiple system atrophy (MSA), inclusion body myositis (IBM), frontotemporal dementia (FTD), Huntington's disease (HD), polyglutamine disease (polyQ), amyotrophic lateral sclerosis (ALS), prion diseases.

### Lysosomal storage disease

As used herein, the term "lysosomal storage disease" means a disease caused by the accumulation of endogenous or exogenous substances in a lysosome due to various reasons, which includes but is not limited to, lysosomal function defects caused by insufficient enzyme activity in the lysosome, or deficiency of processing-correcting enzymes of activator proteins, transporter proteins, or lysosomal protein, resulting in the failure of corresponding substrates to be digested in the secondary lysosome, substrate accumulation, metabolic disorders, and the formation of a storage disease.

Lysosomal storage diseases include, but are not limited to, disorders of sphingolipid metabolism, mucopolysaccharidosis, glycogen storage disease, glycoprotein storage disease, lipid storage disease, post-translational modification defective disorder, intrinsic membrane protein deficiency disorder, neuronal waxy lipofuscinosis, or lysosome-associated organelle disorders. Among these, disorders of sphingolipid metabolism include, but are not limited to, Fabre's disease, metabolic dysgerminosis (Farbe's disease), Gaucher disease types I, II, III, and perinatal death, GM1 gangliosidosis types I, II, and III, GM2 gangliosidosis (familial amaurotic idiocy), GM2 gangliosidosis, and globoid cell leukodystrophy (Krabbe disease), metachromatic leukodystrophy, Niemann-Pick disease types A and B. The mucopolysaccharidoses include, but are not limited to, Hurler-Scheie syndrome and Scheie syndrome (ML I), Hunter syndrome (MPS II), Sanfilippo syndrome A (MPS IIIA), Sanfilippo syndrome B (MPS IIIB), Sanfilippo syndrome C (MPS IIIC), Sanfilippo syndrome D (MPS IIID), Morquio syndrome type A (MPS IVA), Morquio syndrome type B (MPS IVB), Mucopolysaccharidosis type VI (Maroteaux-Lamy syndrome, MPS VI), Sly syndrome (MPS VII), mucopolysaccharidosis type IX (MPS IX). The glycogen storage diseases including, but are not limited to, the rare disease Pompe disease (GSD II). The glycoprotein storage disorders include, but are not limited to, α-mannosidosis, β-mannosidosis, fucosidosis, aspartylglucosaminuria, Schindler's disease type I (infantile-onset neuraxonal dystrophy), Schindler's disease type II (Kanzaki's disease), Schindler's disease type III (moderate severity), sialic acidosis type I (cherry-red dotted myoclonus), sialic acidosis type II (mucopolysaccharidosis I), and galactose salivary acidosis. The lipid storage diseases include, but are not limited to, acid lipase deficiencies such as Wolman's disease and cholesterol ester deposition disease. The post-translational modification defective disorders include, but are not limited to, multiple sulfated lipase deficiencies, mucolipid accumulation disease II α/β (I-cell disease), mucolipid accumulation disease II alpha/beta (pseudo-Heller's syndrome), and mucolipid accumulation disease type III gamma (pseudo-Heller's syndrome variant). The intrinsic membrane protein deficiency disorders include, but are not limited to cystine hyperintensities, Danon's disease, myoclonic renal failure syndrome, salivary acid storage disorders such as ISSD, Salla's disease and moderately severe Salla's disease, Niemann-Pancake disease types C1 and C2, and mucolipid accumulation disease type IV. The neuronal waxy lipofuscinosis including but not limited to waxy lipofuscinosis type 1 (Haltia-Santavuori disease and INCL), neuronal waxy lipofuscinosis type 2 (Jansky-Bielschowsky disease), waxy lipofuscinosis type 3 (Batten-Spielmeyer-Sjogren disease), waxy lipofuscinosis type 4 ( Parry's disease and Kufs types A and B), waxy lipofuscinosis type 5 (Late Infantile Child Finnish Heterozygous), waxy lipofuscinosis type 6 (Lake-Cavanagh or Indian Heterozygous), waxy lipofuscinosis type 7 (Turkish Heterozygous), waxy lipofuscinosis type 8 (Northern Epilepsy, Epileptic Intelligent Disorder), waxy lipofuscinosis 9, waxy lipofuscinosis 10, waxy lipofuscinosis 11, waxy lipofuscinosis 12, waxy lipofuscinosis 13, waxy lipofuscinosis 14. The lysosome-associated organelle disorders include but are not limited to Hermansky-Pudlak disease type 1, Hermansky-Pudlak disease type 2, Hermansky-Pudlak disease type 3, Hermansky-Pudlak disease type 4, Hermansky-Pudlak disease type 5, Hermansky-Pudlak's disease type 6, Hermansky-Pudlak's disease type 7, Hermansky-Pudlak's disease type 8, Hermansky-Pudlak's disease type 9, Griscelli syndrome 1 (Elejalde's syndrome), Griscelli syndrome 2, Chédiak -Higashi disease.

### Drug administration and medicinal use

As used herein, the term "pharmaceutically acceptable" refers to, within the scope of sound medical judgment, those compounds, materials, compositions, and/or dosage forms suitable for use in contact with human and animal tissues without undue toxicity, irritation, allergic reaction, or other problems or complications, with a proper return/risk ratio. In some embodiments, pharmaceutically acceptable compounds, materials, compositions, and/or dosage forms refer to those approved by a regulatory agency (such as the United States Food and Drug Administration, the China Food and Drug Administration, or the European Medicines Agency), or those listed in generally recognized pharmacopoeia (such as the United States Pharmacopoeia, the Chinese Pharmacopoeia, or the European Pharmacopoeia) for use in animals (more particularly, in humans).

As used herein, the term "subject" may include human and non-human animals. The non-human animals include all vertebrates such as mammals and non-mammals. The "subject" may be a livestock (such as cow, pig, sheep, chicken, rabbit, or horse), or a rodent (such as rat or mouse), or a primate (such as gorilla or monkey), or a domesticated animal (such as dog or cat). The "subject" may be male or female, and may also be of different ages. The human "subject" may be Caucasian, African, Asian, Semite, or other races, or a hybrid of different races. The human "subject" may be an elderly, adult, adolescent, child, or infant.

In some embodiments, the subject described herein is a human or a non-human primate.

The deuterated phenylarsine oxide disclosed herein may be administered by routes of administration known in the art, such as injection (including subcutaneous injection, intraperitoneal injection, intravenous injection (drip or infusion), intramuscular injection, or intradermal injection), or non-injection (including oral administration, nasal administration, sublingual administration, rectal administration, or topical administration). In some embodiments, the deuterated phenylarsine oxide described herein is administered orally, subcutaneously, intramuscularly, or intravenously. In some embodiments, the deuterated phenylarsine oxide described herein is administered orally.

As used herein, the term "therapeutically effective amount" refers to an amount of a drug that can alleviate or eliminate a disease or symptom in a subject, or preventatively inhibit or prevent the occurrence of a disease or symptom. The therapeutically effective amount may be an amount of a drug that alleviates one or more diseases or symptoms of a subject to a certain extent; may be an amount of a drug that partially or fully restores one or more physiological or biochemical parameters associated with the cause of a disease or symptom to normal; and/or may be an amount of a drug that reduces the likelihood of a disease or symptom. In some embodiments, the term "therapeutically effective amount" as used herein refers to an amount of a drug that alleviates or eliminates intracellular protein misfolding related disease or lysosomal storage disease in a subject.

The therapeutically effective amount of deuterated phenylarsine oxide provided herein depends on various factors known in the art, such as weight, age, past medical history, current received treatment, the health status of the subject and strength, allergies, hypersensitivity, and side effects of the interaction between drugs, route of administration, and extent of disease progression. A person skilled in the art (such as a physician or veterinarian) may correspondingly reduce or increase the dose according to these or other conditions or requirements.

In some embodiments, the treatment further comprises administering a second agent to the subject in need thereof.

In some embodiments, the second agent is an agent for treating intracellular protein misfolding related diseases, including but not limited to levodopa, riluzole.

In some embodiments, the fluorinated or deuterated phenylarsine oxide is administered before, after, or concurrently with the second agent.

### Example 1. Synthesis of halogenated compounds and their physical properties 1. Synthesis and physicochemical properties of halogenated PAO compounds 1.1 Preparation of 3,4,5-trifluoro-PAO (abbreviated as F3PAO)

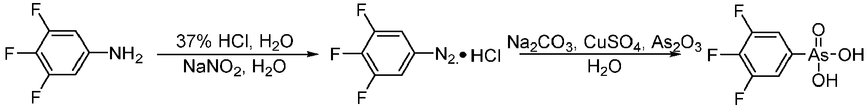

3,4,5-trifluoroaniline (18 mmol, 1.0 eq.) was added to a 100 mL round-bottomed flask with a magnetic stirrer, and 5 mL of water was added, and cooled down to 0 - 5°C. At this temperature, 37% hydrochloric acid (4.1 mL, 41.5 mmol) was slowly added to the reaction solution, diluted by 10 mL of water, and the reaction was carried out with continued stirring for 30 minutes. Then NaNO₂ aqueous solution (1.3 g, 18.9 mmol, 10 mL H₂O) was slowly added to the reaction solution, and the reaction temperature was controlled at 0 - 5°C, and the addition of the NaNO₂ aqueous solution was completed within 30-40 minutes. The solution turned yellow, and the reaction was continued with stirring at a low temperature for 2 h.

Na₂CO₃ (7.25 g, 68.4 mmol, 3.8 eq.), As₂O₃ (3.6 g, 18.0 mmol, 1.0 eq.), CuSO₄·5H₂O (0.27 g, 1.08 mmol, 0.06 eq.), and H₂O (25 mL) were added to a 100 mL round-bottomed reaction flask, and the reaction was carried out at 95°C for 45 min. The solution was a light blue suspension and cooled down to 0 - 5 °C.

The azo hydrochloride prepared in step 1 was added slowly to the reaction solution in step 2, and the temperature of the reaction system was controlled to be lower than 5°C, foam was generated in the process of addition, a small amount of acetone was added to remove the foam, and the addition was continued after the foam disappeared. The addition was completed within 1 hour, and the temperature was naturally increased after completion of the addition and the reaction was stirred overnight.

The reaction solution was filtered through diatomaceous earth, and the filter cake was rinsed with ice water (8 mL × 2). The aqueous phase was concentrated under reduced pressure to 15 mL at 50 °C. The pH was adjusted to 8 by adding 2.8 mL of 6N HCl dropwise in an ice-water bath, a small amount of yellowish-brown solid appeared, filtered, rinsed with 2 mL of ice water, and the solid was discarded. The filtrate was adjusted to pH 7 by adding 8 mL of 6N HCl dropwise and a white solid appeared, filtered, rinsed with 2 mL of ice water, and the solid was collected. The collected filtrate was concentrated to 11 mL, the pH was adjusted to 3 by adding 1.5 mL of 6N HCl, a large amount of solid appeared, filtered, and the solid was collected. The pH of the mother liquor was adjusted to 1 by continuously adding 3 mL of 6N HCl dropwise. Gray-white solid appeared in the system, filtered and the solid 3,4,5-trifluorophenylarsinic acid was collected. The solid contained product and inorganic salts that were not purified and were used directly in the next step of the reaction.

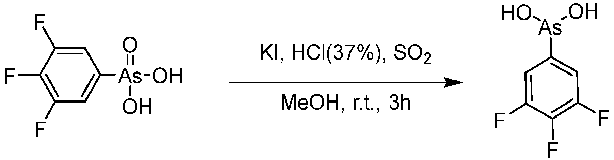

3,4,5-trifluorophenylarsonic acid (4.9 mmol, 1.0 eq.), KI (18.7 mg, 0.11 mmol, 0.023 eq.), 37% HCl (1.8 mL, 22.5 mmol, 4.6 eq.), MeOH (7 mL) were added sequentially to a 25 mL three-necked flask and stirred at room temperature for 5 - 10 min. The reaction was continuously filled with SO₂ for 3 hours and monitored for completeness by TLC.

The reaction solution was filtered and rinsed with MeOH (2 mL × 2). 7 mL of 17% NaOH solution was added dropwise to the liquid in an ice-water bath, and the pH was adjusted to 7, and a yellow oil appeared on the wall of the bottle. Extracted with ethyl acetate (25 mL × 2), the organic phases were combined and dried with anhydrous NaSO₄, filtered, and spun-dried at low temperature (20-28 °C). The solids were loaded dry on a silica gel packed column for 7 cm and passed through the column with EA as the eluent to give about 500 mg of liquid. 500 mg of liquid was taken in a 25 mL round-bottomed flask and added with about 1 mL of ether and stirred vigorously with a spatula to obtain 350 mg of white solid 3,4,5-trifluoro PAO. ¹H NMR (δ, DMSO-d6): 7.47 (t, 2H), 7.02 (s, 2H), MS ES- (m/z): 239.0 [(M-H)-].

### 1.2 Preparation of 3,4-difluoro-PAO (abbreviated as F2oPAO)

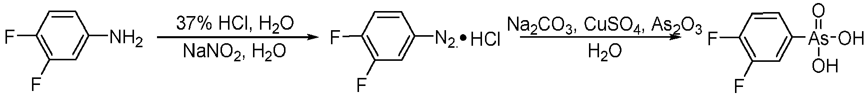

3,4-difluoroaniline (18 mmol, 1.0 eq.) was added to a 100 mL round-bottomed flask with a magnetic stirrer, and 5 mL of water was added and cooled down to 0 - 5°C. At this temperature, 37% hydrochloric acid (4.1 mL, 41.5 mmol) was slowly added to the reaction solution, diluted by 10 mL of water, and the reaction was carried out with continued stirring for 30 minutes. Then NaNO₂ aqueous solution (1.3 g, 18.9 mmol, 10 mL H₂O) was slowly added to the reaction solution, and the reaction temperature was controlled at 0 - 5°C, and the addition of the NaNO₂ aqueous solution was completed within 30-40 minutes. The solution turned yellow, and the reaction was continued with stirring at a low temperature for 2 h.

Na₂CO₃ (7.25 g, 68.4 mmol, 3.8 eq.), As₂O₃ (3.6 g, 18.0 mmol, 1.0 eq.), CuSO₄·5H₂O (0.27 g, 1.08 mmol, 0.06 eq.), and H₂O (25 mL) were added to a 100 mL round-bottomed reaction flask, and the reaction was carried out at 95°C for 45 min. The solution was a blue-white suspension and cooled down to 0 - 5 °C.

The azo hydrochloride prepared in step 1 was added slowly to the reaction solution in step 2, and the temperature of the reaction system was controlled to be lower than 5°C, foam was generated in the process of addition, a small amount of acetone was added to remove the foam, and the addition was continued after the foam disappeared. The addition was completed within 1 hour, and the temperature was naturally increased after completion of the addition and the reaction was stirred overnight.

The reaction solution was filtered through diatomaceous earth, and the filter cake was rinsed with ice water (5 mL × 2). The aqueous phase was concentrated under reduced pressure to 15 mL at 50 °C. The pH was adjusted to 8 by adding 1.4 mL of 6N HCl dropwise in an ice-water bath, a small amount of yellow-brown solid appeared, filtered, rinsed with 2 mL of ice water, and the solid was discarded. The filtrate was adjusted to pH 7 by adding 3 mL of 6N HCl dropwise and a white solid appeared, filtered, rinsed with 2 mL of ice water, and the solid was collected. The collected filtrate was concentrated to 11 mL, the pH was adjusted to 3 by adding 3 mL of 6N HCl, a large amount of solid appeared, filtered, and the solid was collected. The pH of the mother liquor was adjusted to 1 by adding 1 mL of 6N HCl dropwise. White solid appeared in the system, filtered. The mother liquor was rinsed 2-3 times, and the solid was collected to obtain 5.06 g of 3,4-difluorophenylarsinic acid. The solid contained product and inorganic salts that were not purified and were used directly in the next step of the reaction.

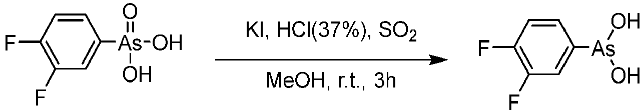

3,4-difluorophenylarsonic acid (6.4 mmol, 1.0 eq.), KI (24.9 mg, 0.15 mmol, 0.023 eq.), 37% HCl (2 mL, 29.5 mmol, 4.6 eq.), MeOH (8.6 mL) were added sequentially to a 25 mL three-necked flask and stirred at room temperature for 5 - 10 min. The reaction was continuously filled with SO₂ for 3 hours and monitored by TLC.

The reaction solution was filtered and rinsed with MeOH (2 mL × 2). The liquid was added in an ice-water bath with 8.5 mL of 17% NaOH solution, and the pH was adjusted to 7, and a yellow liquid appeared on the wall of the bottle. Extracted with ethyl acetate (30 mL × 2), the organic phases were combined and dried with anhydrous NaSO₄, filtered, and spun-dried at low temperature (20-28 °C). The solids were purified with column chromatography to give about 200 mg of liquid. 200 mg of liquid was taken in a 25 mL round-bottomed flask and added with about 1 mL of ether and stirred to obtain 200 mg of white solid 3,4-difluoro PAO. ¹H NMR (δ, DMSO-d6): 7.47 (t, 1H), 7.45-7.43 (m, 2H), 6.89 (s, 2H). MS ES⁻ (m/z): 221.0 [(M-H)⁻].

### 1.3 Preparation of 3,5-difluoro-PAO (abbreviated as F2mPAO)

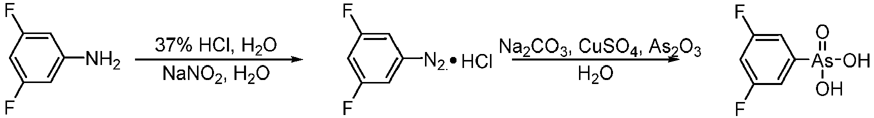

3,5-difluoroaniline (18 mmol, 1.0 eq.) was added to a 100 mL round-bottomed flask with a magnetic stirrer, and 5 mL of water was added and cooled down to 0 - 5°C. At this temperature, 37% hydrochloric acid (4.1 mL, 41.5 mmol) was slowly added to the reaction solution, diluted by 10 mL of water, 4.8 mL of acetone was added and the reaction was carried out with continued stirring for 30 minutes. Then NaNO₂ aqueous solution (1.3 g, 18.9 mmol, 10 mL H₂O) was slowly added to the reaction solution, and the reaction temperature was controlled at 0 - 5°C, and the addition of the NaNO₂ aqueous solution was completed within 30-40 minutes. 5 mL of ice water was supplementally added. The solution turned yellow-white, and the reaction was continued with stirring at a low temperature for 2 h.

Na₂CO₃ (7.25 g, 68.4 mmol, 3.8 eq.), As₂O₃ (3.6 g, 18.0 mmol, 1.0 eq.), CuSO₄·5H₂O (0.27 g, 1.08 mmol, 0.06 eq.), and H₂O (25 mL) were added to a 100 mL round-bottomed reaction flask, and the reaction was carried out at 95°C for 45 min. The solution was a blue-white suspension and cooled down to 0 - 5 °C.

The azo hydrochloride prepared in step 1 was added slowly to the reaction solution in step 2, and the temperature of the reaction system was controlled to be lower than 5°C, foam was generated during the process of addition, and the addition was continued after the foam disappeared. The addition was completed within 1 hour, and the temperature was naturally increased after the addition was completed, and the reaction was stirred overnight.

The reaction solution was filtered through diatomaceous earth, and the filter cake was rinsed with ice water (5 mL × 2). The aqueous phase was concentrated under reduced pressure to 25 mL at 50 °C. The pH was adjusted to 7-8 by adding 7.6 mL of 6N HCl in an ice-water bath, gray solid appeared, filtered, rinsed with 2 mL of ice water, and the solid was discarded. The obtained filtrate was concentrated to 11 mL and yellow-white solid appeared in the system. The system was adjusted to pH 3 by adding 3 mL of 6N HCl, filtered, and the obtained solid was rinsed with 3 mL of ice water to give 4.4 g of solid 3,5-difluorophenylarsinic acid. The solid contained product and inorganic salts that were used directly in the next step of the reaction.

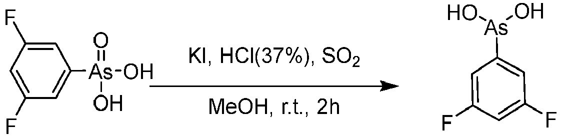

3,5-difluorophenylarsonic acid (5.2 mmol, 1.0 eq.), KI (20 mg, 0.12 mmol, 0.023 eq.), 37% HCl (2 mL, 24 mmol, 4.6 eq.), MeOH (7 mL) were added sequentially to a 25 mL three-necked flask and stirred at room temperature for 5 - 10 min. The reaction was continuously filled with SO₂ for 2 h. The solution changes from orange-yellow to yellow-white. The reaction was carried out for 2 hours and monitored for completeness by TLC.

The reaction solution was filtered and rinsed with MeOH (2 mL × 2). The liquid was added dropwise in an ice-water bath with 6.7 mL of 17% NaOH solution, and the pH was adjusted to 7, and a yellow oily liquid appeared on the wall of the bottle. Extracted with ethyl acetate (25 mL × 2), the organic phases were combined and dried with anhydrous Na₂SO₄, filtered, and spun-dried at low temperature (20-28 °C). 400 mg of the solid was recrystallized with 4 mL of ethanol at 50-60 °C to obtain 240 mg of solid 3,5-difluoro PAO, yield: about 20.8%. ¹H NMR (δ, DMSO-d6): 7.28-7.18 (m, 3H), 6.98 (s, 2H). MS ES⁻ (m/z): 221.0 [(M-H)⁻].

### 1.4 Preparation of 2,3,4-trifluoro-PAO

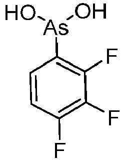

2,3,4-trifluoro-PAO was prepared using a method similar to that of 3,4,5-trifluoro-PAO, ¹H NMR (δ, DMSO-d6): 7.52-7.46 (m, 1H), 7.38-7.32 (m, 1H), 7.07 (s, 2H).

### Example 2. Synthesis of deuterated compounds and PAO and their physical properties

### 1. Synthesis of d5PAO (pentadeuterophenylarsine oxide) and its physical properties

### 1.2 Synthesis of d5PAO

The synthesis route of d5PAO is as follows:

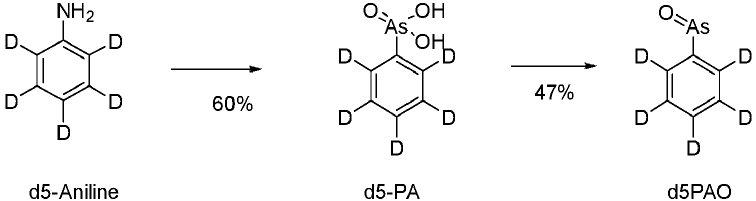

Step 1: Synthesis method of d5-PA:

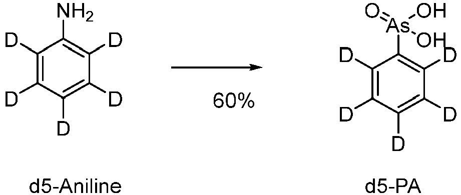

91.35 mL of water, 18.27 g of d5-Aniline was added to a three-necked flask, stirred, cooled down to 0°C-10°C, and 37.45 mL of concentrated hydrochloric acid was added dropwise. After hydrochloric acid was added, sodium nitrite aqueous solution (13.43 g solid sodium nitrite dissolved in 36.5 mL of water) was added dropwise, and the temperature was kept at no more than 5°C; the addition was completed, and the temperature was kept for about 2-3 hours. The preparation of diazonium salt was completed.

274 mL of purified water, 69.06 g of sodium carbonate, 36.82 g of arsenic trioxide, and 2.83 g of copper sulfate pentahydrate (CuSO4·5H2O) were added to another vessel, heated to 90-100°C and stirred at such temperature for 30 minutes, and then cooled down to 5-15°C. The diazonium salt prepared above was added slowly in batches, and the temperature was controlled to be lower than 15°C. The mixture was stirred for 2-3 hours, then naturally heated to room temperature and stirred overnight. The mixture was filtered and the filter cake was washed with water. The filtrates were combined and concentrated hydrochloric acid was added slowly to adjust the pH of the system to 3.0, and a small amount of brown flocculent was precipitated. The mixture was filtered, the filtrate was washed three times with 100 mL of ethyl acetate, and the aqueous phase was concentrated under reduced pressure at 50-60°C to about 170 mL, precipitating a large amount of white solid. The mixture was filtered, the filter cake was rinsed with cold water, drained, and the solid was directly baked in a blast oven at 50 °C for 18 h. 35 g off-white solid, d5-PA, were obtained, yield: 90.83 %, MS ES+ (m/z): 208.0 [(M+H)⁺].

Step 2: Synthesis method of d5PAO (or d5-PAO):

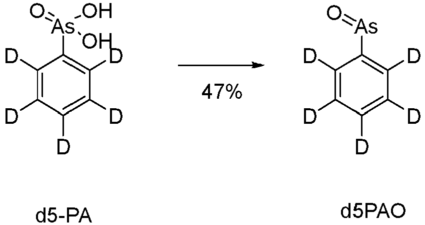

400 mL of purified water, 25 g of d5-PA, 75.4 g of sodium bisulfite, 0.32 g of potassium iodide and 125 mL of methanol were added to a three-necked flask and stirred; the reaction was carried out at 30-40°C overnight, and the reaction was stopped when the raw material was reacted substantially completely. The temperature of the solution was controlled below 30°C, and the pH was adjusted to 7.0 with concentrated hydrochloric acid. The solution was extracted with ethyl acetate for 4 times, and the organic phases were combined and extracted, concentrated to dryness at 35-45°C to give 20 g of white solid wet product. The solid was refluxed with 240 mL of tert-butyl methyl ether at elevated temperature for 1 h, cooled down, filtered and the filter cake was washed with cold MTBE. The filter cake was dried overnight at 50 °C in a blast oven to give 8.7 g white solid d5PAO, ¹³C-NMR (δ, DMSO-d6): 149.9, 129.6, 129.4, 128.2, MS ES+ (m/z): 173.99 [(M+H)⁺].

### 1.3 Physicochemical properties of d5PAO

### 1.2.1 Instrument

Agilent 1260 Prime HPLC, Mettler Toledo XS105 balance (0.01 mg), KQ5200B ultrasonic instrument (Kunshan Ultrasonic Instrument Co., Ltd.), BR2000-GM variable speed oscillator (VWR International), 0.45 µm filter membrane (Shanghai Qingyang Biotechnology Co., Ltd.)

### 1.2.2 Experimental drug

d5PAO (97.9% purity), acetonitrile was chromatographically pure (Sinopharm Chemical Reagent Co., Ltd.), hydrochloric acid, DMSO were analytically pure (Sinopharm Chemical Reagent Co., Ltd.).

### 1.2.3 Solution preparation

2 mL of 0.1 M hydrochloric acid solution was taken and diluted to 20 mL by adding deionized water to obtain a 0.01 M hydrochloric acid solution. The pH of the solution was determined to be 2 by using accurate pH test paper.

2 mL of 0.01 M hydrochloric acid solution was taken and diluted to 20 mL by adding deionized water to obtain 0.001 M hydrochloric acid solution.

2 mL of 0.001 M hydrochloric acid solution was taken and diluted to 20 mL by adding deionized water to obtain 0.0001 M hydrochloric acid solution. The pH of the solution was determined to be 4 using accurate pH test paper.

20 mL of deionized water was taken and the pH was determined to be 6.

7.5 mg of d5PAO was taken into a centrifuge tube, 1 mL of DMSO was added, sonicated and dissolved. The solution was then diluted to 10 mL with acetonitrile/water (1/1) mixture to obtain 0.75 mg/mL of d5PAO stock solution. The d5PAO stock solution was diluted into different d5PAO working solutions at 0.3 mg/mL, 0.15 mg/mL, 0.075 mg/mL, 0.03 mg/mL, and 0.015 mg/mL.

### 1.2.4 Chromatographic conditions

Liquid Chromatograph: Agilent 1260 Infinity II Prime Ultra High Performance Liquid Chromatography System.

Column: ACQUITY UPLC^{®} Peptide C18 130Å 2.1100 mm ID., 1.7 µm (Waters).

Mobile phase A: Water: ACN (v: v, 95: 5) solution containing 0.01% AA and 2 mmol/L NH₄OAc.

Mobile phase B: Water: ACN (v: v, 5: 95) solution containing 0.01% AA and 2 mmol/L NH₄OAc.

Elution gradient:

| Time (min) | flow rate (mL/min) | A (%) | B (%) |
|---|---|---|---|
| Initial | 0.400 | 95 | 5 |
| 2.00 | 0.400 | 2 | 98 |
| 3.00 | 0.400 | 2 | 98 |
| 3.20 | 0.400 | 95 | 5 |
| 4.00 | 0.400 | 95 | 5 |

Column temperature: 40 °C
Injection volume: 4 µL
Detection wavelength: 254 nm

### 1.2.5 Methodological Examination

### Examination of linear relationships

Different d5PAO solutions at 0.75 mg/mL, 0.3 mg/mL, 0.15 mg/mL, 0.075 mg/mL, 0.03 mg/mL, 0.015 mg/mL were taken, injected and measured according to the above chromatographic conditions, and the chromatograms were recorded. Linear regression was performed on the sample concentration by peak area to obtain the regression equation: $y = 1647.7 x + 0.9623 , {R}^{2} = 0.9999 .$

The results showed good linear relationship between the sample concentration of d5PAO and the peak area in the range of 0.015-0.75 mg*mL-1. 1.2.6 Determination of equilibrium solubility

2 mL of different pH buffers were aspirated and placed in 3 mL centrifuge tubes, and an excessive amount of d5PAO powder was added until a large amount of white insoluble precipitate appeared in the solution, ultrasonicated for 30 min, then placed in a thermostatic oscillator and shaken for 24 h at 25°C, then ultrasonicated for 30 min, and filtered through a 0.45 µm filter membrane, and the filtrate was aspirated and diluted 10 times with water, and then injected and determined according to the above chromatographic conditions. The chromatogram was recorded, and the solubility of d5PAO in different pH buffers was calculated to be 5.36 mg/mL, 5.39 mg/mL and 5.98 mg/mL, respectively.

**Table 1. The solubility of d5PAO**

| Drug | pH | Peak Area | Corresponding solubility (mg/mL) |
|---|---|---|---|
| d5PAO | 6 | 884.20 | 5.36 |
| d5PAO | 4 | 889.51 | 5.39 |
| d5PAO | 2 | 986.70 | 5.98 |

### 2. Synthesis and physical properties of PAO

### 2.1 Synthesis of PAO

### Synthesis route:

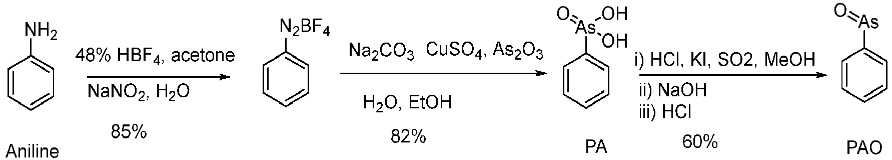

### Step 1:

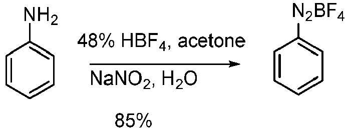

Aniline (10.0 g, 107 mmol, 1.0 eq.) and acetone (20 mL) were added to a 250 mL single-necked flask. The solution was cooled to about -15 °C (high temperatures result in a darker colored product) and 48% HBF4 (30 mL, 29.5 g, 161 mmol, 1.5 eq.) was added slowly. NaNO₂ (11.0 g, 161 mmol, 1.5 eq.) was dissolved in H₂O (20 mL) and slowly added dropwise to the above solution. After addition, the reaction was held at -15 °C for 2 h, then stirred at 0 °C for about 1 h. The solid (white) was filtered and washed with isopropyl ether (50 mL × 2). The product was vacuum dried (temperature 30°C), weight: 17.5 g, yield: 85%, pink solid, and directly used in the next step of the reaction.

### Step two:

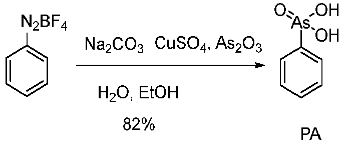

Na₂CO₃ (21.2 g, 200.6 mmol, 3.85 eq.), As₂O₃ (11.3 g, 57.3 mmol, 1.1 eq.), C_{U}SO₄-5H₂O (800 mg, 3.13 mol, 0.06 eq.), and H₂O (60 mL) were added to a 250 mL flask, and the suspension was heated to about 90°C and held for about 20 minutes to dissolve most of the solids. Cooled to about 0°C -15°C, the suspension of azobenzofluoroborate (10.0 g, 52.1 mmol, 1.0 eq.) and H₂O (60 mL) was added slowly in batches, and a small amount of acetone was added to reduce foam buildup. After addition, the mixture was stirred for about 12 h at room temperature. Diatomaceous earth was spread, and the mixture was filtered, and washed with H₂O (20 mL × 3), and the filtrate was decolorized by adding activated carbon if the color was too dark and filtered. Concentrated hydrochloric acid (12 N, 40 mL) was added to the filtrate to neutralize it, and the reaction solution was further adjusted to acidic. The filtrate was concentrated to a mixture volume of about 50 mL. The solid was filtered and washed once with cold water; the filtrate was concentrated and the resulting solid was filtered and washed once with cold water. The solids were combined and dried to give a white solid, weight: 8.6 g, yield: 82%. MS ES⁺ (m/z): 202.9 [(M+H)⁺].

### Step 3:

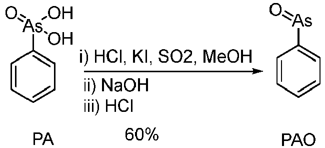

A mixture of phenylarsine acid (8.0 g, 40 mmol, 1.0 eq.), methanol (40 mL), concentrated hydrochloric acid (15 mL), and a catalytic amount of KI (50 mg) was filled with SO₂ until saturation, and stirred for 2 hours at room temperature. NaOH solution (2N) was added to the mixture until the solution was transparent. Concentrated hydrochloric acid was added to neutralize it. A solid was precipitated and filtered to obtain a white solid. The solid was recrystallized with water/ethanol (1/5), dried. weight: 4.0 g, yield: 60%. Product is white powdery solid.¹H NMR (δ, CDCl₃): 7.41-7.62 (m, 3H), 7.75-7.78 (m, 2H). MS ES⁺ (m/z): 168.8 [(M+H)⁺].

### 2.2 Physicochemical properties of PAO

The molecular formula of PAO is C₆H₅AsO and the molecular weight is 168.03.

### 2.2.1 Instrument

Agilent 1260 Prime HPLC, Mettler Toledo XS105 balance (0.01 mg), KQ5200B ultrasonic instrument (Kunshan Ultrasonic Instrument Co., Ltd.), BR2000-GM variable speed oscillator (VWR International), 0.45 µm filter membrane (Shanghai Qingyang Biotechnology Co., Ltd.)

### 2.2.2 Experimental drug

PAO (98% purity), acetonitrile was chromatographically pure (Sinopharm Chemical Reagent Co., Ltd.), hydrochloric acid, DMSO were analytically pure (Sinopharm Chemical Reagent Co., Ltd.).

### 2.2.3 Solution preparation

2 mL of 0.1 M hydrochloric acid solution was taken and diluted to 20 mL by adding deionized water to obtain a 0.01 M hydrochloric acid solution. The pH of the solution was determined to be 2 by using accurate pH test paper.

2 mL of 0.01 M hydrochloric acid solution was taken and diluted to 20 mL by adding deionized water to obtain 0.001 M hydrochloric acid solution.

2 mL of 0.001 M hydrochloric acid solution was taken and diluted to 20 mL by adding deionized water to obtain 0.0001 M hydrochloric acid solution. The pH of the solution was determined to be 4 using accurate pH test paper.

20 mL of deionized water was taken and the pH was determined to be 6.

7.5 mg of PAO was taken into a centrifuge tube, 1 mL of DMSO was added, sonicated and dissolved. The solution was then diluted to 10 mL with acetonitrile/water (1/1) mixture to obtain 0.75 mg/mL of PAO stock solution. The PAO stock solution was diluted into different PAO working solutions at 0.3 mg/mL, 0.15 mg/mL, 0.075 mg/mL, 0.03 mg/mL, and 0.015 mg/mL.

### 2.2.4 Chromatographic conditions

Liquid Chromatograph: Agilent 1260 Infinity II Prime Ultra High Performance Liquid Chromatography System.

Column: ACQUITY UPLC^{®} Peptide C18 130Å 2.1100 mm ID., 1.7 µm (Waters).

Mobile phase A: Water: ACN (v: v, 95: 5) solution containing 0.01% AA and 2 mmol/L NH₄OAc.

Mobile phase B: Water: ACN (v: v, 5: 95) solution containing 0.01% AA and 2 mmol/L NH₄OAc.

Elution gradient:

| Time (min) | flow rate (mL/min) | A (%) | B (%) |
|---|---|---|---|
| Initial | 0.400 | 95 | 5 |
| 2.00 | 0.400 | 2 | 98 |
| 3.00 | 0.400 | 2 | 98 |
| 3.20 | 0.400 | 95 | 5 |
| 4.00 | 0.400 | 95 | 5 |

| | | | |
|---|---|---|---|
| Column temperature: 40 °C Injection volume: 4 µL Detection wavelength: 254 nm | | | |

### 2.2.5 Methodological Examination

### Examination of linear relationships

Different PAO solutions at 0.75 mg/mL, 0.3 mg/mL, 0.15 mg/mL, 0.075 mg/mL, 0.03 mg/mL, 0.015 mg/mL were taken, injected and measured according to the above chromatographic conditions, and the chromatograms were recorded. Linear regression was performed on the sample concentration by peak area to obtain the regression equation: $y = 1834.8 x - 4.2355 , {R}^{2} = 1$

The results showed the good linear relationship between the sample concentration of PAO and the peak area in the range of 0.015-0.75 mg*mL-1.

### 2.2.6 Determination of equilibrium solubility

2 mL of different pH buffers were aspirated and placed in 3 mL centrifuge tubes, and an excessive amount of PAO powder was added until a large amount of white insoluble precipitate appeared in the solution, ultrasonicated for 30 min, then placed in a thermostatic oscillator and shaken for 24 h at 25°C, then ultrasonicated for 30 min, and filtered through a 0.45 µm filter membrane, and the filtrate was aspirated and diluted 10 times with water, and then injected and determined according to the above chromatographic conditions. The chromatogram was recorded, and the solubility of PAO in different pH buffers was calculated to be 1.15 mg/mL, 3.38 mg/mL and 4.52 mg/mL, respectively.

**Table 2. The solubility of PAO**

| Drug | pH | Peak Area | Corresponding solubility (mg/mL) |
|---|---|---|---|
| PAO | 6 | 207.25 | 1.15 |
| PAO | 4 | 616.40 | 3.38 |
| PAO | 2 | 824.35 | 4.52 |

### 3. Synthesis and physicochemical properties of deuterated compounds d1PAO, d2PAO and d3PAO

### 3.1 Preparation of d1PAO

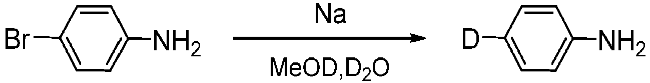

Under the condition of nitrogen, p-bromoaniline (3.44 g) was dissolved in deuterated methanol (5 mL), refluxed for 30 minutes, spun dry, repeated 3 times, spun dry and drained for use. 3 g of sodium metal was added to the deuterated methanol (10 mL) in batches, and when the reaction was completed, heavy water (30 mL) was added slowly to produce a 10% heavy water solution of sodium deuteride oxide. Under the condition of nitrogen, the product of the first step was dissolved in deuterated methanol (5 mL), added with zinc powder (6.5 g) and the obtained 10% heavy water solution of sodium deuteride oxide, heated and refluxed for 3 hours, monitored the disappearance of p-bromoaniline on the spot plate, cooled to room temperature, extracted with ether, and spun-dried at low temperature, to give the product p-deuteraniline.

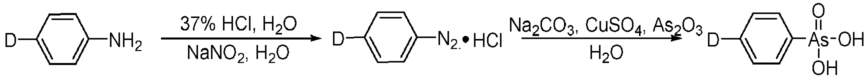

p-deuteraniline (0.94 g) obtained from the step above was added to a round-bottomed flask with a magnetic stirrer and 5 mL of water was added, and cooled to 0-5°C. At this temperature, 37% hydrochloric acid solution (2 mL) was slowly added, diluted by 5 mL of water, and the reaction was carried out with continued stirring for 30 minutes. Then NaNO₂ aqueous solution (724.5 mg, 10.5 mmol, 3 mL H₂O) was slowly added to the reaction solution, and the reaction temperature was controlled at 0 - 5°C, and the addition was completed within 30-40 minutes. The solution turned brown-yellow, and the reaction was continued with stirring at a low temperature for 2 h. Na₂CO₃ (4.0 g, 38.0 mmol, 3.8 eq.), As₂O₃ (1.0 g, 5.0 mmol, 0.5 eq.), CuSO₄·5H₂O (150 mg, 0.6 mmol, 0.06 eq.), and H₂O (13 mL) were added to another round-bottomed reaction flask, and the reaction was carried out at 95°C for 45 min. The solution was green and cooled down to 0 - 5 °C. The azo hydrochloride prepared in step 1 was added slowly to the reaction solution in step 2, and the temperature of the reaction system was controlled to be lower than 5°C, foam was generated in the process of addition, a small amount of acetone was added to remove the foam, and the addition was continued after the foam disappeared. The addition was completed within 1 hour, and the temperature was naturally increased after completion of the addition and the reaction was stirred overnight. The reaction solution was filtered through diatomaceous earth, and the filter cake was rinsed with ice water (2 mL × 2). The aqueous phase was concentrated under reduced pressure to 10 mL at 50 °C. The pH was adjusted to 7-8 by adding 4 mL of 6N HCl dropwise in an ice-water bath, a small amount of yellow-brown solid appeared, filtered, rinsed with 2 mL of ice water, and the solid was discarded. The filtrate was adjusted to pH 2-3 by adding 2.5 mL of 6N HCl, a large amount of bubble was generated. The filtrate was concentrated under reduced pressure, cooled and filtered when a large amount of solid appeared in the system, and the solids were collected. The resulting filtrate was adjusted to pH 1 by adding 6N HCl, rotary evaporated, and then cooled down and filtered when a large amount of solid appeared in the system. The mother liquor was washed twice and the solid was collected. A total of 900 mg of pure solid p-deuterophenylarsinic acid was obtained, yield: 44 %.1H NMR (δ, DMSO-d6): 7.60 (d, 2H), 7.53 (d, 2H).

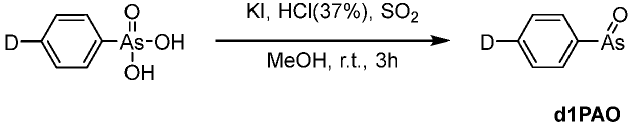

p-deuterophenylarsinic acid (880mg, 4.3 mmol, 1.0 eq.), KI (16.6 mg, 0.1 mmol, 0.023 eq.), 37% HCl (1.7 mL, 20.0 mmol, 4.6 eq.), MeOH (5.8 mL) were added sequentially to a 25 mL three-necked flask and stirred at room temperature for 5 - 10 min. The reaction was continuously filled with SO₂ for 3 h, and monitored for completeness by TLC. The reaction solution was filtered and rinsed with MeOH (1.5 mL × 2). The liquid was added dropwise in an ice-water bath with 15% NaOH solution, and the pH was adjusted to 14, and the system is orange suspension. Extracted with ethyl acetate (50 mL × 2), the organic phases were combined and dried with anhydrous Na₂SO₄, filtered, and spun-dried at low temperature (20-28 °C), and crude product was obtained. The crude product was pulped with about 4 mL of ether for about 30 min and then filtered and drained to obtain about 430 mg of d1PAO as an off-white solid, yield: about 59%. ¹H NMR (δ, DMSO-d6): 7.70 (d, 2H), 7.46 (d, 2H), MS ES⁺ (m/z): 169.9 [(M+H)⁺].

### 3.2 Preparation of d2PAO

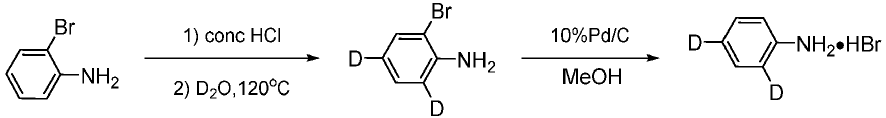

Concentrated hydrochloric acid (5 mL) was added dropwise to an ether solution of o-bromoaniline (3.44 g), a solid was produced, filtered through a sand-core funnel, washed with ether, and drained to obtain the hydrochloride of aniline. The solid was dissolved in heavy water (7 mL) in a closed tube under nitrogen, heated to 120°C, reacted for 24 h, the heavy water was spun-dried, replaced with nitrogen, reintroduced with heavy water (7 mL), and the reaction was carried out for 48 h. The reaction was cooled to room temperature, the pH was adjusted to pH = 7 using 30% sodium hydroxide, extracted with ether, dried over anhydrous sodium sulfate, and spun-dried to give 2-bromo-4,6-dideuterio-aniline. Under nitrogen, 22 mL of methanol was added to a mixture of 2-bromo-4,6-dideuterium-aniline and 10% Pd/C (300 mg), the nitrogen was replaced with hydrogen and the reaction was carried out for 3 h. The system was monitored by spot plate until the disappearance of 2-bromo-4,6-dideuterium-aniline, filtered through diatomaceous earth, washed with a small amount of methanol, and spun-dried directly to give the hydrobromide salt of 2,4-dideuterium-aniline.

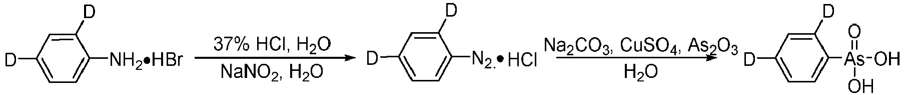

The hydrobromide salt of 2,4-dideuterium-aniline obtained from the step above was added to a 25mL round-bottomed flask with a magnetic stirrer and 5 mL of water was added, and cooled to 0-5 °C. At this temperature, 37% hydrochloric acid (1.1 mL) was slowly added, diluted by 5 mL of water, and the reaction was carried out with continued stirring for 30 minutes. Then NaNO₂ aqueous solution (724.5 mg, 10.5 mmol, 3 mL H₂O) was slowly added to the reaction solution, and the reaction temperature was controlled at 0 - 5°C, and the addition was completed within 30-40 minutes. The solution turned brown-yellow, and the reaction was continued with stirring at a low temperature for 2 h. Na₂CO₃ (4.0 g, 38.0 mmol, 3.8 eq.), As₂O₃ (1.0 g, 5.0 mmol, 0.5 eq.), CuSO₄·5H₂O (150 mg, 0.6 mmol, 0.06 eq.), and H₂O (13 mL) were added to a 50mL round-bottomed reaction flask, and the reaction was carried out at 95°C for 45 min. The solution was green and cooled down to 0 - 5 °C. The azo hydrochloride prepared in step 1 was added slowly to the reaction solution in step 2, and the temperature of the reaction system was controlled to be lower than 5°C, foam was generated in the process of addition, a small amount of acetone was added to remove the foam, and the addition was continued after the foam disappeared. The addition was completed within 1 hour, and the temperature was naturally increased after completion of the addition and the reaction was stirred overnight. The reaction solution was filtered through diatomaceous earth, and the filter cake was rinsed with ice water (2 mL × 2). The aqueous phase was concentrated under reduced pressure to 10 mL at 50 °C. The pH was adjusted to 7-8 by adding 4 mL of 6N HCl dropwise in an ice-water bath, a small amount of yellow-brown solid appeared, filtered, rinsed with 2 mL of ice water, and the solid was discarded. The filtrate was adjusted to pH 3-4 by adding 2 mL of 6N HCl, viscous solid appeared. The system was filtered and the solid was discarded (NMR showed that this solid contained no product). The resulting filtrate was concentrated to 8 mL, the pH was adjusted to 2-3 by the addition of 6 N HCl (0.5 mL), a large amount of solid appeared, and the system was filtered to obtain 1.15 g of 2,4-dideuterium-benzenearsonic acid solid, yield: 56.4%. ¹H NMR (δ, DMSO-d6): 7.72 (d, 1H), 7.56-7.59 (m, 2H).

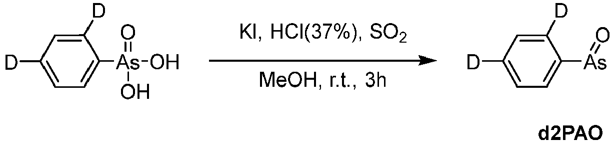

2,4-dideuterium-benzenearsonic acid (1.1g, 5.4 mmol, 1.0 eq.), KI (20.6 mg, 0.124 mmol, 0.023 eq.), 37% HCl (2.1 mL, 25.0 mmol, 4.6 eq.), MeOH (7.3 mL) were added sequentially to a 25 mL three-necked flask and stirred at room temperature for 5 - 10 min. The reaction was continuously filled with SO₂ for 3 h and monitored for completeness by TLC. The pH was adjusted to 7 by dropwise addition of a 15% NaOH solution in an ice-water bath, and a large amount of insoluble material appeared in the system. The system was extracted with ethyl acetate (50 mL × 2), the organic phases were combined and dried with anhydrous Na₂SO₄, filtered, and spun-dried at low temperature (20-28 °C) to obtain the crude product. About 3 mL of ethyl acetate was added to the crude product and pulped for about 30 min and then filtered and drained to obtain an off-white solid d2PAO (about 400 mg), yield: about 48%. ¹H NMR (δ, DMSO-d6): 7.58 (d, 1H), 7.36-7.39 (m, 2H). MS ES+ (m/z): 170.7 [(M+H)+].

### 3.3 Preparation of d2PAO

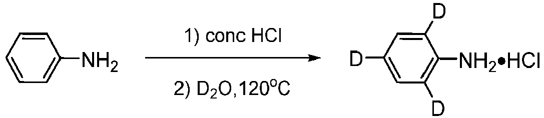

Concentrated hydrochloric acid (5 mL) was added dropwise to aniline solution (2.65 g aniline, 7 mL heavy water), a solid was produced, filtered through a sand-core funnel, washed with ether, and drained to obtain the hydrochloride of aniline, which was used directly in the next step. The solid was dissolved in heavy water (7 mL) in a closed tube under nitrogen, heated to 120°C, and reacted for 24 h. The heavy water was spun-dried, the nitrogen was replaced, and heavy water (7 mL) was reintroduced and reacted for 48 h. The resulting heavy aqueous solution of 2,4,6-trideuterium-aniline was used directly in the next step.

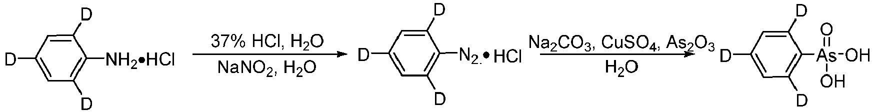

The heavy aqueous solution of 2,4,6-trideuterium-aniline (4.33 mL) was added to a 25 mL round-bottomed flask with a magnetic stirrer and 2.5 mL of water was added, and cooled to 0-5 °C. At this temperature, 37% hydrochloric acid solution (1.1 mL) was slowly added, diluted by 5 mL of water, and the reaction was carried out with continued stirring for 30 minutes. Then NaNO₂ aqueous solution (724.5 mg, 10.5 mmol, 3 mL H₂O) was slowly added to the reaction solution, and the reaction temperature was controlled at 0 - 5°C, and the addition was completed within 30-40 minutes. The solution changed from purple to yellow, and the reaction was continued with stirring at a low temperature for 2 h. Na₂CO₃ (4.0 g, 38.0 mmol, 3.8 eq.), As₂O₃ (1.0 g, 5.0 mmol, 0.5 eq.), CuSO₄·5H₂O (150 mg, 0.6 mmol, 0.06 eq.), and H₂O (13 mL) were added to a 50 mL round-bottomed reaction flask, and the reaction was carried out at 95°C for 45 min. The solution was green and cooled down to 0 - 5 °C. The azo hydrochloride prepared in step 1 was added slowly to the reaction solution in step 2, and the temperature of the reaction system was controlled to be lower than 5°C, foam was generated in the process of addition, a small amount of acetone was added to remove the foam, and the addition was continued after the foam disappeared. The addition was completed within 1 hour, and the temperature was naturally increased after completion of the addition and the reaction was stirred overnight. The reaction solution was filtered through diatomaceous earth, and the filter cake was rinsed with ice water (2 mL × 2). The aqueous phase was concentrated under reduced pressure to 10 mL at 50 °C. The pH was adjusted to 7-8 by adding 1.8 mL of 6N HCl dropwise in an ice-water bath, a small amount of yellow-brown solid appeared, filtered, rinsed with 2 mL of ice water, and the solid was discarded. The filtrate was adjusted to pH 3 by adding 1.8 mL of 6N HCl, light yellow solid was appeared. The system was filtered and washed with 2 mL of ice water and the solid was collected. The obtained filtrate was concentrated to 8 mL, pH was adjusted to 1 by adding 6 N HCl (0.8 mL), a large amount of solid appeared, filtered and the solid was collected. A total of 860 mg of solid 2,4,6-trideuterium-phenylarsonic acid was obtained. yield: 39 %. ¹H NMR (δ, DMSO-d6): 7.56 (s, 2H).

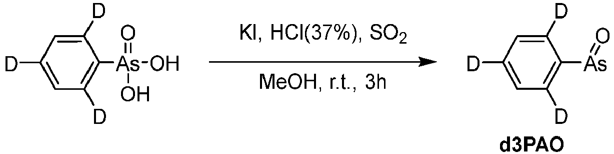

2,4,6-trideuterium-phenylarsonic acid (3.9 mmol, 1.0 eq.), KI (15 mg, 0.09 mmol, 0.023 eq.), 37% HCl (1.3 mL, 18.0 mmol, 4.6 eq.), MeOH (5.3 mL) were added sequentially to a 25 mL three-necked flask and stirred at room temperature for 5 - 10 min. The reaction was continuously filled with SO₂ for 3 h, and monitored for completeness by TLC. The reaction solution was filtered, and rinsed with methanol (1.5 mL × 2). The pH was adjusted to 7 by dropwise addition of 4.3 mL of 17% NaOH solution in an ice-water bath, and a yellow oil appears on the wall of the bottle. The system was extracted with ethyl acetate (25 mL × 2 ), the organic phases were combined and dried with anhydrous Na₂SO₄, filtered, and spun-dried at low temperature (20-28 °C). About 3 mL of ethyl acetate was added to the obtained solid and pulsed for about 30 minutes, then filtered and drained to obtain an off-white solid d3PAO (about 200 mg). The mother liquor obtained in the previous step was spun-dried, and the obtained solid was added with about 1.5 mL Et₂O and pulsed for about 30 minutes, then filtered and drained to obtain an off-white solid d3PAO (about 170 mg). d3PAO was combined to give a yield of about 55%. ¹H NMR (δ, DMSO-d6): 7.46 (s, 2H). MS ES⁺ (m/z): 171.9 [(M+H)⁺].

### Example 3. Pharmacokinetic studies of compounds

F2mPAO was prepared by the method described in Example 1 and d5PAO (d5-PAO) and PAO (PAO) were prepared by the method described in Example 2. After a single intravenous injection of PAO, d5PAO and F2mPAO (administered at a dose of 0.1 mg/Kg, the compounds were dissolved in 0.1% DMSO) or a single oral gavage of a mixture of the above reagents (administered at a dose of 0.2 mg/Kg) in a test adult male rat, venous blood was drawn at 0, 0.083, 0.25, 0.5, 1, 2, 4, 6, 8, 24, 32, and 48 hours respectively after adminstration, for pharmacokinetic testing. PAO and d5PAO in the test samples were extracted by protein precipitation, and the treated samples were injected into liquid-mass spectrometry (LC-MS/MS) and detected by ESI negative ion mode after liquid phase separation.

### Sample processing (blood samples)

1) 40 µL of Unknown, Calibration Standard, Quality Control, Single Blank, and Double Blank samples are added to a 96-well plate;
2) 120 µL of 0.1 mg/mL sodium dimercaptopropanesulfonate dissolved in water is added to each sample;
3) 40 µL of 0.2% formic acid dissolved in water was added to each sample. The samples were mixed well and incubated at 45 °C for 15 minutes with shaking and centrifuged at 4 °C for 5 minutes (×3220 g);
4) Each sample (except double blanks) was quenched with 200 µL of IS1 (double blank samples were quenched with 240 µL of MeOH), shaken and mixed for 15 minutes and then centrifuged at 4°C for 15 minutes (×3220 g);
5) 50 µL of supernatant was transferred to a 96-well plate and centrifuged at 4 °C for 5 min (×3220 g), then the supernatant was used for LC-MS/MS analysis. The analysis was performed by a Triple Quad 6500⁺ LC-MS/MS (SCIEX) system.

### Results:

Male SD rats (n=3) were administered with the same dose of PAO, d5PAO and F2mPAO by single intravenous injection (0.1 mg/Kg) or single oral gavage (0.2 mg/Kg), and there were no significant differences in the pharmacokinetic parameters of PAO and d5PAO. However, F2mPAO was significantly better than PAO and d5PAO in terms of bioavailability, maximal blood concentration, half-life, and systemic exposure (see Table 3, Table 4 and Figures 1 and 2).

**Table 4. Pharmacokinetic indices of single oral gavage (0.2 mg/Kg) in male SD rats**

| **PK Parameters** | **PAO (0.11mg/kg, IV)** | | | **F2mPAO (0.08mg/kg, IV)** | | | **d5PAO (0.09mg/kg, IV)** | | |
|---|---|---|---|---|---|---|---|---|---|
| | **Mean** | SD | CV (%) | **Mean** | SD | CV (%) | **Mean** | SD | CV (%) |
| **No. points used for T_{1/2}** | **3** | -- | -- | **3** | -- | -- | **3.00** | -- | -- |
| **C₀ (ng/mL)** | **1345** | 211 | 15.7 | **1943** | 375 | 19.3 | **1488** | 142 | 9.53 |
| **T_{1/2} (h)** | **11.4** | 0.300 | 2.63 | **17.0** | 2.858 | 16.81 | **12.4** | 0.651 | 5.23 |
| **Vdₛₛ (L/kg)** | **0.163** | 0.0161 | 9.88 | **0.155** | 0.01044 | 6.74 | **0.155** | 0.0219 | 14.1 |
| **CL (mL/min/kg)** | **0.411** | 0.0256 | 6.22 | **0.239** | 0.0046 | 1.92 | **0.379** | 0.0230 | 6.07 |
| **Tₗₐₛₜ (h)** | **48.0** | -- | -- | **48.0** | -- | -- | **48.0** | -- | -- |
| **AUC₀₋ₗₐₛₜ (ng.h/mL)** | **4010** | 253 | 6.30 | **6636** | 58 | 0.88 | **4337** | 281 | 6.47 |
| **AUC_{0-inf} (ng.h/mL)** | **4062** | 258 | 6.35 | **6976** | 145 | 2.08 | **4404** | 276 | 6.26 |
| **MRT₀₋ₗₐₛₜ (h)** | **5.84** | 0.436 | 7.46 | **7.62** | 0.628 | 8.25 | **5.87** | 0.488 | 8.32 |
| **MRT_{0-inf} (h)** | **6.58** | 0.445 | 6.76 | **10.81** | 0.878 | 8.12 | **6.79** | 0.648 | 9.54 |
| **AUC_{Extra} (%)** | **1.27** | 0.0513 | 4.05 | **4.86** | 1.1433 | 23.51 | **1.54** | 0.272 | 17.7 |
| **AUMC_{Extra} (%)** | **12.5** | 0.666 | 5.34 | **32.7** | 7.481 | 22.90 | **14.9** | 1.457 | 9.80 |

| **PK Parameters** | **PAO (0.22mg/kg, PO)** | | | **F2mPAO (0.17mg/kg, PO)** | | | **d5PAO (0.19mg/kg, PO)** | | |
|---|---|---|---|---|---|---|---|---|---|
| | **Mean** | SD | CV (%) | **Mean** | SD | CV (%) | **Mean** | SD | CV (%) |
| **No. points used for T_{1/2}** | **3** | -- | -- | **3** | -- | -- | **3** | -- | -- |
| **Cₘₐₓ (ng/mL)** | **156** | 12.5 | 8.03 | **356** | 53.1 | 14.9 | **161** | 10.4 | 6.48 |
| **Tₘₐₓ (h)** | **8.00** | 0.00 | 0.00 | **8.00** | 0.00 | 0.00 | **7.33** | 1.15 | 15.7 |
| **T_{1/2} (h)** | **6.87** | 0.942 | 13.7 | **8.99** | 1.34 | 15.0 | **7.19** | 0.778 | 10.8 |
| **Tₗₐₛₜ (h)** | **48.0** | -- | -- | **48.0** | -- | -- | **48.0** | -- | -- |
| **AUC₀₋ₗₐₛₜ (ng.h/mL)** | **2314** | 420 | 18.1 | **6194** | 774 | 12.5 | **2445** | 475 | 19.4 |
| **AUC_{0-inf} (ng.h/mL)** | **2341** | 426 | 18.2 | **6406** | 746 | 11.7 | **2479** | 483 | 19.5 |
| **MRT₀₋ₗₐₛₜ (h)** | **12.5** | 0.611 | 4.88 | **14.8** | 1.31 | 8.86 | **12.6** | 0.777 | 6.15 |
| **MRT_{0-inf} (h)** | **13.0** | 0.751 | 5.76 | **16.3** | 1.68 | 10.3 | **13.3** | 0.902 | 6.80 |
| **AUC_{Extra} (%)** | **1.16** | 0.467 | 40.4 | **3.37** | 1.31 | 38.8 | **1.35** | 0.444 | 32.8 |
| **AUMC_{Extra} (%)** | **5.09** | 1.99 | 39.0 | **12.5** | 4.49 | 36.0 | **5.92** | 1.80 | 30.3 |
| **Bioavailability (%)^{a}** | **28.8** | -- | -- | **45.9** | -- | -- | **28.1** | -- | -- |

### Example 4. Study of the inhibitory effect of fluorinated compounds on pulmonary fibrosis

Pulmonary fibrosis (PF) is a chronic, fibrotic lung disease that can be caused by a variety of reasons, mainly manifested by dry cough and progressive dyspnea, with a poor prognosis and still difficult to cure. The main pathological feature of PF is the excessive scarring after the destruction of the normal lung structure, which ultimately leads to respiratory insufficiency. Although research on the pathophysiological mechanisms of pulmonary fibrosis has made great progress, its pathogenesis has not been fully elucidated.

Human embryonic lung fibroblast MRC-5 cells are important tool cells for studying the pathological changes of PF and conducting drug development. During the development of PF, related transcription factors such as transforming growth factor-1 (TGF-β1) can regulate the abnormal activation, proliferation, and migration of fibroblasts, causing abnormal deposition of extracellular matrix and destruction of alveolar structure, which ultimately leads to the formation of PF. TGF-β1 is one of the key factors in the induction of PF, which can regulate the transformation of fibroblasts into myofibroblasts by binding to the corresponding receptor, therefore, a certain concentration of TGF-β1 is usually used to treat MRC-5 cells to induce fibrosis in the experimental process.

### 1. Materials

MRC-5 cells were purchased from the Innovation Center of Excellence in Molecular Cell Science, Chinese Academy of Sciences. Human recombinant TGF-β1 protein was purchased from Proteintech Group (Catalog No. #HZ-1011). Rabbit anti-Calponin1 primary antibody was purchased from Cell Signaling Technology (Catalog No. #17819). Rabbit anti-α-Smooth Muscle Actin primary antibody was purchased from Cell Signaling Technology (Catalog No. #19245). Alexa Flour 555 goat anti-rabbit IgG was purchased from Molecular Probes. High-sugar DMEM medium, MEM medium and fetal bovine serum FBS were purchased from Gibco. Olympus FV1000 inverted fluorescence microscope was purchased from Olympus (Tokyo, Japan). Dako Cytomation fluorescent mounting medium was purchased from Dako (Copenhagen, Denmark).

### 2. Methods

### 2.1 MRC-5 cell culture and processing

MRC-5 cells were cultured in DMEM medium containing 10% fetal bovine serum (FBS) at a constant temperature of 37°C, 5% CO₂, and saturated humidity in an incubator for 24 hours to allow for adherence to the wall according to the Cell Culture Guidelines. On the following day, DMEM medium (without FBS) containing 5 ng/mL TGF-β1 and different concentrations of F2oPAO, F2mPAO, or F3PAO according to the grouping was added, and the incubation was continued for 24 h according to the experimental needs. The groupings were as follows: control (ctrl) group, 5 ng/mL TGF-β1 group, 5 ng/mL TGF-β1 and 12.5 nM F2oPAO co-treated group (5 ng/mL TGF-β1+12.5 nM F2oPAO group), 5 ng/mL TGF-β1 and 25 nM F2oPAO co-treated group (5 ng/mL TGF-β1+ 25 nM F2oPAO group), 5 ng/mL TGF-β1 and 50 nM F2oPAO co-treatment group (5 ng/mL TGF-β1+50 nM F2oPAO group), 5 ng/mL TGF-β1 and 12.5 nM F2mPAO co-treated group (5 ng/mL TGF-β1+12.5 nM F2mPAO group), 5 ng/mL TGF-β1 and 25 nM F2mPAO co-treated group (5 ng/mL TGF-β1+25 nM F2mPAO group), 5 ng/mL TGF-β1 and 50 nM F2mPAO co-treated group (5 ng/mL TGF-β1+50 nM F2mPAO group), 5 ng/mL TGF-β1 and 12.5 nM F3PAO co-treated group (5 ng/mL TGF-β1+12.5 nM F3PAO group), 5 ng/mL TGF-β1 and 25 nM F3PAO co-treated group (5 ng/mL TGF-β1+25 nM F3PAO group), 5 ng/mL TGF-β1 and 50 nM F3PAO co-treated group (5 ng/mL TGF-β1+50 nM F3PAO group).

### 2.2 Thiazolyl Blue (MTT) Assay for Cell Viability

Cells were treated with drugs for 24 hours, MTT at a final concentration of 0.5 mg/mL was added, and after 4 hours of incubation, the incubation solution was aspirated, 100 µL of DMSO was added to dissolve the adsorbed MTT, and the absorbance value was read after 15 minutes of shaking.

### 2.3 Immunofluorescence staining

MRC-5 cells were treated for 24 hours and the supernatant was aspirated, cells were washed 3 times with phosphate buffer saline (PBS); 4% Paraformaldehyde (PFA) was added and allowed to stand for 30 minutes at room temperature; 4% PFA was discarded and washed 3 times with PBS for 10 minutes each time; 0.3% non-ionic detergent Triton-X (dissolved in PBS) was added, and stand at room temperature for 30 minutes; 0.3% Triton-X was removed, PBS blocking solution containing 10% goat serum (GS) was added and left to stand at room temperature for 1 hour; the primary antibody was incubated overnight at 4°C (the primary antibody was diluted with 10% GS blocking solution, α-Smooth Muscle Actin dilution ratio 1:200, Calponin1 dilution ratio 1:100). On the following day, the cells were washed 3 times with PBS for 10 min each time; the secondary antibody Alexa Flour 555 goat anti-rabbit IgG (diluted with the blocking solution at a dilution ratio of 1:500 and added with 1 µg/mL 4',6-diamidino-2-phenylindole (DAPI)) was incubated and left to stand at room temperature for 2 h. The cells were washed with PBS 3 times for 10 min each time. The slices were sealed using mounting medium and then observed by microscope. The immunofluorescence staining of bone marrow MSCs was processed as the same as above.

### 2.4 Statistics

Fluorescence intensity was processed using Image J software, data processing and statistics were performed using GraphPad Prism5 software, and data were expressed as mean ± standard error (mean ± SEM). Differences between groups were compared using one-way ANOVA, and p<0.03 was considered statistically different.

### 3. Results

### 3.1 Inhibition of the expression of Calponin1 and α-Smooth Muscle Actin by a series of fluorinated compounds in TGF-β1-treated MRC-5 Cells

MRC-5 cells were treated with DMEM (without FBS) medium containing 5 ng/mL TGF-β1 for 24 h and were treated with different concentrations of a series of fluorinated compounds according to groupings. After MRC-5 cells were treated with 5 ng/mL TGF-β1 for 24 h, different concentrations of F2mPAO, F2oPAO or F3PAO were added according to groupings, and the cell viability was detected by MTT assay after incubation for 4 or 24 hours. The results showed that after 4 hours of treatment with the a series of fluorinated compounds, the cell viability of 5 ng/mL TGF-β1-treated group decreased significantly compared with that of the control group (Figure 3A), and other groups had no significant change in the cell viability compared with the 5 ng/mL TGF-β1-treated group; after 24 hours of treatment with the a series of fluorinated compounds, the cell viability of 5 ng/mL TGF-β1-treated group decreased significantly compared with that of the control group (Figure 3B); both the 5 ng/mL TGF-β1 and 100 nM F2mPAO co-treated group and the 5 ng/mL TGF-β1 and 100 nM F3PAO co-treated group resulted in a significant decrease in cell viability compared to the 5 ng/mL TGF-β1-treated group (Figure 3B).

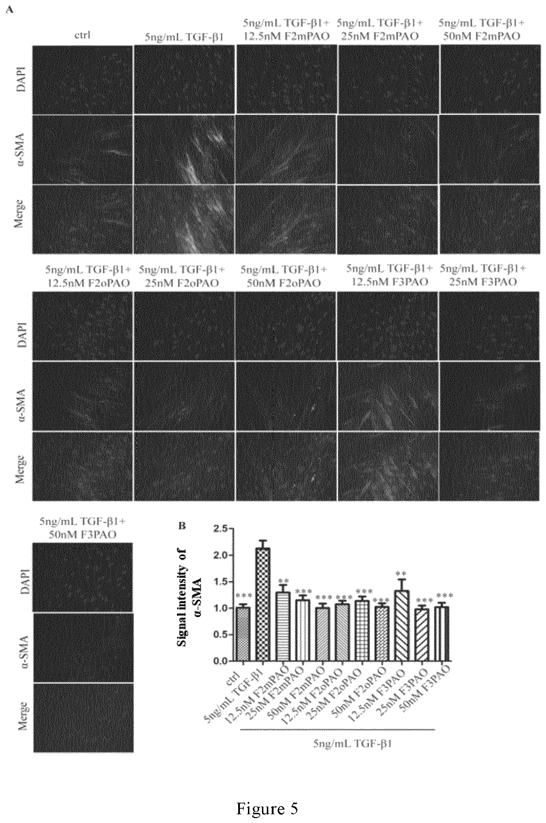

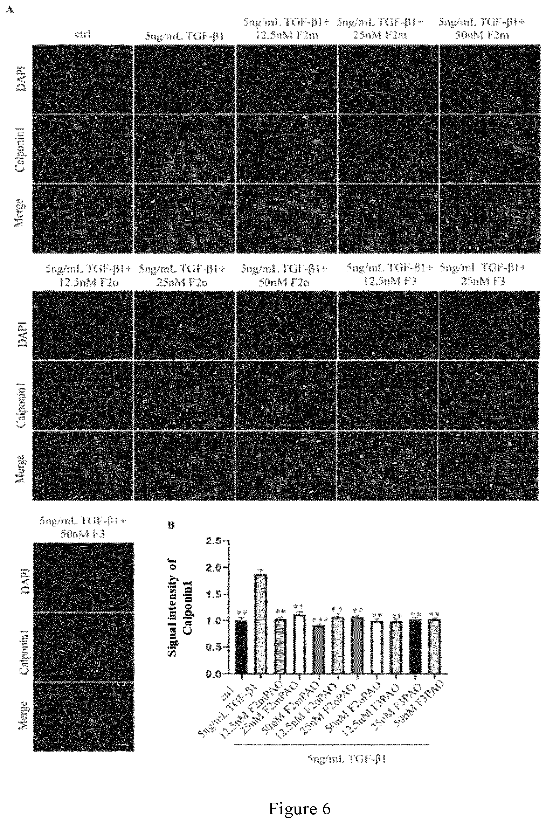

α-Smooth Muscle Actin (α-SMA) and actin-binding protein Calponin1 are markers of myofibroblasts, and in order to investigate whether the series of fluorinated compounds had a regulatory effect on lung fibrosis, α-SMA and Calponin1 were detected after 24 h of treatment with the series of fluorinated compounds, respectively. The results of protein immunoblotting experiments showed that: α-SMA expression was significantly increased in the 5 ng/mL TGF-β1-treated group compared with the control (ctrl) group, suggesting that the treatment of MRC-5 cells with 5 ng/mL TGF-β1 for 24 h resulted in cellular fibrosis, and that 50 nM F2mPAO, 25 nM F2oPAO, as well as 12.5 nM, 25 nM and 50 nM F3PAO significantly inhibited 5 ng/mL TGF-β1-induced α-SMA overexpression (Figure 4A, C). The results of protein immunoblotting experiments for Calponin1 were similar to those of α-SMA described above, and Calponin1 expression was significantly increased in the 5 ng/mL TGF-β1-treated group compared with the ctrl group, and certain concentrations of F2mPAO, F2oPAO or F3PAO and 5 ng/mL TGF-β1 co-treated groups showed a significant decrease in Calponin1 expression compared with the 5 ng/mL TGF-β1 alone treated group (Figures 4A, D). The study showed that Collagen Type I (COL1) was one of the important components of the extracellular matrix and was involved in the occurrence and development of lung fibrous processes. Similarly, it was also investigated whether the series of fluorinated compounds had a regulatory effect on the expression of COL1 in MRC-5 cells. The results showed that the expression of Collagen Type I was consistent with the results of α-SMA and Calponin1 described above (Figures 4A, B). In addition, the immunofluorescence results were also consistent with the protein immunoblotting results, and α-SMA expression was significantly higher in the 5 ng/mL TGF-β1-treated group compared with the control group (Figures 5A, B). 5 ng/mL TGF-β1 and 25 nM F2mPAO, 5 ng/mL TGF-β1 and 50 nM F2mPAO, 5 ng/mL TGF-β1 and 25 nM F2oPAO, 5 ng/mL TGF-β1 and 50 nM F2oPAO, 5 ng/mL TGF-β1 and 25 nM F3PAO, and 5 ng/mL TGF-β1 and 50 nM F3PAO co-treated groups had no significant difference in α-SMA fluorescence signal intensity compared with the control group, and had significant decrease compared with the 5 ng/mL TGF-β1-treated group (Figures 5A, B). The immunofluorescence results of the actin-binding protein Calponin1 are consistent with the above results of α-SMA (Figures 6A, B). The above results indicated that a certain concentration of F2mPAO, F2oPAO and F3PAO could significantly inhibit the TGF-β1-induced fibrotic process in MRC-5 cells.

### 3.2 Knockdown (or PI4KA) inhibits MRC-5 cell fibrosis

The series of fluorinated compounds are novel compounds modified based on their parent compounds, while the previous study showed that the representative compound of its parent compound, PAO, mainly acts on phosphatidylinositol 4-kinase PI4KIIIα at low concentration (<5 µM), and in order to further investigate the role of the corresponding target of action, PI4KIIIα, in the process of fibrosis, a shRNA interference lentiviral vector (with green fluorescent protein GFP expression sequence) against a gene sequence PI4KA encoding the PI4KIIIα protein was designed. The results of protein immunoblotting experiments showed that after the three shRNA interference lentiviral vectors against PI4KA transfected MRC-5 cells for 48 hours, the interference sequences sh1, sh2, and sh3 significantly reduced the expression level of PI4KIIIα in the treatment group (Figures 7A, B). The expression of Calponin1 and α-SMA was observed by immunofluorescence after treatment with shRNA interference lentiviral vectors for 48 hours. The expression level of α-SMA was significantly higher in the sh-ctrl and 5 ng/mL TGF-β1 co-treated group compared with the vector control (sh-ctrl) group. Compared with the sh-ctrl and 5 ng/mL TGF-β1 co-treated group, the α-SMA expression level was significantly reduced in the sh1 and 5 ng/mL TGF-β1 co-treated group and sh3 and 5 ng/mL TGF-β1 co-treated group, and there was a trend of decreasing in the α-SMA expression level in the sh2 and 5 ng/mL TGF-β1 co-treated group (Figures 8A, C). The observed results of Calponin1 were consistent with those of α-SMA (Figures 8B, D), both indicating that knockdown of PI4KA inhibited MRC-5 cell fibrosis.

### Example 5. Role of Fluorinated Derivatives in a TGF-β-Induced Fibroblast Model of Heart, Liver, and Kidney

### 1. Normal human ventricular cardiac fibroblasts (NHCF-V)

NHCF-V cells (Normal human ventricular cardiac fibroblasts, at a density of 5000 cells/well, in 2% FBS complete medium, 200 µL/well) were seeded in 96-well culture plates (Corning), incubated at 37°C/5% CO₂ for 24 hours. On day 1, cells were starved with 0.5% FBS-FGM for 24 hours. On day 2, drugs were added to the cell culture medium as: blank, Nintedanib (positive control), F2mPAO, F2oPAO, F3PAO, AsIII (2-mercaptopropyl-PAO) and A1 (PI4KA inhibitor, Naveen Bojjireddy et al. THE JOURNAL OF BIOLOGICAL CHEMISTRY VOL. 289, NO. 9, pp. 6120-6132, February 28, 2014*).* All treatment group culture mediums contained TGF-β (as a fibrosis inducer with a final concentration of 1 ng/mL). Incubation was continued at 37°C/5% CO₂. On day 3, after treatment with TGF-β for 24 hours, cells were washed with cold DPBS (Mg²⁺/Ca²⁺-free). Lysis was performed by adding 50 µL lysis solution (0.5 µL DNase I +49.5 µL lysis lysate) and incubating for 5 min at room temperature on a horizontal shaker (900 rpm) with shaking. After adding 5 µL of termination solution, incubation was continued for 2 min. 4 µL of sample lysate was added to 16 µL of reverse transcription master mix, mixed well, and reverse transcription was run on the PCR instrument. The reverse transcription products would be used for subsequent RT-qPCR. PCR master mixes were prepared for the target genes (ACTA2, CTGF, PAI-1) as well as the internal reference control gene (RPLP0), respectively. 8 µL of PCR master mix and 2 µL of sample reverse transcription product were mixed well on a centrifugal incubator plate (1000 rpm, 2 min), and finally PCR reactions were performed on an Applied BiosystemsTM ViiA7 instrument. Each sample was replicated twice for testing. The analyzed data were derived from the Ct values generated from the RT-PCR experiments. The Ct values generated from the amplification of individual genes in each sample were normalized by the internal reference gene RPLP0. The same data processing method was used for the following experiments.

### Experimental Results

Addition of TGF-β to NHCF-V cell culture solution increased mRNA expression of genes encoding α-actinin (ACTA2), connective tissue growth factor (CTGF), and plasminogen activator inhibitor (PAI-1) (ranging from 130%-300%, data not shown), suggesting that TGF-β induced fibrosis in cultured cells. In contrast, the simultaneous addition of the transforming growth factor β type I receptor inhibitor Nintedanib to the solution (positive control) could reverse the mRNA expression of the above genes (see Table 5): the mRNA expression of the three genes in the Nintedanib-treated group was decreased by 67%, 61%, and 82%, respectively, compared with the TGF-β-treated group. Similarly, in the PAO derivatives F2mPAO, F2oPAO, F3PAO and ASIII treated groups, the mRNA expression levels of these genes were inhibited to varying degrees, suggesting that these compounds could effectively inhibit TGF-β-induced cellular fibrosis. In addition, PI4KA inhibitor A1 was also known to inhibit TGF-β-induced elevation of ACTA2 mRNA expression, but the inhibition to the expression of CTGF and PAI-1 mRNA suggested that its inhibitory effect was not as strong as that of PAO derivatives.

**Table 5. Effect of PAO derivatives on gene mRNA expression in TGF-β-treated cardiac fibroblasts**

| NHCF-V cell | Relative changes in mRNA levels (vs. TGF-β treatment) | | |
|---|---|---|---|
| Test item (100nM) | ACTA2 | CTGF | PAI-1 |
| Nintedanib | -67% | -61% | -82% |
| F2mPAO | -93% | -74% | -17% |
| F2oPAO | -69% | -51% | -35% |
| F3PAO | -94% | -72% | -9% |
| ASIII | -31% | 7% | -19% |
| A1 | -81% | 7% | 116% |

### 2. Human hepatic stellate (LX-2) cells

LX-2 cells (human hepatic stellate cell line, Sigma-Aldrich SCC064) were seeded in a 96-well plate at a density of 20,000 cells/well (2% FBS complete medium, 200 µL/well). On day 1, the cells were incubated at 37°C and 5% CO₂ for 24 hours. On day 2, drug treatments were performed by adding drugs to the cell culture medium: blank, Nintedanib (positive control), F2mPAO, F2oPAO, F3PAO, AsIII, and A1 (PI4KA inhibitor). 30 minutes later, TGF-β was added for fibrosis induction treatment, and the final concentration of TGF-β was 5 ng/mL. After 24 hours of TGF-β treatment, cells were washed with cold DPBS (Mg²⁺/Ca²⁺ free). Lysis was performed by adding 50 µL lysis solution (0.5 µL DNase I + 49.5 µL lysis lysate), and incubated for 5 min at room temperature on a shaker (900 rpm) with shaking. 5 µL of termination solution was added, and the incubation was continued for 2 min. 15 µL of sample lysate was added to 35 µL of reverse transcription master mix, mixed well, and then reverse transcription was run on a PCR instrument, and the resulting reverse transcription product was used for subsequent real-time quantitative PCR (RT-qPCR). PCR master mixes were prepared for the target genes (ACTA2, COL1A1, TGFB1) and the internal reference control gene (GAPDH), respectively. 8 µL of PCR master mix and 2 µL of sample reverse transcription product were mixed well by centrifugation (1000 rpm, 2 min) on a centrifuge plate, and finally the PCR reaction was performed on an Applied BiosystemsTM QuantStudioTM 7 Flex Real-Time PCR instrument. The analyzed data were derived from Ct values generated from RT-qPCR experiments.

### Experimental Results:

The addition of TGF-β to the cell culture solutions of LX-2 cells increased the expression of mRNA of genes encoding α-actinin (ACTA2), collagen type 1 α-1 chain (COL1A), and transforming growth factor type 1-β (TGFB1) (ranging from 300%-400%, data not shown), suggesting that TGF-β induced cultured stellate hepatocytes' fibrosis. Whereas the solution, if it also contained Nintedanib (400nM), reversed the mRNA expression of the above genes (see Table 6). In the F2mPAO, F2oPAO, and F3PAO treated groups, the mRNA expression levels of the above genes were inhibited to varying degrees, suggesting that these PAO derivatives could effectively inhibit TGF-β-induced cellular fibrosis, while ASIII showed no obvious inhibitory effect. A1, a PI4KA inhibitor, also inhibited the TGF-β-induced increase in mRNA expression of several A genes.

**Table 6. effect of PAO derivatives on gene mRNA expression in TGF-β-treated stellate hepatocytes**

| LX-2 cell | Relative changes in mRNA levels (vs. TGF-β treatment) | | |
|---|---|---|---|
| Test item | ACTA2 | COL1A1 | TGFB1 |
| Nintedanib (400nM) | -55% | -77% | -78% |
| F2mPAO (100nM) | -69% | -54% | -36% |
| F2oPAO (100nM) | 21% | -15% | 3% |
| F3PAO (100nM) | -65% | -54% | -29% |
| ASIII (100nM) | 48% | -3% | 7% |
| A1 (100nM) | -92% | -53% | -20% |

### 3. Human renal mesangial (HRMC) cells

HRMC cells (human renal mesangial cells, at a density of 6000 cells/well, in 2% FBS complete medium, 100 µL/well) were seeded in 96-well cell culture plates (Corning), and incubated at 37°C and 5% CO₂ for 24 hours. On day 1, the cells were starved with 0.1% FBS for 6 hours. On day 2, individual drugs to be tested (F2mPAO, F2oPAO, F3PAO, AsIII, and A1; containing TGF-β at a final concentration of 1 ng/mL in 0.1% FBS) were added to the cell culture medium. Incubation was continued at 37°C and 5% CO₂. On day 3, 24 hours after TGF-β treatment, cells were washed with cold DPBS (Mg²⁺/Ca²⁺-free). Lysis was performed by adding 30 µL of lysis solution (0.3 µL DNase I + 29.7 µL lysis lysate) and incubating with shaking at room temperature on a shaker (900 rpm) for 5 minutes. 3 µL of termination solution was added, and the incubation was continued for 2 min. 6 µL of sample lysate was added to 24 µL of reverse transcription master mix, mixed well, and then reverse transcription was run on a PCR instrument, and the resulting reverse transcription product was used for subsequent real-time quantitative PCR (RT-qPCR). PCR master mixes were prepared for the target genes (COL4A1, COL16A1 and CTGF) as well as the internal reference control gene (RPLP0), respectively. 8 µL of each PCR master mix and 2 µL of reverse transcription product of the lysed samples were mixed, the plates were centrifuged (1000 rpm, 2 min), and then the PCR reactions were performed on an Applied Biosystems ViiA7 instrument.

### Experimental Results:

The addition of TGF-β to HRMC cell cultures increased the mRNA expression of genes encoding collagen type 16 Alpha 1 chain (COL16A1), collagen type 4 Alpha 1 chain (COL4A1α-1), and connective tissue growth factor (CTGF) in the cells (ranging from 200%-300%, data not shown), suggesting that TGF-β might induce fibrosis in cultured renal mesangial cells. And the solution, if it also contained Nintedanib 100 nM, reversed the mRNA expression of the above genes (see Table 7). In treatment groups of F2mPAO, F2oPAO, and F3PAO at the same concentration, the mRNA expression levels of the above genes were inhibited to different degrees, and the inhibition was stronger than that of the positive control Nintedanib, which suggested that these PAO derivatives could effectively inhibit TGF-β-induced cellular fibrosis, but the inhibition of ASIII was relatively weak. The PI4KA inhibitor A1 only inhibited the TGF-β-induced elevation in mRNA expression of CTGF gene.

**Table 7. effect of PAO derivatives on gene mRNA expression in TGF-β treated liver fibroblasts.**

| HRMC cell | Relative changes in mRNA levels (vs. TGF-β treatment) | | |
|---|---|---|---|
| Test item (100nM) | COL16A1 | COL4A1 | CTGF |
| Nintedanib | -57% | -55% | -53% |
| F2mPAO | -63% | -55% | -81% |
| F2oPAO | -78% | -74% | -70% |
| F3PAO | -72% | -62% | -83% |
| ASIII | -28% | -7% | -20% |
| A1 | 2% | -2% | -66% |

### Example 6. Study of PAO and its derivatives in inhibiting cardiac fibrosis

### 1. Myocardial primary fibroblast cell extraction:

Myocardial primary fibroblast isolation and culture: C57BL/6J suckling rat myocardial primary fibroblasts were extracted using 0.2% collagenase type 2.

C57BL/6J suckling rats 0-3 days after birth (males, Shanghai Jihui) were sterilized in 75% ethanol. After isolating the suckling rat hearts, the skin and ribs were cut open and the suckling rat hearts were isolated, the isolated hearts were put into sterilized PBS and washed 3 times, the hearts were cut into fine tissue pieces with scissors, washed 3 times with PBS, and then digested with 0.025% trypsin (Gbico) at 4°C overnight.

The washed tissue pieces were re-digested using 0.2% collagenase type 2 at 37°C for 30 minutes; they were neutralized using DMEM (Gibco) medium containing 10% serum and centrifuged for 5 minutes. The supernatant was gently discarded and the precipitate was blown up with DMEM medium containing 10% serum and seeded in 25 cm² culture flasks (Corning). After 2 hours of adherence, the medium was changed. The cells were observed under microscope at 24, 48 and 72 hours for wall adherence and growth, and the 2nd-3rd generation of cells were taken for experiments.

### 2. Establishment of hypoxic conditions in cultured cardiac fibroblasts:

The experiments were divided into control group, 24 h anoxic group and 24 h anoxia + d5PAO or PAO intervention group. Myocardial primary fibroblasts were spread into cell culture dishes (Corning) in six-well plates, and when the cells were completely attached to the wall and fused to 70%-80%, the cells were starved with serum-free medium for 24 hours, then the serum-free medium was changed and d5PAO/PAO at various concentrations was added, and then put into anoxic box (Mitsubishi, Japan) for anoxia, and then the cells were stimulated for 24 hours with microdemanding AnaeroPack (AnaeroPack of Mitsubishi, Japan were used). When the anaerobic indicator changed from pink to purple and back to pink, it indicated successful anoxia, and the cells were collected for spare use.

### 3. Detection of α-SMA and Col 1A gene expression by fluorescence quantitative PCR:

Total RNA was extracted by RNA extraction kit (B0004DP, Suzhou Yingze Biomedical Co., Ltd.), and the concentration and purity of RNA were determined by spectrophotometer (Thermo Fisher). cDNA was obtained by reverse transcription reaction of 1000 ng of total RNA using a reverse transcription kit (Shanghai Yi Sheng). cDNA of α-SMA and Col 1A was amplified by fluorescence quantitative PCR using a SYBR Green kit (Shanghai Yi Sheng), and the GAPDH gene was used as an internal reference. The cDNA sequences of the above genes were downloaded from NCBI website, and the PCR primers were synthesized by Shanghai Sangong Biological Engineering Co. The primer sequences were as follows:
α-SMA: forward GTCCCAGACATCAGGGAGTAA, reverse
   TCGGATACTTCAGCGTCAGGA;
Col 1A: forward TAAGGGTCCCCAATGGTGAGA, reverse
   GGGTCCCTCGACTCCTACAT;
GAPDH: forward CCAGGCGCCCAATACG, reverse
   CCACATCGCTCAGACACCAT.

### 4. Detection of SMA and Col 1A protein expression by immunoblotting (Western Blotting) of cell extracts:

The medium was aspirated and the dishes were washed 2 times with PBS. An appropriate amount of RIPA lysis (Beyotime) containing protease inhibitor was added according to the total amount of cultured cells and lysed on ice for 30 minutes. During this time, the lysate was vortexed and shaken several times to ensure adequate protein lysis. The lysate was then centrifuged at 12,000g for 15 min at 4°C, and the supernatant was the total cellular protein obtained. The protein was quantified using BCA kit (Beyotime). SDSPAGE gel kit (Epizyme) gel was applied to electrophoresis of 30 µg of protein. Primary antibody was used as Col 1A antibody (Abeam), α-SMA (Abeam) antibody; secondary antibody was used as HRP-labeled antibody (Beyotime), and GAPDH, Tubulin were used as internal reference. Image-Pro Plus 6.0 software was applied to analyze the bands in gray scale.

### 5. Detection of the viability of cultured cardiac fibroblasts by CCK8 method:

The first generation of myocardiac fibroblasts were spread into 96-well plates (Corning), and when the cells were completely adhered to the wall and fused to 70%-80%, they were starved with serum-free DMEM medium for 24 hours, and then put into the anoxia box for anoxia, and cells were stimulated with microdemanding anoxia packages for 24 hours. When the anoxia indicator changed from pink to purple and then back to pink, it indicated that the anoxia was successful. 10 µL of CCK8 reagent (Beyotime) and 90 µL of DMEM medium were then added to each well, and the cells were incubated at 37°C for 2 hours, and then a microplate reader was applied to detect the absorbance (OD) value at 450 nm to evaluate the cell proliferation and cell viability. Five replicate wells were set up in each group.

### 6. Myocardial fibroblast migration ability test (scratch assay):

Myocardial primary fibroblasts were inoculated into 6-well plates with parallel lines drawn, and when the cell density was about 80%, after starvation (serum-free medium culture) for 24 hours, scratches were created along the previously drawn parallel lines with a 200 µL sterilized gun tip. The cells were washed three times with PBS solution, and the shed cells were aspirated and discarded. The cells were then subjected to anoxia and pharmacological interventions, and three replicate wells were set up in each group, respectively. The migration of cells in the scratched area was observed under an inverted microscope at 0 hour and 24 hours. ImageJ software was used to calculate the migrated scratch width and scratch area for each microscopic field, and the migration rate was calculated as the percentage of the migrated scratch area to the initial scratch area.

### 7. Construction of mouse infarction model:

Adult male mice (C57BL/6J Shanghai Jihui) at 7-8 weeks were used, and fasted for 10 hours before surgery. After hair removal and skin exposure, they were gas-anesthetized with isoflurane (400 ml/min), the skin of the left side of the chest was clipped (about 0.5 cm), the mouse heart was exposed, and the anterior descending to the proximal end of the left coronary artery of the mice was ligated with a #7.0 non-invasive suture to suture the chest, and an electrocardiogram and cardiac ultrasound were taken to assess the effect of the surgery.

### 8. Detection of the degree of fibrosis in the peri-infarct area of mice (HE staining and Masson staining):

Mice were treated with d5PAO/PAO gavage (0.3 mg/kg) intervention the day after infarction surgery, and 2 weeks later, mice in each experimental group were treated with gas anesthesia followed by decapitation, and after 4% paraformaldehyde perfusion, cardiac tissues were removed and then continued to be fixed in 4% paraformaldehyde for 48 hours, and then the tissues were paraffin-embedded, sectioned and stained.

### Reagents and instrument

Reagents: 0.5% alcohol soluble eosin staining solution, modified Harris hematoxylin, 0.5% hydrochloric acid alcohol solution.
1.1 Eosin: preparation method: neutral red 0.5 g, 80% alcohol 100 mL, Eosin (sigma) dissolved in alcohol and stirred until all dissolved for further use.
1.2 hematoxylin: preparation: liquid A: hematoxylin (sigma) 1 g, anhydrous alcohol (Sinopharm) 10 mL; liquid B: potassium aluminum sulfate (Sinopharm) 20 g, 200 mL of distilled water two liquids were dissolved and mixed, heated to a boil, and then the red mercuric oxide 0.5 g was slowly added, at this time there is a large number of bubbles, so the container should be large in order to prevent the liquid from overflowing, and then the dyeing solution was quickly cooled, filtered and 6 ml of glacial acetic acid and 10 mL of glycerol were added to 100 mL solution.
1.3 preparation method of 0.7% hydrochloric acid alcohol solution: hydrochloric acid 0.7 mL, 70% alcohol 100 mL.

| Reagent Name | Manufacturer | Catalog No. | Place of Origin |
|---|---|---|---|
| Acid Fuchsin | Sinopharm | 71019360 | China |
| Ponceaux | Sinopharm | 71033761 | China |
| Hydrochloric acid | Sinopharm | 10011018 | China |
| Anhydrous ethanol | Sinopharm | 10009218 | China |
| Aniline Blue | Sinopharm | 71003644 | China |
| Phosphomolybdic acid | OKA | XW514297441 | China |
| Glacial Acetic Acid | Sinopharm | 10000208 | China |

### Instrument

Semi-automatic dehydrator, manufacturer: Leica, Model: TP1020;
Fully automatic paraffin embedding machine, manufacturer: Leica, model: EG1150;
Fully automatic paraffin sectioning machine, manufacturer: Leica, model RM2235;
Nikon micrographic image processing software Nikon H500S electron microscope;
NIS-Element image analysis software;
Microscope: Nikon Eclipse 50i, Model: H550S;
Digital camera: Nikon, Model: DS-FiI;
Professional image analysis software Image pro plus 6.0.

### Experimental Methods

HE staining of paraffin sections
1) Place 4 µm paraffin sections in an oven at 60°C for 1 to 2 hours, and dewax with conventional xylene, ethanol;
2) Stain with hematoxylin (Sigma) for 10 minutes, rinse with running water to remove the remaining color;
3) Differentiate with 0.7% hydrochloric acid ethanol (Sinopharm) for several seconds, rinse with running water;
4) Allow the sections to turn blue for about 15 minutes and soak in 7.95% ethanol for 30 seconds;
5) Stain with alcoholic eosin (Sigma) for 30 seconds;
6) fix with 95% ethanol for 30+30 seconds, followed by 100% ethanol (Sinopharm) for 30+30 seconds;
7) treat with xylene carbolic acid (1:4 carbolic acid 1-xylene 4) for 30 seconds;
8) Treat with xylene for 30 seconds + 30 seconds;
9) Final sealing with neutral gum.

### Masson staining

1) Place the paraffin sections in an oven at 60°C for 1 to 2 hours;
2) Dewax Paraffin sections with conventional xylene, ethanol to water;
3) Stain with Weigert iron hematoxylin (Weigert A liquid and Weigert B liquid equal volume mixture) for 10 minutes, slightly rinsed with running water;
4) Differentiate with 1% hydrochloric acid alcohol, rinse with running water for a few minutes;
5) Stain with Acid red - Li Chun red solution for about 5-10 minutes (it is recommended to try staining a slice to find the best staining time), slightly rinsed with running water;
6) Slightly wash with 0.2% acetic acid;
7) Differentiate and stain with phosphomolybdic acid solution for 3-5 minutes, until under the microscope observing the tissue differentiate into different degrees of red;
8) Slightly wash with 0.2% acetic acid;
9) Stain with aniline blue solution for 1-5 minutes;
10) Slightly wash with 0.2% acetic acid, slightly wash with 95% alcohol;
11) Dehydrate with anhydrous alcohol, xylene transparency, seal the slice with neutral resin, examine under microscopy.

### 9. Detection of SMA and Col 1A protein expression by immunoblotting (WB) of animal tissue extracts.

Mice with myocardial infarction were intervened with d5PAO/PAO gavage treatment (0.3 mg/kg) on the next day of infarction construction, and after 2 weeks, mice in each experimental group were decapitated by gas anesthesia, and after 4% paraformaldehyde perfusion, cardiac tissues were taken out and then continued to be fixed with 4% paraformaldehyde for 48 hours, and then the tissues were paraffin-embedded, sectioned and stained. The infarcted area of the modeling group and the infarcted area of the intervention group were taken for comparison: the tissues were cut into pieces, and the tissues were lysed by adding an appropriate amount of RIPA lysis (Beyotime), which contains protease and phosphatase inhibitors (Beyotime), during which the tissues were vortexed and shaken several times to ensure adequate protein cleavage. The lysates were then centrifuged at 12,000g for 15 min at 4°C, and the supernatant was the total cellular protein obtained. The protein was quantified by using the BCA kit (Beyotime). SDSPAGE gel kit (Epizyme) gel was applied to electrophoresis of 30 µg of protein. Col 1A antibody (Abeam) and α-SMA (Abeam) were used as primary antibodies, HRP-labeled antibody (Beyotime) was used as secondary antibody, and GAPDH and tubulin were used as internal reference. Image-Pro Plus 6.0 software was applied to analyze the bands in grayscale.

### 10. Statistical processing

SPSS22.0 software was used for statistical analysis. Measurement data were expressed as x ± s, and independent samples T-test was used for comparison between two groups; count data were expressed as rate (%), and χ2 test was used for comparison between groups, p < 0.05 was considered as statistically significant difference.

### 11. Results

# denoted compared with control, ^{#}p<0.05, ^{##}p<0.001, ^{###}p<0.0001. *denotes compared with model, *p<0.05,**p<0.001,***p<0,0001.

### Effects of d5PAO and PAO on cultured myocardiac fibroblasts

### 11.1 d5PAO and PAO inhibit anoxia-stimulated growth of cultured myocardiac fibroblasts (CCK8 assay)

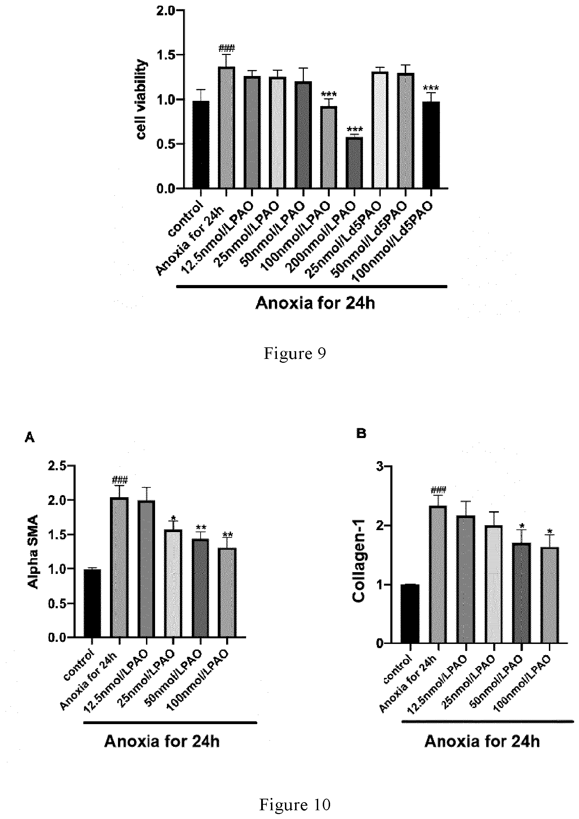

The results showed (Figure 9) that the proliferative capacity of myocardiac fibroblasts was significantly elevated under anoxic 24-hour incubation conditions compared with the control group, from 100%±5.0% in the control group to about 137%±5.3% in the hypoxic group (^{###}p<0.0001) indicating that the hypoxic conditions stimulated fibroblasts proliferation and migration (and also proved the success of the fibroblast model). The addition of different concentrations of d5PAO/PAO under hypoxic conditions, respectively, revealed that the proliferation and migration ability of fibroblasts also decreases, and when the concentration of d5PAO/PAO reaches 100 nM, it had a statistically significant difference compared with that of the hypoxic group (100 nM/L, ^{###}p<0.0001). PAO/d5PAO, below 100 nM, did not significantly change cell viability. Similar to PAO, d5PAO at 100 nM could also inhibit anoxia-induced fibroblast proliferation and migration (97.5%±3.9%, ^{####}p<0.0001, compared with the hypoxic group). The experiments showed that both PAO and d5PAO can effectively inhibit anoxia-stimulated growth of cultured myocardiac fibroblasts.

### 11.2 PAO inhibits anoxia-stimulated induced cellular SMA/Col 1A gene expression (quantitative PCR, Q-PCR)

SMA and Collagen-1 are typical marker proteins for cellular fibrosis. Q-PCR (Figure 10) revealed that the expression of the SMA gene was significantly increased by anoxia for 24 h (increase from 100% ± 0.9% to 202% ± 10.2%, P<0.0001), while this expression-enhancing effect could be inhibited by PAO (25, 50, 100 nM) , which was a dose-dependent inhibitory effect: the higher the PAO concentration, the more significant the inhibitory effect (SMA expression is 157%±6.9%, P<0.05; 144%±5.5%, P<0.001; and 131%±8.5%, P<0.001, respectively).

The expression of gene Col 1A encoding the Collagen-1 was also significantly increased by anoxia for 24 hours (increase from 100%±0.6% to 234%±9.8%), while this expression-enhancing effect was inhibited by PAO (50, 100 nM) , which was a dose-dependent inhibitory effect: the higher the PAO concentration, the more significant the inhibitory effect (Col 1A expression is 171%±12.5%, P<0.05, and 163%±12%, P<0.05, respectively).

### 11.3 PAO inhibits anoxia-stimulation induced cellular Collagen-1 and SMA protein expression (WB)

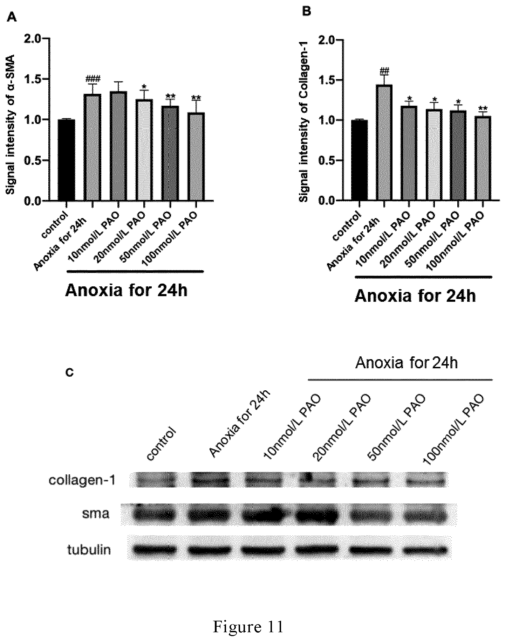

WB results (Figure 11) showed that the expression of Collagen-1 protein in cardiomyocytes was significantly increased by 24 hours of anoxia (increase from 100%±0.6% to 144%±7.0%, P<0.005), while this expression-enhancing effect could be inhibited by PAO (12.5, 25, 50, 100nM) , which was a dose-dependent inhibitory effect: the higher the concentration of PAO, the more significant the inhibitory effect, and the expression of Collagen-1 is 118%±3.2%, P<0.05; 114%±4.8%, P<0.05; 112%±4.0%, P<0.05; and 105%±3.0%, P<0.001, respectively.

The expression of SMA protein was also significantly increased by anoxia for 24 hours (increase from 100%±0.6% to 131%±7.0%, P<0.05), whereas this expression-enhancing effect could be inhibited by PAO (20, 50, 100nM) , which was a dose-dependent inhibitory effect: the higher the concentration of PAO, the more significant the inhibitory effect (SMA expression is 125%±6.4%, P<0.05, 117%±4.6%, P<0.001, 109%±8.7%, P<0.001, respectively).

### A1 and G1 inhibit anoxia-stimulation induced cellular Collagen-1 and SMA protein expression (WB)

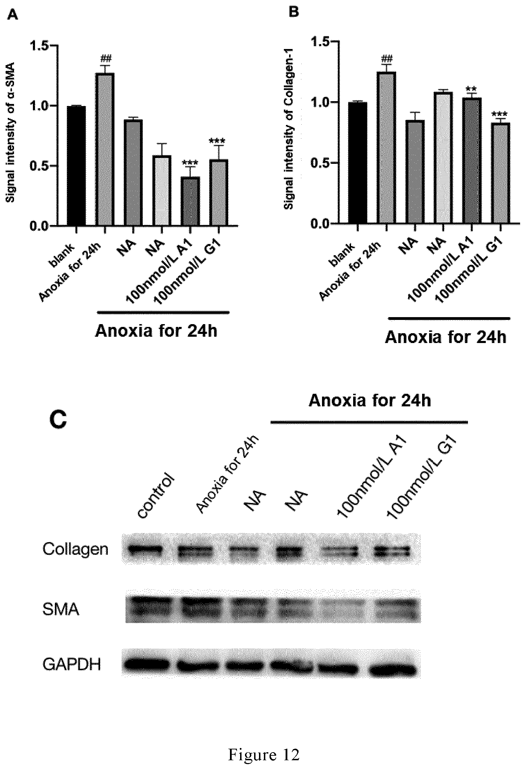

WB results (Figure 12) showed the expression of Collagen-1 protein in cardiomyocytes was significantly increased by 24 hours of anoxia (increase from 100%±0.6% to 125%±3.2%, P<0.005), while this expression-enhancing effect could be inhibited by the known inhibitors of PI4Klllα, A1 and G1 (100 nM/L). Collagen-1 expression was 104%±2.1%, P<0.001, and 83%±2.0%, P<0.0001, respectively;

The expression of SMA protein was also significantly increased by anoxia for 24 hours (increase from 100%±0.3% to 127%±3.4%, P<0.05), while this expression-enhancing effect could be inhibited by A1 and G1 (100 nM/L), (SMA expression was 41%±4.6%, P<0.0001, and 55%±8.7%, P<0.0001, respectively). Considering A1 and G1 as inhibitors of PI4Klllα, the WB results suggested that PI4Klllα was likely to be closely associated with fibrosis, and inhibition of PI4Klllα or PI4KA may improve fibrosis.

### 11.4 PAO inhibits anoxia-induced enhancement of myocardial fibroblast migration (scratch assay)

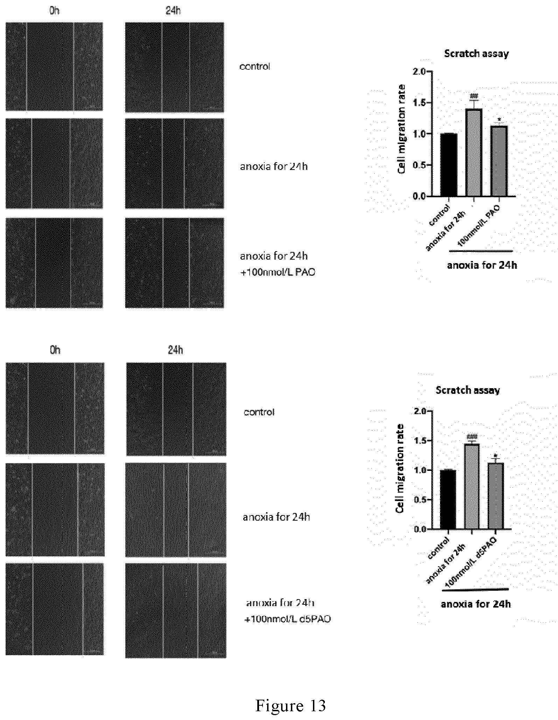

Scratch experiments showed (Figure 13) that the migratory capacity of cells was significantly increased by 24 hours of anoxia from 100%±0.6% to 140%±7.9% in the control group (P<0.001). In contrast, PAO (100 nM) effectively inhibited the increase in migratory capacity (113%±2.6%, P<0.05). d5PAO had a similar function and effectively inhibited the anoxia-induced increase in migratory capacity at a concentration of 100 nM (113%±3.8%, P<0.05). Migration % = (initial scratch area - scratch area after 24 h)/initial scratch area × 100%.

### 11.5 Efficacy of d5PAO and PAO in a mouse model of myocardial infarction

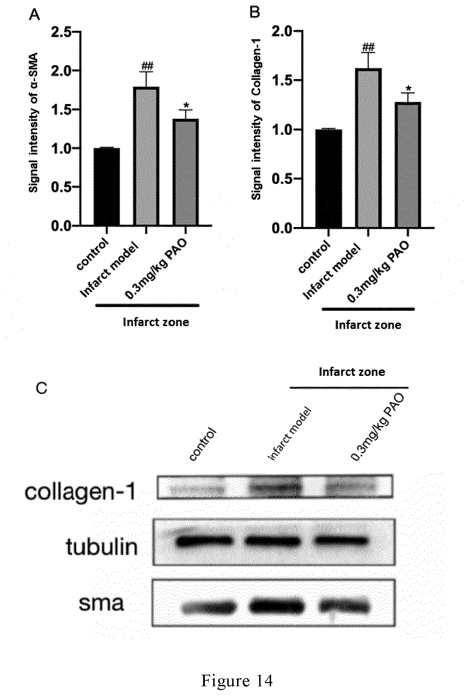

1) PAO effectively inhibited the enhancement of SMA and Collagen-1 protein expression in the infarct zone
   WB results (Figure 14) showed that the expression of SMA and Collagen-1 proteins in cardiac tissues in the infarct mouse model was significantly higher than that in the control group, increasing from control group 100%±0.6% to 180%±11.2% (P<0.001),162%±9.2% (P<0.001). In contrast, PAO (gavage, 0.3 mg/Kg) significantly inhibited the increase of both proteins, with SMA levels decreasing to 138%±6.7% (P<0.05) and Collagen levels decreasing to 128%±5.2% (P<0.05).
2) PAO effectively inhibited the increase of fibrotic area containing Collagen area in the infarct zone

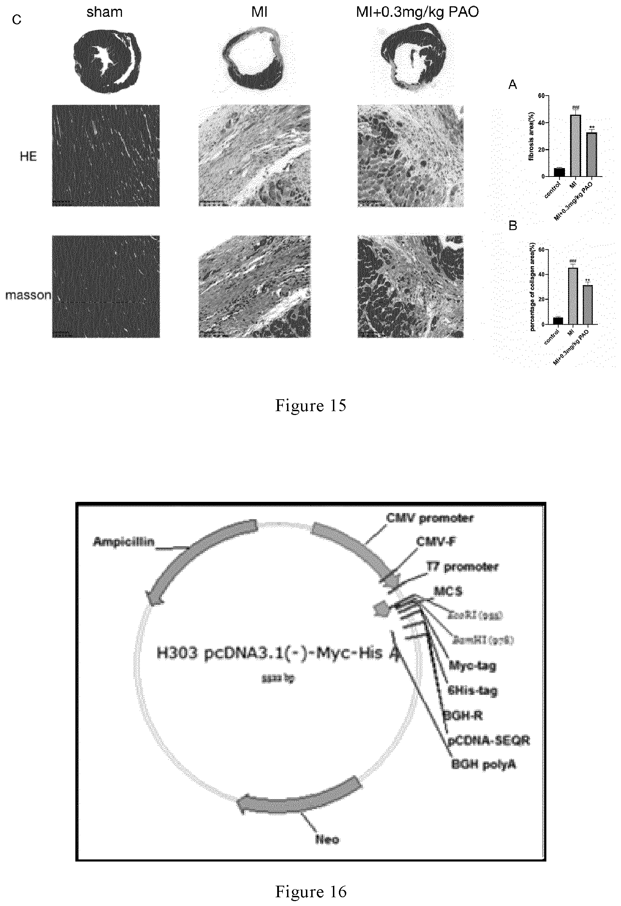

The hearts of mice in the infarct group (MI) showed obvious trauma, and immunohistochemical staining showed that the fibrotic area of the hearts of mice in the infarct group was substantially elevated (46.13%±2.0%, P<0.0001), whereas the gavage of PAO (0.3mg/Kg) inhibited the fibrotic area to a certain extent, decreasing to 32.9%±1.3% (P<0.001). Masson staining, on the other hand, showed (Figure 15) that the Collagen-containing area in the heart tissues of mice in the infarct group was also substantially increased (45.47%±1.7%, P<0.0001), and, similarly, PAO causes the Collagen-containing area to decrease to 31.30%±1.48% (P<0.001).

### Conclusion:

The above results showed that PAO and d5PAO inhibited anoxia-induced proliferation of myocardiac fibroblasts cultured in vitro. PAO also inhibited anoxia-stimulation induced increases in the expression of SMA and Collagen-1 proteins, markers of cellular fibrosis, and their encoding genes, SMA and Col 1A, as well as the migratory capacity of cardiomyocytes after damage. A1 and G1, known inhibitors of PI4Klllα or PI4KA, also inhibited anoxia-induced increases in SMA and Collagen-1 proteins. In vivo experiments had shown that PAO was also effective in inhibiting the enhancement of SMA and Collagen-1 protein expression in the infarct zone, as well as the increase in the Collagen-containing area of the fibrotic area of the infarct zone. These results suggested that PAO and d5PAO were effective in preventing cardiac fibrogenesis after anoxia, and thus were potential therapeutic agents for related diseases.

### Example 7. Comparison of the efficacy of the series of fluorinated compounds on α-synuclein and Aβ42

### 1. Experimental materials

### 1.1 Reagents and consumables

α-synuclein ELISA Kit was purchased from Thermo Fisher Scientific (Catalog No. KHB0061);.
Amyloid beta 42 Human ELISA Kit was purchased from Thermo Fisher Scientific (Catalog No. KHB3544).
Fugene HD Transfection Reagent was purchased from Promega (Beijing) Biotech Co., Ltd. (Catalog No. E2311).
α-synuclein monoclonal antibody (Mouse monoclonal) was purchased from Sigma-Aldrich (Shanghai, Catlog No. S5566);

### 1.2 Instrument

Olympus IX51 inverted microscope, FV1000 laser confocal microscope were purchased from Olympus (Japan); NovoStar microplate reader was purchased from BMG (Germany);

### 1.3 Plasmid Information

The plasmid was purchased from Obio Technology (Shanghai) Corp, Ltd.

The information of α-synuclein overexpression plasmid is as follows:
Sequence:

ATGGATGTATTCATGAAAGGACTTTCAAAGGCCAAGGAGGGAGTTGTGGC
TGCTGCTGAGAAAACCAAACAGGGTGTGGCAGAAGCAGCAGGAAAGACA
AAAGAGGGTGTTCTCTATGTAGGCTCCAAAACCAAGGAGGGAGTGGTGCA
TGGTGTGGCAACAGTGGCTGAGAAGACCAAAGAGCAAGTGACAAATGTT
GGAGGAGCAGTGGTGACGGGTGTGACAGCAGTAGCCCAGAAGACAGTGG
AGGGAGCAGGGAGCATTGCAGCAGCCACTGGCTTTGTCAAAAAGGACCA
GTTGGGCAAGAATGAAGAAGGAGCCCCACAGGAAGGAATTCTGGAAGAT
ATGCCTGTGGATCCTGACAATGAGGCTTATGAAATGCCTTCTGAGGAAGG
GTATCAAGACTACGAACCTGAAGCCTAA

Vector map was shown in Figure 16.

### 2. Experimental methods

### 2.1 Cell culture and drug treatments:

The complete culture system for SH-SY5Y was high sugar DMEM with 15% FBS. The plasmids were transfected using Fugene HD transfection reagent and incubated for 24 hrs and then starved, i.e., removing the serum and using only DMEM high sugar medium. After 24 hrs of incubation, the culture was changed to complete culture system and treated with drugs.

Stable trans APP (SW) HEK293 cell line (gifted by Haiyan Zhang's group, Shanghai Institute of Pharmaceutical Sciences, Chinese Academy of Sciences) is a human embryonic kidney cell line transfected with Swedish double mutant APP695cDNA, and the well plates need to be treated with 20 µg/mL Poly-D-Lysine (PDL) for 24 hours before seeding the plates. The culture medium was high-sugar DMEM with 10% FBS and screened with 200 µg/mL G418 at the same time. The seeded plates were incubated for 48 h and then starved, i.e., serum was removed and only DMEM high sugar medium was used. After 24 h of incubation, the culture was changed to complete culture system and treated with drugs.

### 2.2 Cell viability by thiazolyl blue (MTT) assay

After 12 hours of drug treatment, MTT at a final concentration of 0.5 mg/mL was added, incubated for 4 hours and then the incubation solution was aspirated, 100 µL of DMSO was added to dissolve the adsorbed MTT, and the absorbance value was read after 15 minutes of shaking.

### 2.3 ELISA assay

### a) α-synuclein ELISA assay

50 µL of Hu α-synuclein Detection Antibody solution was added to each well (except chromogen blanks empty), then 50 µL of sample and standards (see Figure 17 for standard preparation) were added to each well (except chromogen blanks empty); each well was mixed with gentle shaking and incubated at 4°C overnight with membrane covered. Each well was washed four times with 100 µL of 1X Wash buffer; each well except the blank colorimetric wells was added with 100 ul of Anti-Rabbit IgG HRP and incubated for 30 minutes at room temperature with membrane covered and then washed four times with 1X Wash buffer. Each well was added with 100 µL of Stabilized Chromogen to each well, the solution was turned blue, and each well was incubated for 30 minutes at room temperature away from light. Each well was added 100 µL of Stop Solution and mixed with gentle shaking; the solution was turned yellow; and the absorbance value was read immediately on an microplate reader (absorption wavelength 450 nm).

### b) Aβ ELISA assay

100 µL of diluted standard, blank and sample was added to the corresponding wells of the assay plate; the wells were incubated at 37°C for 2 hours with membrane covered; the liquid in each well was discarded, and each well was added with 100 µL of Detection Reagent A Working Solution and incubated at 37°C for 1 hour with membrane covered. The supernatant was discarded and each well was washed with 1X Wash buffer 3 times for 2 minutes each time and left with as little liquid as possible. Each well was added with 100 µL of Detection Reagent B Working Solution was incubated at 37°C for 1 h with membrane covered; the washing operation was repeated 5 times. Each well was added with 90 µL of Substrate Solution and incubated at 37°C for 20 min away from light with membrane covered. The solution was turned blue; each well was added with 50 µL of Stop Solution and mix with gentle shaking; the solution was turned yellow; and the absorbance value was read with an microplate reader (absorption wavelength 450 nm) as soon as possible.

### 2.4 Statistical analysis

Data processing was analyzed using GraphPad Prism 5 software. Processing was done using one-way ANOVA, mean±SEM, p<0.03 statistically different.

### 3. Results

### 3.1 Effect of the series of fluorinated compounds on SH-sy5y cell viability and α-synuclein

SH-SY5Y cells were treated with high sugar DMEM with 15% FBS, transfected with empty (vehicle) plasmid or α-synuclein overexpression plasmid by Fugene HD transfection reagent, and incubated for 24 h and then starved, i.e., removing the serum and using only DMEM high sugar medium for incubation for 24 hours. According to the grouping, cells were respectively treated with different concentrations of F2mPAO, F2oPAO and F3PAO. Cell viability was detected by MTT assay after 4 and 24 hours of treatment with the serious of fluorinated compounds, respectively. The experimental results showed that the cell viability of each group did not show any significant change compared with that of the α-synuclein overexpression (α-syn OE) group after 4 hours of treatment with the series of fluorinated compounds (Figure 18A); the cell viability of the 100 nM F2oPAO and 100 nM F3PAO treated groups decreased significantly compared with that of the α-syn OE group after 24 hours of treatment with the series of fluorinated compounds (Figure 18B).

The supernatant of cell culture medium was collected after 4 h of treatment with different concentrations of F2mPAO, F2oPAO or F3PAO, and the α-synuclein content in cell culture medium was detected by ELISA assay. The results showed that the α-synuclein content was significantly higher in cell culture supernatants of the α-syn OE and 25 nM F2mPAO co-treated group, the α-syn OE and 50 nM F2mPAO co-treated group, and the α-syn OE and 25 nM F2oPAO co-treated group, when compared with the α-synuclein overexpression (α-syn OE) group (Figure 18C-D)

### 3.2 Effect of the series of fluorinated compounds on the viability and Aβ release of stable transfection APP (SW) HEK293 cells

The Stable Transfection APP (SW) HEK293 cell line (a gift from Haiyan Zhang's group at the Shanghai Institute of Pharmaceutical Sciences, Chinese Academy of Sciences) is a human embryonic kidney cell line transfected with the Swedish double mutant amyloid precursor protein (APP) 695 cDNA and bearing the G418 screening label. Well plates were treated with 20 µg/mL Poly-D-Lysine (PDL) for 24 hours before seeding. The culture medium was high-sugar DMEM with 10% FBS and screened with 200 µg/mL G418 at the same time. After 48 hours of culture, different concentrations of fluorinated compounds were added and treated for 4 or 24 hours, respectively, and the cell viability of different groups was detected by MTT assay. The results showed that treatments of HEK293 cell lines with certain concentrations of F2mPAO, F2oPAO and F3PAO for 4 h and 24 h had no significant effect on cell viability (Figure 19A-B).

In the experiment to detect the effect of the series of fluorinated compounds on Aβ release from stable transfection APP (SW) HEK293 cells. Cells are cultured for 48 h and starved for 24 h, and then the culture was replaced with a complete culture system and treated with compounds for 4 h. Aβ levels in the supernatant of the cytosolic culture fluid were detected by ELISA. The results showed that Aβ concentration is significantly increased in the cell supernatants of the 50 nM and 75 nM F3PAO-treated groups compared with the control group (Figure 19C-D).

### Example 8. Effect of the Series of Fluorinated Compounds on Huntington's Tract (HTT) Protein and Superoxide Dismutase 1 (SOD1)

### 1. Experimental material

### 1.1 Reagents and consumables

Human Superoxide Dismutase 1 Kit was purchased from Signalway Antibody LLC. (SAB, Catalog No. EK16688);.

Human Huntingtin (HTT) ELISA Kit was purchased from Signalway Antibody LLC. (SAB, Catalog No. EK2869).

Fugene HD Transfection Reagent was purchased from Promega (Beijing) Biotech Co., Ltd. (Catalog No.E2311).

Fugene HD Transfection Reagent was purchased from Promega (Beijing) Biotech Co., Ltd. (Catalog No.E2311).

### 1.2 Instrument

The NovoStar Microplate reader was purchased from BMG (Germany);

### 1.3 Plasmid Information

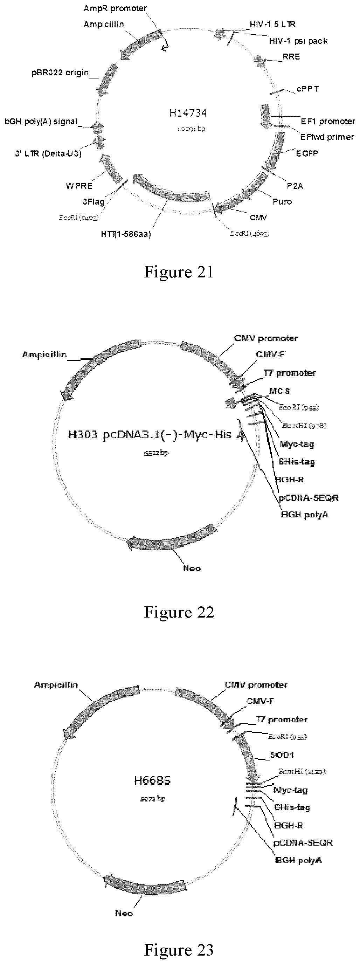

The following plasmids were purchased from Obio Technology (Shanghai) Corp., Ltd.
1) Gene name: HTT(1-586aa); GenBank ID:NM_002111; empty vector map and full map are shown in Figures. 20-21;
2) Gene name: SOD1, gene ID: 6647; empty vector map and full map are shown in Figures 22-23.

### 2. Methods

### 2.1 Cell culture and drug treatments:

The complete culture system for SH-SY5Y was high sugar DMEM with 15% FBS. Cells were transfected with the plasmids by Fugene HD transfection reagent and incubated for 24 hrs and then starved, i.e., removing the serum and using only DMEM high sugar medium. After 24 hrs of incubation, the culture was changed to complete culture system and treated with drugs.

### 2.2 Detection of cell viability by thiazolyl blue (MTT) assay

After 12 hours of treatment with drugs, the system was added with MTT at a final concentration of 0.5 mg/mL, and incubated for 4 hours and then the culture solution was aspirated; the system was added with 100 µL of DMSO to dissolve the adsorbed MTT, and the absorbance value was read after 15 minutes of shaking.

### 2.3 ELISA assay

### a) SOD1 ELISA assay

100 uL of diluted standards, blank control and samples were added to the wells of the assay plate. The membrane was covered and cells were incubated at 37°C for 2 h and the liquid in each well was discarded, 100 ul of Detection Reagent A Working Solution was added to each well, and the membrane was covered and the plate was incubated at 37°C for 1 h. the supernatant was discarded, and the wells was washed with 1X Wash buffer 3 times for 2 min each time and was left with as little liquid as possible. Each well was added with 100 ul of Detection Reagent B Working Solution and incubated for 1 hour with membrane covered; the washing operation was repeated for 5 times. Each well was added with 90 µL of Substrate Solution and incubate at 37°C for 20 minutes away from light with membrane covered; the solution was turned blue. Each well was added with 50 µL of Stop Solution, mixed with gentle shaking; the solution was turned yellow; and the absorbance value was read as soon as possible with an microplate reader (absorption wavelength 450 nm).

### b) Human Huntingtin ELISA assay

50 µL of diluted standard, 50 µL of blank and 50 µL of sample were added to the corresponding wells of the assay plate; each well was immediately added with 50 µL of Detection A Working Solution, mixed with gentle shaking and incubate at 37°C for 1 h with membrane covered. The supernatant was discarded; each well was washed three times with 1X Wash buffer for 2 minutes each time and left with as little liquid as possible. Each well was added with 100ul of Detection Reagent B Working Solution and incubate at 37°C for 45 minutes with membrane covered; the washing operation was repeated for 5 times. Each well was added with 90 µL Substrate Solution and incubate at 37°C for 10-20 minutes away from light with membrane covered. Each well was added with 50 µL of Stop Solution, mixed with gentle shaking; the solution was turned yellow; and the absorbance value was read immediately on an microplate reader (absorption wavelength 450 nm).

### 2.4 Statistical analysis

Data processing was analyzed using GraphPad Prism 5 software. Processing was performed using one-way ANOVA, mean±SEM, p<0.03 statistically different.

### 3. Results

### 3.1 The series of fluorinated compounds promote Huntington's protein (HTT) secretion

SH-SY5Y cell line was cultured in complete medium (15% FBS dissolved in high sugar DMEM) and transfected with HTT overexpression (HTT-OE) plasmid for 24 hours, starved (high sugar DMEM) for 24 hours, and then treated with a series of fluorinated compounds for 24 hours. The cell viability of each group was detected by MTT assay and compared with HTT-OE group by One-way ANOVA analysis. The results showed that the cell viability of HTT-OE group is significantly lower than that of the control (ctrl) group (Figure 24A), and there is no significant change in the cell viability between the series of fluorinated compounds-treated groups and HTT-OE group.

SH-SY5Y cell line was transfected with HTT overexpression plasmid using Fugene HD and cultured for 24 h. After starvation treatment for 24 h, the cells were treated with compounds for 4 h according to the grouping, and the level of HTT in the supernatant of the cell culture solution was detected by ELISA. The results showed that the HTT protein concentration in the cell culture medium of the HTT-OE group was significantly higher than that of the control group; the HTT protein level in the cell culture medium of the HTT-OE and 25 nM F3PAO co-treated group was significantly increased compared with that of the HTT-OE group, and other treatment groups also showed a tendency to promote the secretion of HTT (Figure 24B). The results above suggested that a certain concentration of the series of fluorinated compounds could promote HTT protein secretion.

### 3.2 The series of fluorinated compounds promote superoxide dismutase 1 (SOD1) secretion

SH-SY5Y cell line was cultured in complete medium (15% FBS dissolved in high sugar DMEM) and transfected with SOD1 overexpression (SOD1-OE) plasmid for 24 hours, starved (high sugar DMEM) for 24 hours, and then treated with the series of fluorinated compounds for 24 hours. The cell viability of each group was detected by MTT assay and compared with SOD1-OE group by One-way ANOVA analysis. The results showed that the cell viability of the SOD1-OE group was significantly lower than that of the control (ctrl) group (Figure 25A), and there was no significant change in the cell viability between the series of fluorinated compounds-treated groups and the HTT-OE group.

SH-SY5Y cell line was transfected with SOD1 overexpression plasmid using Fugene HD and cultured for 24 h. After starvation treatment for 24 h, the cells were treated with compounds for 4 h according to the groupings. The level of SOD1 in the supernatant of the cell culture solution was detected by ELISA. The results showed that the level of HTT protein in the cell culture medium was significantly increased in the SOD1-OE group compared with the control group; the series of fluorinated compound-treated groups showed a tendency to promote the secretion of SOD1 compared with the SOD1-OE group (Figure 25B).

### Example 9. Therapeutic effect of the series of fluorinated compounds on Gaucher disease

### 1. Cell culture

The complete culture system for SH-sy5y was high sugar DMEM with 15% FBS. SH-SY5Y cells were treated with 100 µM CBE for 24 h and then co-treated with starvation (high sugar DMEM without FBS) and 100 µM CBE for 24 h, and then the cells were treated with different concentrations of fluorinated compounds for 24 h.

### 2. Detection of cell viability by thiazolyl blue (MTT) assay

After 24 hours of drug treatment, the system was added with MTT at a final concentration of 0.5 mg/mL, and incubated for 4 hours and then the culture solution was aspirated; the system was added with 100 µL of DMSO to dissolve the adsorbed MTT, and the absorbance value was read after 15 minutes of shaking.

### 3. Lyso-tracker staining

SH-SY5Y cells were treated with 100 µM CBE for 24 h, co-treated with starvation (high sugar DMEM without FBS) and 100 µM CBE for 24 h, and then treated with different concentrations of the series of fluorinated compounds for 24 h, and then treated with different concentrations of a series of fluorinated compounds for another 24 h. The system was treated with Lyso-tracker for 30 min, replaced with fresh culture medium and meanwhile treated with different concentrations of a series of fluorinated compounds. The system was incubated for 10 minutes, treated with 4% paraformaldehyde for 30 minutes and then washed with PBS three times for 10 minutes each time. The slices was sealed with sealer and were visualized by microscope.

### 4. Results

### 4.1 The series of fluorinated compounds inhibit CBE-induced apoptosis or death of SH-SY5Y cells

Conduritol B epoxide (CBE), an inhibitor of the GBA1 enzyme encoded by the lysosomal glucocerebrosidase GBA gene, is commonly used in the construction of cellular and animal models of Gaucher disease (GD). SH-SY5Y cells were treated with 100 µM CBE for 24 h. The cells were incubated with a certain concentration of the series of fluorinated compounds according to the groupings for 24 h. The cell viability was detected by MTT assay. The results showed that compared with the 100 µM CBE-treated group, the cell viability of the 50 nM F2mPAO and 100 µM CBE co-treated group, and the 50 nM F3PAO and 100 µM CBE co-treated group was significantly increased (Figure 26), which suggested that a certain concentration of fluorinated compounds had a protective effect on the CBE-induced apoptosis or cell death of SH-SY5Y cells.

### 4.2 The series of fluorinated compounds promote lysosomal exocytosis

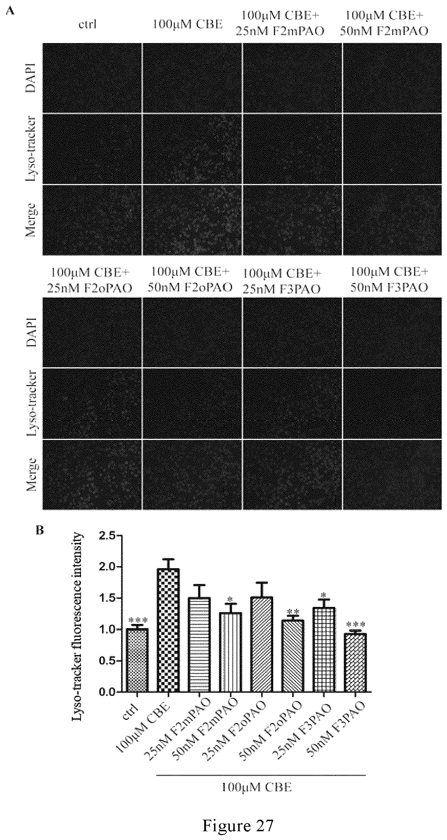

GD is a common class of lysosomal storage disease, and it was investigated whether the series of fluorinated compounds attenuated CBE-induced lysosomal storage by the lysosomal marker DND99 (Lyso-tracker red DND99). The results showed that 100 µM CBE-treated group cause the fluorescence intensity of 100 µM CBE Lyso-tracker to elevate when compared with the control group (ctrl) (Figure 27A-B), suggesting that CBE treatment led to lysosomal storage in SH-SY5Y cells. Compared with the 100 µM CBE-treated group, cell viability significantly increased and the fluorescence intensity of Lyso-tracker significantly decreased in the 50 nM F2mPAO and 100 µM CBE co-treated group, 50 nM F2oPAO and 100 µM CBE co-treated group, 25 nM F3PAO and 100 µM CBE co-treated group, and 50 nM F3PAO and 100 µM CBE co-treated group significantly decreased the fluorescence intensity of the Lyso-tracker (Figure 27A-B).

### Example 10. Inhibition of inflammatory factor release by the series of fluorinated compounds

BV2 cells are immortalized from mouse microglia by retroviral-mediated transfection of v-raf/v-myc and retain a variety of morphological, phenotypic and functional characteristics of microglia. Lipopolysaccharide (LPS) stimulation of BV2 cells is commonly used to obtain inflammatory cell models in experiments related to inflammation in the nervous system. In this experiment, LPS-stimulated BV2 cells were used as an inflammatory cell model to observe the effects of fluorinated compounds on the expression and secretion of inflammatory factors such as tumor necrosis factor α (TNF-α) and CD206.

### 1. Cell culture and processing

BV2 cells were cultured in high-sugar DMEM with 10% FBS and incubated in a 37°C and 5% CO₂ cell culture incubator for 48 h. The medium was removed and replaced with high-sugar DMEM medium containing 1 µg/mL of LPS (lipopolysaccharide, purchased from Sigma, Catalog No. L2880) (without FBS), and the cells were also co-incubated with different concentrations of fluorinated compounds according to the grouping. After 4 hours, the supernatant was collected and centrifuged for subsequent experiments.

### 2. Mouse Tumor Necrosis Factor Alpha (TNF-α) ELISA assay

Mouse Tumor Necrosis Factor Alpha (TNFα) ELISA Kit was purchased from Signalway Antibody LLC, Catalog No. EK16997. The following experiments are performed according to the product instructions:
1) Prepare standards using Diluent buffer at concentrations of 10 ng/mL, 5 ng/mL, 2.5 ng/mL, 1.25 ng/mL, 0.625 ng/mL, 0.312 ng/mL, 0.156 ng/mL, and 0;
2) Gently oscillate Detection Reagent A and Detection Reagent B, and use Diluent buffer to dilute Reagent A and Reagent B to 1/100 of the mother liquor to reach the concentration of working solution, respectively;
3) Dilute the Wash solution with deionized water to 1/30 of the mother liquor to form the working solution;
4) Add 100 µL of the sample to be tested or standard into each well, seal the plate with a sealing film and incubate at 37°C for 2 hours;
5) Remove the supernatant without washing;
6) Add 100 µL of Detection Reagent A working solution to each well respectively, seal the well plate with a sealing film and incubate at 37°C for 1 hour;
7) Discard the supernatant and wash 3 times using 300 µL Wash solution working solution, each time resting for 2 min, and remove as much of the wash solution as possible by inverting the well plate on absorbent dust-free paper;
8) Add 100 µL Detection Reagent B working solution to each well, seal the plate with a sealing film, and incubate at 37°C for 1 hour;
9) Wash the well plate 5 times according to step 7);
10) Add 90 µL of Substrate solution into each well, observe the blue liquid in the well plate, seal the plate with a sealing film, and incubate at 37°C for 20 minutes, protected from light;
11) Add 50 µL of Stop solution into each well, observe the blue liquid turns yellow, seal the plate with sealing film, and mix with shaker for 10 minutes;
12) Read the absorbance value of the samples at 450 nM wavelength using a microplate reader (NOVOstar trans-liquid microplate reader).

### 3. Immunofluorescence staining assay

BV2 cells were treated for 12 hours, supernatant was aspirated, and washed three times with phosphate buffer saline (PBS); 4% Paraformaldehyde (PFA) was added and allowed to stand for 30 minutes at room temperature; 4% PFA was discarded and washed three times with PBS for 10 minutes each time; 0.3% non-ionic detergent Triton-X (dissolved in PBS) was added and allowed to stand for 30 min at room temperature; 0.3% Triton-X was aspirated, and PBS blocking solution containing 10% goat serum (GS) was added and allowed to stand for 1 h at room temperature; the primary antibody was incubated overnight at 4°C (the primary antibody was diluted with 10% GS blocking solution at a dilution ratio of 1:100 for TNF-α, and 1:100 for CD206). On the following day, cells were washed with PBS 3 times for 10 minutes each time; incubated with secondary antibody Alexa Flour 555 goat anti-rabbit IgG (diluted using a blocking solution at a dilution ratio of 1:500, added with 1 µg/mL 4',6-diamidino-2-phenylindole (DAPI)), left at room temperature for 2 hours, and added with PBS for washing three times, each time for 10 minutes, sealed using a sealer and then observed by microscope.

### 4. Statistical analysis

Data processing and statistics were performed using GraphPad Prism5 software, and data were expressed as mean ± standard error (mean ± SEM). Differences between groups were compared using one-way ANOVA statistics, with p<0.03 considered statistically different.

### 5. Results

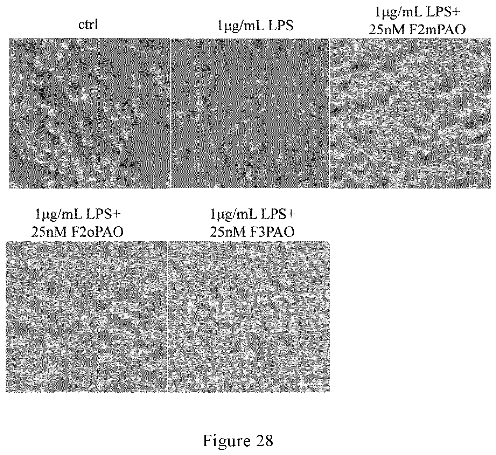

BV2 cells were cultured in high-sugar DMEM medium with 10% FBS and incubated in a 37°C 5% CO2 cell culture incubator for 48 h. The medium was removed and replaced with high-sugar DMEM medium containing 1 µg/mL LPS, and the cells were also co-incubated with different concentrations of the series of fluorinated compounds according to the grouping for 12 h (Figure 28). Microglia are the most important intrinsic immune cells and major effector cells in the CNS. They secrete both neurotoxic and neurotrophic factors in the immune response to Alzheimer's disease, Parkinson's disease, and multiple sclerosis, and exhibit both pro-inflammatory and anti-inflammatory effects, with two polarization states, M1 and M2. In order to examine the role played by the series of fluorinated compounds in the LPS-induced BV2 cell model, immunofluorescence staining was performed for the M1-type microglial cell marker TNFα and the inflammation-suppressing M2-type microglial cell marker CD206. The results showed that the expression level of TNFα in BV2 cells in the 1 µg/mL LPS-treated group was significantly elevated compared with the control group, whereas compared with the 1 µg/mL LPS-treated group, the TNFα expression level in the 25 nM F2mPAO and 1 µg/mL LPS co-treated group, the 12.5 nM F3PAO and 1 µg/mL LPS co-treated group, and the 25 nM F3PAO and 1 µg/mL LPS co-treated group was significantly decreased (Figure 29A-B). Unlike the TNFα expression level in each treatment, the CD206 expression level in BV2 cells in the 1 µg/mL LPS-treated group tended to decrease compared with the control group, whereas there was a decreasing trend of CD206 in the groups co-treated with a certain concentration of F2mPAO, F2oPAO, and F3PAO with 1 µg/mL LPS, compared with the 1 µg/mL LPS-treated group (Figure 29A and 29C). In addition, by calculating the ratio of CD206 signal intensity to the corresponding TNFα fluorescence intensity, the analysis reveals that compared with the 1 µg/mL LPS-treated group, the ratio of CD206 fluorescence intensity to TNF-α fluorescence intensity in the 25 nM F2mPAO and 1 µg/mL LPS co-treated group, 12.5 nM F3PAO and 1 µg/mL LPS co-treated group, and 25 nM F3PAO and 1 µg/mL LPS co-treated group was significantly elevated, indicating that a certain concentration of the series of fluorinated compounds promoted the polarization of M1-type BV2 cells to M2 type (Figure 30).

To further test whether the the series of fluorinated compounds have the effect of inhibiting inflammation, the release of inflammatory factors was examined in the LPS-induced BV2 cell model, and the concentration of TNF-α in the culture medium was measured using the ELISA (enzyme-linked immunosorbent assay) method. The results were as follows: the concentration of TNF-α in the cell culture medium was significantly elevated in the 1 µg/mL LPS-treated group compared with the control group (Figure 31). While compared with the 1 µg/mL LPS-treated group, the concentration of TNF-α in the cell culture medium in 25 nM F2mPAO and 1 µg/mL LPS co-treated group, 50 nM F2mPAO and 1 µg/mL LPS co-treated group, 12.5 nM F2oPAO and 1 µg/mL LPS co-treated group, 25 nM F2oPAO and 1 µg/mL LPS co-treated group, 50 nM F2oPAO and 1 µg/mL LPS co-treated group, 12.5 nM F3PAO and 1 µg/mL LPS co-treated group, 25 nM F3PAO and 1 µg/mL LPS co-treated group as well as 50 nM F3PAO and 1 µg/mL LPS co-treated group was significantly reduced (Figure 31).

### Example 11: Pharmacological research of fluorinated, deuterated compounds in tumor cell lines

### 1. Experimental methods:

### Cell planking

Complete culture medium was prepared and mixed well. Resuscitated tumor progenitor cells (Shanghai ChemPartner Co., Ltd.) was passed for about two generations and selected for the cell line with good growth status. Adherent cells: the medium was removed by being aspirated; the cells were washed with trypsin once; the waste liquid was discarded; 3ml of fresh trypsin was added to the culture flask for digestion. When the cells are loosened to be detached from the wall of the bottle, 8 mL of complete medium was added to abort trypsin digestion and mixed gently. The cell suspension was pipetted into a centrifuge tube and centrifuged at 1000 rpm for 4 minutes. Suspend cells: the cell suspension was aspirated to a centrifuge tube and centrifuged at 1000 rpm for 4 minutes. The supernatant was discarded. An appropriate volume of medium was added to the centrifuge tube and gently blown to resuspend the cells. The cell number was counted using a Vi-Cell XR cell counter. The cell suspension was adjusted to the appropriate concentration.

### Preparation and addition of compound plates

Compounds to be tested: the compounds were prepared as a 10 mM solution in DMSO and the compounds were diluted to a 0.5 mM solution in DMSO. The compounds were added to the corresponding cell wells using an HPD300 instrument and incubated for 72 hours in a CO2 incubator.

### Reagent preparing and testing

CellTiter-Glo Buffer was thawed at room temperature. Lyophilized CellTiter Glo substrate was equilibrated to room temperature. CellTiter-Glo Buffer was added to CellTiter Glo Substrate and mixed thoroughly. The cell plate was taken out and equilibrated to room temperature. 100 µL of mixed CellTiter Glo Reagent was added to each well, shaken for 10 minutes away from light, and incubated for 10 minutes. The culture plate was placed into the Envision for reading plate, and the results of the luminescence reading were recorded; the inhibition rate was calculated according to the following formula: inhibition rate (%) = (1-(RLU compound-RLU blank) / (RLU DMSO - RLU blank)) × 100%. The pharmacodynamic inhibition rate curve was plotted and IC50 values were calculated using XLFit. A 4-parameter model was used [fit = (A+ ((B-A)/ (1+ (C/x) ^D)))].

### 2. Results:

**Table 8. Comparison of deuterated compounds in inhibiting tumor cell proliferation**

| Cell line | | d1PAO | d2PAO | d3PAO | Staurosporine |
|---|---|---|---|---|---|
| | | IC₅₀(nM) | IC₅₀(nM) | IC₅₀(nM) | IC₅₀(nM) |
| CCRF-CEM | Acute lymphoid T-cell leukemia | 33.51 | 31.92 | 23.85 | 8.05 |
| KS-1 | Glioblastoma | 43.47 | 45.94 | 30.94 | 2.25 |
| NOMO-1 | Acute myeloid leukemia (AML) | 58.08 | 59.40 | 40.98 | 16.02 |
| RD | Malignant embryonal rhabdomyosarcoma | 26.67 | 25.74 | 18.58 | 4.79 |
| SUP-B15 | Acute lymphoid B-cell leukemia | 34.24 | 33.57 | 22.94 | 10.78 |
| TT | Medullary thyroid carcinoma | 109.41 | 107.06 | 75.39 | 83.92 |
| CCF-STTG1 | Cerebral astrocytoma | 107.52 | 130.27 | 94.68 | 9.10 |
| IMR-32 | Neuroblastoma | 23.28 | 24.08 | 14.44 | 4.19 |
| REH | Acute non-B or non-T cell lymphoma | 22.81 | 22.43 | 18.55 | 8.08 |
| AN3 CA | Adenocarcinoma of the endometrium | 39.86 | 40.66 | 28.59 | 35.83 |

As shown in Table 8: d1PAO, d2PAO, and d3PAO all showed inhibitory effects on the tumor cells tested. d1PAO and d2PAO both had similar inhibitory IC₅₀, while d3PAO was superior to the first two. All three demonstrated the strongest inhibitory effects on glioblastoma, neuroblastoma, acute lymphoid T-cell leukemia, acute non-B or non-T-cell lymphoma, acute lymphoid B-cell leukemia, malignant embryonal rhabdomyosarcoma, and adenocarcinoma of the endometrium, with IC₅₀s of less than 50 nM; relatively strong inhibitory effect on acute myeloid leukemia cells, with IC₅₀ of 50-100nM; weakest inhibitory effect on medullary thyroid carcinoma and cerebral astrocytoma cells, IC₅₀>100 nM.

**Table 9. Comparison of fluorinated compounds in inhibiting tumor cell proliferation.**

| Cell line | | F2oPAO | F3PAO | Staurosporine |
|---|---|---|---|---|
| | | IC₅₀(nM) | IC₅₀ (nM) | IC₅₀ (nM) |
| CCRF-CEM | Acute lymphoid T-cell leukemia | 33.79 | 34.81 | 8.05 |
| KS-1 | Glioblastoma | 42.15 | 36.27 | 2.25 |
| NOMO-1 | Acute myeloid leukemia (AML) | 60.18 | 48.23 | 16.02 |
| RD | Malignant embryonal rhabdomyosarcoma | 28.90 | 26.68 | 4.79 |
| SUP-B15 | Acute lymphoid B-cell leukemia | 25.86 | 25.99 | 10.78 |
| TT | Medullary thyroid carcinoma | 119.51 | 94.77 | 83.92 |
| CCF-STTG1 | Cerebral astrocytoma | 104.32 | 106.61 | 9.10 |
| IMR-32 | Neuroblastoma | 24.88 | 19.08 | 4.19 |
| REH | Acute non-B or non-T cell lymphoma | 22.31 | 26.75 | 8.08 |
| AN3 CA | Adenocarcinoma of the endometrium | 40.83 | 39.72 | 35.83 |

As shown in Table 9: F2oPAO and F3PAO both show inhibitory effects on the tumor cells tested with similar inhibitory IC₅₀. Both demonstrated the strongest inhibitory effects on glioblastoma, neuroblastoma, acute lymphoid T-cell leukemia, acute non-B or non-T-cell lymphoma, acute lymphoid B-cell leukemia, malignant embryonal rhabdomyosarcoma, and adenocarcinoma of the endometrium, with IC₅₀s of less than 50 nM; relatively strong inhibitory effect on acute myeloid leukemia cells, with IC₅₀ of 50-100nM; weakest inhibitory effect on medullary thyroid carcinoma and Cerebral astrocytoma cells, IC₅₀>100 nM.

**Table 10. Comparison of fluorinated compounds in inhibiting tumor cell proliferation.**

| Cell line | | F2oPAO | F2mPAO | F3PAO | Staurosporine |
|---|---|---|---|---|---|
| | | IC₅₀(n M) | IC₅₀(nM) | IC₅₀(nM) | IC₅₀(nM) |
| HeLa | Cervical cancer | 105.12 | 126.27 | 113.40 | 13.5 |
| SK-HEP-1 | Liver cancer | 42.98 | 50.43 | 60.43 | 16.9 |
| MCF7 | Breast cancer | 101.80 | 116.52 | 150.03 | 39.4 |
| MDA-MB-231 | Breast cancer | 101.26 | 46.02 | 150.36 | 13.4 |
| A549 | Lung cancer | 126.86 | 160.27 | >200 | 13.1 |
| NCI-H1299 | Lung cancer | 99.93 | 146.70 | 165.32 | 9.2 |
| HT-29 | Colon cancer | 92.66 | 139.68 | 155.07 | 15.5 |
| A-431 | Skin cancer | 139.58 | >200 | >200 | 21.1 |
| NCIN87 [N87] | Gastric cancer | 80.89 | 117.15 | 110.96 | 10.6 |
| HCC827 | Lung cancer | 95.68 | 144.27 | 155.97 | 16.1 |
| EL4 | T cell lymphoma | 49.83 | 65.58 | 68.83 | 24 |
| Daudi | Blood cancer | 40.84 | 59.16 | 69.53 | 15.5 |
| U-2 OS | Bone cancer | 29.23 | 42.36 | 44.92 | 5.3 |
| Jurkat, Clone E6-1 | Blood cancer | 72.48 | 74.96 | 105.05 | 21.4 |
| K-562 | Myeloma | 48.59 | 59.32 | 86.50 | 47 |
| HL-60 | Blood cancer | 53.72 | 48.23 | 64.85 | 24.5 |
| SK-OV-3 | Ovarian cancer | 112.52 | 159.35 | 185.11 | 28 |
| U-937 | Lymphoma | 86.74 | 139.53 | 120.58 | 13.9 |
| NAMALWA | Blood cancer | 31.27 | 39.30 | 48.80 | 19.9 |
| KG-1 | Myeloma | 84.22 | 85.27 | 118.49 | 15.6 |
| A-375 | Melanocarcinoma | 29.62 | 41.73 | 45.57 | 18 |

As shown in Table 10: The three fluorinated compounds all showed inhibitory effects on the tumor cells tested with similar inhibitory IC₅₀s, while F2oPAO had a slightly lower IC₅₀. All three demonstrated the strongest inhibitory effects on U2-OS, NAMALWA and A-375 cells, with IC₅₀s of less than 50 nM; relatively strong inhibitory effect on SK-HEP-1, Daudi, K562, HL-60, Jurkat, Clone E6-1, NAMALWA cells, with IC₅₀ of 50-100nM; weakest inhibitory effect on A-431 cells. The IC₅₀ for the remaining cells was in the range of 100-200 nM.

### Example 12. Effect of downregulation of PI4KIIIα and the PI4KIIIα inhibitors PAO and F3PAO on cellular tissue damage caused by radiation exposure

6 - 8-week-old, 20 - 23g C57BL/6 mice were to be used for this experiment. The mice were grouped according to their body weight. After grouping, the mice were anesthetized and then given a one-time total body irradiation of 15 Gy using X-rays to mice in the G2 - G4 groups. The day of modeling was defined as Day 0. Peripheral blood was collected from all animals for leukocyte counting on the day of grouping, 1, 3, 5, 7 days after irradiation, and on the day of sampling. Subsequently, mice were weighed weekly, and salivary secretion was examined.

### Experimental materials and methods

Compound to be tested: Phenylarsine oxide (PAO) and fluorinated phenylarsine oxide (F3PAO)

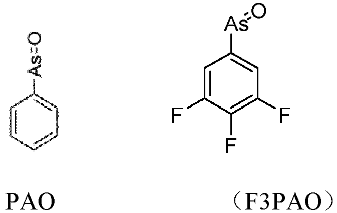

### Preparation of compound to be tested

Since the test compound is sensitive to light, the experimental process was carried out with care to avoid strong light.
a. Preparation of F3PAO mother liquor (0.9 mg/mL)
   Under low light, 9 mg of F3 powder was weighed on weighing paper, put into a brown glass bottle, and added into 10 mL of solvent medium-chain fatty acid (MCT); a clean stirrer was put in the mixture, and the mixture was mixed using ultrasonic for 1 - 5 minutes away from light. The solution was magnetic stirred for 1 - 2 hours in a water bath (not higher than 50°C) away from light until the solution was turned clear and transparent. The mother liquor was stored under the following conditions: stored under refrigeration (2 - 8°C) away from light for one year.
b. Preparation of F3PAO (0.15 mg/kg/day) working solution (60 mL) 0.015 mg/mL:
   Under low light, 1.0 mL of F3 mother liquor was taken, added with 59 mL of solvent (MCT) and mixed. The solution was stored under refrigeration (2 - 8°C) away from light for one year.
c. Preparation of PAO mother liquor (0.9 mg/mL)
   Under low light, 9 mg of PAO powder was weighed on weighing paper, put it into a brown glass bottle, and added into 10 mL of solvent Medium chain triglycerides (MCT) (Beijing Fengli Jingqiu Pharmaceutical Co., Ltd.); a clean stirrer was put in the mixture, and the mixture was mixed using ultrasonic for 1 - 5 minutes away from light. The solution was magnetic stirred for 1 - 2 hours in a water bath (not higher than 50°C) away from light until the solution was turned clear and transparent. The mother liquor was stored under the following conditions: stored under refrigeration (2 - 8°C) away from light for one year.
d. Preparation of PAO (0.3 mg/kg/day) working solution (90 mL) 0.03 mg/mL:
   Under low light, 3.0 mL of PAO mother liquor was taken, added with 87 mL of solvent (MCT) and mixed. The solution was stored under refrigeration (2 - 8°C) away from light for one year.

### Animals, grouping and dosing regimen

A total of 44 C57BL/6 mice, males, of which 4 were spare animals, were purchased from Shanghai Jihui. After acclimatization in the PharmaLegacy SPF animal house for 3 days, the animals in the appropriate weight range were selected for grouping. After grouping, the remaining spare animals were euthanized by receiving excess carbon dioxide at the endpoint of the experiment. The animals are free of specific pathogens and arrive at the PharmaLegacy animal house at approximately 6 - 8 weeks. The experimental protocols designed to be applied to the animals will be approved by the PharmaLegacy IACUC (Experimental Animal Care and Use Committee).

Clean-grade environment-cultured PI4KA (encoding PI4KIIIα) mutant heterozygous mice [(Pi4kaGt(RRO073)^{Byg/+}] mice (MMRRC, Cat. #016351-UCD) of approximately 7 months of age and their littermates in the wild-type were used for the PI4K^{-/+} group and the control group, respectively.

Animals were randomly assigned to each treatment group based on their body weights by the BioBook random assignment function to approximate the mean weight of each group and to reduce inter-group bias. Grouping and dosing information is detailed in the following table:

### Grouping and dosing regimen

| Group^{a} | X-ray irradiation | Gavage | Animal number | X-ray (Gy/mouse) | Concentration mg/mL | Gavage parameters^{a} | |
|---|---|---|---|---|---|---|---|
| | | | | | | mL/kg | mg/kg |
| Normal group | No | N/A | 10 | N/A | N/A | 10 | N/A |
| Model-vehicle group | Yes | MCT | 10 | 15 | N/A | 10 | N/A |
| PAO group | Yes | PAO | 10 | 15 | 0.03 | 10 | 0.3 p.o., qd |
| F3PAO group | Yes | F3PAO | 10 | 15 | 0.015 | 10 | 0.15 p.o., qd |

| | | | | | | | |
|---|---|---|---|---|---|---|---|
| a. Dosing began on the day of grouping and continued for 2 weeks. | | | | | | | |

### X-ray irradiation

The mice were anesthetized for 4 h. Blood was taken for subsequent blood leukocyte testing, and then each group of mice was given a 15 Gy dose of irradiation using X-rays. The day of modeling was defined as Day 0.

### X-ray post-irradiation inspection

**Body weight measurement:** body weight was measured twice a week before X-ray irradiation and three times a week for 3 weeks after irradiation;
**Salivary secretion:** C57BL/6 mice were grouped and salivary secretion was measured by collecting saliva within 10 minutes after injection of pilocarpine (Aladdin, Lot No. D1929095, Catalog No. P129614) on day -1 before modeling, and on days 7, 14 and 90 after modeling, respectively;
**Leukocyte count:** after the animals received 2 - 5% isoflurane anesthesia, 50 microliters of whole blood was collected from the orbits of all the animals, anticoagulated with EDTA-K2, and routine leukocyte counts were measured using a routine blood instrument. Routine blood counts were performed on the day of grouping before dosing, 4 hours after dosing and before irradiation, and on days 3, 5, 7, 14, 30, 60 and 90 after irradiation.
**Sample collection:** at the endpoint of the test (day 90 post-irradiation), after anesthesia with 2 - 5% isoflurane, the largest amount of whole blood was collected from the orbits of all animals, anticoagulated with EDTA-K2, and the plasma was separated by centrifugation using a centrifuge at 1000 g for 10 min at 4°C, and stored at -80°C for later use. After blood samples were collected, mice received transcardial perfusion with pre-cooled PBS and 4% paraformaldehyde, and then tissues and organs such as salivary glands, lung tissues, etc. were stripped and collected, photographed and weighed, and then re-fixed with formalin in vitro, dehydrated, and embedded in paraffin wax for subsequent pathology testing.
**Clinical observation:** during the test, if the animals showed significant weight loss (more than 15%) or other abnormalities, agar and glucose saline (10 mL/kg, subcutaneous injection, 1 - 2 times a day) were given promptly, and the fluid infusion was recorded.

### Statistical analysis

Results would be presented as Mean ± SEM. Tests will be performed using the appropriate statistical methods. A statistically significant difference will be considered if P < 0.05.

### Results

As shown in Table 11, on day 7 after X-ray irradiation, the salivary weight and volume of the model-vehicle group significantly decreased compared with those of the normal group, while those of the PAO group had no significant change and are significantly higher than those of the model-vehicle group; the salivary volume of the F3PAO group had no significant change but the salivary weight had a significant decrease, but all of them tended to increase compared with those of the model-vehicle group. On day 7 after X-ray irradiation, compared with their own pairs on day -1, the salivary weight and volume of each animal did not decrease in the normal group and both had a significant increase; there was a large and significant decrease in the model-vehicle group; there was no significant change in the PAO group; and there was a significant decrease in the F3PAO group but the magnitude was relatively small. These results suggested that PAO and F3PAO treatments could attenuate the damage to salivary glands and the decrease in salivary secretion caused by radioactive irradiation.

As shown in Table 11, the leukocyte levels in the blood of the model-vehicle group, the PAO group and the F3PAO group decreased significantly compared with those of the normal group at 3, 5 and 7 days after X-ray irradiation, with the lowest level at day 3, and then recovering gradually at days 5 and 7. However, on days 3 and 5, the leukocyte levels in the blood of the PAO group and F3PAO group significantly increased or tended to increase compared with those in the model-vehicle group; on day 7, there was no significant difference in the leukocyte levels in the blood of the model-vehicle group, PAO group and F3PAO group. These results suggested that PAO and F3PAO treatments could attenuate the decrease of leukocytes caused by radioactive irradiation.

As shown in Table 11, the body weights of the model-vehicle group, PAO group and F3PAO group significantly decreased compared to those of the normal group on days 3, 5 and 7 after X-ray irradiation, with the lowest on day 5 and some recovery on day 7. However, on days 3 and 5, the body weights of the PAO group and the F3PAO group significantly increased or tended to increase compared with the body weights of the model-vehicle group. These results suggested that PAO and F3PAO treatments attenuated the weight loss caused by radioactive irradiation.

Since the dose of PAO given to the PAO group was 0.3 mg/kg/day and the dose of F3PAO given to the F3PAO group is 0.15 mg/kg/day, and since the molecular weight of F3PAO was 30% higher than that of PAO, the molar amount of the drug given to the F3PAO group was only 35% of that given to the PAO group, the above results could not indicate that the anti-radioactive ray-induced damage effect of PAO was stronger than F3PAO.

**Table 11. Effects of PAO and F3PAO on cellular tissue damage caused by X-ray irradiation**

| Time | Test item | Normal group (10 mice) | Model-vehicle group (10 mice) | PAO group (10 mice) | F3PAO group (10 mice) |
|---|---|---|---|---|---|
| Day -1 | Leukocyte count: (10^9/L) | 8.75 ± 0.6 | 9.36 ± 0.71 | 10.17 ± 0.82 | 9.95 ± 0.74 |
| | Body weight (g) | 20.97 ± 0.19 | 20.89 ± 0.21 | 20.89 ± 0.19 | 20.94 ± 0.19 |
| | Salivary weight (g) | 0.147 ± 0.006 | 0.148 ± 0.004 | 0.146 ± 0.003 | 0.145 ± 0.002 |
| | Salivary volume (µL) | 95.3 ± 5.6 | 93.4 ± 3.5 | 92.0 ± 2.9 | 93.2 ± 2.3 |
| Day 0 | Leukocyte count: (10^9/L) | 8.25 ± 0.73 | 6.53 ± 0.45 | 8.67 ± 0.85 | 8.67 ± 0.54 |
| Day 3 | Leukocyte count: (10^9/L) | 8.05 ± 0.46 | ^{###}0.42 ± 0.02 | ^{###}0.71 ± 0.09* | ^{###}0.49 ± 0.07 |
| | Body weight (g) | 20.42 ± 0.17 | ^{###}18.82 ± 0.23 | ^{###}19.23 ± 0.19 | ^{###}19.4 ± 0.17 |
| Day 5 | Leukocyte count: (10^9/L) | 9.93 ± 0.54 | ^{###}1.09 ± 0.1 | ^{###}1.44 ± 0.13* | ^{###}1.15 ± 0.07 |
| | Body weight (g) | 20.71 ± 0.21 | ^{###}18.58 ± 0.18 | ^{###}18.74 ± 0.23 | ^{###}19.22 ± 0.19* |
| Day 7 | Leukocyte count: (10^9/L) | 10.03 ± 0.52 | ^{###}1.36 ± 0.11 | ^{###}1.39 ± 0.14 | ^{###}1.36 ± 0.13 |
| | Body weight (g) | 21.15 ± 0.2 | ^{###}19.13 ± 0.24 | ^{###}19.31 ± 0.24 | ^{###}19.2 ± 0.28 |
| | Salivary weight (g) | 0.178 ± 0.014^{Y} | ^{###}0.089±0.01^{Y YY} | 0.142 ± 0.013** | 0.107 ± 0.016 ^{Y} |
| | Salivary volume (µL) | 127.0 ± 12.3 ^{Y} | ^{###}47.1 ± 6.3 ^{Y YY} | 97.6 ± 11.5** | ^{##}64.3 ± 11.8 ^{Y} |
| Day 14 | Leukocyte count: (10^9/L) | 8.66 ± 0.73 | ^{###}1.13 ± 0.11 | ^{###}1.09 ± 0.15 | ^{###}1.17 ± 0.13 |
| | Body weight (g) | 22.44 ± 0.29 | ^{###}20.41± 0.25 | ^{###}20.33 ± 0.28 | ^{###}20.29 ± 0.29 |
| | Salivary weight (g) | 0.21 ± 0.02^{Y} | ^{###}0.09 ± 0.02 | ^{#}0.14 ± 0.01*** | ^{##}0.13 ± 0.01* |
| | Salivary volume (µL) | 155.52±22.44 ^{Y} | ^{###}44.73 ± 3.75 | ^{#}94.33± 9.12*** | ^{##}81.99±12.06* ** |

T-test, "^{###}"= P<0.001 and "^{#}"= P<0.05 (compared with normal group), "*"= P<0.1 and "**"= P<0.01 (compared with model-vehicle group), "^{YYY}"= P<0.001 and "^{Y}"= P<0.05 (compared with its own pairs on day -1)_{∘}

### Example 13. Effect of PAO on atherosclerotic foam cell formation

Atherosclerosis is the most common type of cardiovascular disease, characterized by the formation of atheroma or fibrous plaque in the endothelium of blood vessels, which mainly involves the large arteries and middle arteries, resulting in hardening of the wall, narrowing of the lumen and weakening of elasticity, causing ischemic changes in the corresponding organs. Foam cell formation occurs throughout the development of atherosclerosis and is the hallmark of atherosclerosis. Foam cell formation is caused by the imbalance of three major interrelated biological processes: lipid uptake, cholesterol esterification and cholesterol efflux. Increased lipid uptake and cholesterol esterification and inadequate cholesterol efflux result in excessive accumulation of cholesteryl esters (CE) in macrophages, resulting in the formation of foam cells.

The most central aspect of the pathomechanism of atherosclerosis is mediated by macrophages. As blood low-density lipoproteins (LDL) pass through gaps in the arterial endothelium or through active transport by vascular endothelial cells, they are deposited sub-endothelially and oxidized to produce oxidized low-density lipoproteins (ox-LDL). Subendothelial ox-LDL can bind to receptors on macrophage membranes (e.g., SR-A, CD36, LOX-1) and then be endocytosed into the cell, where it is degraded by lysosomal acid lipase to produce cholesteryl esters. Cholesteryl esters can further generate free cholesterol and free fatty acids. Excess free cholesterol has toxic effects on cells. Free cholesterol is in turn re-esterified to cholesteryl esters in the endoplasmic reticulum by acetyl coenzyme A acetyltransferase 1 (ACAT1) and acetyl coenzyme A acetyltransferase 2 (ACAT2), resulting in the formation of lipid droplets present in large quantities in foamy cells, which can be revealed by oil red O staining. Macrophage-derived foam cells can form when the cholesteryl ester content in macrophages exceeds 50% of the total cholesterol content. As the disease progresses, smooth muscle cells from the arterial intima migrate into the intima via the endoelastic membrane window pore and phagocytose lipids to form myogenic foam cells. Smooth muscle cells proliferate and migrate and synthesize extracellular matrix to form a fibrous cap. Foam cells necrolyze and disintegrate to form atheromatous necrotic material, and an atheromatous plate is formed.

### 1. PAO and its derivatives reduced lipid droplet formation after uptake of oxidized low-density lipoprotein by macrophages.

### Experimental methods:

1) Cell culture: THP-1 cells were purchased from the Shanghai Cell Bank of the Chinese Academy of Sciences, placed in a 37°C, 5% CO2 incubator using RPMI 1640 (Gibco C11875500BT) + 10% FBS (Gibco 10099141C), and passaged every 3 days.
2) Induction of differentiation: Log phase THP-1 cells were taken, blown and mixed thoroughly, and PMA (Sigma P1585) was added to the medium so that the final concentration of PMA was 100 nM. The cell suspension was added to 24-well plates and incubated at 37°C for 72 h. Subsequently, the cells were observed under the microscope, and the cells changed from the suspended state to the wall adhesion state and extended pseudopods.
3) Grouping and treatment: After induced differentiation, the cells were randomly divided into six groups, Control group, Model group, Model+10 nM PAO, Model+20 nM PAO, Model+40 nM PAO, Model+60 nM PAO. The Control group was incubated with serum-free medium for 2 days, and the Model group was incubated with serum-free medium containing 60 ug/ml ox-LDL (Guangzhou Yiyuan Bio, YB-002) for 2 days. The drug treatment group was cultured with serum-free medium containing 60 ug/ml ox-LDL for 1 day, and then changed to serum-free medium containing 60 ug/ml ox-LDL and different concentrations of PAO to continue the culture for 1 day.
4) Oil red O staining: after the end of treatment, the cells were rinsed twice with PBS, added with 4% paraformaldehyde solution for fixation of 30 minutes at room temperature, and rinsed twice with PBS after the fixation. Subsequently, the cells were stained using a modified oil red O kit (Byotime C0158S). The cells were covered with added staining wash solution for 20 seconds. The staining wash solution was removed; Oil Red O Staining Working Solution was added; and the cells were stained for 30 minutes at room temperature; and then the Oil Red O Staining Working Solution was removed. An appropriate amount of Staining Wash Solution was added; the cells were rested for 30 seconds; then the Staining Wash Solution was removed; and the cell was washed with PBS for 20 seconds. The cells were evenly covered with PBS added, observed and photographed under microscope.

### Results:

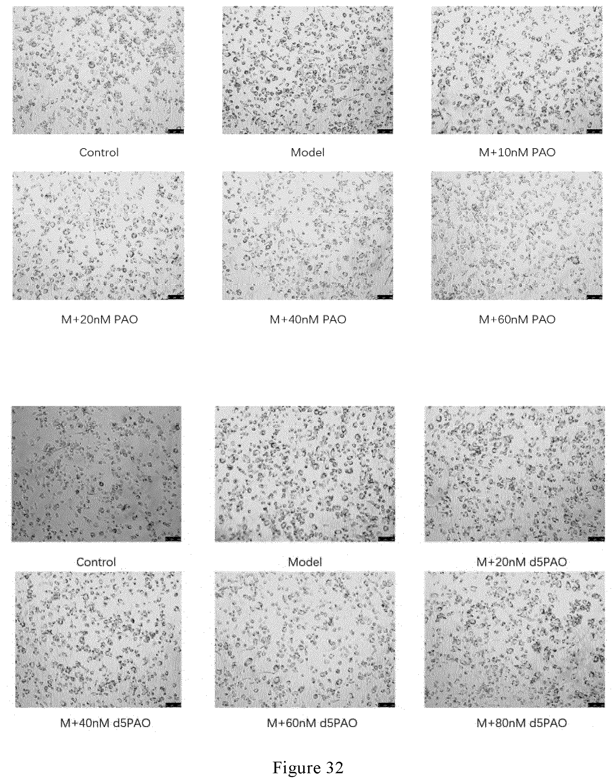

As shown in Figure 32, the signal of oil red O staining in THP-1 cells differentiated by ox-LDL treatment (model) was stronger than that in cells not treated with ox-LDL (control), suggesting intracellular lipid droplet accumulation, and this intracellular lipid droplet accumulation could be markedly attenuated by PAO in a dose-dependent manner (M+PAO). F2mPAO and F3PAO also had the same function.

### 2. PAO and its derivatives promoted clearance of fluorescently labeled oxidized low-density lipoproteins taken up by macrophages

### Experimental methods:

1) Cell culture: THP-1 cells were purchased from the Shanghai Cell Bank of the Chinese Academy of Sciences, placed in a 37°C, 5% CO2 incubator using RPMI 1640 (Gibco C11875500BT) + 10% FBS (Gibco 10099141C), and passaged every 3 days.
2) Induction of differentiation: Log phase THP-1 cells were taken, blown and mixed thoroughly, and PMA (Sigma P1585) was added to the medium so that the final concentration of PMA was 100 nM. The cell suspension was added to 24-well plates with cell crawlers and incubated at 37°C for 72 h. Subsequently, the cells were observed under the microscope, and the cells changed from the suspended state to the wall adhesion state and extended pseudopods.
3) Grouping and treatment: After induced differentiation, the cells were treated with serum-free medium for 18 hrs and then randomly divided into four groups: Dil-ox-LDL group, PAO 20 nM group, PAO 40 nM group, PAO 60 nM group. The Dil-ox-LDL group was incubated with serum-free medium containing 40 µg/mL Dil-ox-LDL (Guangzhou Yiyuan Bio, YB-010) for 7 hrs. The drug treatment group was cultured with serum-free medium containing 40 ug/ml Dil-ox-LDL for 6 hrs, and then different concentrations of PAO were added to the medium and incubation was continued for 1 hour.
4) Fixation and section: After the treatment, the cells were rinsed twice with PBS, added with 4% paraformaldehyde solution for fixation of 30 minutes at room temperature, and rinsed twice with PBS after the fixation. The cells were stained with DAPI staining solution for 10 minutes at room temperature and rinsed twice with PBS after the staining. The crawls were removed from the 24-well plate for section.
5) Laser confocal microscope observation and photography.

### Results:

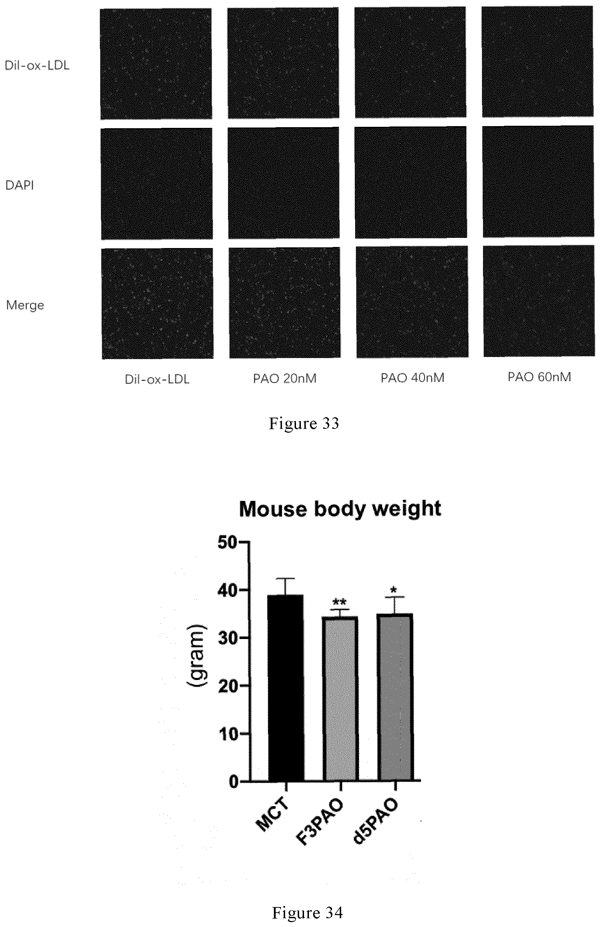

As shown in Figure 33, THP-1 cells could take up a large amount of fluorescently labeled ox-LDL (Dil-ox-LDL), and PAO promoted the clearance of Dil-ox-LDL in THP-1 cells in a dose-dependent manner.

### Example 14. In vitro bacteriostatic activity of PAO and its derivatives

The strains to be tested as well as the tests were provided and performed by WuXi AppTec Shanghai. The tested compounds PAO, d5PAO and F3PAO were prepared as 64 µg/mL mother liquor, and then added to the petri dishes of pathogenic bacteria by gradient dilution. The in vitro Minimum inhibitory concentration (MIC) of the compounds against pathogenic bacteria refers to the standard method provided by the Clinical & Laboratory Standards Institute (CLSI). In vitro drug susceptibility testing for aerobic bacteria refers to CLSI M07, M100; in vitro drug susceptibility testing for anaerobic bacteria refers to CLSI M11, M100; in vitro drug susceptibility testing for Helicobacter pylori refers to CLSI M100; in vitro drug susceptibility testing for fungi refers to CLSI M27, M38, M60; and MIC testing for Trichomonas vaginalis refers to the reference (Mohammad MATINI & Amir Hossein MAGHSOOD.2016. In Vitro Susceptibility of Iranian Isolates of Trichomonas vaginalis to Metronidazole. Iran JParasitol. Vol. 11, No. 1, Jan Mar 2016, pp. 46-51*.* UPCROFT, J.A. & UPCROFT P. 2001. Drug susceptibility testing of anaerobic protozoa. Antimicrob Agents Chemother, 45,. 1810-4*.)*

Microfluidic dilution method: bacterial growth was observed visually after incubation in media containing graded dilution compounds (e.g., ion-regulated Miller-Hinton broth media). The lowest compound concentration point, at which bacterial growth was significantly or completely inhibited by visual observation, was defined as the minimum inhibitory concentration of the compound.

Agar dilution method: Bacterial growth was observed visually after incubation in solid media plates containing graded dilutions of the compounds (e.g., Brucella solid medium supplemented with 5 µg/mL hemin, 1 µg/mL vitamin K1, and 5% aseptic defibrillated lysed horse blood). The lowest compound concentration point, at which bacterial growth was significantly or completely inhibited by visual observation, was defined as the minimum inhibitory concentration of the compound.

Different strains of broad-spectrum antimicrobials that are routinely used in clinical practice are used as positive controls. Linezolid is an antibacterial agent that inhibits infections caused by Gram-positive (G+) cocci; colistin has a broad antibacterial spectrum, especially against Gram-negative bacteria, and is less toxic. Voriconazole is a triazole antifungal compound that acts on severe fungal infections. Ciprofloxacin is a quinolone antibacterial drug with broad-spectrum antibacterial activity against Enterobacteriaceae, Pseudomonas aeruginosa, Haemophilus influenzae, Gonococcus gonorrhoeae, Streptococcus, Legionella, Staphylococcus aureus. The antibacterial spectrum of Clarithromycin is similar to that of Erythromycin. Clindamycin is clinically used for abdominal and gynecological infections caused by anaerobic bacteria, and is the drug of first choice for the treatment of Staphylococcus aureus osteomyelitis. Metronidazole is mainly used for the treatment or prevention of systemic or localized infections caused by anaerobic bacteria, and is also effective in sepsis, endocarditis, meningeal infections, and colitis caused by the use of antibiotics.

### Results:

**Table 12. Minimum Inhibitory Concentration (MIC) (µg/mL) of d5PAO, PAO, and F3PAO against Pathogenic Bacteria**

| **Name and strain number of the resistant strain** | ***d5P AO** | **PAO** | **F3P AO** | **Linezolid** | **Colistin** | **Voricona zole** | **Ciproflox acin** |
|---|---|---|---|---|---|---|---|
| *Enterococcus faecalis ATCC 51575* | 0.125 | 0.125 | 0.25 | 2 | - | - | - |
| *Enterococcus faecium ATCC 700221* | 0.125 | 0.25 | 0.25 | 4 | - | - | - |
| *Staphylococcus aureus NRS 384* | <0.06 25 | <0.00 2 | <0.00 2 | 4 | - | - | - |
| *Staphylococcus aureus VRS1* | <0.00 2 | <0.00 2 | <0.00 2 | 4 | - | - | - |
| *Staphylococcus epidermidis ATCC 35983 (* | <0.00 2 | <0.00 2 | <0.00 2 | 2 | - | - | - |
| *Acinetobacter baumannii ATCC-BAA-160 5* | 2 | 1 | 0.5 | - | 0.5 | - | - |
| *Escherichia coli ATCC BAA-2469* | <0.06 25 | <0.06 25 | 0.125 | - | 0.25 | - | - |
| *Klebsiella pneumoniae ATCC BAA-1705* | 0.5 | 0.25 | 1 | - | 0.5 | - | - |
| *Enterobacter cloacae ATCC BAA-1143* | 1 | 1 | 1 | - | 0.25 | - | - |
| *Pseudomonas aeruginosa NCTC 13437* | 0.25 | 0.25 | 1 | - | 0.5 | - | - |
| *Candida albicans ATCC MYA-2876* | 2 | 2 | 0.5 | - | - | 0.03 | - |
| *Candida albicans ATCC-MYA-574* | 2 | 2 | 1 | - | - | 1 | - |
| *Candida parapsilosis ATCC 22019* | 1 | 1 | 1 | - | - | 0.03 | - |
| *Aspergillus fumigatus ATCC MYA-4609* | 8 | 4 | 2 | - | - | 1 | - |
| *Neisseria gonorrhoeae ATCC 49226* | <0.06 25 | <0.06 25 | 0.125 | - | - | - | 0.008 |
| | | | | | | | |

| **Name and strain number of the resistant strain** | ***d5P AO** | **PAO** | **F3P AO** | **Voricona zole** | **Clarithrom ycin** | **Clindam ycin** | **Metronida zole** |
|---|---|---|---|---|---|---|---|
| *Cryptococcus neoformans ATCC 208821* | 0.06 | 0.06 | 0.016 | 0.03 | - | - | |
| *Helicobacter pylori ATCC 43504* | 0.125 | 0.25 | 0.25 | - | 0.03 | - | |
| *Helicobacter pylori ATCC 700392* | 0.125 | 0.125 | 0.25 | - | 0.03 | - | |
| *Helicobacter pylori ATCC 700824* | 0.25 | 0.25 | 0.25 | - | 0.03 | - | |
| *Bacterioides fragilis ATCC 25285* | 0.125 | 0.125 | 0.25 | - | - | 1 | |
| *Clostridium difficile ATCC 700057* | 0.5 | 0.5 | 0.5 | - | - | >2 | |
| *Clostridium tetani ATCC 17891* | 0.125 | 0.125 | 0.25 | - | - | 0.03 | |
| *Gardnerella vaginalis (Gardner and Dukes) ATCC 14018* | 0.125 | 0.125 | 0.25 | - | - | 0.125 | |
| *Propionibacteriu m αcnes (Cutibacterium acnes) ATCC 11827* | 0.125 | 0.125 | 0.25 | - | - | 0.0625 | |
| *Atopobium vaginae Rodriguez Jovita et al. ATCC BAA-55* | 0.006 | 0.003 | 0. 0 06 | - | - | 0.008 | |
| *Peptostreptococ cus anaerobius ATCC 27337* | 0.25 | 0.25 | 0.25 | - | - | 0.5 | |
| *Trichomonas vaginalis ATCC 30236* | 0.25 | 0.25 | 0.125 | - | - | - | 0.25 |
| | | | | | | | |

| **Name and strain number of the resistant strain** | **d5PA O** | **PAO** | **F3P AO** | **Colistin** | **Levofloxac in** | | |
|---|---|---|---|---|---|---|---|
| *Escherichia coli ATCC BAA-2469* | 0.062 5 | 0.062 5 | 0.25 | 0.5 | 16 | | |
| *Acinetobacter baumannii WX-AB003* | 0.125 | 0.125 | 0.25 | 0.5 | 16 | | |
| *Acinetobacter baumannii WX-AB004* | 0.25 | 0.125 | 0.25 | 1 | 0.125 | | |
| *Acinetobacter baumannii WX-AB006* | 0.5 | 0.5 | 0.5 | 0.5 | 0.25 | | |
| *Acinetobacter baumannii WX-AB019* | 0.25 | 0.125 | 0.25 | 0.25 | 1 | | |
| *Citrobacter* | <0.06 | <0.06 | 0.25 | 0.25 | >16 | | |
| *kirschner WX-C.kirschner 001* | 25 | 25 | | | | | |
| *Enterobacter cloacae WX-E.cloαcαe00 1* | <0.06 25 | 0.125 | 0.25 | 0.25 | 0.0625 | | |
| *Enterobacter cloacae WX-E.cloαcαe00* 2 | 2 | 2 | 2 | 0.25 | 0.03125 | | |
| *Enterobacter cloacae WX-E.cloαcαe00* 3 | 0.125 | 0.125 | 0.25 | 0.25 | 0.5 | | |
| *Escherichia coli WX-EC008* | <0.06 25 | 0.125 | 0.125 | 0.5 | 16 | | |
| *Escherichia coli WX EC018* | <0.06 25 | <0.06 25 | 0.25 | 0.25 | 16 | | |
| *Escherichia coli WX-EC021* | 1 | 1 | 1 | 0.25 | 16 | | |
| *Escherichia coli WX-EC022* | 0.125 | 0.125 | 0.25 | 0.25 | 0.5 | | |
| *Klebsiella oxytoca WX-K.oxytoca001* | 0.125 | 0.125 | 0.5 | 0.25 | 16 | | |
| *Klebsiella oxytoca WX-K.oxytoca00* 2 | 0.125 | 0.125 | 0.5 | 0.25 | 0.5 | | |
| *Klebsiella oxytoca WX-K.oxytoca003* | 0.125 | 0.125 | 0.5 | 0.25 | 0.5 | | |
| *Klebsiella pneumoniae WX-KP013* | 0.5 | 0.5 | 1 | 0.5 | >16 | | |
| *Klebsiella pneumoniae WX-KP021* | 0.125 | 0.125 | 0.5 | 0.25 | 0.0625 | | |
| *Klebsiella pneumoniae WX-KP037* | <0.06 25 | 0.125 | 0.25 | 0.25 | >16 | | |
| *Klebsiella pneumoniae WX-KP041* | <0.06 25 | <0.06 25 | 1 | 0.25 | 1 | | |
| *Morganella morgani WX-M.morgani0 01* | 0.5 | 0.5 | 2 | >16 | 1 | | |
| *Pseudomonas aeruginosa WX-PA008* | 0.5 | 0.5 | 4 | 1 | >16 | | |
| *Pseudomonas aeruginosa WX-PA013* | 0.5 | 0.5 | 2 | 1 | 1 | | |
| *Pseudomonas aeruginosa WX-PA015* | 0.5 | 0.5 | 1 | 1 | 1 | | |
| *Pseudomonas aeruginosa WX-PA025* | 1 | 1 | 32 | 1 | 8 | | |
| *Serratia marcescens WX-S.marcescen s001* | 0.5 | 0.5 | 4 | >16 | 0.25 | | |
| | | | | | | | |

| **Strain name and strain number** | **d5PA O** | **PAO** | **F3P AO** | **Linezolid** | **Levofloxac in** | **Voricona zole** | |
|---|---|---|---|---|---|---|---|
| *Staphylococcus aureus WX-SA007* | <0.06 | <0.06 | <0.06 | 8 | - | - | |
| *Staphylococcus aureus WX-SA008* | <0.06 | <0.06 | <0.06 | 8 | - | - | |
| *Staphylococcus aureus WX-SA01 2* | <0.06 | <0.06 | <0.06 | 8 | - | - | |
| *Staphylococcus aureus WX-SA033* | <0.06 | <0.06 | <0.06 | 4 | - | - | |
| *Enterococcus faecalis WX-E.faecalis00 2* | 0.125 | 0.125 | 0.125 | 8 | - | - | |
| *Enterococcus faecalis WX-E.faecalis00 7* | 0.25 | 0.125 | 0.125 | 4 | - | - | |
| *Enterococcus faecalis WX-E.faecalis00 7* | 0.125 | 0.125 | <0.06 | 4 | - | - | |
| *Enterococcus faecalis WX-E.faecium01 0* | 0.25 | 0.25 | 0.25 | 4 | - | - | |
| *Streptococcus pneumoniae WX-SP001* | 0.125 | 0.125 | 0.125 | 1 | 1 | - | |
| *Streptococcus pneumoniae WX-SP002* | 0.125 | 0.125 | 0.25 | 1 | 1 | - | |
| *Candida albicans WX-CA007* | 64 | 64 | 8 | - | - | 0.0039 | |
| *Candida albicans WX-CA008* | 64 | 64 | 8 | - | - | 0.0078 | |
| *Candida albicans WX-CA009* | 64 | 64 | 8 | - | - | 0.5 | |
| *Candida albicans WX-CA011* | 64 | 64 | 8 | - | - | 0.5 | |
| | | | | | | | |

| **Strain name and strain number** | **d5PA O** | **PAO** | **F3P AO** | **Ciproflox acin** | **Clindamyc in** | | |
|---|---|---|---|---|---|---|---|
| *Neisseria gonorrhoeae ATCC 700825* | <0.06 | <0.06 | <0.06 | 0.008 | - | | |
| *Neisseria gonorrhoeae 35*/*02* | <0.06 | <0.06 | 0.125 | 1 | - | | |
| *Neisseria gonorrhoeae FA6140* | <0.06 | <0.06 | <0.06 | 0.03 | - | | |
| *Neisseria gonorrhoeae FA19* | <0.06 | <0.06 | <0.06 | 0.008 | - | | |
| *Neisseria gonorrhoeae ATCC 49226* | 0.03 | 0.03 | 0.06 | 0.008 | - | | |
| *Clostridium tetani ATCC 454* | 0.125 | 0.125 | 0.125 | - | 0.016 | | |
| *Clostridium tetani ATCC 10779* | 0.125 | 0.125 | 0.125 | - | 0.016 | | |
| *Clostridium tetani ATCC BAA-1870* | 1 | 1 | 1 | - | >2 | | |
| *Clostridium tetani ATCC 19406* | 0.125 | 0.125 | 0.125 | - | 0.016 | | |
| *Clostridium tetani ATCC 43255* | 0.5 | 0.5 | 1 | - | 0.5 | | |
| *Clostridium tetani ATCC BAA-1382* | 0.5 | 0.5 | 0.5 | - | >2 | | |
| *Propionibacteriu m acnesATCC 6919* | 0.5 | 0.5 | 0.5 | - | 0.06 | | |
| *Clostridium tetani ATCC 453* | 0.125 | 0.125 | 0.5 | - | 0.016 | | |

The results showed that PAO and its deuterium derivative, d5PAO, as well as the fluorinated derivative, F3PAO, showed inhibitory effects on the different kinds of strains tested, especially the drug-resistant strains. The minimum inhibitory concentration (MIC) was less than 8 µg/mL, and most of the MICs were in the range of 0.06-1 µg/mL, thus having a broad-spectrum inhibitory effect. Among the three, PAO and deuterated PAO showed similar or stronger inhibitory effects than fluorinated PAO.

In terms of inhibitory effects on certain bacteria, these compounds showed significant advantages over positive control antimicrobials, e.g., the minimum inhibitory concentration MIC of linezolid against Enterococcus faecalis, Enterococcus faecium, and Staphylococcus aureus was 2-4 µg/mL, whereas most of the MICs of PAO and its derivatives against these bacteria were less than or equal to 0.0625 µg/mL. Relative to colistin, these tested compounds also show a significant inhibitory advantage against E. coli. Relative to clindamycin, they also show strong advantage against Bacterioides fragilis, Clostridium difficile.

The PAO tested compounds also show good inhibitory effects against bacteria such as Acinetobacter baumannii, Klebsiella pneumoniae, Pseudomonas aeruginosa, Neisseria gonorrhoeae, Cryptococcus neoformans, Gardnerella vaginalis, Propionibacterium acnes, Peptostreptococcus anaerobius, as well as Trichomonas vaginalis, with effects similar to those of the control antimicrobials.

Finally, the tested compounds fail to show significant advantages against bacteria such as Enterobacter cloacae, Candida albicans, Helicobacter pylori, Clostridium tetani, and Atopobium vaginae.

In conclusion, PAO and its deuterated and fluorinated derivatives have broad-spectrum antipathogenic effects. They have a better inhibitory effect on many strains than the clinically widely used antimicrobials.

### Example 15. Regulation of d5PAO, PAO and F3PAO to Blood Glucose Levels/Body Weight in an Animal Model of Diabetes Mellitus

ApoE^{-/-} mice are one of the models for studying the formation of diabetes as well as its preventive treatment. Adult ApoE^{-/-} male mice of 8 weeks of age were used in this experiment (Saiye Bio). Medium-chain fatty acids MCT were used as solvent for PAO, d5PAO and F3PAO: PAO (0.015 mg/mL), d5PAO (0.015 mg/mL) and F3PAO (0.015 mg/mL). Mice were fed with a high-fat diet (Dyets, high fat (40 kcal%) high cholesterol (1.5%). ApoE^{-/-} male mice were randomly divided into 4 groups: the MCT blank control group (MCT, n=6), the PAO group (0.15 mg/kg/day, po, n=8), the d5PAO group (0.15 mg/kg/day, po, n=8), and the F3PAO group (0.05 mg/kg/day, po, n=7). There was no significant difference in mean body weight between the 4 groups. After that, ApoE^{-/-} mice in the 4 groups were given the same housing environment as well as dietary and drinking conditions. ApoE^{-/-} mice were administered by gavage at an dose of 0.2 mL, which was administered Monday through Friday each week and discontinued over the weekend. After 12 weeks of high-fat feeding and drug administration, mice were measured for body weight and blood glucose. For blood glucose measurement, blood was taken from the tail vein of the mice by clipping the mice with hand, and blood glucose was measured and recorded using Roche blood glucose test strips and a blood glucose meter, which was random blood glucose; After 24 hours of fasting (water supplied), the same technique was used to measure blood from the tail vein of mice clipped by hand, and blood glucose was measured and recorded with Roche blood glucose test strips and blood glucose meter, which was fasting blood glucose; after 24 hours of starvation, the high-fat diet was resumed uniformly, and 2 hours later, blood was taken from the tail vein of mice clipped by the same technique, and blood glucose was measured and recorded with Roche blood glucose test strips and blood glucose meter, which was the 2-hour postprandial blood glucose.

SPSS22.0 software was used for statistical analysis. Measurement data were expressed as x±s, and independent samples t-test was used for comparison between the two groups; count data were expressed as rate (%), and χ2 test was used for comparison between the groups. p < 0.05 was regarded as statistically different.

### Results:

### 1) F3PAO and d5PAO reduced the body weight of tested ApoE^{-/-} mice

As shown in Figure 34, the mean body weight of high-fat diet-fed APOE^{-/-}mice administered with F3PAO and d5PAO for 12 weeks was significantly lighter than that of the MCT group.

### 2) PAO reduced the body weight of tested ApoE^{-/-} mice

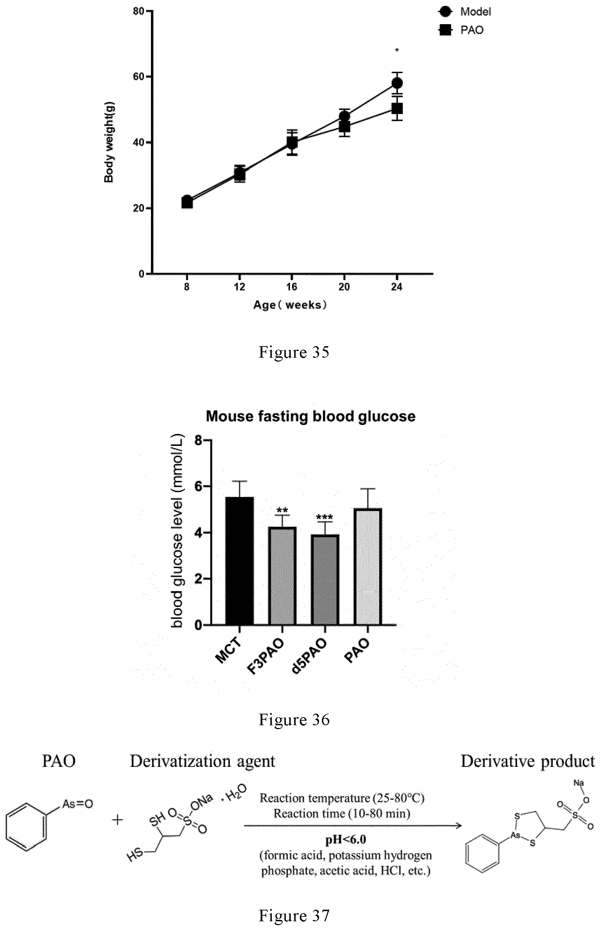

As shown in Figure 35, the mean body weight of high-fat diet-fed APOE^{-/-}mice administered with PAO for 20 weeks was significantly lighter than that of the MCT group.

### 3) PAO, F3PAO and d5PAO reduced blood glucose levels of ApoE^{-/-} mice

As shown in Figure 36, after 12 weeks of administration, the mean fasting blood glucose of the F3PAO and d5PAO groups was significantly lower than that of the MCT group, and the mean fasting blood glucose of the PAO group was also decreased compared with that of the MCT group.

The above results indicated that the weight gain of ApoE^{-/-} mice fed with high fat and high cholesterol was significantly inhibited by the intervention of PAO, F3PAO or d5PAO. PAO, d5PAO and F3PAO reduced or tended to reduce the random, postprandial and fasting blood glucose in ApoE3 knockout mice, and the reduction of fasting blood glucose by d5PAO and F3PAO was especially significant, with the potency of F3PAO being 3 times or more than that of d5PAO. Although not showing statistical significance, PAO also presented a trend of inhibitory effect on fasting blood glucose levels in high-fat diet-fed ApoE^{-/-} mice. In addition, there was evidence showing similar effects of these compounds on another animal model of diabetes, the Db/Db mouse.

### Example 16. Method for quantitative analysis of arsenic compounds in biological samples

Since there are still many arsenic-containing compounds in biological samples and environmental samples, there have been insurmountable difficulties in quantitatively analyzing arsenic compounds in these samples, especially organic arsenic compounds, such as phenylarsinic compound. First of all, some arsenic compounds are tightly bound to proteins, especially binding to proteins through covalent bonds (e.g., arsenic in compounds forms covalent bonds with the adjacent dithio group in the protein) leading to: on the one hand, the extraction rate is low; and secondly, in the liquid chromatography separation of organic arsenic compounds, the polarity of organic arsenic compounds, and the interference of a large number of unknown arsenic compounds in the samples make it difficult to achieve the separation of organic arsenic compounds. Thus, derivatization methods were adopted. In 2007 Cathum et al. improved the extraction rate by derivatizing the arsenic compounds in the samples with dimercaptopropanol and using high performance liquid chromatography (HPLC) for examination. But the resolution was only 1.0-2.0 µg/g (Cathum, 2007 #5256), Eom et al. in 2015 improved the resolution a little bit by derivatizing the arsenic compounds in samples with dimercaptotoluene (toluene-3,4-ditihol) and using HPLC for examination, the resolution was calculated based on the amount of arsenic in the arsenic compounds. The lower limit of quantitative analysis was about 0.06-0.21 µg/g (Eom, 2015 #3263).

The present disclosure employs sodium dimercaptopropanesulfonate (CnH₍₂ₙ₊₁₎NaO₃S₃) and others to derivatize the arsenic compounds in the samples, which greatly improves the resolution (see Figure 37). The lower limit of quantitative analysis for phenylarsine oxide (PAO) can be as low as 0.45 ng/mL by calculating the resolution based on the amount of arsenic in the arsenic compounds. However, the derivatization method has some drawbacks due to the fact that different compounds produce the same derivatization products upon derivatization, e.g., both PAO and phenylarsonic acid (PAA) may be derivatized by sodium dimercaptopropanesulfonate to produce the same derivatization products. Unexpectedly, in blood samples, sodium dimercaptopropanesulfonate could only derivatize PAO but not PAA. Thus, derivatization with sodium dimercaptopropanesulfonate allows for the high and specific detection of PAO in blood.

### I. Liquid chromatography-mass spectrometry (LC-MS/MS) method for the determination of PAO concentration in whole blood with EDTA-K2

This present application describes the partial validation of an analytical method for the determination of PAO concentration in whole blood of SD rats by liquid chromatography-mass spectrometry (LC-MS/MS). The results included linearity, intra- and inter-batch precision and accuracy, sensitivity, specificity, matrix effect, dilution reliability, extraction recovery, residuals, system suitability, and repeated injection stability of analytes in treated samples. The linear range of the method was 1.00 ng/mL - 500 ng/mL at 30 µL of SD rat whole blood, and the validation results demonstrated that the method is reliable and suitable for the determination of PAO concentration in the whole blood matrix of SD rats.

| **Methodology validation item** | **Methodological validation results** | **Cited table** |
|---|---|---|
| Description of analytical methods | A liquid chromatography-mass spectrometry (LC-MS) method was developed for the determination of PAO concentration in whole blood of SD rats. Tolbutamide was used as the internal standard, and the extraction method of protein precipitation in acetonitrile solution was adopted. The reverse phase chromatography column was ACQUITY UPLC^{®} Peptide C18 130 Å 2.1*100 mm I.D., 1.7 µm (Waters), electrospray negative ionization mode, and the ion pairs were m/z 336.8→152.9 for PAO and m/z 269.0→169.8 for internal standard. | - |
| Sample volume | 30 µL | - |
| Analyte | PAO | - |
| Internal standard | Tolbutamide | - |
| Linear range | 1.00 - 500 ng/mL | Table 14 |
| Lower limit of quantification (LLOQ) | 1.00 ng/mL | |
| Regression mode | Linear regression | Table 15 |
| Weighting factor | 1/x² | |
| Coefficient of determination (CoD) | R²≥0.9944 | |
| QC sample concentration | 3.00, 80.0 and 400 ng/mL | Table 16 |
| Intra-batch accuracy range (% deviation) | -15.5% -7.8% | |
| Intra-batch precision range (% coefficient of variation) | 4.1%-8.3% | |
| Inter-batch accuracy range (% deviation) | -5.9% -7.4% | |
| Inter-batch precision range (% coefficient of variation) | 4.6% -9.9% | |
| Dilution reliability | 4000 ng/mL, 10-fold dilution reliability verified | Table 17 |
| Specificity | No quantitatively interfering peaks at the retention times of analytes and internal standards in six different batches of blank whole blood matrices from SD rats | Table 18 |
| | No interference between analyte and internal standard in blank whole blood matrix of SD rats | Table 19 |
| Matrix effect | No significant matrix effect in whole blood of six different batches of SD rats | Table 20 |
| Extraction recovery (total % coefficient of variation) | 5.2% | Table 21 |
| Residues (%) | Analyte and internal standard residues meet acceptance criteria | Table 22 |
| Repeated injection stability of analytes in treated samples | Stable at 10°C for at least 53 hours | Table 23 |

### Abbreviations:

| | |
|---|---|
| LC-MS/MS | Liquid Chromatography Tandem Triple Quadrupole Mass Spectrometry |
| HPLC | High Performance Liquid Chromatography |
| V | volt |
| eV | electronvolt |
| psi | pounds per square inch |
| m/z | mass-to-charge ratio |
| msec. | millisecond |
| ACN | acetonitrile |
| MeOH | methanol |
| IPA | isopropanol |
| TFA | trifluoroacetic acid |
| FA | formic acid |
| DMSO | dimethyl sulfoxide |
| NH₄OAc | ammonium acetate |
| AR | analytically pure reagents |
| MΩ | megohm |
| mM | millimoles per liter |
| mol/L | mole per liter |
| µm | micron meter |
| µg/mL | micrograms per milliliter |
| v | volume |
| ng/mL | nanograms per milliliter |
| rpm | rotations per minute |
| g | gram |
| *g* | Centrifugal force |
| R² | coefficient of determination |
| MF | matrix factor |
| LLOQ | lower limit of quantification |
| ULOQ | upper limit of quantification |

The present disclosure establishes a liquid chromatography-mass spectrometry (LC-MS/MS) analytical method and partially validates the analytical method in accordance with the analytical research program "Methodological validation of the liquid chromatography-mass spectrometry (LC-MS/MS) method for the determination of PAO concentration in whole blood of SD rats".

The stability of the analytes in the stock solution and the working solution, as well as the stability of PAO in whole blood samples from SD rats under wet ice conditions, the stability of storage in an ultra-low temperature refrigerator, and the stability after 4 circles of freezings and thawings can be referred to the GLP BAS protocol of Suzhou.

### 1. Experimental materials and analytical methods

### 1.1 Reference standards and internal standards

| **Reference standard information** | |
|---|---|
| Name of reference standard | PAO |
| Molecular formula/Molecular weight | C₆H₅AsO/168.03 |
| Calibration factor | 1.005 |
| Source | Kaihui Pharmaceutical Shanghai Co., Ltd. |
| Batch number | SP-0020182-007 |
| Purity | 99.5% |
| Storage conditions | Sealed at room temperature |

| **Internal standard information** | |
|---|---|
| Name of internal standard | Tolbutamide |
| Molecular formula/Molecular weight | C₁₂H₁₈N₂O₃S/270.35 |
| Source | Sigma |
| Batch number | MKBR6717V |
| Purity | 99.9% |
| Storage conditions | Sealed, refrigerated and dry storage |

### 1.2 Reagents

| | **Test Reagent Information** | |
|---|---|---|
| | dimethyl sulfoxide / DMSO | HPLC Grade Reagent, Sigma, Batch No. STBH8372 |
| | acetonitrile / ACN | HPLC Grade Reagent, Merck, Batch No. JA083330 |
| | methanol / MeOH | HPLC Grade Reagent, Merck, Batch No. 11003307913 |
| | formic acid / FA | HPLC Grade Reagent, Honeywell Fluka, Batch No. I240DIL |
| | acetic acid / AA | HPLC Grade Reagent, Sigma, Batch No. SHBJ2597 |
| | isopropanol / IPA | HPLC Grade Reagent, Sigma, Batch No. WXBD0243V |
| | trifluoroacetic acid / TFA | HPLC Grade Reagent, Fluka, Batch No. I2550IL |
| | ammonium acetate / NH₄OAc | HPLC Grade Reagent, Sigma, Batch No. SLBV5241 |
| | test-used water / Water | ELGA PureLabUVF+OptionS15 (≥18.2MΩ), Shanghai WuXi AppTec in-house |

| **1.3 Preparation of blank matrix** | | |
|---|---|---|
| | **Blank matrix information** | |
| | Matrix | Blank EDTA-K₂ whole blood of SD rat |
| | Provider | Laboratory preparation by WuXi AppTec Shanghai |
| | Storage conditions | <-60°C |

### 1.4 Preparation of stock solution

Preparation of PAO stock solution: two appropriate amount of reference standards were accurately weighed, placed in a brown glass vial, and then prepared to form a control stock solution at a concentration of 1.00 mg/mL with DMSO. The stock solution was vortexed until completely dissolved and stored at -20°C in refrigeration. One portion was used as the calibration standard stock solution; the other portion was used as the quality control (QC) sample stock solution.

Preparation of tolbutamide internal standard stock solution: an appropriate amount of internal standard was accurately weighed and placed in a brown glass vial, and then prepared to form an internal standard stock solution at a concentration of 1.00 mg/mL with DMSO. The stock solution was vortexed until completely dissolved and stored at -20°C in refrigeration.

Preparation of sodium 2,3-dimercaptopropanesulfonate derivative reagent stock solution: an appropriate amount of sodium 2,3-dimercaptopropanesulfonate standard powder was accurately weighed and placed in a glass vial, and then prepared to form a stock solution at a concentration of 1.00 mg/mL with pure water. The stock solution was vortexed until completely dissolved and stored at room temperature.

### 1.5 Preparation of working solutions for calibration standards, QC samples and internal standards

The working solution for calibration standards (CWS) and the working solution for QC samples (WS) were prepared as follows, and stored at -20°C in refrigeration.

Note: The preparation volume can be adjusted according to actual needs, but the final concentration should be kept constant.

| **Solution source** | | | **Working solution (solvent: water: acetonitrile (v: v, 50:50))** | | |
|---|---|---|---|---|---|
| **Solution Source No.** | **Concentration (µg/mL)** | **Pipetting volume (µL)** | **Final volume (µL)** | **Concentration (µg/mL)** | **Working solution No.** |
| Calibration standard stock solution | 1000 | 20 | 800 | 25.0 | CWS-C1 |
| Calibration standard stock solution | 1000 | 22 | 1000 | 22.0 | CWS-C2 |
| Calibration standard stock solution | 1000 | 12 | 1000 | 12.0 | CWS-C3 |
| CWS-C1 | 25.0 | 100 | 500 | 5.00 | CWS-C4 |
| CWS-C1 | 25.0 | 60 | 600 | 2.50 | CWS-C5 |
| CWS-C1 | 25.0 | 20 | 1000 | 0.500 | CWS-C6 |
| CWS-C4 | 5.00 | 10 | 500 | 0.100 | CWS-C7 |
| CWS-C4 | 5.00 | 10 | 1000 | 0.0500 | CWS-C8 |
| | | | | | |
| Quality control sample stock solution | 1000 | 16 | 800 | 20.0 | WS-HQC |
| Quality control sample stock solution | 1000 | 4 | 1000 | 4.00 | WS-MQC |
| WS-MQC | 4.00 | 15 | 400 | 0.150 | WS-LQC |

| | | | | | |
|---|---|---|---|---|---|
| WS-LQC | 0.150 | 100 | 300 | 0.0500 | WS-LLOQ |
| | | | | | |
| Quality control sample stock solution | 1000 | 20 | 100 | 200 | WS-DQC |

Tolbutamide (1.00 mg/mL in DMSO) internal standard stock solution was further diluted with acetonitrile solution to prepare an internal standard working solution at a concentration of 10.0 ng/mL and stored at room temperature.

### 1.6 Preparation of calibration standards and QC samples

Calibration standards and QC samples were prepared in whole blood matrix as follows and needed to be used within the stability expiration date.

Note: The preparation volume can be adjusted according to actual needs, but the final concentration should be kept constant.

| **Working solution No.** | **Concentration (µg/mL)** | **Pipetting volume (µL)** | **Matrix volume (µL)** | **Concentration (ng/mL)** | **Calibration standard/QC sample No.** |
|---|---|---|---|---|---|
| CWS-C1 | 25.0 | 2 | 98 | 500 | C1 |
| CWS-C2 | 22.0 | 2 | 98 | 440 | C2 |
| CWS-C3 | 12.0 | 2 | 98 | 240 | C3 |
| CWS-C4 | 5.00 | 2 | 98 | 100 | C4 |
| CWS-C5 | 2.50 | 2 | 98 | 50.0 | C5 |
| CWS-C6 | 0.500 | 2 | 98 | 10.0 | C6 |
| CWS-C7 | 0.100 | 2 | 98 | 2.00 | C7 |
| CWS-C8 | 0.0500 | 2 | 98 | 1.00 | C8 |
| | | | | | |
| WS-HQC | 20.0 | 2 | 98 | 400 | HQC |
| WS-MQC | 4.00 | 2 | 98 | 80.0 | MQC |
| WS-LQC | 0.150 | 2 | 98 | 3.00 | LQC |
| WS-LLOQ | 0.0500 | 2 | 98 | 1.00 | LLOQ |
| WS-DQC | 200 | 2 | 98 | 4000 | DQC |

### 1.7 Pre-treatment of samples

Note: The volume of the sample can be adjusted according to actual needs, but the final concentration should be kept constant. Polyethylene centrifuge tubes may be used in place of the 96-well plate for sample processing, but be sure to use the same type of container to process the calibration standards, QC samples, and samples.

30 µL of whole blood sample, 90 µL of 1.00 mg/mL aqueous sodium 2,3-dimercaptopropanesulfonate and 30 µL of 0.2% formic acid were added to a 96-well plate, mixed well, and then incubated for 15 minutes at 45°C. 240 µL of the internal standard working solution (10.0 ng/mL of tolbutamide in acetonitrile solution) was added to precipitate proteins. The 96-well plate was shaken for 15 minutes, and then centrifuged at 4°C, 3220 g for 15 minutes. 160 µL of the supernatant was placed in a new 96-well plate, which was then centrifuged at 4°C, 3220 g for 5 minutes, and the samples were directly loaded.

### 1.8 Experimental instruments and conditions

### Liquid phase analysis conditions

Liquid Chromatograph: Waters ACQUITY Ultra High Performance Liquid Chromatography System
Column: ACQUITY UPLC^{®} Peptide C18 130 Å 2.1*100 mm I.D., 1.7 µm (Waters)
Mobile phase A: 0.01% AA and 2 mmol/L NH4OAc-containing Water: ACN (v: v, 95: 5)
Mobile Phase B: 0.01% AA and 2 mmol/L NH4OAc-containing Water: ACN (v: v, 5: 95)

### Elution gradient:

| **Time (min)** | **Flow rate (mL/mim)** | **A (%)** | **B (%)** |
|---|---|---|---|
| Initial | 0.400 | 60 | 40 |
| 1.40 | 0.400 | 2 | 98 |
| 1.70 | 0.400 | 2 | 98 |
| 1.71 | 0.400 | 60 | 40 |
| 2.00 | 0.400 | 60 | 40 |

Column temperature: 40°C
Sample injector weak wash solvent: ACN: Water (v: v, 10: 90)
Sample injector strong wash solvent: ACN: MeOH: IPA: Water (v: v: v: v, 1: 1: 1: 1) solution containing 0.2% TFA
Temperature of sample injector: 10°C
Injection volume: 4 µL (the injection volume can be adjusted appropriately to obtain good mass spectral behavior)

### Mass Spectrometry Analysis Conditions

Mass Spectrometer: AB SCIEX Triple Quad 6500+
Scanning Mode: Negative Ion Multi-Reactive Ion Monitoring

| | | | |
|---|---|---|---|
| Ion Source: | Turbo Spray | Ionization Mode: | Electrospray Ionization |
| Nebulizing gas: | 60 psi | Auxiliary heating gas: | 60 psi |
| Curtain gas: | 40 psi | Crash gas: | 10 |
| Spray voltage: | -4500 V | Ion source temperature: | 500°C |

### Multi-response monitoring parameter tables

| **Compound Name** | **Parent ion (*m*/*z*)** | **Daught er ion (m/z)** | **Dwell time (msec.)** | **Declusteri ng potential (eV)** | **Collision energy (eV)** |
|---|---|---|---|---|---|
| PAO (derivative) | 336.8 | 152.9 | 100 | -80 | -19 |
| Tolbutamide | 269.0 | 169.8 | 100 | -80 | -23 |

### 1.9 Data acquisition and processing

Retention times of compounds and internal standards, chromatogram acquisition and chromatogram integration were processed by the software Analyst (Applied Biosystems, version number 1.6.3, Foster City, CA, USA).

The statistics of the data were processed by the software Watson LIMS (Thermo Fisher Scientific, version no. 7.4.2, Philadelphia, PA, USA) and Microsoft Office Excel (Microsoft Corporation, version 2013, Redmond, WA, USA).

### 1.10 Formula

### 1.10.1 Accuracy (% deviation)

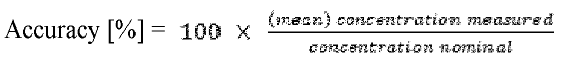

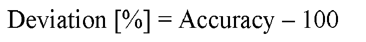

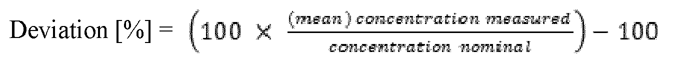

### 1.10.2 Precision (% coefficient of variation)

Precision is expressed by the coefficient of variation, calculated by the formula:

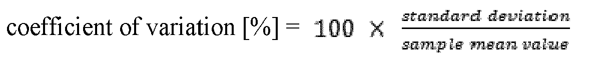

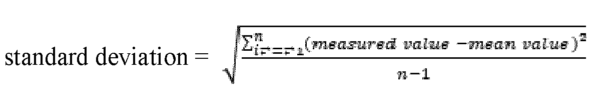

### 1.10.3 Interference

The interferences of analytes and internal standards in blank matrices were calculated as follows:

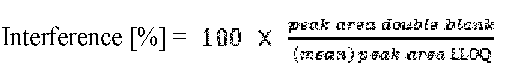

### 1.10.4 Extraction recovery rate

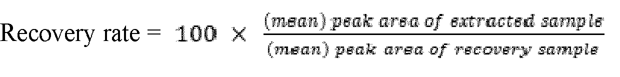

### 1.10.5 Stability

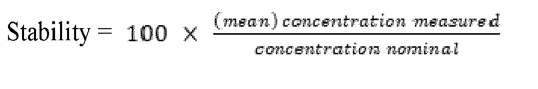

### 2. Analytical method validation

The analytical method for the determination of PAO concentration in whole blood of SD rats was fully validated using tolbutamide as an internal standard.

**Table 13: Method validation schedule**

| **Analytical batch number** | **Acceptance or not** | **Analytical batch test content** |
|---|---|---|
| 1 | Yes | Calibration curve, QC samples, specificity, interference, dilution reliability (1/10), residues, intra- and inter-batch accuracy and precision |
| 2 | No* | Calibration curve, QC samples, extraction recovery rate, matrix effect, residues, and inter-batch accuracy and precision |
| 3 | Yes | Calibration curve, QC samples, residues, and inter-batch accuracy and precision |
| 4 | Yes | Calibration curve, QC samples, extraction recovery rate, matrix effect, residues, and inter-batch accuracy and precision |
| 5 | No ** | Calibration curve, QC samples, and stability of treated samples at 79 hours |
| 6 | Yes | Calibration curve, QC samples, and stability of treated samples at 23 hours |
| 7 | Yes | Calibration curve, QC samples, and stability of treated samples at 53 hours |

| | | |
|---|---|---|
| * LLQC does not meet the analysis criteria **peak pattern abnormal | | |

### 2.1 Calculated concentration of calibration standards

Calibration standards should yield concentrations within ±15.0% of the nominal value, except for the lowest calibration standard, which should be within ±20.0%. The calculated concentrations for the calibration standards were shown in Table 14, and the calibration standards for the three analytical batches in this test met the above acceptance criteria.

**Table 14: Calculated concentrations of PAO calibration standards in whole blood of SD rats.**

| **Analyt ical batch No.** | **1** | **%deviat ion*** | **3** | **%deviat ion*** | **4** | **%deviat ion*** | **Aver age value** | **Stand ard ion** | **% coeffici ent of deviat variati on** | **%inter-b atch deviation** | **Sam ple size** |
|---|---|---|---|---|---|---|---|---|---|---|---|
| 1.00 ng/mL | 0.99 6 | -0.4 | 1. 01 | 1.0 | 1. 01 | 1.0 | 1.01 | 0.0080 8 | 0.8 | 1.0 | 3 |
| 2.00 ng/mL | **1. 45 | | 1. 96 | -2.0 | 1. 97 | -1.5 | 1.97 | | | -1.5 | 2 |
| 10.0 ng/mL | 10.5 | 5.0 | 9. 91 | -0.9 | 9. 75 | -2.5 | 10.1 | 0.395 | 3.9 | 1.0 | 3 |
| 50.0 ng/mL | 50.0 | 0.0 | 47 .7 | -4.6 | 51 .0 | 2.0 | 49.6 | 1.69 | 3.4 | -0.8 | 3 |
| 100 ng/mL | 86.9 | -13.1 | 10 8 | 8.0 | 10 5 | 5.0 | 100 | 11.4 | 11.4 | 0.0 | 3 |
| 240 ng/mL | 265 | 10.4 | 23 7 | -1.3 | 23 1 | -3.8 | 244 | 18.1 | 7.4 | 1.7 | 3 |
| 440 ng/mL | 425 | -3.4 | 43 7 | -0.7 | 45 7 | 3.9 | 440 | 16.2 | 3.7 | 0.0 | 3 |
| 500 ng/mL | 507 | 1.4 | 50 3 | 0.6 | 47 7 | -4.6 | 496 | 16.3 | 3.3 | -0.8 | 3 |

| | | | | | | | | | | | |
|---|---|---|---|---|---|---|---|---|---|---|---|
| *% The deviation should be within ±15.0%, except for LLOQ, which should be within ±20.0%. % The deviation exceeded ±15.0%, and thus was not included in the linear fit. | | | | | | | | | | | |

### 2.1.1 Calibration curve (linear)

The linear regression of PAO concentration in whole blood of SD rats was performed with a weighting factor of 1/x², and the linear range was 1.00-500 ng/mL. The parameters of the calibration curves for the three analytical batches of this test were shown in Table 15. The coefficients of determination of the calibration curves for all the three analytical batches of the test were R²≥0.9944. The results of the test indicated that the linearity met the acceptance criteria, and that the calibration curves were suitable for the quantitative analysis.

**Table 15. Parameters of the regression equation for the calibration curve of PAO in whole blood of SD rats**

| **Anal ytical batc h No.** | **Slope (a)** | **Intercept (b)** | **Coefficie nt of determin ation (R²)** | **LLOQ (ng/mL)** | **ULOQ (ng/mL)** |
|---|---|---|---|---|---|
| 1 | 0.00588 | 0.001695 | 0.9944 | 1.00 | 500 |
| 3 | 0.007681 | 0.0009535 | 0.9985 | 1.00 | 500 |
| 4 | 0.008219 | 0.001715 | 0.9985 | 1.00 | 500 |

### 2.1.2 Inter- and intra-batch accuracy and precision

The formulae were described in Sections 1.10.1 and 1.10.2. Inter-batch, intra-batch accuracy and precision were evaluated using three analytical batches and six determinations per QC sample concentration level. The results of the inter-batch and intra-batch accuracy and precision were shown in Table 16. The results indicated that the inter-batch and intra-batch accuracy and precision met the acceptance criteria, and that the analytical method was reliable.

### 2.1.3 Analytical method sensitivity

The LLOQ of PAO had good signal-to-noise ratio and sensitivity. The LLOQ in whole blood of SD rats was 1.00 ng/mL. The mean precision and accuracy of the intra- and inter-batch LLOQ were within ±20.0%, and the results were shown in Table 16.

The mean precision and accuracy of intra- and inter-batch LLOQs were within ±20.0%, and the results were shown in Table 16.

**Table 16. Precision and accuracy of PAO in whole blood of SD rats**

| **Analytical batch No.** | **LLOQ** | **% devia tion** | **Low concent ration** | **% devia tion** | **Mediu m concent ration** | **% devia tion** | **High concent ration** | **% devia tion** |
|---|---|---|---|---|---|---|---|---|
| | **(1.00 n g/mL)** | | **(3.00 ng /mL)** | | **(80.0 ng /mL)** | | **(400 ng/ mL)** | |
| 1 | 0.873 | -12.7 | 2.82 | -6.0 | 82.6 | 3.3 | 394 | -1.5 |
| | 0.907 | -9.3 | 2.86 | -4.7 | 84.2 | 5.3 | 460 | 15.0 |
| | 0.818 | -18.2 | 3.12 | 4.0 | 90.6 | 13.3 | 445 | 11.3 |
| | 0.845 | -15.5 | 3.14 | 4.7 | 83.1 | 3.9 | 406 | 1.5 |
| | 0.835 | -16.5 | 2.77 | -7.7 | 84.3 | 5.4 | 455 | 13.8 |
| | #0.792 | -20.8 | 3.42 | 14.0 | 90.0 | 12.5 | 423 | 5.8 |
| Intra-batch mean value | 0.845 | | 3.02 | | 85.8 | | 431 | |
| Intra-batch standard deviation | 0.0407 | | 0.250 | | 3.55 | | 27.1 | |
| Intra-batch %coefficien t of variation * | 4.8 | | 8.3 | | 4.1 | | 6.3 | |
| Intra-batch %deviation ** | -15.5 | | 0.7 | | 7.3 | | 7.8 | |
| Sample size | 6 | | 6 | | 6 | | 6 | |
| 3 | 1.06 | 6.0 | 2.92 | -2.7 | 81.6 | 2.0 | 400 | 0.0 |
| | 1.08 | 8.0 | 3.29 | 9.7 | 82.8 | 3.5 | 444 | 11.0 |
| | 1.09 | 9.0 | 3.19 | 6.3 | 88.6 | 10.8 | 433 | 8.3 |
| | 0.921 | -7.9 | 3.07 | 2.3 | 83.8 | 4.8 | 393 | -1.8 |
| | 1.04 | 4.0 | 3.21 | 7.0 | ##92.3 | 15.4 | 411 | 2.8 |
| | 0.939 | -6.1 | 3.00 | 0.0 | 87.4 | 9.3 | 408 | 2.0 |
| Intra-batch mean value | 1.02 | | 3.11 | | 86.1 | | 415 | |
| Intra-batch standard deviation | 0.0733 | | 0.140 | | 4.07 | | 19.7 | |
| Intra-batch %coefficien t of variation | 7.2 | | 4.5 | | 4.7 | | 4.7 | |
| Intra-batch %deviation | 2.0 | | 3.7 | | 7.6 | | 3.8 | |
| Sample size | 6 | | 6 | | 6 | | 6 | |

**Table 16. (Continued) Precision and accuracy of PAO in whole blood of SD rats**

| **Analytical batch No.** | **LLOQ** | **% devia tion** | **Low concent ration** | **% devia tion** | Mediu **m concent ration** | **% devia tion** | **High concent ration** | **% devia tion** |
|---|---|---|---|---|---|---|---|---|
| | **(1.00 n g/mL)** | | **(3.00 ng /mL)** | | **(80.0 ng /mL)** | | **(400 ng/ mL)** | |
| 4 | 0.997 | -0.3 | 3.00 | 0.0 | 83.8 | 4.8 | 409 | 2.3 |
| | 0.911 | -8.9 | 2.92 | -2.7 | 84.9 | 6.1 | 408 | 2.0 |
| | 0.906 | -9.4 | 3.25 | 8.3 | ##95.4 | 19.3 | 407 | 1.8 |
| | 0.978 | -2.2 | 3.32 | 10.7 | 83.6 | 4.5 | ##273 | -31.8 |
| | 0.922 | -7.8 | 3.24 | 8.0 | 82.8 | 3.5 | 384 | -4.0 |
| | 1.03 | 3.0 | 3.19 | 6.3 | 83.8 | 4.8 | 403 | 0.8 |
| Intra-batch mean value | 0.957 | | 3.15 | | 85.7 | | 381 | |
| Intra-batch standard deviation | 0.0516 | | 0.157 | | 4.79 | | 53.6 | |
| Intra-batch %coefficie nt of variation | 5.4 | | 5.0 | | 5.6 | | 14.1 | |
| Intra-batch %deviation | -4.3 | | 5.0 | | 7.1 | | -4.8 | |
| Sample size | 6 | | 6 | | 6 | | 6 | |
| Inter-batch mean value | 0.941 | | 3.10 | | 85.9 | | 409 | |
| Inter-batch standard deviation | 0.0922 | | 0.186 | | 3.92 | | 40.4 | |
| Inter-batch %coefficie nt of variation *** | 9.8 | | 6.0 | | 4.6 | | 9.9 | |
| Inter-batch %deviation **** | -5.9 | | 3.3 | | 7.4 | | 2.3 | |
| Sample size | 18 | | 18 | | 18 | | 18 | |

| | | | | | | | | |
|---|---|---|---|---|---|---|---|---|
| * The intra-batch % coefficient of variation should be ≤ 15.0% and the LLOQ should be ≤ 20.0%. ** The intra-batch % deviation should be within ±15.0%, and the LLOQ should be within ±20.0%. *** The inter-batch % coefficient of variation should be ≤ 15.0% and the LLOQ should be ≤ 20.0%. **** The inter-batch % deviation should be within ±15.0%, and the LLOQ should be within ±20.0%. # % deviation was not within ±20.0%, but the data was still involved in the calculation of intra-batch precision and accuracy, and all results met analytical requirements. ## % deviation was not within ±15.0%, but the data was still involved in the calculation of intra-batch precision and accuracy, and all results met analytical requirements. | | | | | | | | |

### 2.2 Lower limit of quantification and upper limit of quantification (LLOQ and ULOQ)

For all three analyzed batches, the mean accuracy of the LLOQ was within ±20.0% and the mean precision of the LLOQ was less than 20.0%; the mean accuracy of the ULOQ was within ±15.0% and the mean precision of the ULOQ was less than 15.0%.

### 2.3 Dilution reliability

The dilution test was used to verify the dilution reliability of diluted samples at concentrations higher than ULOQ.

Quality control samples at a concentration of 4000 ng/mL were prepared and diluted 10-fold with blank SD rat whole blood, six duplicate, to verify the dilution reliability.

The mean accuracy (% deviation) and precision (% coefficient of variation) were shown in Table 17. The results of this test met the acceptance criteria and the 10-fold dilution reliability was verified when diluting samples at concentrations higher than ULOQ.

**Table 17. Precision and accuracy of the diluted QC samples of PAO in whole blood of SD rats**

| **Date of analysis** | **Dilution factor** | **Concentration 4000 ng/mL** | **%deviation** |
|---|---|---|---|
| - | 10 | 4150 | 3.8 |
| | | 4600 | 15.0 |
| | | 4320 | 8.0 |
| | | 4250 | 6.3 |
| | 4210 | 5.3 | |
| | 5430 | 35.8 | |
| Intra-batch mean value | 4490 | | |
| Intra-batch standard deviation | 485 | | |
| Intra-batch % coefficient of variation * | 10.8 | | |
| Intra-batch % deviation** | 12.3 | | |
| Sample size | 6 | | |

| | | | |
|---|---|---|---|
| * The intra-batch % coefficient of variation should be ≤ 15.0%. ** The intra-batch % deviation should be within ±15.0%. | | | |

### 2.4 Specificity and interference

Six different batches of blank whole blood from SD rats were used to evaluate the specificity (selectivity) of the method using the formula in Section 2.10.3.

The specificity results for analytes in blank matrices from six different sources were shown in Table 18; the analyte interference fraction in all six different sources of blank matrices in the test was less than 20.0% of the analyte response in the LLOQ QC sample, and the internal standard interference fraction was less than 5.0% of the internal standard response in the QC sample. The results of this test met the acceptance criteria and the specificity of the analytical method was verified.

Six batches of mixed blank matrices from the specificity test described above were taken to evaluate analyte and internal standard interferences. In the six batches of mixed blank matrices, the interference component of the internal standard to the analyte was less than 20.0% of the analyte response in the LLOQ sample, and the interference component of the analyte to the internal standard was less than 5.0% of the internal standard response in the LLOQ sample. The results indicated that there was no mutual interference between the analyte and the internal standard. The results were shown in Table 19.

**Table 18. Specificity evaluation of SD rat blank whole blood from six different sources**

| **Sample name** | **PAO (analyte)** | | | | **Tolbutamide (internal standard)** | | | |
|---|---|---|---|---|---|---|---|---|
| | **Peak area in the blank matrix** | **Peak area in the LLOQ sample** | **% interfer ence*** | **<20. 0% Yes/ No** | **Peak area in the blank matrix** | **Peak area in the LLOQ sample** | **% interfere nce**** | **<5.0 % Yes/ No** |
| Rat EDTA-K₂ blood source 1 | 0 | 4900 | 0.0 | Yes | 0 | 882000 | 0.0 | Yes |
| Rat EDTA-K₂ blood source 2 | 0 | 4950 | 0.0 | Yes | 0 | 901000 | 0.0 | Yes |
| Rat EDTA-K₂ blood source 3 | 0 | 5520 | 0.0 | Yes | 0 | 877000 | 0.0 | Yes |
| Rat EDTA-K₂ blood source 4 | 0 | 5470 | 0.0 | Yes | 0 | 882000 | 0.0 | Yes |
| Rat EDTA-K₂ blood source 5 | 0 | 4970 | 0.0 | Yes | 0 | 894000 | 0.0 | Yes |
| Rat EDTA-K₂ blood source 6 | 0 | 4360 | 0.0 | Yes | 0 | 908000 | 0.0 | Yes |
| | Mean | 5030 | | | Mean | 891000 | | |

| | | | | | | | | |
|---|---|---|---|---|---|---|---|---|
| * The analyte % interference component in the blank matrix should be less than 20.0% of the analyte response in the LLOQ sample. ** The internal standard % interference component in the blank matrix should be less than 5.0% of the internal standard response in the LLOQ sample. Rat EDTA-K₂ blood source 1-6: Blank whole blood of SD rats not containing PAO or tolbutamide from six different sources | | | | | | | | |

**Table 19. Interference evaluation of analytes and internal standards in blank whole blood of SD rats**

| **Sample name** | **PAO (analyte)** | | | | **Sample name** | **Tolbutamide (internal standard)** | | | |
|---|---|---|---|---|---|---|---|---|---|
| | **Pea k are a in the bla nk mat rix** | **Pea k area in the LL OQ sam ple** | **% interfer ence*** | **<20. 0% Yes/ No** | | **Peak area in the blank matrix** | **Peak area in the LLOQ sample** | **% interfer ence **** | **<5.0 % Yes/ No** |
| Interference QC0-1 | 0 | 679 0 | 0.0 | Yes | Interference ULOQ-1 | 0 | 899000 | 0.0 | Yes |
| Interference QC0-2 | 0 | | 0.0 | Yes | Interference ULOQ-2 | 0 | | 0.0 | Yes |
| Interference QC0-3 | 0 | | 0.0 | Yes | Interference ULOQ-3 | 0 | | 0.0 | Yes |
| Interference QC0-4 | 0 | | 0.0 | Yes | Interference ULOQ-4 | 0 | | 0.0 | Yes |
| Interference QC0-5 | 0 | | 0.0 | Yes | Interference ULOQ-5 | 0 | | 0.0 | Yes |
| Interference QC0-6 | 0 | | 0.0 | Yes | Interference ULOQ-6 | 0 | | 0.0 | Yes |

| | | | | | | | | | |
|---|---|---|---|---|---|---|---|---|---|
| * % The analyte interference component should be less than 20.0% of the analyte response in the LLOQ sample. **% The internal standard interference component should be less than 5.0% of the internal standard response in the LLOQ sample. Interference QC0: QC samples added with only internal standard tolbutamide and without PAO Interference ULOQ: QC samples added with only ULOQ concentration of PAO and without internal standard tolbutamide | | | | | | | | | |

### 2.5 Matrix effect

Six different sources of SD rat EDTA-K2 blank whole blood were taken to evaluate the matrix effect.

Six different sources of SD rat blank whole blood were used to prepare low concentration QC samples and high concentration QC samples according to the sample treatment method, which were used to evaluate the matrix effect and batch-to-batch variation of the method. The total % coefficient of variation for the high and low concentration QC samples from each batch was 7.2%, which showed that there was no significant matrix effect between different batches of biological matrix. The results were shown in Table 20.

**Table 20. Evaluation of matrix effects of SD rat blank whole blood from six different sources**

| **Conce ntrati on** | **Matrix source** | **Peak area** | | **Matrix factor (MF)** | | **MFs norma lized by the intern al standa rd** | **% coeffici ent of variatio n for each concent ration*** | **Total % coeffic ient of variati on*** |
|---|---|---|---|---|---|---|---|---|
| | | **PAO (analyte )** | **Tolbu tamid e (inter nal stand ard)** | **PAO (anal yte)** | **Tolbu tamid e (inter nal standa rd)** | | **(MFs normali zed by the internal standar d)** | **(MFs norma lized by the intern al standa rd)** |
| Low: | Reference (mean value) | 9860 | 10200 00 | - | - | - | | |
| 2.00 n g/mL | Rat EDTA-K₂ blood source 1 | 20700 | 10600 00 | 2.10 | 1.04 | 2.02 | | |
| | Rat EDTA-K₂ blood source 2 | 18200 | 99900 0 | 1.85 | 0.979 | 1.89 | | |
| | Rat EDTA-K₂ blood source 3 | 17900 | 99600 0 | 1.82 | 0.976 | 1.86 | 4.8 | |
| | Rat EDTA-K₂ blood source 4 | 18000 | 10400 00 | 1.83 | 1.02 | 1.79 | | 7.2 |
| | Rat EDTA-K₂ blood source 5 | 18000 | 10400 00 | 1.83 | 1.02 | 1.79 | | |
| | Rat EDTA-K₂ blood source 6 | 19000 | 10100 00 | 1.93 | 0.990 | 1.95 | | |
| High: | Reference (mean value) | 2600000 | 99700 0 | - | - | - | 5.3 | |
| 500 ng | Rat EDTA-K₂ | 4820000 | 10300 | 1.85 | 1.03 | 1.80 | | |
| /mL | blood source 1 | | 00 | | | | | |
| | Rat EDTA-K₂ blood source 2 | 4340000 | 10200 00 | 1.67 | 1.02 | 1.64 | | |
| | Rat EDTA-K₂ blood source 3 | 4630000 | 97900 0 | 1.78 | 0.982 | 1.81 | | |
| | Rat EDTA-K₂ blood source 4 | 3990000 | 96600 0 | 1.53 | 0.969 | 1.58 | | |
| | Rat EDTA-K₂ blood source 5 | 4200000 | 96600 0 | 1.62 | 0.969 | 1.67 | | |
| | Rat EDTA-K₂ blood source 6 | 4360000 | 98600 0 | 1.68 | 0.989 | 1.70 | | |

| | | | | | | | | |
|---|---|---|---|---|---|---|---|---|
| * % The coefficient of variation should be ≤ 15.0%. Rat EDTA-K₂ blood source 1-6: SD rat blank whole blood from six different sources | | | | | | | | |

### 2.6 Recovery rates for biological sample treatment processes

Analyte extraction recovery from biological samples was calculated by dividing the peak areas of analytes obtained from three concentration levels of PAO extracted from biological samples (3.00, 80.0, and 400 ng/mL, three duplicates of each concentration level), respectively, by the peak area of analytes obtained from a PAO control solution of the same concentration prepared with supernatant after extraction from a blank matrix. The formula was shown in Section 1.10.4.

The overall % coefficient of variation for the recovery of analyte PAO in the matrix to be tested under the existing biological sample processing methods was 5.2%. The results showed no significant difference in the recoveries at low, medium and high concentrations and the results were shown in Table 21.

**Table 21. recovery of PAO during processing in whole blood of SD rats**

| **Sample name** | **Peak area of control** | **Sample name** | **Peak area of extract** | **Recover y rate (%)** | **%co effici ent of vari ance *** | **Total % coefficient of variation for all concentrat ion recoveries** |
|---|---|---|---|---|---|---|
| | | | | | | * |
| Recovery LQC 1 | 16200 | Extraction LQC 1 | 11200 | 67.1 | 5.8 | |
| Recovery LQC 2 | 17100 | Extraction LQC 2 | 12000 | 71.9 | | |
| Recovery LQC 3 | 16900 | Extraction LQC 3 | 12600 | 75.4 | | |
| Mean value | 16700 | Mean value | 11900 | | | |
| Standard deviation | 473 | Standard deviation | 702 | | | |
| %coefficient of variation | 2.8 | %coefficient of variation | 5.9 | | | |
| Recovery MQC 1 | 404000 | Extraction MQC 1 | 298000 | 71.0 | 2.7 | 5.2 |
| Recovery MQC 2 | 428000 | Extraction MQC 2 | 303000 | 72.1 | | |
| Recovery MQC 3 | 429000 | Extraction MQC 3 | 314000 | 74.8 | | |
| Mean value | 420000 | Mean value | 305000 | | | |
| Standard deviation | 14200 | Standard deviation | 8190 | | | |
| %coefficient of variation | 3.4 | %coefficient of variation | 2.7 | | | |
| Recovery HQC 1 | 1920000 | Extraction HQC 1 | 1540000 | 78.2 | 0.6 | |
| Recovery HQC 2 | 2020000 | Extraction HQC 2 | 1530000 | 77.7 | | |
| Recovery HQC 3 | 1980000 | Extraction HQC 3 | 1550000 | 78.7 | | |
| Mean value | 1970000 | Mean value | 1540000 | | | |
| Standard deviation | 50300 | Standard deviation | 10000 | | | |
| %coefficient of variation | 2.6 | %coefficient of variation | 0.6 | | | |

| | | | | | | |
|---|---|---|---|---|---|---|
| *% Coefficient of variation should be ≤ 15.0%. Recovery QC: PAO working solution was added after blank matrix extraction Extraction QC: PAO working solution was added to the blank matrix before extraction. | | | | | | |

### 2.7 Residue

System residues were verified by entering a blank sample immediately after the ULOQ sample. The PAO peak areas in the residue samples were all within 20.0% of the average peak area in the LLOQ; the internal standard peak areas in the residue samples were all within 5.0% of the average peak area in the LLOQ. The results are shown in Table 22, and the residues of this analytical method met the standard.

**Table 22. Evaluation of PAO and Tolbutamide residues in SD rat whole blood**

| **Analytic al batch No.** | **Sample name** | **PAO (analyte)** | | **Tolbutamide (internal standard)** | |
|---|---|---|---|---|---|
| | | **Peak area** | **%residu e*** | **Peak area** | **%residue*** |
| 1 | C8 Double Blank (after C1) | 6790 | - | 899000 | - |
| | | 0 | 0.0 | 0 | 0.0 |
| 3 | C8 Double Blank (after C1) | 8600 | - | 986000 | - |
| | | 0 | 0.0 | 0 | 0.0 |
| 4 | C8 Double Blank (after C1) | 10000 | - | 1000000 | - |
| | | 0 | 0.0 | 0 | 0.0 |

| | | | | | |
|---|---|---|---|---|---|
| *% Analyte residues should be less than 20.0% of the peak area of the analyte in the LLOQ sample. **% Internal standard residues should be less than 5.0% of the peak area of the internal standard in the LLOQ sample. | | | | | |

### 2.8 System applicability

The chromatographic behavior and mass spectral response of the analytes were confirmed by three LLOQ concentration samples before examining the method validation samples.

### 3. Stability

All stability tests were evaluated using low and high concentration levels with three samples at each concentration level. The acceptance criterion was that the % deviation of the ratio of the measured concentration to the nominal concentration should be within ±15.0%.

The formula was shown in Section 1.10.5.

### 3.1 Repeated injection stability of PAO in treated samples (autosampler)

Treated PAO calibration curve samples and QC samples were placed in 96-well plates in an autosampler for 23 and 53 hours at 10°C. Then the calibration curve samples and QC samples were re-injected and analyzed by the existing LC-MS/MS method, and the repeated injection stability of the treated QC samples in the autosampler was evaluated by the ratio of the average concentration of the analytes to the nominal concentration. The results were shown in Table 23, and the treated samples were stable for at least 53 hours when placed in a 10°C sampler.

**Table 23. Repeated injection stability of PAO in treated SD rat whole blood**

| **Sample name** | **Nominal concentration** | **Measured concentration (ng/mL in whole blood)** | | | | **Stan dard devia tion** | **%coeffi cient of variatio n*** | **% deviation from nominal concentration* *** |
|---|---|---|---|---|---|---|---|---|
| | **(ng/mL in whole blood)** | **Repe at 1** | **Repe at 2** | **Repe at 3** | **Mea n valu e** | | | |
| Low concentration | | | | | | | | |
| Place at 10°C for 23 hours | 3.00 | 3.19 | 2.96 | 2.90 | 3.02 | 0.153 | 5.1 | 0.7 |
| Place at 10°C for 53 hours | 3.00 | 2.84 | 2.84 | 2.91 | 2.86 | 0.040 4 | 1.4 | -4.7 |
| | | | | | | | | |
| High concentration | | | | | | | | |
| Place at 10°C for 23 hours | 400 | 367 | 362 | 367 | 365 | 2.89 | 0.8 | -8.8 |
| Place at 10°C for 53 hours | 400 | 370 | 377 | 380 | 376 | 5.13 | 1.4 | -6.0 |

| | | | | | | | | |
|---|---|---|---|---|---|---|---|---|
| *% Coefficient of variation should be ≤ 15.0%. **% Deviation from nominal concentration should be within ±15.0%. | | | | | | | | |

### 4. Conclusion

The LC-MS/MS method for the quantitative determination of PAO in SD rat whole blood was successfully validated. The linear range of the method was 1.00-500 ng/mL, and samples above the ULOQ could be diluted up to 10-fold in blank SD rat whole blood. The analytical method was validated for calibration curve, intra- and inter-batch precision and accuracy, sensitivity, specificity, matrix effect, extraction recovery, dilution reliability and residue. The stability of the treated samples was also investigated.
1) The validation results of the LC-MS/MS method for the quantitative determination of PAO in SD rat whole blood met all analytical acceptance criteria. The results showed that the validated LC-MS/MS method can be used for the determination of PAO concentration in SD rat whole blood matrix.
2) The LLOQ of PAO in SD rat whole blood could reach 1.00 ng/mL, and the precision and accuracy of intra- and inter-batch LLOQ met the acceptance criteria.
3) The calibration curve of PAO in SD rat whole blood showed good linearity (R2 ≥ 0.9944) and accuracy. The linear range was 1.00-500 ng/mL.
4) The intra- and inter-batch analytical results of the samples with four different concentration levels (n=6) met the acceptance criteria, and the analytical method had good intra- and inter-batch precision and accuracy.
5) The specificity of the analytical method was verified by the absence of any potential endogenous interference of PAO in the six different sources of blank matrices examined. There was also no quantitative interference between analytes and internal standards.
6) PAO showed no significant matrix effects in the six different sources of blank matrices examined.
7) The overall % coefficient of variation for the extraction recoveries of PAO in the examined matrices with the available analytical methods at various concentrations was 5.2%.
8) Under the conditions of the existing LC-MS/MS analytical method, the residue of PAO in of SD rat whole blood was less than 20% LLOQ; the residue of internal standard was less than 5% LLOQ.
9) Stability test results were as follows: PAO from SD rat whole blood treated samples was stable in an autosampler at 10°C for at least 53 hours.

In conclusion, the currently validated LC-MS/MS analytical method was suitable for the determination of PAO concentration in SD rat whole blood.

### II. Effect of phenylarsonic acid on the liquid chromatography-mass spectrometry (LC-MS/MS) method for the determination of PAO concentration in EDTA-K2 whole blood

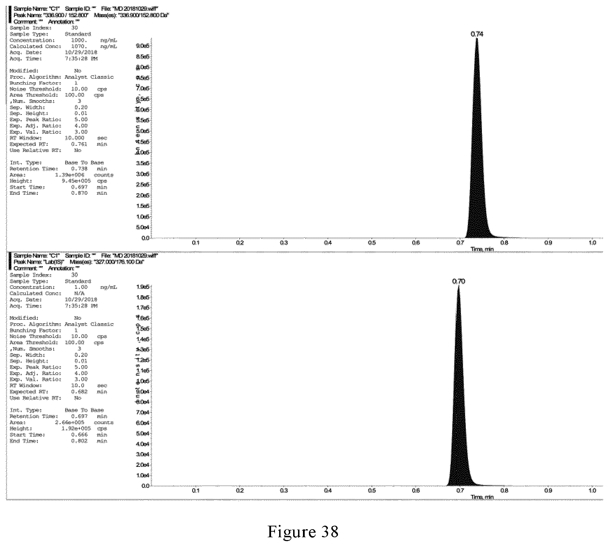

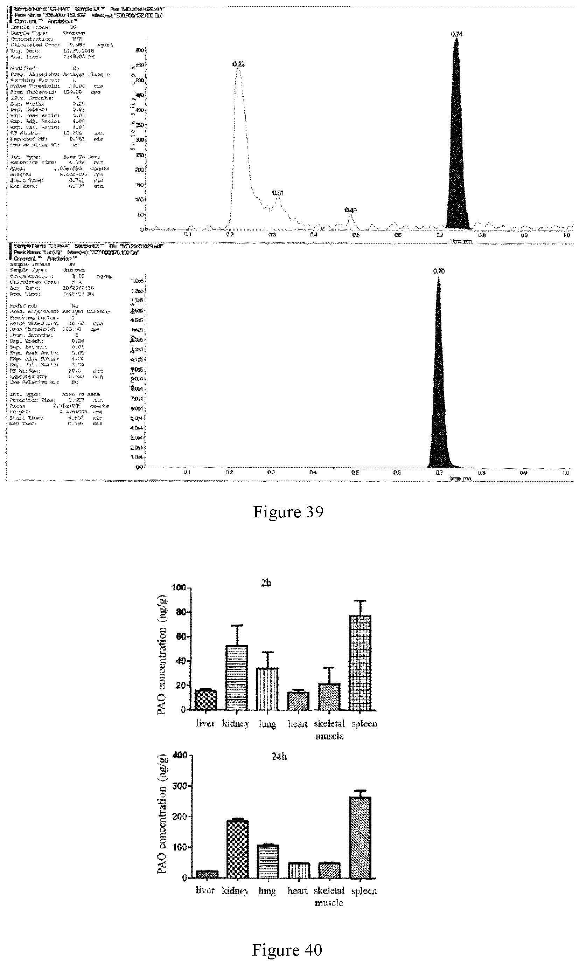

This example showed that phenylarsonic acid in blood could not be derivatized with sodium dimercaptopropanesulfonate or the amount of the derivative product of phenylarsonic acid was four orders of magnitude smaller than that of the derivative product of the same amount of PAO, see Figure 38 and Figure 39.

### Example 17. Tissue distribution characteristics of PAO in SD rats after single gavage administration

In this experiment, 30 SD rats were divided into 5 groups (3/sex/group). Each group was given a single gavage of 0.9 mg/kg PAO in the solvent MIGLYOL^{®} 812N. The rats in groups 1 to 5 were euthanized by carbon dioxide inhalation at 2, 4, 10, 24 and 36 hours after the administration of PAO. Whole blood as well as various tissues were collected and tissue homogenate samples were prepared and analyzed by a validated LC/MS/MS method.

The results showed that after a single administration of 0.9 mg/kg PAO to SD rats, PAO was rapidly distributed in the tissues, reaching most of the tissues within 2 hours after administration (Figure 40).

In all tissues of both male and female rats, the peak concentration of PAO was reached between 2 and 24 hours after administration, and a significant decrease in concentration was observed after 36 hours of administration.

**Table 24. Concentration of PAO (ng/g) in various organs of male rats after 2h of single administration**

| **Animal No.** | **Liver** | **Kidney** | **Lung** | **Heart** | **Skeletal muscle** | **Spleen** |
|---|---|---|---|---|---|---|
| R1 | 13.3 | 33.2 | 12.8 | 12.5 | 48 | 57 |
| R2 | 15.1 | 37.4 | 31.5 | 12.1 | 10.9 | 75 |
| R3 | 18.6 | 86.5 | 58.5 | 18.9 | 5.55 | 100 |
| mean value | 15.7 | 52.4 | 34.3 | 14.5 | 21.5 | 77.3 |
| standard error | 1.6 | 17.1 | 13.3 | 2.2 | 13.4 | 12.5 |
| coefficient of variation (%) | 17.2 | 56.5 | 67.1 | 26.3 | 107 | 27.9 |

**Table 25. Concentration of PAO (ng/g) in various organs of male rats after 24h of single administration**

| **Animal No.** | **Liver** | **Kidney** | **Lung** | **Heart** | **Skeletal muscle** | **Spleen** |
|---|---|---|---|---|---|---|
| R19 | 21.6 | 188 | 103 | 44.2 | 45.9 | 305 |
| R20 | 18 | 199 | 114 | 45.8 | 54 | 260 |
| R21 | 23 | 170 | 103 | 52 | 44.8 | 229 |
| mean value | 20.9 | 186 | 107 | 47.3 | 48.2 | 265 |
| standard error | 1.5 | 8.5 | 3.7 | 2.4 | 2.9 | 22.1 |
| coefficient of variation (%) | 12.3 | 7.85 | 5.93 | 8.71 | 10.4 | 14.4 |

### Example 18. PAO Inhibited Atherosclerotic Plaque Formation in ApoE^{-/-} Mice - A Novel Therapeutic Regimen for Atherosclerosis

The present experiment tested the effects of the PI4KIIIa inhibitor PAO on body weight and intra-arterial plaque formation in ApoE^{-/-} mice, a model of atherosclerosis induced by a high-fat diet. ApoE^{-/-} mice are commonly used atherosclerosis model mice, and their development of atherosclerotic lesions is similar to that observed in pathologic studies of human disease. PAO inhibited high-fat diet-induced weight gain, as well as the number and area of intra-aortic atheromatous plaques in ApoE^{-/-} mice. These results suggested that PAO reduced atherosclerotic plaque formation, thus becoming a potentially effective therapeutic option against atherosclerosis.

### 1. Experimental methods

### 1.1 Laboratory animal

ApoE^{-/-} mice (purchased from Suzhou Saiye Biologicals), SPF grade, 8 weeks old, body weight (20±2) g, were housed in the Laboratory Animal Center of Xinhua Hospital, Shanghai Jiaotong University School of Medicine. The animals were kept under 12 hours of light and 12 hours of darkness at a constant temperature of 22°C. Model group: 7 8-week-old male ApoE^{-/-} mice were fed with high-fat diet (Dyets ASHF3 high fat (40%kcal%) and high cholesterol (1.25%) mouse food). PAO group: 7 8-week-old male ApoE^{-/-} mice were fed with high-fat diet (Dyets ASHF3 high-fat (40% kcal%) and high cholesterol (1.25%) mouse food), and were administered with 0.15 mg/kg PAO by gavage once a day. Both groups of mice were fed for a total of 20 weeks.

### 1.1.1 Mouse artery oil red O staining

### Preparation of Oil Red O staining related solutions

(1) Preparation of Oil Red O stock solution: 5g of Oil Red O reagent (Sigma, catalog No. SLBG0158V) was diluted with isopropyl alcohol to 1000 ml. Oil Red O was dissolved by shaking gently in a water bath, cooled to room temperature and filtered through filter paper.
(2) Preparation of Oil Red O working solution: Oil Red O stock solution was mixed with distilled water in the ratio of 6:4, and then residues were removed and used.
(3) Preparation of 60% isopropanol solution: distilled water was used to prepare the solution, freshly prepared.

### Mouse aortic stripping and sampling

(1) Mouse heart perfusion fixation:
   1. The mice were anesthetized and immediately fixed on a foam plate with a needle. The skin of the chest was clamped with forceps, and the skin and ribs of the chest cavity were cut with scissors to fully expose the heart.
   2. The needle was inserted into the left ventricle of the mouse, and the right atrium of the mouse was cut to allow blood to flow out. The mice were perfused with 0.9% sodium chloride solution for about 10 minutes, causing the liver and tongue to turn white.
   3. After the limbs, liver and tongue turned white, the mice were fixed by 4% paraformaldehyde and perfused with 4% paraformaldehyde for 20 minutes.
   4. At the end of the perfusion, the needle was removed and the mice were placed on ice for subsequent operations.
(2) Aortic dissection in mice: under a body-view microscope, the abdominal and thoracic cavities of mice were opened with blunt shears to free the aortic arch. The diaphragm and kidneys were cut, and the fat exposed on the surface of the aorta was removed. The proximal aorta was lifted with forceps, and the aorta was separated along the spine to the branch of the iliac artery. Micro-spring scissors were inserted into the lumen of the aorta and cut longitudinally along the inner curvature of the aortic arch to the bifurcation of the iliac arteries, and the right and left iliac arteries were cut longitudinally, and the aortic arch, the innominate artery, the left common carotid artery, the left subclavian artery, and finally the right and left iliac arteries were cut from the outer curvature of the aortic arch, and the entire aorta was dissected out.
(3) Oil red O staining: the dissected aorta was rinsed with 0.9% saline and transferred to a 5 ml centrifuge tube pre-filled with 4% paraformaldehyde and fixed for 12 hours at room temperature. At the end of fixation, the aorta was rinsed 3 times with saline and placed in a petri dish. The periarterial connective tissue and fat were carefully peeled off under a stereomicroscope. The aorta was then submerged in Oil Red O working solution and stained for 30 minutes at room temperature. After staining, the aorta was decolorized by rinsing with 60% isopropyl alcohol solution for 30 seconds, followed by 3 rinses in saline.
(4) Photography and measurement of aortic plaque area: after staining, the aorta was spread out on a plastic plate with a black background, and then photographed. The surface area of the whole artery and the area of atherosclerotic lesions were calculated using Image pro plus 6.0 software.

### 1.2 Statistical analysis

The data were analyzed using SPSS 23.0, and the measurements were expressed as mean ± standard deviation, and the body weights of the mice and the area occupied by atherosclerotic plaques were analyzed using the t-test, and P<0.05 indicated statistically different.

### 2. Results

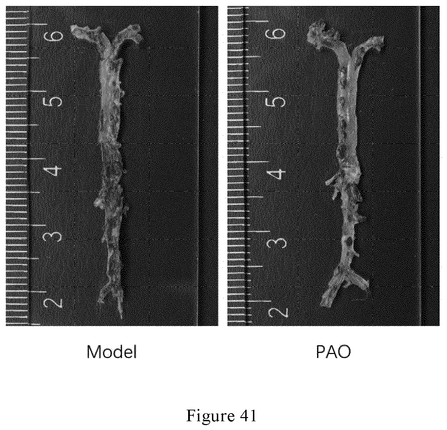

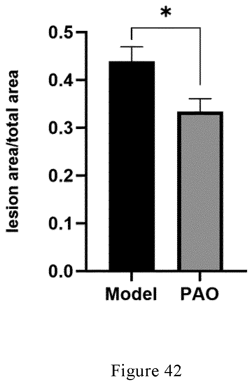

Model group: 8-week-old male ApoE^{-/-} mice were fed with high-fat diet (Dyets ASHF3 high-fat (40%kcal%) and high cholesterol (1.25%) mouse food). PAO group: 8-week-old male ApoE^{-/-} were simultaneously administered with 0.15 mg/kg PAO by gavage once daily. Both groups of mice were fed for a total of 20 weeks. At the end of feeding, transcardiac perfusion fixation was performed under anesthesia, followed by freeing the full-length aorta of the mice under a body-vision microscope, which was subsequently clipped and placed in 4% paraformaldehyde solution for 12 hours of fixation. The peri-arterial connective tissue was stripped after rinsing in saline and subsequently stained with Oil Red O. The results showed that a large number of atheromatous plaques were generated in the aorta of the animals in the model group. In contrast, the number of atherosclerotic plaques in the PAO group was significantly less than that in the model group, and the area of the plaques in the whole artery was also smaller than that in the model group (Figure 41), P<0.05 indicated statistically different. (Figure 42).

### Example 19. Evaluation of PAO efficacy in ovalbumin OVA-induced BALB/c mouse asthma model

### 1. Experimental methods and materials

### 1.1 Laboratory animal treatment

A total of 40 female BALB/c mice were purchased from Shanghai Jihui Laboratory Animal Breeding Co., Ltd. or other qualified suppliers (including 5 spares, wherein the spares were only used for screening the appropriate weight range of the animals to be randomly grouped, without modeling or drug administration, and would be euthanized at the end of the experiment). Animals were all free of specific pathogens and were approximately 5 - 6 weeks old when arrived at the PharmaLegacy Animal House. Experimental protocols designed to be applied to the animals would be approved by PharmaLegacy's IACUC (Experimental Animal Care and Use Committee).

Based on the weight of the animals, the animals were randomly assigned to each treatment group using the BioBook random assignment function. The 35 animals were randomly divided into 4 groups: G1 group, i.e., normal group, 5 BALB/c mice; G2 group, i.e., model group, 10 BALB/c mice; G3 group, i.e., model treatment + PAO 0.1 mpk group; and G4 group, i.e., model treatment + PAO 0.3 mpk group.

Preparation of sensitization solution: 2 mg of ovalbumin was dissolved in 5 mL of phosphate buffer. The final concentration was 0.4 mg/mL. A 1:1 solution was prepared by mixing 5 mL of ovalbumin solution and 5 mL of aluminum hydroxide (40 mg/mL), 37 °C, and placed on a horizontal shaker for 30 min to shake and mix. Preparation of attack solution: 0.5 g of ovalbumin was dissolved in 50 mL of phosphate buffer. The final concentration was 10 mg/mL.

On day 1 and 14, mice in groups G2 - G4 were sensitized by intraperitoneal injection of sensitization solution (0.1 mL/mouse); mice in group G1 were injected intraperitoneally with 0.1 mL of phosphate buffer. On days 28 - 31, mice in groups G2 - G4 were attacked with an attack solution (1% OVA attack solution, BUXCO aerosolizing dosing system, 30 min). The animals in group G1 were attacked with phosphate buffer for 30 min.

### 1.2 Airway reactivity monitoring-Penh Value

On day 32, 4 hours after administration, animals were tested for airway hyperresponsiveness using a whole-body plethysmography WBP (Buxco BFL0100 WBP). All animals were nebulized to inhale 1.5625, 3.125, 6.25, 12.5, 25, and 50 mg/mL multiplicative concentrations of acetylmethacholine, and the augmented expiratory interval values were determined at the corresponding concentrations.

On day 32, after the WBP assay, animals were tracheally intubated after anesthesia using 25 - 50 mg/kg of Sutent and 5 - 10 mg/kg of mephenothiazine, and the lungs were lavaged for the first time with 0.4 mL of PBS (containing 1% FBS). The lungs were lavaged with another 0.4 mL of PBS (containing 1% FBS) for the second and third times. The bronchoalveolar lavage fluid (BALF) from the three lavages was placed on ice. BALF was centrifuged for 5 min at 300 g at 4°C, and the cell-free BALF supernatant was collected and stored at -80°C.

Total leukocyte count: 100 µL of trypan blue solution was added to 100 µL of cell-containing BALF solution, and 10 µL of the mixture was added to the cell counting plate. The total number of cells in the four large squares was counted under the microscope. Total cell count (cells/mL) = absolute count * 100 µL / total BALF volume (mL).

Differential cell count: cell slides were wiped with alcohol. 100 µL of cell-containing BALF was added to a centrifugal coater. After centrifugation, the cells on the slide were allowed to dry naturally and then fixed in methanol for 1 minute. The cells were stained using Wright-Giemsa staining solution in order to distinguish eosinophils, neutrophils, macrophages and lymphocytes. The cells were found under a microscope with a 4x field of view and magnified by 20x. Under the microscope, the number of differentiated cells was counted until the count contained 200 cells in a randomly selected area with an even distribution of cells. Cell count = absolute count / 200 * total cell count (cells/mL).

After the animals were euthanized by cervical dislocation, the right lung tissue was collected and frozen at -80°C after liquid nitrogen flash freezing. Left lung tissue was collected, fixed in 10% neutral formaldehyde solution, embedded in a wax block using paraffin wax, and one section was cut longitudinally along the largest section for H&E staining, and scored for airway diameter, mucosal thickness, and inflammatory infiltrate by a pathologist.

### 1.3 Body weight

Animals were weighed and recorded twice a week throughout the experimental cycle.

### 1.4 Statistical analysis

Experimental data were expressed as mean ± standard error (mean ± S.E.M). Data were analyzed using SPSS or Graphpad Prism. The specific analysis methods used were described in the figure legends and in the notes below the tables. p < 0.05 was considered statistically different.

### 2. Results

### 2.1 Body weight analysis of experimental animals

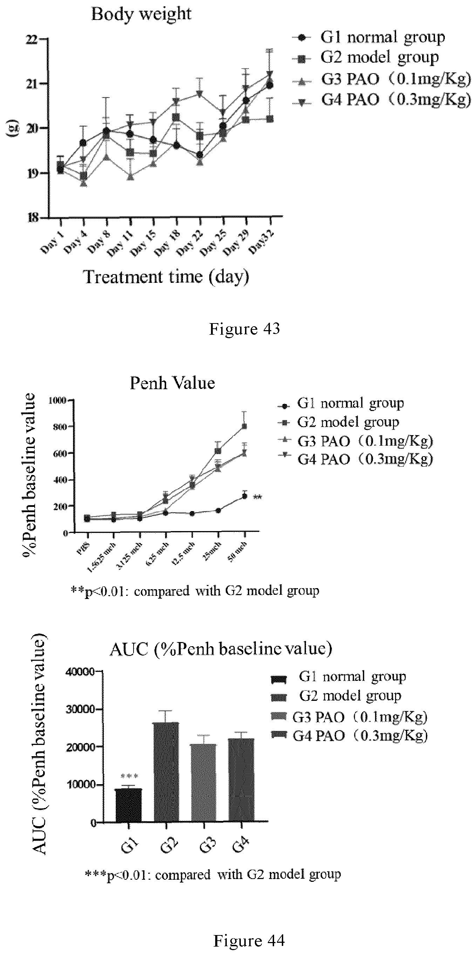

The body weights of mice in each group on days 1, 4, 8, 11, 15, 18, 22, 29 and 32 of the experiment were statistically analyzed. The experimental results showed that the body weight of mice in each group increased gradually with the increase of experimental duration. On the 32nd day of the experiment, there was no significant difference in the body weights of mice in the G1 normal group, G3 model + PAO 0.1 mpk group and G4 model + PAO 0.3 mpk group when compared with those in the G2 model group; there was a decreasing trend in the body weight of mice in the G2 model group compared with the G1 normal group, and there was no significant decreasing trend in the G3 model + PAO 0.1 mpk group and the G4 model + PAO 0.3 mpk group (Figure 43).

### 2.2 PAO improved respiratory function in model group mice

On day 32 of the experiment, 4 hours after administration, the animals were tested for airway hyperresponsiveness using a whole-body plethysmography WBP (Buxco BFL0100 WBP). All animals were nebulized to inhale 1.5625, 3.125, 6.25, 12.5, 25, 50 mg/mL multiplying concentrations of acetylmethacholine, and enhanced expiratory interval values were determined at the corresponding concentrations. Compared with the G1 normal group, the Penh Value and area under the curve (AUC) statistics were significantly higher in the G2 model group, suggesting that the enhanced expiratory interval values were significantly higher and the respiratory function was decreased in the model group mice; compared with the G2 model group, the Penh Value and AUC statistical values of the G3 model + PAO 0.1 mpk group and the G4 model + PAO 0.3 mpk group showed a decreasing trend (Figure 44), suggesting that a certain concentration of PAO could improve the respiratory function of mice in the model group.

### 2.3 PAO reduced the number of multiple types of leukocytes in mouse lung lavage fluid

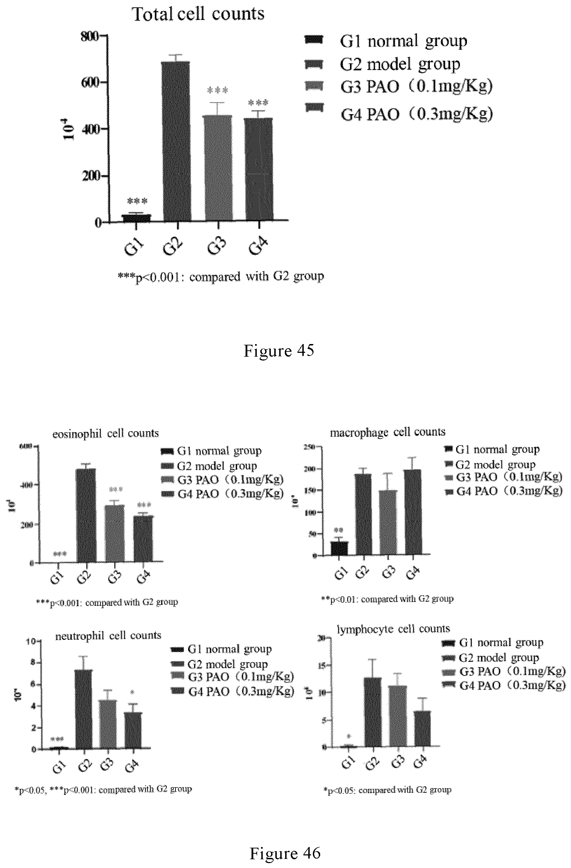

On the 32nd day of the experiment, the lungs of mice were lavaged three times using PBS, and the total leukocyte number and leukocyte classification cells in the lavage fluid were counted and analyzed, respectively. The experimental results showed that the total leukocyte count in the G2 model group was significantly increased compared with that in the G1 normal group; and the total leukocyte count in the G3 model + PAO 0.1 mpk group and the G4 model + PAO 0.3 mpk group was significantly decreased compared with that in the G2 model group (Figure 45), suggesting that PAO 0.1 mpk and 0.3 mpk treatments were able to significantly inhibit the rise in the total number of leukocytes in the lavage fluids caused by modeling. Statistical analysis of the different types of leukocytes in the lavage fluid showed that the number of eosinophils (EOS), macrophages (Mac), neutrophils (Neu) and lymphocytes (Lym) were significantly increased in the G2 model group compared with that in the G1 normal group; compared with the G2 model group, the number of eosinophils was significantly reduced in the G3 model + PAO 0.1mpk group and the G4 model + PAO 0.3mpk group, the number of neutrophils was significantly reduced in the G4 model + PAO 0.3mpk group, and the number of lymphocytes tended to decrease in the G3 model + PAO 0.1mpk group and the G4 model + PAO 0.3mpk group (Figure 46).

### 2.4 PAO improved respiratory pathology caused by modeling

After mice were euthanized and processed, right lung tissues were collected, snap-frozen in liquid nitrogen and frozen at -80°C. The left lung tissue was collected, fixed in 10% neutral formaldehyde solution, embedded in a wax block using paraffin wax, and one section was cut longitudinally along the largest section for H&E staining, and scored for airway diameter, mucosal thickness, and inflammatory infiltration by a pathologist.

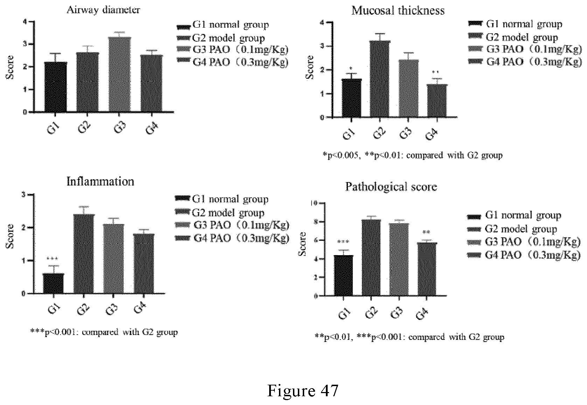

The results showed that the mucosal thickness score, inflammatory infiltration score and pathology score were significantly higher in the G2 model group compared with the G1 normal group; compared with the G2 model group, the mucosal thickness score in the G4 model + PAO 0.3 mpk group and the pathology score in the G4 model + PAO 0.3 mpk group were significantly decreased, and the inflammatory score tended to decrease in the G3 model + PAO 0.1 mpk group and the G4 model + PAO 0.3 mpk group (Figure 47).

### Example 20. Study of the Therapeutic Effects of PAO and d5PAO on Non-Alcoholic Steatohepatitis

### 1. Experimental Methods

### 1.1 HepG2 cell culture and processing

Human hepatoma cells HepG2 cells were cultured with DMEM containing 15% fetal bovine serum (FBS) at 37°C, 5% CO2, saturated humidity in an incubator, and cultured for 24 hours to allow wall adhesion. On the following day, 0.5 mM oleic acid (OA) was added, and then incubated for 24 hours according to the experimental needs. After 24 hours of modeling treatment, the cells were treated with different concentrations of PAO, d5PAO or f3PAO for 24 hours according to the experimental grouping.

### 1.2 Oil Red O staining

### 1.2.1 Preparation of staining solution

### a) Preparation of Oil Red O staining solution

Formulation of Oil Red O stock solution: 100% isopropyl alcohol 100 mL + 0.5 g Oil Red O dry powder, fully dissolved, filtered, and stored at 4°C away from light.

Formulation of Oil Red O working solution: before the experiment, the stock solution was diluted with distilled water according to 3:2, filtered with filter paper or polar filter membrane with 1mL syringe. The working solution was in burgundy color with no precipitation. The working solution was used immediately after prepared to avoid precipitation in the working solution.

### b) Preparation of 60% isopropanol

Formulation of 60% isopropanol: 60 mL of 100% isopropanol + 40 mL of distilled water, stored at 4 °C.

### 1.2.2 Staining

a) The cell culture solution was slowly aspirated and the cells were washed once by PBS;
b) The cells were fixed by 4% paraformaldehyde fixative (P0099) or 10% formaldehyde solution for 10 minutes and rinsed twice with PBS;
c) The cells were covered with appropriate amount of staining washing solution for 20 seconds;
d) The staining washing solution was aspirated, and an appropriate amount of Oil Red O staining working solution was added, and the cells were stained for 10-20 minutes;
e) The Oil Red O staining working solution was removed; an appropriate amount of staining washing solution was added; the cells were allowed to stand for 30 seconds, then the staining washing solution was removed and the cells were washed with PBS for 20 seconds;
f) The cells were covered evenly by appropriate amount of PBS, and then observed and photographed under the microscope.

### 2. Results

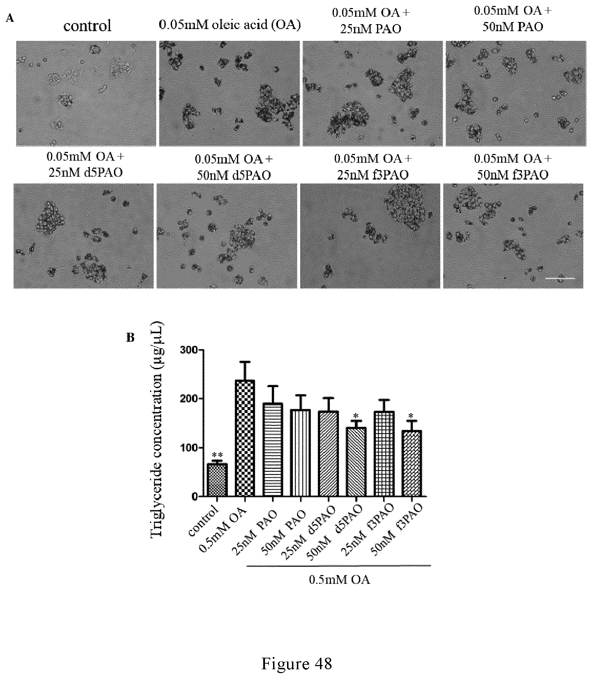

The effect of PAO, d5PAO, and f3PAO compounds on the oleic acid-treated HepG2 cell model was examined by oil red O staining and detection of triglyceride concentrations in cells. The results showed that PAO, d5PAO and f3PAO were able to reduce the accumulation of lipid droplets in HepG2 cells caused by 0.5 mM oleic acid treatment (Figure 48A). 0.5 mM oleic acid treatment resulted in a significant increase in triglyceride amount of the cells compared to the control, whereas 50 nM d5PAO and 50 nM f3PAO significantly reduced the accumulation of triglycerides caused by 0.5 mM oleic acid treatment (Figure 48B).

HepG2 cells were incubated for 24 hr with the addition of 0.5 mM oleic acid and then treated with different concentrations of PAO, d5PAO, or f3PAO for 24 hr according to experimental groupings. (A) treated with oil red O staining and photographed for analysis, scale bar: 50 µM; (B) triglycerides were detected and analyzed by One-way ANOVA, *p<0.03, **p<0.01, compared with 0.5 mM oleic acid-treated group (Figure 48).

This experiment revealed that PAO, d5PAO and f3PAO could prevent the accumulation of lipid droplets and triglycerides caused by oleic acid in hepatocytes, wherein the preventing effect was significant when the concentration of d5PAO and f3PAO was 50nM, thus alleviating the damage to hepatocytes.

### Example 21. Study of the effects of PAO and F3PAO on arterial stenosis

### 1. Experimental methods

### 1.1 Proliferation and migration of rat vascular smooth muscle cells (A7R5) induced by Ang2 in vitro:

Proliferation and migration model of rat thoracic aortic smooth muscle cells (A7R5) was established by using angiotensin 2 (1 µg/ml, Ang2), and was divided into (1) control group; (2) model group; and (3) drug-treated group (low, medium and high concentration). Cells were digested with trypsin and seeded in plates, and when the cell density reached 70%, the cells were starved with serum-free medium for 24 hours, and then Ang2 was added for modeling and intervened with different concentration gradients of PAO and F3PAO for 12-24 hours, and then the overall viability of the cells was examined by CCK8 assay; the changes of smooth muscle cell proliferation index (PCNA) and migration index (MMP9, collagen-1) were observed by qPCR, western, and cell immunofluorescence.

### 1.2 Detection of cell viability by CCK8 assay:

A7R5 cells were seeded uniformly at a density of 5*10³ into 96-well plates (Coming), and when the cells were completely adhered to the wall and fused to 70%-80%, the cells were starved with serum-free DMEM medium for 24 hours, then incubated with 1ug/ml Ang2 for about 48 hours to induce cell proliferation, after which, each well was added with 10 µl of CCK8 reagent (Beyotime) and 90 µL DMEM medium. After each well was incubated at 37°C for 2 hours, cell proliferation and cell viability were evaluated by detecting the absorbance (OD) value at 450 nm with a microplate reader. Five replicate wells were set in each group.

### 1.3 Detection of PCNA and MMP9 gene expression by fluorescence quantitative PCR:

Total RNA was extracted by RNA extraction kit (B0004DP, Suzhou EZBioscience Co., Ltd.), and the concentration and purity of RNA were determined by spectrophotometer (Thermo Fisher). cDNA was obtained by reverse transcription reaction of 1000 ng of total RNA using a reverse transcription kit (Shanghai Yi Sheng). The cDNA was used to amplify PCNA and MMP9 gene by fluorescence quantitative PCR using SYBR Green kit (Shanghai Yi Sheng), and GAPDH gene was used as internal reference. The cDNA sequences of the above genes were downloaded from NCBI website, and the PCR primers were synthesized by Sangon Biotech (Shanghai) Co., Ltd. The primer sequences were as follows: PCNA: forward TGGACTTAGACGTTGAGCAACTTGG, reverse GCACAGGAGATCACCACAGCATC; MMP9: GATCCCCAGAGCGTTACTCG, GTTGTGGAAACTCACACGCC; Col 1A: forward TAAGGGTCCCCAATGGTGAGA, reverse GGGTCCCTCGACTCCTACAT; GAPDH: forward CCAGGCGCCCAATACG, reverse CCACATCGCTCAGACACCAT.

### 1.4 Detection of PCNA, MMP9, and Col 1A protein expression by immunoblotting (Western Blotting) of cell extracts:

The medium was aspirated, and the petri dishes were washed 2 times with PBS. According to the total amount of cultured cells, appropriate amount of RIPA lysis containing protease inhibitor (Beyotime) was added, and the cells were lysed on ice for 30 minutes, during which the cells were vortexed and shaken several times to ensure sufficient protein lysis. The lysate was then centrifuged at 12,000g for 15 min at 4°C, and the supernatant was the total cellular protein obtained. The protein was quantified by using BCA kit (Beyotime). 30 µg of the protein was electrophoresed by applying SDSPAGE gel kit (Epizyme) gel. Col 1A antibody (Abcam), PCNA (Abcam), and MMP9 (Abcam) antibodies were used for primary antibody; HRP-labeled antibody (Beyotime) was used for secondary antibody, and GAPDH was used as internal reference. The bands were analyzed in grayscale by using Image-Pro Plus 6.0 software.

### 1.5 A7R5 cell migration capacity test (scratch assay):

A7R5 cells were inoculated into 6-well plates with parallel lines drawn. When the cell density was about 80%, the cells were starved (cultured in serum-free medium) for 24 h. After the starving, scratches were made along the previously drawn parallel lines with a 200 µL sterilized pipette tip. The cells were washed three times with PBS solution, and the shed cells were aspirated and discarded, after which the cells were subjected to Ang2 modeling and pharmacological intervention, with three replicate wells in each group, respectively. The migration of cells in the scratched area was observed under an inverted microscope at 0 h, 48 h, respectively. The scratch width and scratch area after migration in each microscopic field of view were analyzed using ImageJ software, and the migration rate was calculated as the percentage of the migrated scratch area to the initial scratch area.

### 1.6. Statistical processing:

Statistical analysis was performed using SPSS22.0 software. Measurement information was expressed as x±s, and independent sample t-test was used for comparison between two groups; more than 3 groups were statistically analyzed by one-way ANOVA, and inter-groups were analyzed using Bonferroni test, and count information was expressed as rate (%), and χ2 test was used for comparison between groups, p < 0.05 was considered as statistically different. # indicated comparison with control, #p<0.05, ##p<0.001, ###p<0.0001. * indicated comparison with model, *p<0.05, **p<0.001, ***p<0,0001.

### 2. Results

### 2.1 F3PAO inhibited Ang2-stimulated vascular smooth muscle cell growth (CCK8 assay)

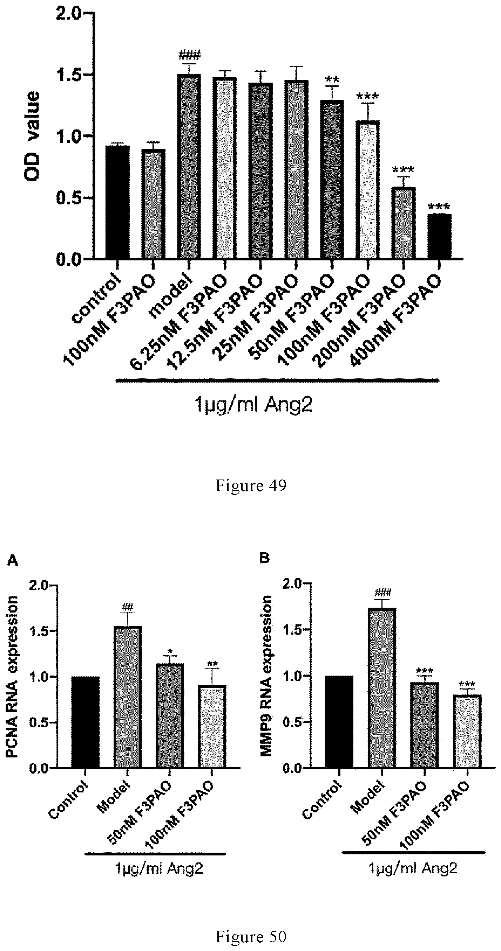

The results showed (Figure 49) that under the intervention of 1 µg/ml Ang2, the proliferation capacity of vascular smooth muscle cells was significantly elevated compared to the control group, from 92% ± 0.9% in the control group to about 150% ± 3.5% in the model group (###p<0.0001), indicating that Ang2 intervention stimulated the proliferation and migration of vascular smooth muscle cells. The addition of different concentrations of F3PAO under the condition of Ang2 intervention, respectively, revealed that the proliferation and migration ability of vascular smooth muscle cells also decreased, and when the concentration of F3PAO reached 50 nM, there was statistical difference compared with the model group (50 nM,**p<0.001, >100 nM,***p<0,0001). Below 50 nM F3PAO, on the other hand, did not significantly change the inhibition of cell viability. The experiments indicated that F3PAO could effectively inhibit the growth of Ang2-stimulated vascular smooth muscle cells. n=3, processed using one-way ANOVA with T-test analysis between groups, and data were expressed as mean±SEM.

### 2.2 F3PAO inhibited Ang2-induced PCNA, MMP9 gene expression in A7R5 cells (Quantitative PCR, Q-PCR)

PCNA, MMP9 is a typical proliferation and migration marker protein. Q-PCR revealed that the expression of MMP9 gene was significantly increased after Ang2 intervention (from 100% to 173%±10.2%, P<0.0001), while this expression-enhancing effect could be inhibited by F3PAO (50, 100nM), which was a dose-dependent inhibitory effect: the higher the concentration of F3PAO, the more significant the inhibitory effect. MMP9 expression were 93%±7.6%, P<0.05; and 80%±6.1%, P<0.001, respectively. (Figure 50A).

The expression of PCNA was also significantly increased after Ang2 intervention (from 100% to 156%±8.2%), while this expression-enhancing effect could be inhibited by F3PAO (50, 100nM), which was a dose-dependent inhibitory effect: the higher the concentration of F3PAO, the more significant the inhibitory effect (PCNA expression was 115%±4.7%, P<0.0001, and 91%±10.7%, P<0.0001, respectively) (Figure 50B). n=3, processed using one-way ANOVA with T-test analysis between groups, and data were expressed as mean±SEM.

### 2.3 F3PAO inhibited Ang2-induced expression of PCNA and MMP9 proteins in A7R5 cells (WB)

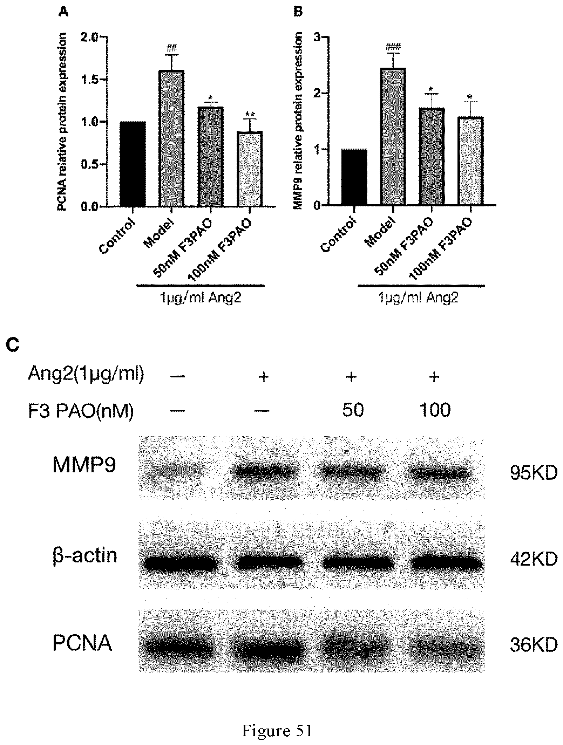

The expression of PCNA protein was significantly increased after Ang2 intervention (from 100% to 161%±10.2%, P<0.0001), while this expression-enhancing effect could be inhibited by F3PAO (50, 100nM), which was a dose-dependent inhibitory effect: the higher the concentration of F3PAO, the more significant the inhibitory effect (PCNA expression was 118%±3.0%, P<0.05; and 89%±8.3%, P<0.001, respectively) (Figure 51A, C).

The expression of MMP9 protein was significantly increased after Ang2 intervention (from 100% to 245%±15.2%, P<0.0001), while this expression-enhancing effect could be inhibited by F3PAO (50, 100nM), which was a dose-dependent inhibitory effect: the higher the concentration of F3PAO, the more significant the inhibitory effect. (MMP9 expression was 174%±14.4%, P<0.05, and 158%±15.3%, P<0.05, respectively) (Figure 51B, C). n=3, processed using one-way ANOVA with T-test analysis between groups, and data were expressed as mean±SEM.

### 2.4 PAO/F3PAO/GSK-A1 inhibited Ang2 stimulation-induced cellular Collagen-1 protein expression (WB)

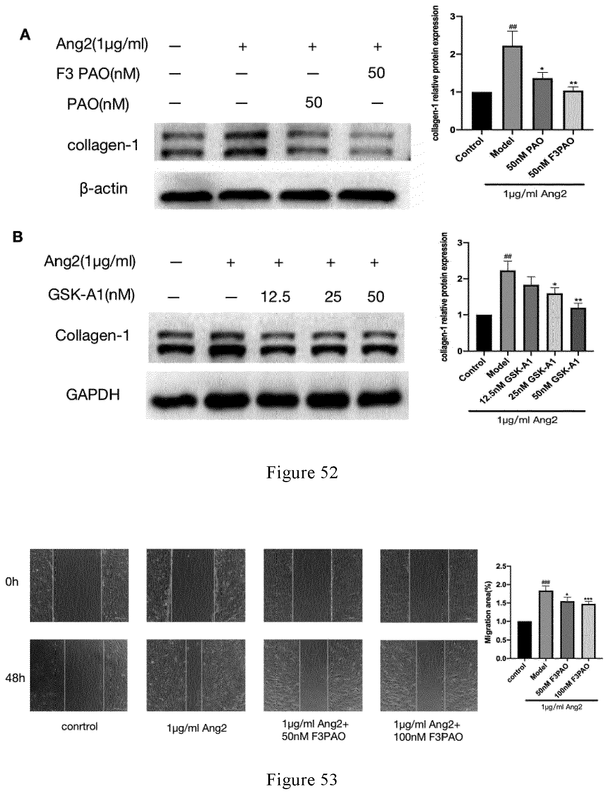

In Figure 52A, WB results showed that Ang2 significantly increased the expression of Collagen-1 protein in vascular smooth muscle (from 100% to 220%±22.4%, P<0.001), whereas this expression-enhancing effect could be inhibited by PAO/F3PAO (50nM). The expression of Collagen-1 was 136%±8.7%, P<0.05 and 104%±5.8%, P<0.001, respectively. In Figure 52B, WB results showed that Ang2 significantly increased the expression of Collagen-1 protein in vascular smooth muscle (from 100% to 223%±15.0%, P<0.001), whereas this expression-enhancing effect could be inhibited by GSK-A1 (25nM, 50nM). The expression of Collagen-1 was 160%±9.0%, P<0.05, and 120% 7.2%, P<0.001, respectively. n=3, processed using one-way ANOVA with T-test analysis between groups, and data were expressed as mean±SEM, (*p<0.05, **p<0.001). Considering that GAK-A1 is an inhibitor of PI4KIIIα, the WB results suggested that PI4KIIIα was likely related to collagen deposition in vascular smooth muscle cells or vascular sclerosis, suggesting that inhibition of PI4KIIIα might improve vascular sclerosis.

### 2.5 F3PAO inhibited Ang2-induced enhancement of vascular smooth muscle cell migration (scratch assay)

Scratch assay showed that the migration capacity of cells was significantly increased after Ang2 intervention from 100% in control group to 184%±7.4% (P<0.0001), while the growth of migration capacity could be effectively inhibited by F3PAO (50 nM and 100 nM) (155%±6.4%, P<0.05, and 147%±4.1%, P<0.0001) (Figure 53). n=3, T-test analysis was used between groups and data were expressed as mean±SEM. (Migration% = (initial scratch area - scratch area after 24 h)/initial scratch area × 100%.)

The above results showed that F3PAO could inhibit Ang2-induced proliferation and migration of vascular smooth muscle cells cultured in vitro. It was also found that PAO/F3PAO/GSK-A1 could all inhibit the Ang2 stimulation-induced increase in the expression of collagen in vascular smooth muscle cells at the protein level, and these results suggested that F3PAO might have an inhibitory effect on the stenosis of the vasculature after endothelial injury caused various reasons. PAO/F3PAO/GSK-A1 can inhibit collagen on vascular smooth muscle and thus might be a novel drug on inhibition of hypertension as well as late vascular sclerosis after PCI.

It should be apparent to persons skilled in the art that although specific embodiments of the present disclosure are described herein for purposes of illustration, various modifications may be made thereto without departing from the spirit and scope of the present disclosure. Therefore, the specific embodiments and examples of the present disclosure should not be regarded as limiting the scope of the present disclosure. The present disclosure is limited only by the appended claims. All literature cited herein is incorporated herein by reference in its entirety.

## Claims

1. A compound of Formula (I) or a pharmaceutically acceptable salt thereof, 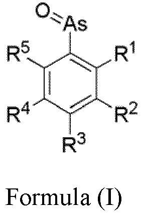 wherein R¹, R², R³, R⁴, R⁵ are independently selected from hydrogen, deuterium, fluorine, chlorine, bromine, iodine, methyl, amino, nitro, mono-deuteromethyl, di-deuteromethyl, tri-deuteromethyl, mono-fluoromethyl, di-fluoromethyl, or tri-fluoromethyl, and at least one of R¹, R², R³, R⁴, R⁵ is halogen or deuterium.

2. The compound of claim 1, or a pharmaceutically acceptable salt thereof, wherein R¹, R², R³, R⁴, R⁵ are independently selected from hydrogen or deuterium or halogen, and at least one of R¹, R², R³, R⁴, R⁵ is halogen, at least two of R¹, R², R³, R⁴, R⁵ are halogens, preferably at least two, three, four or five of R¹, R², R³, R⁴, R⁵ are fluorine.

3. The compound of claim 1, or a pharmaceutically acceptable salt thereof, wherein the compound of Formula (I) optionally forms a compound of Formula (I') when dissolved in a solvent, in particular an aqueous solvent, wherein R¹, R², R³, R⁴, R⁵ have the same meaning as described in the preceding claims.

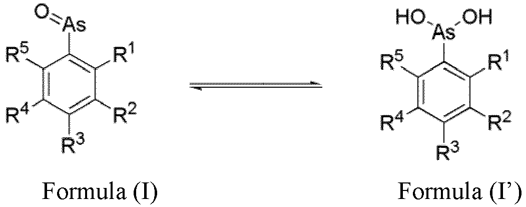

4. The compound of claim 2 or 3, or a pharmaceutically acceptable salt thereof, wherein the compound is selected from the group consisting of the following compounds:

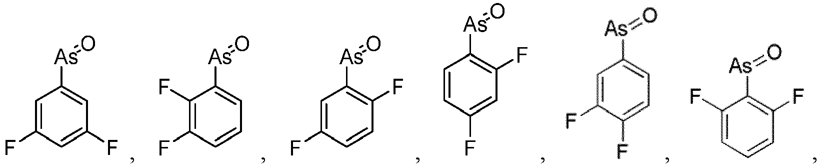

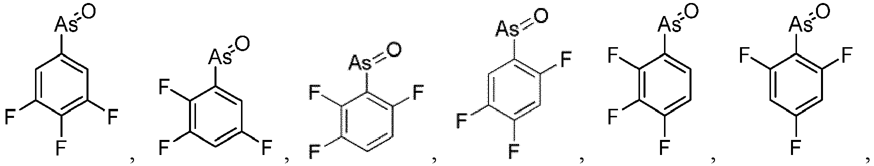

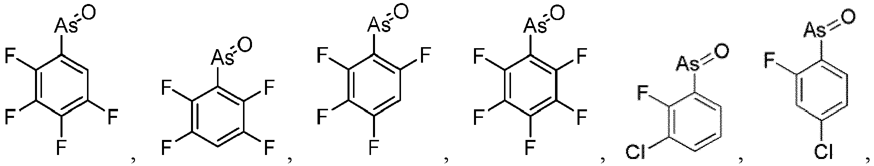

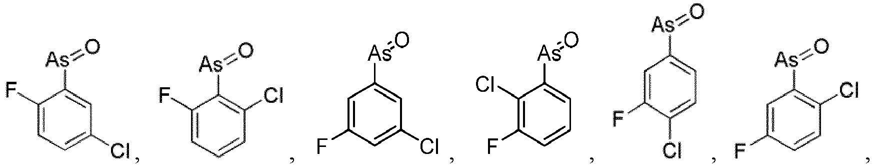

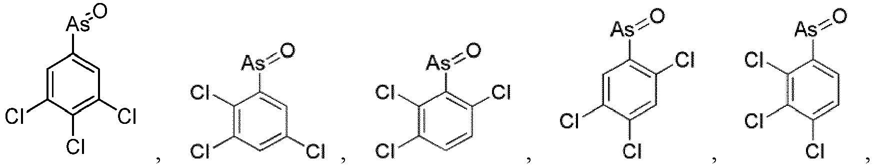

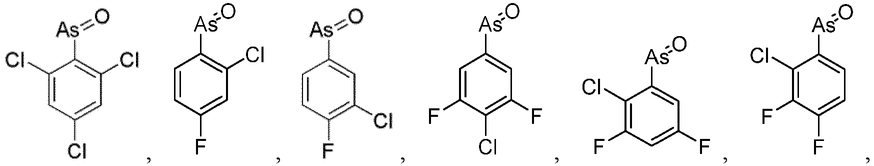

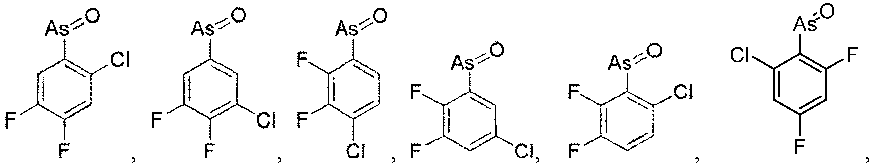

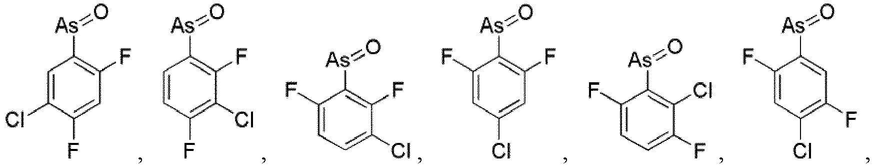

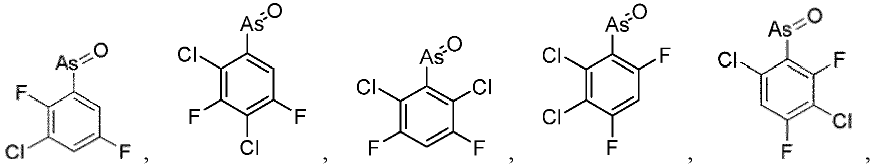

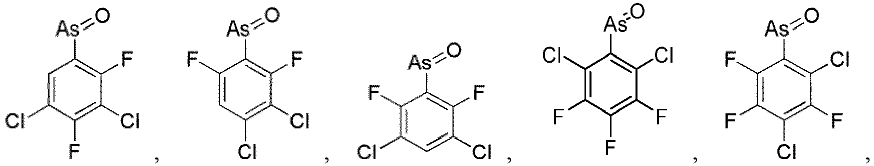

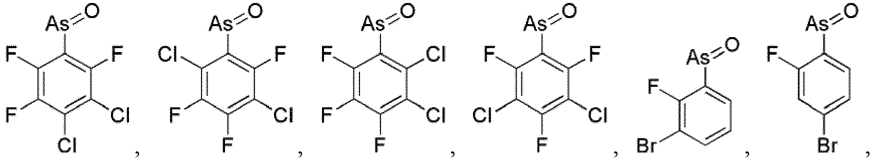

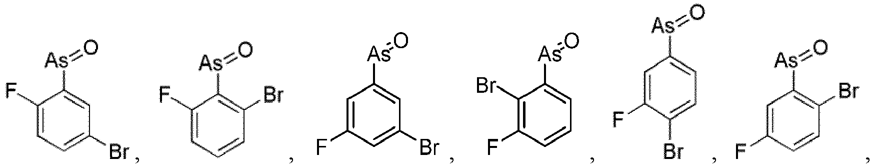

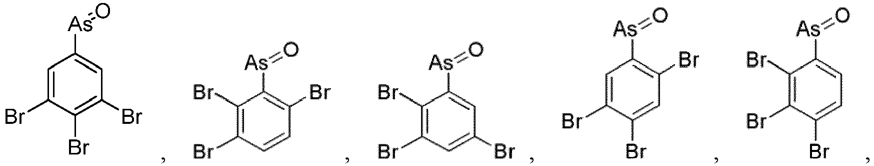

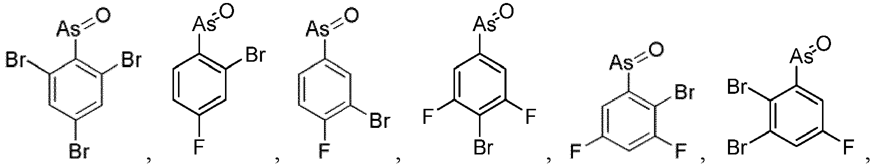

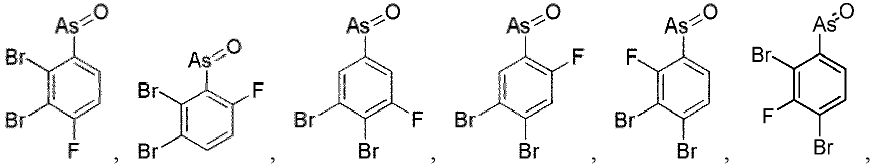

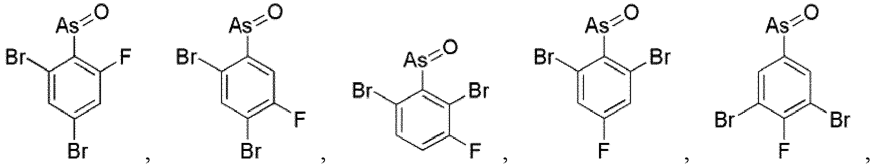

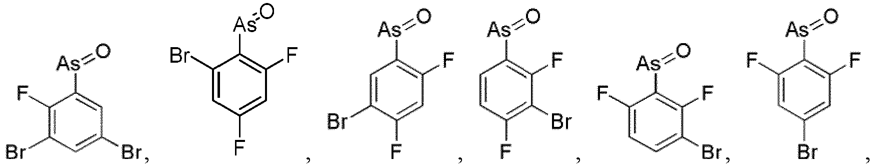

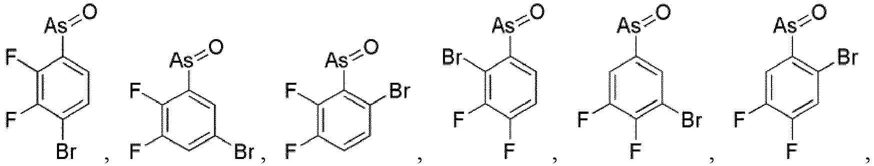

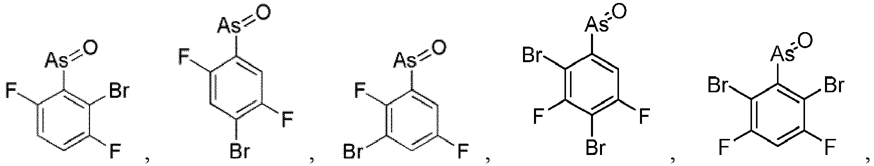

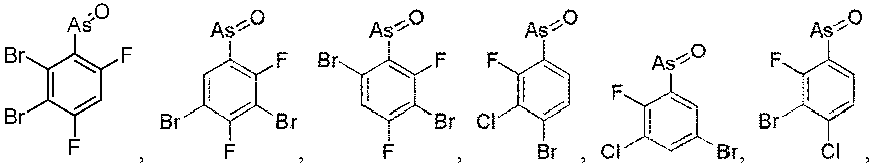

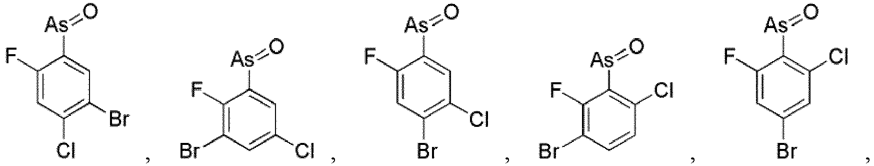

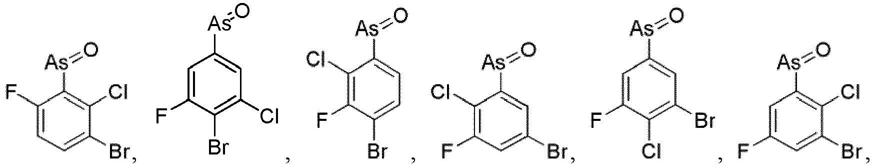

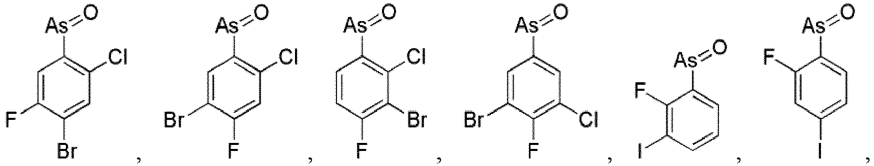

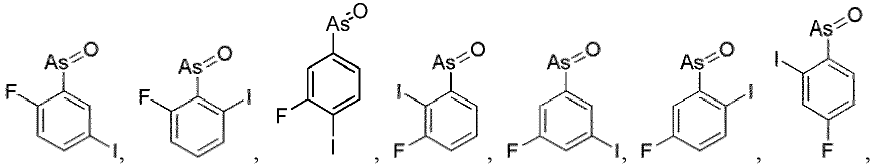

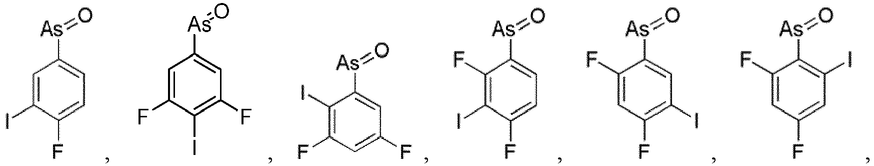

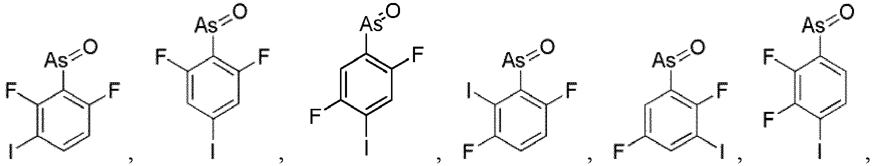

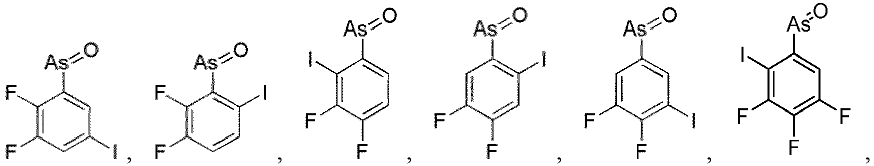

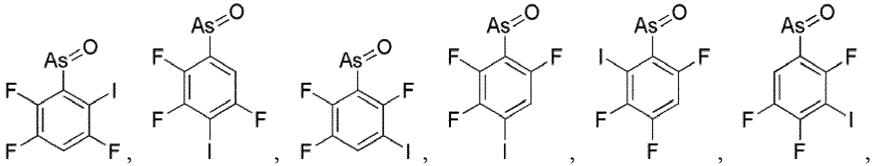

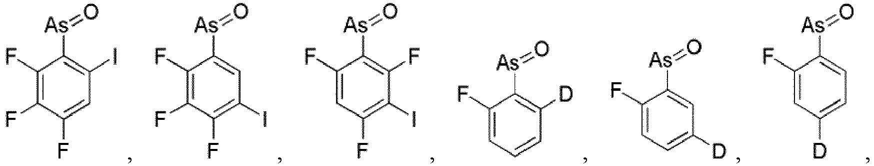

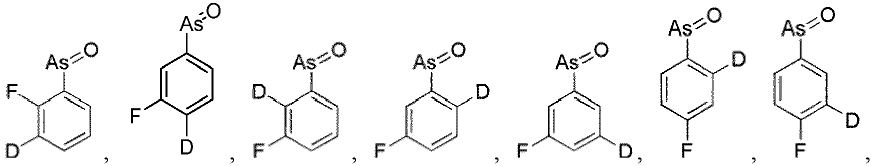

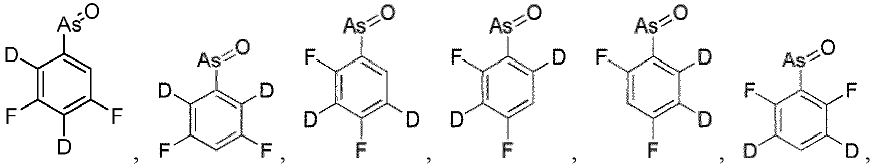

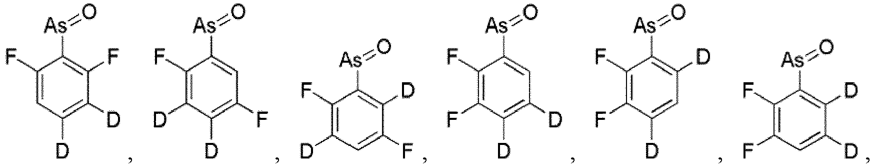

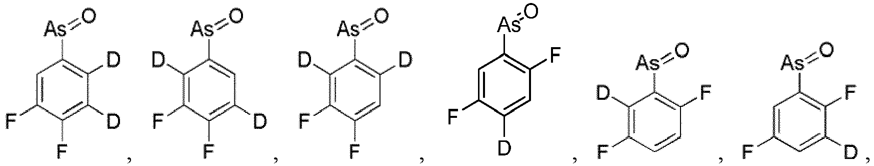

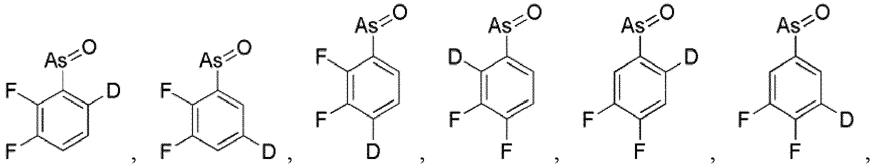

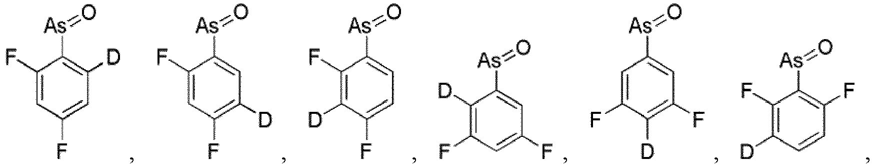

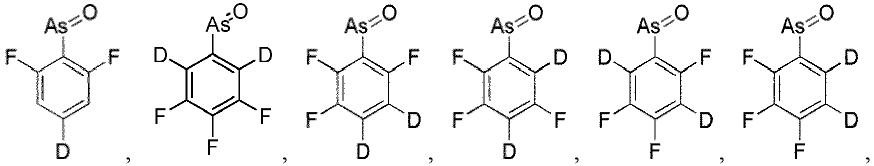

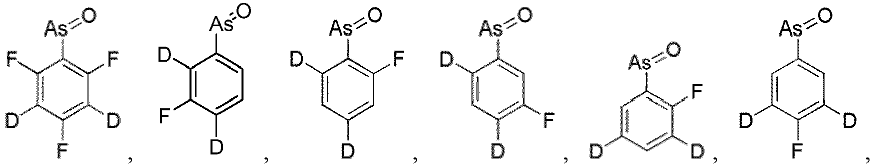

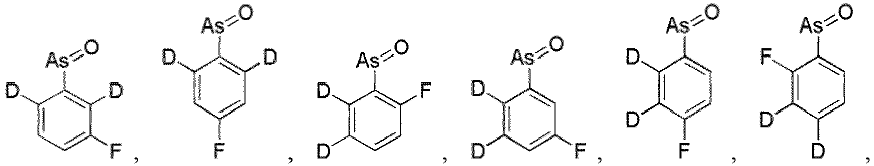

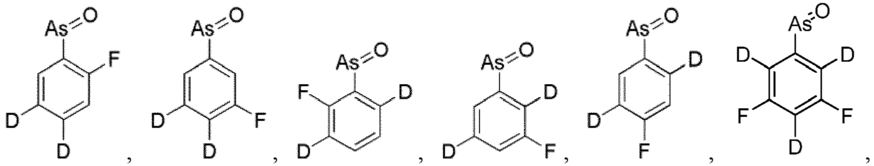

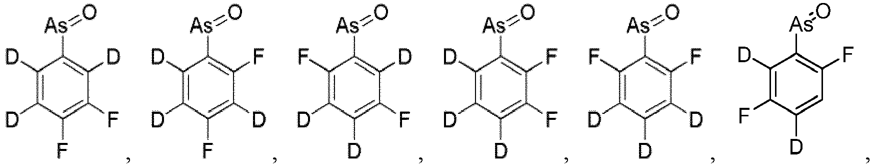

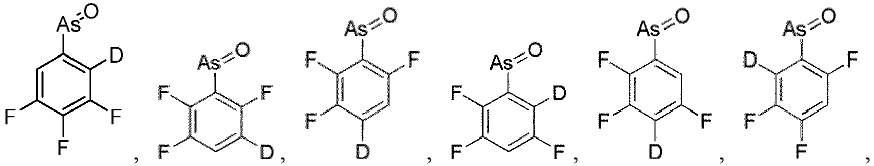

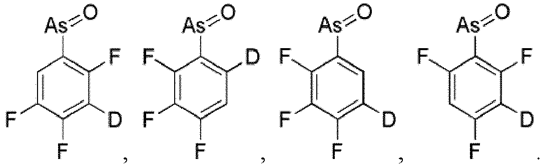

5. Use of the compound of any one of claims 1 to 4, or a pharmaceutically acceptable salt thereof, in the manufacture of a medicament for preventing or treating a disease or a pathological reaction in a subject.

6. The use of claim 5, wherein the disease is selected from at least one of tumor, malignant disease such as malignant tumor or malignant disease caused by chemotherapeutic drug for the treatment of tumor, Alzheimer's disease, intracellular protein misfolding related disease, lysosomal storage disease, inflammatory response, tissue or organ fibrosis, virus-infected disease, neurosis, microbial infection such as infection of drug-resistant microorganism, cellular tissue damage resulting from exposure to radiation, atherosclerosis, diabetes and asthma.

7. The use of claim 5, wherein the subject is human or non-human mammal.

8. The use of claim 6, wherein the tumor is selected from lymphoma (e.g., acute non-B or non-T cell lymphoma), cervical cancer, endometrial cancer, liver cancer, breast cancer (e.g., triple-negative breast cancer), lung cancer (e.g., non-small cell lung cancer or small cell lung cancer), colorectal cancer, gastric cancer, skin cancer (e.g., melanoma), bone cancer, osteosarcoma, myeloma, blood cancer (e.g., acute or chronic leukemia), ovarian cancer, prostate tumor, brain tumor (e.g., cerebral astrocytoma), neuroblastoma, thyroid cancer, malignant embryonal rhabdomyosarcoma.

9. The use of claim 6, wherein the intracellular protein misfolding related disease is Parkinson's disease, dementia with Lewy bodies, multiple system atrophy, inclusion body myositis, frontotemporal dementia, Huntington's disease, polyglutamine disease, amyotrophic lateral sclerosis, or prion disease.

10. The use of claim 6, wherein the lysosomal storage disease is a disorder of sphingolipid metabolism such as Gaucher disease, Niemann's disease type C, mucopolysaccharidosis, glycogen storage disease, glycoprotein storage disease, lipid storage disease, post-translational modification defects, intrinsic membrane protein deletion disorder, neuronal waxy plasma lipofuscinosis, or lysosome-associated organelle disorder.

11. The use of claim 6, wherein the inflammatory response is an increase of inflammatory factors such as TNFα or IL-6 in a local tissue or in blood throughout a body.

12. The use of claim 6, wherein the tissue or organ fibrosis is selected from cardiac fibrosis, renal fibrosis, pulmonary fibrosis, liver fibrosis, fibrosis during wound healing, or restenosis due to scar formation or fibrosis after arterial stent placement.

13. The use of claim 6, wherein the virus comprises coronavirus and non-coronavirus, preferably the coronavirus is selected from chicken infectious bronchitis virus, porcine epidemic diarrhea virus, porcine infectious gastroenteritis virus, porcine hemagglutinating encephalomyelitis virus, porcine δ-coronavirus, canine respiratory coronavirus, murine hepatitis virus, feline coronavirus, human coronavirus, human papillomavirus, severe acute respiratory syndrome virus, Middle East Respiratory Syndrome virus, or novel coronavirus; the non-coronavirus is selected from Hepatitis C virus or HIV.

14. The use of claim 6, wherein the neurosis is selected from neurasthenia, anxiety, depression or mania.

15. The use of claim 6, wherein the microbial infection is selected from bacterial or fungal infection, preferably drug-resistant bacterial infection or drug-resistant fungal infection, more preferably wherein the microorganism is selected from Acinetobacter baumannii, Enterobacter cloacae, Escherichia coli, Klebsiella oxytoca, Klebsiella pneumoniae, Morganella morgani, Pseudomonas aeruginosa, Serratia marcescens, Staphylococcus aureus, Enterococcus faecalis, Enterococcus faecium, Streptococcus pneumoniae, Candida albicans, Candida parapsilosis, Aspergillus fumigatus, Neisseria gonorrhoeae, Clostridium tetani, Propionibacterium acnes, Cryptococcus neoformans, Helicobacter pylori, Bacterioides fragilis, Clostridium difficile, Gardnerella vaginalis, Atopobium vaginae, Peptostreptococcus anaerobius, Trichomonas vaginalis, and at least one of the drug-resistant organisms thereof; or, preferably, the microbial infection is selected from a microorganism infecting surface of body or mucosal site such as the site of acne wound, pimple, wound, burn or scald.

16. The use of any one of claims 6 to 15, further comprising administering a second reagent to a subj ect in need thereof.

17. The use of claim 16, wherein the compound of any one of claims 1 to 4, or a pharmaceutically acceptable salt thereof, is administered before, after or simultaneously with the second reagent.

18. A pharmaceutical composition, comprising the compound of any one of claims 1 to 4, or a pharmaceutically acceptable salt thereof, and a pharmaceutically acceptable carrier.

19. The pharmaceutical composition of claim 18, wherein the pharmaceutical composition further comprises a second agent such as a drug for treating tumors, wherein the drug for treating tumors is preferably an antineoplastic agent interfering with mitosis such as paclitaxel or albumin paclitaxel, a drug directly affecting DNA structure and function such as carboplatin or cisplatin, or a Kras inhibitor.

20. A method of preparing the compound of any one of claims 1 to 4, or a pharmaceutically acceptable salt thereof, comprising the steps of: 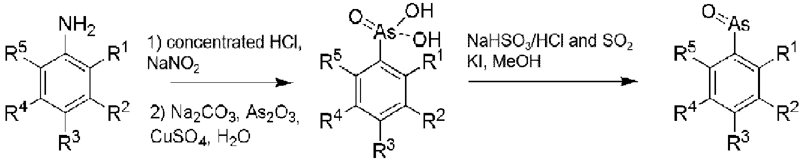
1) adding concentrated hydrochloric acid, aqueous sodium nitrite to an aqueous solution of aniline having the structure of Formula (I) or the salt thereof at 0°C~10°C and keeping the temperature below 5°C;
2) heating an aqueous solution of sodium carbonate, arsenic trioxide, copper sulfate to 90~100°C and then cooling down the aqueous solution, adding the solution prepared in step 1) into the aqueous solution, stirring, filtering, and adding acid to the filtrate to adjust the pH, and separating precipitated solid;
3) stirring the above precipitated solid, potassium iodide, sodium bisulfite or hydrochloric acid and sulfur dioxide in methanol until the reaction is complete and post-treating to obtain the compound.

21. Use of a PI4KIIIα-specific inhibitor in the manufacture of a medicament for preventing or treating cardiac fibrosis.

22. Use of a PI4KIIIα-specific inhibitor in the manufacture of a medicament for preventing or treating cellular tissue damage caused by radiation exposure in a tumor patient.

23. Use of a PI4KIIIα-specific inhibitor of phenylarsine oxide and a derivative thereof in the manufacture of a medicament for preventing or treating atherosclerosis or atherosclerosis-induced disease, wherein the atherosclerosis-induced disease is preferably coronary artery disease, cardiac infarction, or cerebral infarction.

24. Use of a PI4KIIIα-specific inhibitor in the manufacture of a medicament for preventing or treating diabetes in a tumor patient.

25. Use of a PI4KIIIα-specific inhibitor in the manufacture of a medicament for preventing or treating a microbial infection, wherein the microbial infection is selected from bacterial or fungal infection, preferably drug-resistant bacterial infection or drug-resistant fungal infection, more preferably wherein the microorganism is selected from Acinetobacter baumannii, Enterobacter cloacae, Escherichia coli, Klebsiella oxytoca, Klebsiella pneumoniae, Morganella morgani, Pseudomonas aeruginosa, Serratia marcescens, Staphylococcus aureus, Enterococcus faecalis, Enterococcus faecium, Streptococcus pneumoniae, Candida albicans, Candida parapsilosis, Aspergillus fumigatus, Neisseria gonorrhoeae, Clostridium tetani, Propionibacterium acnes, Cryptococcus neoformans, Helicobacter pylori, Bacterioides fragilis, Clostridium difficile, Gardnerella vaginalis, Atopobium vaginae, Peptostreptococcus anaerobius, Trichomonas vaginalis, and at least one of the drug-resistant organisms thereof; or, preferably, the microbial infection is selected from a microorganism infecting surface of body or mucosal site such as the site of acne wound, pimple, wound, burn or scald.

26. Use of a PI4KIIIα-specific inhibitor in the manufacture of a medicament for preventing or treating asthma.

27. Use of a PI4KIIIα-specific inhibitor the manufacture of a medicament for preventing or treating nonalcoholic steatohepatitis.

28. The use of any one of claims 21 to 27, wherein phenylarsine oxide and a derivative thereof are an antibody, a small molecule compound, a RNAi molecule or an antisense nucleic acid, preferably the antibody is a monoclonal or polyclonal antibody, more preferably the antibody is a chimeric antibody, a humanized antibody or a fully human antibody; preferably the RNAi molecule is a small interfering RNA (siRNA), a short hairpin RNA (shRNA) or microRNA (miRNA), more preferably the RNAi molecule has a length of 18-100 bases and/or the RNAi molecule is modified to enhance its stability; preferably the small molecule compound is phenylarsine oxide or a derivative thereof, G1 and an analogue thereof, A1 and an analogue thereof (J Med Chem 2014 Vol. 57 Issue 5 Pages 2091-106 and J Biol Chem 2014 Vol. 289 Issue 9 Pages 6120-32), or simepivir and an analogue thereof (Int J Radiation Oncol Biol Phys, Vol. 96, No. 4, pp. 867e876, 2016), TG-1478 ( Tyrphostin AG-1478, NSC-693255), more preferably phenylarsine oxide and the derivative thereof have a structure of Formula (II), or a pharmaceutically acceptable salt thereof: 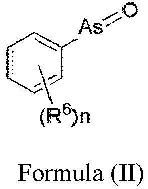
wherein R⁶ is each independently selected from (a) hydrogen, deuterium, methyl, mono-deuteromethyl, di-deuteromethyl, tri-deuteromethyl, halogen, nitro, cyano, hydroxyl, amino, carbamoyl, C1-6 alkylsulfonyl, C1-6 alkyl, C1-6 cycloa lkyl, C2-6 alkynyl, C2-6 alkenyl, C1-6 alkoxyl, C1-6 haloalkyl, C1-6 alkylene-NH2, C1-6 alkylene-NH-C(O)H, -As(O), -N=NH, N-(C1-6 alkyl) amino, N,N-(C1-6 alkyl)₂ amino, -NH-C(O)H, -NH-S(O)2H, -C(O)OH, -OC(O)H, -SH, -S(O) 2H, -S(O)2-NH2 or heterocyclyl, which is optionally substituted by R⁷ or R⁸, wherein R⁷ and R⁸ are each independently selected from amino, C1-6 alkyl, C 1-6 alkoxyl, C1-6 haloalkyl, N-(C1-6 alkyl) amino, N-(6-12-membered-aryl) ami no, N,N-(C1-6 alkyl)2 amino, C3-6 cycloalkyl, 6-12-membered aryl, or 3-12-me mbered heterocyclyl, which is optionally substituted by one or more halogen, n itro, cyano, hydroxyl, amino, carbamoyl, -NH-C(O)-R¹⁰, -C(O)OR⁹, 6-12-membe red aryl, C1-6 alkyl, C2-6 alkynyl, C2-6 alkenyl, C1-6 alkoxyl, C1-6 haloalkyl, 3-6-membered heterocyclyl, C3-6 cycloalkyl, or Bn-O-, and R⁹ is C1-6 alkyl, which is optionally substituted by one or more halogen, nitro, cyano, hydroxyl, amino, carbamoyl, 6-12-membered aryl, C1-6 alkyl, C2-6 alkynyl, C2-6 alkeny l, C1-6 alkoxyl, C1-6 haloalkyl, 3-6-membered heterocyclyl, C3-6 cycloalkyl, or Bn-O-, and R¹⁰ is selected from H, C1-6 alkyl, C2-6 alkynyl, C2-6 alkenyl, C1-6 alkoxyl or C1-6 haloalkyl, and/or
(b) R⁶ on two adjacent carbon atoms forms 5-12-membered cycloalkyl, ary l or heterocyclyl, which is optionally substituted by one or more halogen, nitro, cyano, hydroxyl, amino, carbamoyl, 6-12-membered aryl, C1-6 alkyl, C2-6 alk ynyl, C2-6 alkenyl, C1-6 alkoxyl, C1-6 haloalkyl, 3-6-membered heterocyclyl, C3-6 cycloalkyl or Bn-O-,
wherein n is an integer from 0 to 5.

29. The use of claim 28, wherein n is an integer from 0 to 2, R⁶ is each independently selected from H, deuterium, methyl, mono-deuteromethyl, di-deuteromethyl, tri-deuteromethyl, halogen, nitro, cyano, hydroxyl, amino, carbamoyl, C1-6 alkylsulfonyl, C1-6 alkyl, C1-6 cycloalkyl, C1-6 alkoxyl, C1-6 haloalkyl, -As(O), N-(C1-6 alkyl) amino, N,N-(C1-6 alkyl)2 amino, -NH-C(O)H or -NH-S(O)2H, which is optionally substituted by R⁷ or R⁸.

30. The use of claim 28, wherein n is an integer from 0 to 2, R¹ is each independently selected from H, deuterium, methyl, mono-deuteromethyl, di-deuteromethyl, tri-deuteromethyl, halogen, nitro, cyano, hydroxyl, amino, C1-6 alkylsulfonyl, C1-6 alkyl, C1-6 cycloalkyl, C1-6 alkoxyl, C1-6 haloalkyl, -As(O), -NH-C(O)H or -NH-S(O)2H, which is optionally substituted by R⁷ or R⁸.

31. The use of claim 28, wherein n is 1 or 2, R⁶ is each independently selected from H, deuterium, methyl, mono-deuteromethyl, di-deuteromethyl, tri-deuteromethyl, halogen, amino, C1-6 alkylsulfonyl, C1-6 cycloalkyl, C1-6 alkoxyl, C1-6 haloalkyl, -NH-C(O)R⁷ or -NH-S(O)2R⁸, wherein R⁷ is C1-6 alkyl which is optionally substituted by 6-12-membered aryl, R⁸ is 6-12-membered aryl which is optionally substituted by one halogen, C1-6 alkoxyl or C1-6 haloalkyl.

32. The use of claim 31, wherein the R⁶ is located at an ortho position and/or para position of the -As(O) group.

33. The use of claim 28, wherein n is 0.

34. The use of claim 28,
wherein, R¹, R², R³, R⁴, R⁵ are independently selected from H, deuterium, halogen, methyl, mono-deuteromethyl, di-deuteromethyl or tri-deuteromethyl, and at least one of R¹, R², R³, R⁴, R⁵ is deuterium or deuterated;
preferably, R¹, R², R³, R⁴, R⁵ are independently selected from H or deuterium, and at least one of R¹, R², R³, R⁴, R⁵ is deuterium, at least two of R¹, R², R³, R⁴, R⁵ are deuterium, preferably, at least three, four or five of R¹, R², R³, R⁴, R⁵ are deuterium;
more preferably, the compound is selected from the group consisting of the following compounds:

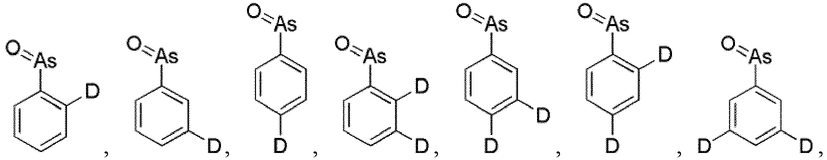

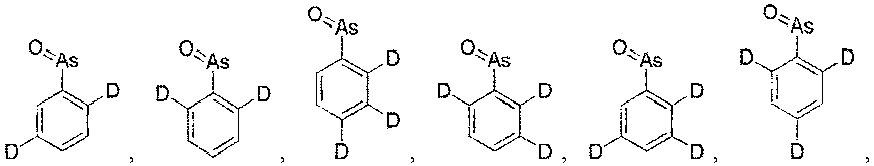

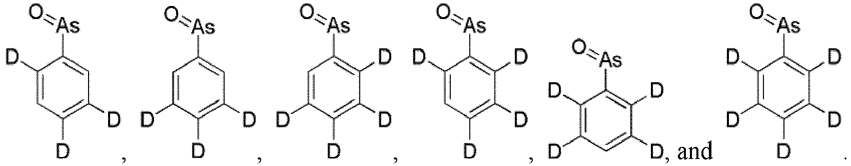

35. The use of claim 28, wherein the compound is selected from the group consisting of the following compounds:

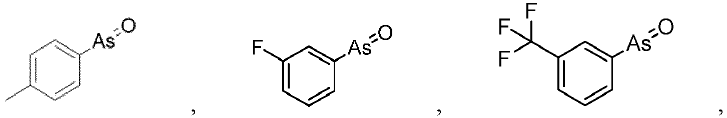

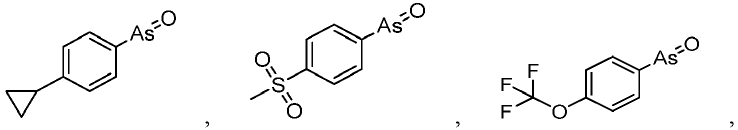

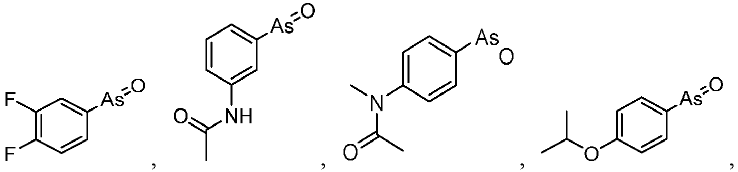

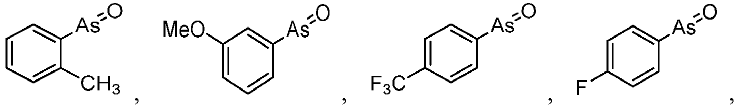

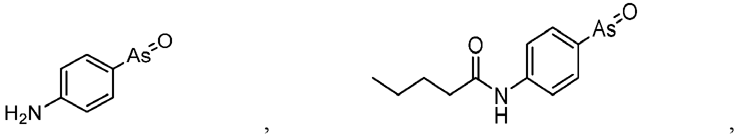

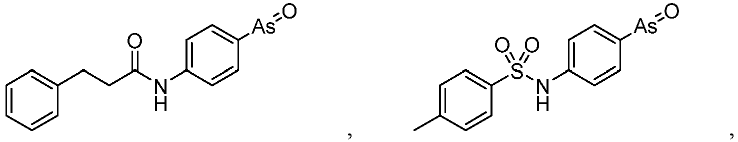

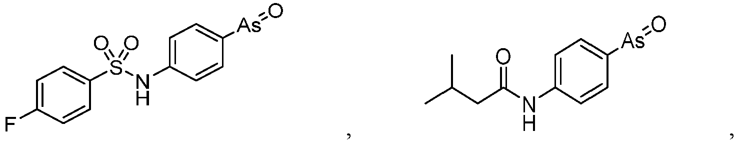

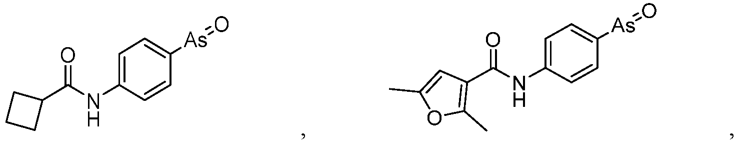

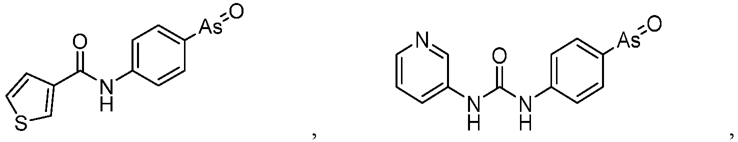

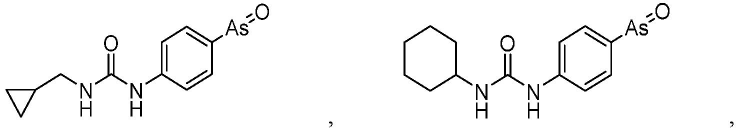

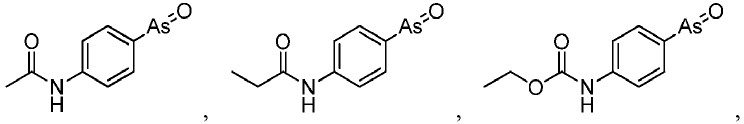

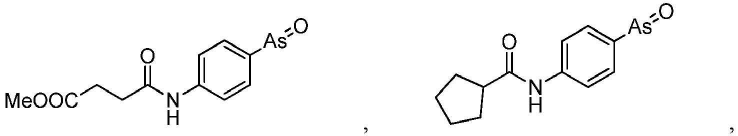

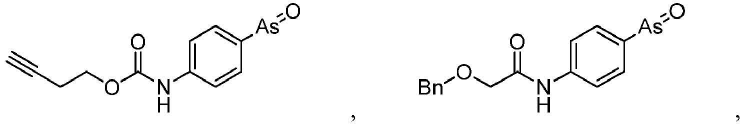

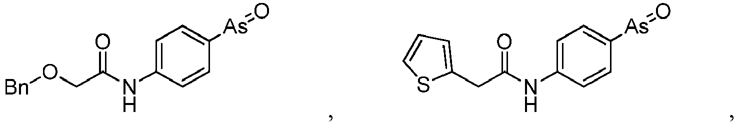

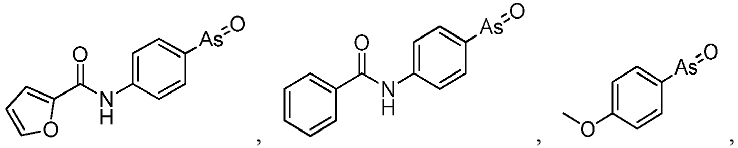

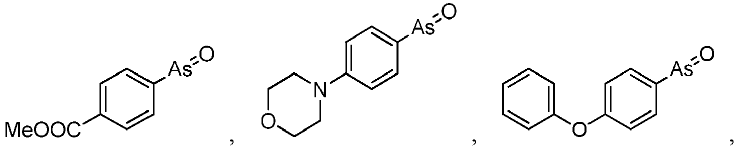

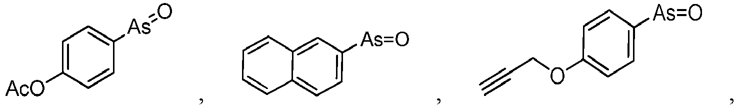

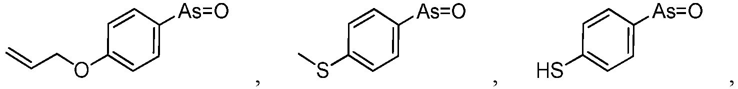

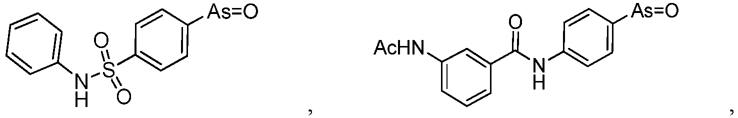

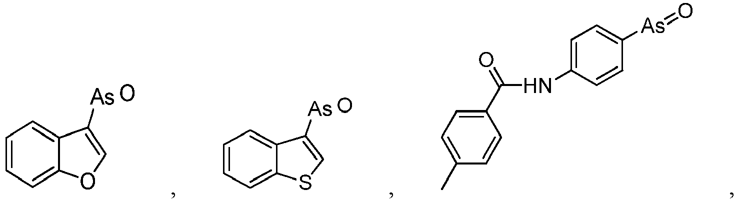

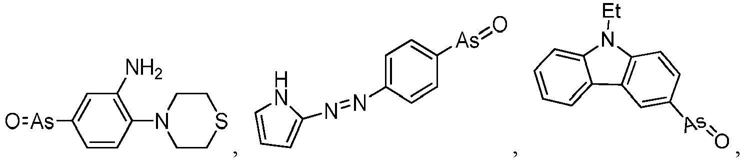

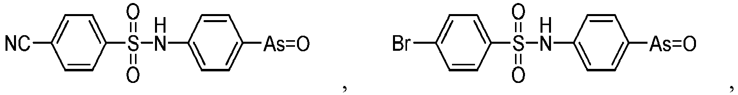

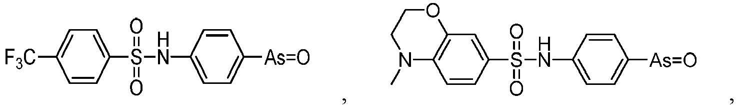

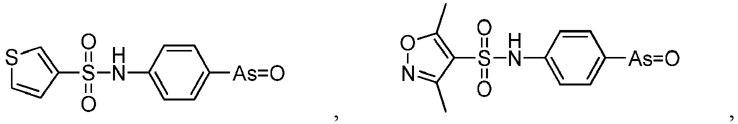

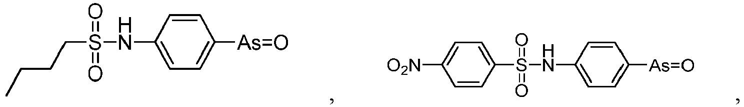

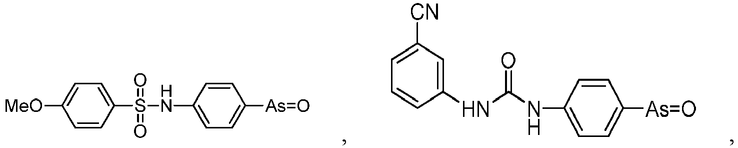

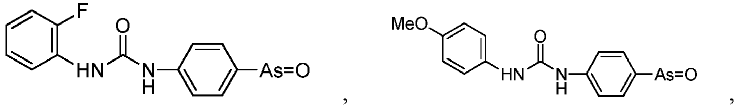

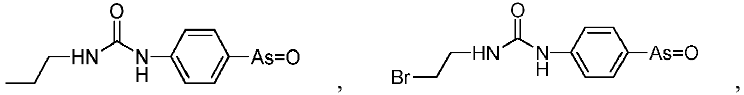

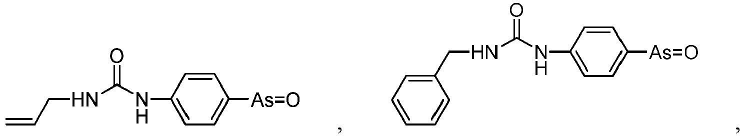

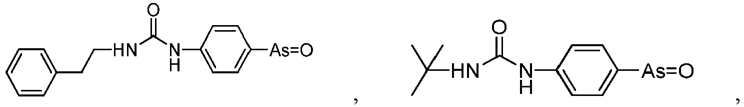

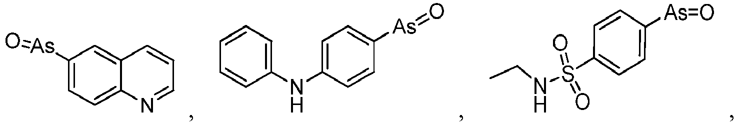

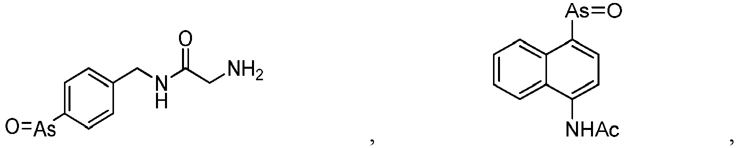

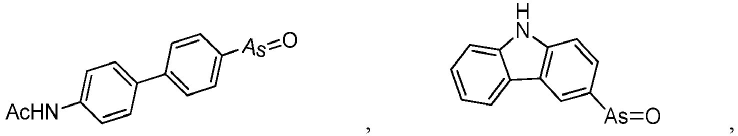

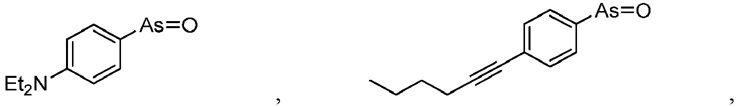

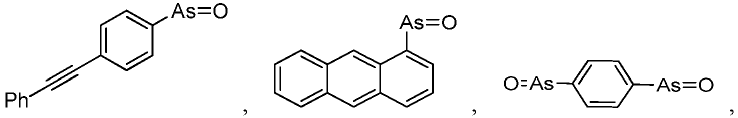

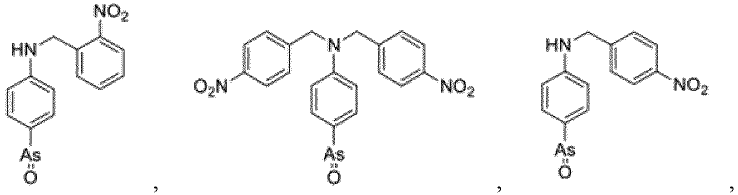

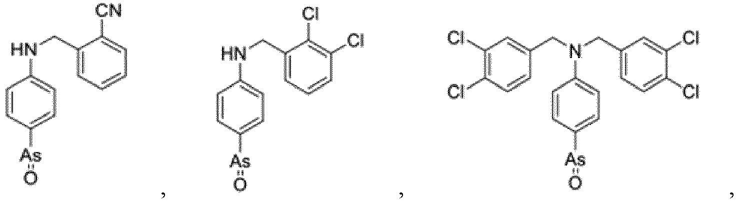

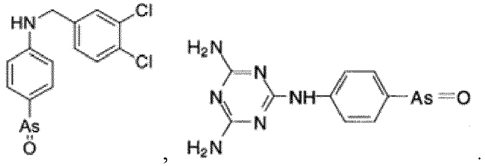

36. The use of any one of claims 21-27, wherein the subject is human or non-human mammal.

37. The use of claim 24, wherein the tumor is selected from lymphoma (e.g., acute non-B or non-T cell lymphoma), cervical cancer, endometrial cancer, liver cancer, breast cancer (e.g., triple-negative breast cancer), lung cancer (e.g., non-small cell lung cancer or small cell lung cancer), colorectal cancer, gastric cancer, skin cancer (e.g., melanoma), bone cancer, osteosarcoma, myeloma, blood cancer (e.g., acute or chronic leukemia), ovarian cancer, prostate tumor, brain tumor (e.g., cerebral astrocytoma), neuroblastoma, thyroid cancer, malignant embryonal rhabdomyosarcoma.

38. The use of claim 24, further comprising administering a second reagent to a subject in need thereof, preferably the second reagent is a reagent used for treating tumor.

39. The use of claim 38, wherein the compound is administered before, after or simultaneously with the second reagent.

40. An analytical method for measuring the concentration of PAO or a derivative thereof in blood, the derivative comprise a compound of Formula (I) or Formula (II), comprising the steps of:
Step 1: preparing three stock solutions by dissolving PAO or the derivative thereof, a control standard and sodium 2,3-dimercaptopropanesulfonate, respectively, in separate containers;
Step 2: mixing the blood comprising PAO or the derivative thereof to be tested with the stock solution of sodium 2,3-dimercaptopropanesulfonate, adjusting the solution to acidity, and incubating at 25-80°C for an appropriate time;
Step 3: adding the stock solution of the control standard to the solution obtained in step 2, shaking for a period of time and centrifuging, and taking the supernatant to test the derivatization product of PAO or the derivative thereof.

41. The analytical method of claim 40, wherein in the step 2, the incubation time is 10-80 minutes, the acidity is pH less than 6, the solution is adjusted to acidity by adding appropriate amount of an acid such as formic acid, potassium hydrogen phosphate, acetic acid or hydrochloric acid, and the reaction in the step 2 is as follows:

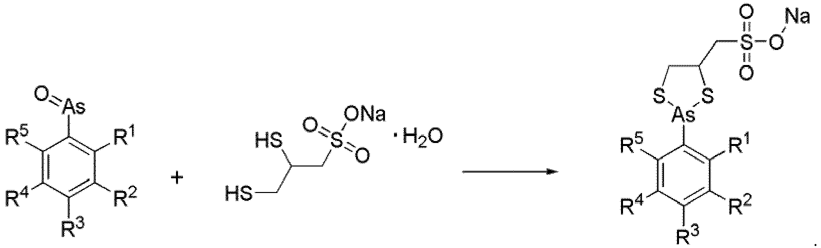

42. The analytical method of claim 40, wherein in the step 1, PAO or the derivative thereof and the control standard are dissolved in DMSO, the DMSO solution of the control standard is diluted with acetonitrile, and sodium 2,3-dimercaptopropanesulfonate is dissolved in pure water.

43. The analytical method of claim 40, wherein the control standard is tolbutamide, and in the step 3, the derivatization product of PAO or the derivative thereof is measured by a liquid chromatography-mass spectrometry method.
